# EUROPEAN PATENT SPECIFICATION

(11) **EP 1 144 642 B1**
(45) Date of publication and mention of the grant of the patent: **26.05.2010**
(21) Application number: 99963037.9
(22) Date of filing: 08.12.1999
(51) Int. Cl.: C12N 15/31, C07K 14/295, C12N 1/21, C12N 1/19, C12N 5/10, C07K 19/00, C12N 15/62, C07K 16/02, A61K 39/118, A61K 31/70, G01N 33/569, C12Q 1/68, A61K 48/00

(54) **COMPOUNDS AND METHODS FOR TREATMENT AND DIAGNOSIS OF CHLAMYDIAL INFECTION**
VERBINDUNGEN UND VERFAHREN ZUR BEHANDLUNG UND DIAGNOSE VON CHLAMYDIA-INFEKTIONEN
COMPOSES ET PROCEDES POUR LE TRAITEMENT ET LE DIAGNOSTIC D'INFECTIONS PAR LE CHLAMYDIA

(30) Priority: 08.12.1998 US 208277; 08.04.1999 US 288594; 01.10.1999 US 410568; 22.10.1999 US 426571
(43) Date of publication of application: 17.10.2001
(73) Proprietor: CORIXA CORPORATION, Wilmington, DE 19808 (US)
(72) Inventor: PROBST, Peter, Seattle, WA 98117 (US); BHATIA, Ajay, Seattle, WA 98104 (US); SKEIKY, Yasir, A., W., Bellevue, WA 98006 (US); FLING, Steven, P., Bainbridge Island, WA 98110 (US); JEN, Shyian, Seattle, WA 98122 (US); STROMBERG, Erica, Jean, Seattle, WA 98103 (US)
(74) Representative: Teuten, Andrew John
(86) International application number: PCT/US1999/029012
(87) International publication number: WO 2000/034483

(56) References cited:
- EP-A- 0 784 059
- WO-A-98/02546
- WO-A-98/10789
- WO-A-99/28475
- STEPHENS R ET AL: "Genome sequence of an obligate intracellular pathogen of humans: Chlamydia trachomatis" SCIENCE, vol. 282, 23 October 1998 (1998-10-23), pages 754-759, XP002136477 cited in the application -& EMBL DATABASE Accession no O84466 Sequence reference O84466 1 November 1998 XP002136478 -& EMBL DATABASE Accesion no AE001320 Sequence ID AE001320 22July 1998 XP002136479

### SEQUENCE LISTING

<110> Corixa Corporation
   Probst, Peter
   Bhatia, Ajay
   Skeiky, Yasir
   Fling, Steve
   Maisonneuve, Jeff
<120> COMPOSITIONS AND METHODS FOR TREATMENT AND DIAGNOSIS OF CHLAMYDIAL INFECTION
<130> 210121.469PC
<140> PCT
   <141> 1399-12-08
<160> 303
<170> FastSEQ for Windows Version 3.0/4.0
<210> 1
   <211> 481
   <212> DNA
   <213> Chlamydia trachomatis
<400> 1
   ctgaagactt ggctatgttt tttattttga cgataaacct agttaaggca taaaagagtt     60
   gcgaaggaag agccctcaac ttttcttatc accttcttta actaggagtc atccatgagt    120
   caaaataaga actctgcttt catgcagcct gtgaacgtat ccgctgattt agctgccatc    180
   gttggtgcag gacctatgcc tcgcacagag atcattaaga aaatgtggga ttacattaag    240
   gagaatagtc ttcaagatcc tacaaacaaa cgtaatatca atcccgatga taaattggct    300
   aaagtttttg gaactgaaaa acctatcgat atgttccaaa tgacaaaaat ggtttctcaa    360
   cacatcatta aataaaatag aaattgactc acgtgttcct cgtctttaag atgaggaact    420
   agttcattct ttttgttcgt ttttgtgggt attactgtat ctttaacaac tatcttagca    480
   g                                                                    481
<210> 2
   <211> 183
   <212> DNA
   <213> Chlamydia trachomatis
<400> 2
   atcgttggtg caggacctat gcctcgcaca gagatcatta agaaaatgtg ggattacatt 60
   aaggagaata gtcttcaaga tcctacaaac aaacgtaata tcaatcccga tgataaattg 120
   gctaaagttt ttggaactga aaaacctatc gatatgttcc aaatgacaaa aatggtttct 180
   caa 183
<210> 3
   <211> 110
   <212> DNA
   <213> Chlamydia trachomatis
<400> 3
   gctgcgacat catgcgagct tgcaaaccaa catggacatc tccaatttcc ccttctaact 60
   cgctctttgg aactaatgct gctaccgagt caatcacaat cacatcgacc 110

<210> 4
   <211> 555
   <212> DNA
   <213> Chlamydia trachomatis
<400> 4
   cggcacgagc ctaagatgct tatactactt taagggaggc ccttcgtatg ccgcgcatca 60
   ttggaataga tattcctgcg aaaaagaaat taaaaataag tcttacatat atttatggaa 120
   tagggccagc tctttctaaa gagattattg ctagattgca gttgaatccc gaagctagag 180
   ctgcagagtt gactgaggaa gaggttggtc gactaaacgc tcttttacag tcggattacg 240
   ttgttgaagg ggatttgcgc cgtcgtgtgc aatctgatat caaacgtctg attactatcc 300
   atgcttatcg tggacaaaga catagacttt ctttgcctgt tcgtggtcag agaacaaaaa 360
   caaattctcg cacgcgtaag ggtaaacgta aaactattgc aggtaagaag aaataataat 420
   ttttaggaga gagtgttttg gttaaaaatc aagcgcaaaa aagaggcgta aaaagaaaac 480
   aagtaaaaaa cattccttcg ggcgttgtcc atgttaaggc tacttttaat aatacaattg 540
   taaccataac agacc 555
<210> 5
   <211> 86
   <212> PRT
   <213> Chlamydia trachomatis
<400> 5
   Met Ser Gln Asn Lys Asn Ser Ala Phe Met Gln Pro Val Asn Val Ser
   1               5                   10                  15
   Ala Asp Leu Ala Ala Ile Val Gly Ala Gly Pro Met Pro Arg Thr Glu
               20                  25                  30
   Ile Ile Lys Lys Met Trp Asp Tyr Ile Lys Glu Asn Ser Leu Gln Asp
           35                  40                  45
   Pro Thr Asn Lys Arg Asn Ile Asn Pro Asp Asp Lys Leu Ala Lys Val
       50                  55                  60
   Phe Gly Thr Glu Lys Pro Ile Asp Met Phe Gln Met Thr Lys Met Val
   65                  70                  75                  80
   Ser Gln His Ile Ile Lys
                   85
<210> 6
   <211> 61
   <212> PRT
   <213> Chlamydia trachomatis
<400> 6
   Ile Val Gly Ala Gly Pro Met Pro Arg Thr Glu Ile Ile Lys Lys Met
   1               5                   10                  15
   Trp Asp Tyr Ile Lys Glu Asn Ser Leu Gln Asp Pro Thr Asn Lys Arg
               20                  25                  30
   Asn Ile Asn Pro Asp Asp Lys Leu Ala Lys Val Phe Gly Thr Glu Lys
           35                  40                  45
   Pro Ile Asp Met Phe Gln Met Thr Lys Met Val Ser Gln
       50                  55                  60
<210> 7
   <211> 36
   <212> PRT
   <213> Chlamyida trachomatis
<400> 7
   Ala Ala Thr Ser Cys Glu Leu Ala Asn Gln His Gly His Leu Gln Phe
   1               5                   10                  15
   Pro Leu Leu Thr Arg Ser Leu Glu Leu Met Leu Leu Pro Ser Gln Ser
               20                  25                  30
   Gln Ser His Arg
           35
<210> 8
   <211> 18
   <212> PRT
   <213> Chlamydia trachomatis
<400> 8
   Leu Arg His His Ala Ser Leu Gln Thr Asn Met Asp Ile Ser Asn Phe
   1               5                   10                  15
   Pro Phe
<210> 9
   <211> 5
   <212> PRT
   <213> Chlamydia trachomatis
<400> 9
   Leu Ala Leu Trp Asn
   1               5
<210> 10
   <211> 11
   <212> PRT
   <213> Chlamydia trachomatis
<400> 10
   Cys Cys Tyr Arg Val Asn His Asn His Ile Asp
    1               5                   10
<210> 11
   <211> 36
   <212> PRT
   <213> Chlamydia trachomatis
<400> 11
   Val Asp Val Ile Val Ile Asp Ser Val Ala Ala Leu Val Pro Lys Ser
    1               5                   10                  15
   Glu Leu Glu Gly Glu Ile Gly Asp Val His Val Gly Leu Gln Ala Arg
               20                  25                  30
   Met Met Ser Gln
           35
<210> 12
   <211> 122
   <212> PRT
   <213> Chlamydia trachomatis
<400> 12
   Met Pro Arg Ile Ile Gly Ile Asp Ile Pro Ala Lys Lys Lys Leu Lys
    1               5                  10                  15
   Ile Ser Leu Thr Tyr Ile Tyr Gly Ile Gly Pro Ala Leu Ser Lys Glu
               20                  25                  30
   Ile Ile Ala Arg Leu Gln Leu Asn Pro Glu Ala Arg Ala Ala Glu Leu
           35                  40                  45
   Thr Glu Glu Glu Val Gly Arg Leu Asn Ala Leu Leu Gln Ser Asp Tyr
       50                  55                  60
   Val Val Glu Gly Asp Leu Arg Arg Arg Val Gln Ser Asp Ile Lys Arg
   65                  70                  75                  80
   Leu Ile Thr Ile His Ala Tyr Arg Gly Gln Arg His Arg Leu Ser Leu
                   85                  90                  95
   Pro Val Arg Gly Gln Arg Thr Lys Thr Asn Ser Arg Thr Arg Lys Gly
               100                 105                 110
   Lys Arg Lys Thr Ile Ala Gly Lys Lys Lys
           115                 120
<210> 13
   <211> 20
   <212> PRT
   <213> Chlamydia trachomatis

<400> 13
   Asp Pro Thr Asn Lys Arg Asn Ile Asn Pro Asp Asp Lys Leu Ala Lys
   1               5                   10                  15
   Val Phe Gly Thr
               20
<210> 14
   <211> 20
   <212> PRT
   <213> Chlamydia trachomatis
<400> 14
   Asp Asp Lys Leu Ala Lys Val Phe Gly Thr Glu Lys Pro Ile Asp Met
   1               5                   10                  15
   Phe Gln Met Thr
               20
<210> 15
   <211> 161
   <212> DNA
   <213> Chlymidia trachomatis
<400> 15
   atctttgtgt gtctcataag cgcagagcgg ctgcggctgt ctgtagcttc atcggaggaa 60
   ttacctacct cgcgacattc ggagctatcc gtccgattct gtttgtcaac aaaatgctgg 120
   cgcaaccgtt tctttcttcc caaactaaag caaatatggg a 161
<210> 16
   <211> 897
   <212> DNA
   <213> Chlymidia trachomatis
<400> 16
   atggcttcta tatgcggacg tttagggtct ggtacaggga atgctctaaa agcttttttt 60
   acacagccca acaataaaat ggcaagggta gtaaataaga cgaagggaat ggataagact 120
   attaaggttg ccaagtctgc tgccgaattg accgcaaata ttttggaaca agctggaggc 180
   gcgggctctt ccgcacacat tacagcttcc caagtgtcca aaggattagg ggatgcgaga 240
   actgttgtcg ctttagggaa tgcctttaac ggagcgttgc caggaacagt tcaaagtgcg 300
   caaagcttct tctctcacat gaaagctgct agtcagaaaa cgcaagaagg ggatgagggg 360
   ctcacagcag atctttgtgt gtctcataag cgcagagcgg ctgcggctgt ctgtagcatc 420
   atcggaggaa ttacctacct cgcgacattc ggagctatcc gtccgattct gtttgtcaac 480
   aaaatgctgg caaaaccgtt tctttcttcc caaactaaag caaatatggg atcttctgtt 540
   agctatatta tggcggctaa ccatgcagcg tctgtggtgg gtgctggact cgctatcagt 600
   gcggaaagag cagattgcga agcccgctgc gctcgtattg cgagagaaga gtcgttactc 660
   gaagtgccgg gagaggaaaa tgcttgcgag aagaaagtcg ctggagagaa agccaagacg 720
   ttcacgcgca tcaagtatgc actcctcact atgctcgaga agtttttgga atgcgttgcc 780
   gacgttttca aattggtgcc gctgcctatt acaatgggta ttcgtgcgat tgtggctgct 840
   ggatgtacgt tcacttctgc aattattgga ttgtgcactt tctgcgccag agcataa 897
<210> 17
   <211> 298
   <212> PRT
   <213> Chlamydia trachomatis
<400> 17
   Met Ala Ser Ile Cys Gly Arg Leu Gly Ser Gly Thr Gly Asn Ala Leu
    1               5                  10                  15
   Lys Ala Phe Phe Thr Gln Pro Asn Asn Lys Met Ala Arg Val Val Asn
               20                  25                  30
   Lys Thr Lys Gly Met Asp Lys Thr Ile Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Ser Ile Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Lys Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Glu Arg Ala Asp Cys Glu Ala
           195                 200                 205
   Arg Cys Ala Arg Ile Ala Arg Glu Glu Ser Leu Leu Glu Val Pro Gly
       210                 215                 220
   Glu Glu Asn Ala Cys Glu Lys Lys Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 18
   <211> 18
   <212> PRT
   <213> Chlamydia trachomatis
<400> 18
   Arg Ala Ala Ala Ala Ala Ala Val Cys Ser Phe Ile Gly Gly Ile Thr
    1               5                  10                  15
   Tyr Leu
<210> 19
   <211> 18
   <212> PRT
   <213> Chlamydia trachomatis
<400> 19
   Cys Ser Phe Ile Gly Gly Ile Thr Tyr Leu Ala Thr Phe Gly Ala Ile
    1               5                  10                  15
   Arg Pro
<210> 20
   <211> 216
   <212> PRT
   <213> Chlamydia trachomatis
<400> 20
   Met Arg Gly Ser Gln Gln Ile Phe Val Cys Leu Ile Ser Ala Glu Arg
    1               5                  10                  15
   Leu Arg Leu Ser Val Ala Ser Ser Glu Glu Leu Pro Thr Ser Arg His
               20                  25                  30
   Ser Glu Leu Ser Val Arg Phe Cys Leu Ser Thr Lys Cys Trp Gln Asn
           35                  40                  45
   Arg Phe Phe Leu Pro Lys Leu Lys Gln Ile Trp Asp Leu Leu Leu Ala
       50                  55                  60
   Ile Leu Trp Arg Leu Thr Met Gln Arg Leu Trp Trp Val Leu Asp Ser
   65                  70                  75                  80
   Leu Ser Val Arg Lys Glu Gln Ile Ala Lys Pro Ala Ala Leu Val Leu
                   85                  90                  95
   Arg Glu Lys Ser Arg Tyr Ser Lys Cys Arg Glu Arg Lys Met Leu Ala
               100                 105                 110
   Arg Arg Lys Ser Leu Glu Arg Lys Pro Arg Arg Ser Arg Ala Ser Ser
           115                 120                 125
   Met His Ser Ser Leu Cys Ser Arg Ser Phe Trp Asn Ala Leu Pro Thr
       130                 135                 140
   Phe Ser Asn Trp Cys Arg Cys Leu Leu Gln Trp Val Phe Val Arg Leu
   145                 150                 155                 160
   Trp Leu Leu Asp Val Arg Ser Leu Leu Gln Leu Leu Asp Cys Ala Leu
                   165                 170                 175
   Ser Ala Pro Glu His Lys Gly Phe Phe Lys Phe Leu Lys Lys Lys Ala
               180                 185                 190
   Val Ser Lys Lys Lys Gln Pro Phe Leu Ser Thr Lys Cys Leu Ala Phe
           195                 200                 205
   Leu Ile Val Lys Ile Val Phe Leu
       210                 215
<210> 21
   <211> 1256
   <212> DNA
   <213> Chlamydia trachomatis

<400> 21
   ctcgtgccgg cacgagcaaa gaaatccctc aaaaaatggc cattattggc ggtggtgtga     60
   tcggttgcga attcgcttcc ttattccata cgttaggctc cgaagtttct gtgatcgaag    120
   caagctctca aatccttgct ttgaataatc cagatatttc aaaaaccatg ttcgataaat    180
   tcacccgaca aggactccgt ttcgtactag aagcctctgt atcaaatatt gaggatatag    240
   gagatcgcgt tcggttaact atcaatggga atgtcgaaga atacgattac gttctcgtat    300
   ctataggacg ccgtttgaat acagaaaata ttggcttgga taaagctggt gttatttgtg    360
   atgaacgcgg agtcatccct accgatgcca caatgcgcac aaacgtacct aacatttatg    420
   ctattggaga tatcacagga aaatggcaac ttgcccatgt agcttctcat caaggaatca    480
   ttgcagcacg gaatataggt ggccataaag aggaaatcga ttactctgct gtcccttctg    540
   tgatctttac cttccctgaa gtcgcttcag taggcctctc cccaacagca gctcaacaac     600
   atctccttct tcgcttactt tttctgaaaa atttgataca gaagaagaat tcctcgcaca    660
   cttgcgagga ggagggcgtc tggaagacca gttgaattta gctaagtttt ctgagcgttt    720
   tgattctttg cgagaattat ccgctaagct tggttacgat agcgatggag agactgggga    780
   tttcttcaac gaggagtacg acgacgaaga agaggaaatc aaaccgaaga aaactacgaa    840
   acgtggacgt aagaagagcc gttcataagc cttgctttta aggtttggta gttttacttc    900
   tctaaaatcc aaatggttgc tgtgccaaaa agtagtttgc gtttccggat agggcgtaaa    960
   tgcgctgcat gaaagattgc ttcgagagcg gcatcgcgtg ggagatcccg gatactttct   1020
   ttcagatacg aataagcata gctgttccca gaataaaaac ggccgacgct aggaacaaca   1080
   agatttagat agagcttgtg tagcaggtaa actgggttat atgttgctgg gcgtgttagt   1140
   tctagaatac ccaagtgtcc tccaggttgt aatactcgat acacttccct aagagcctct   1200
   aatggatagg ataagttccg taatccatag gccatagaag ctaaacgaaa cgtatt       1256
<210> 22
   <211> 601
   <212> DNA
   <213> Chlamydia trachomatis
<400> 22
   ctcgtgccgg cacgagcaaa gaaatccctc aaaaaatggc cattattggc ggtggtgtga     60
   tcggttgcga attcgcttcc ttattccata cgttaggctc cgaagtttct gtgatcgaag    120
   caagctctca aatccttgct ttgaataatc cagatatttc aaaaaccatg ttcgataaat    180
   tcacccgaca aggactccgt ttcgtactag aagcctctgt atcaaatatt gaggatatag    240
   gagatcgcgt tcggttaact atcaatggga atgtcgaaga atacgattac gttctcgtat    300
   ctataggacg ccgtttgaat acagaaaata ttggcttgga taaagctggt gttatttgtg    360
   atgaacgcgg agtcatccct accgatgcca caatgcgcac aaacgtacct aacatttatg    420
   ctattggaga tatcacagga aaatggcaac ttgcccatgt agcttctcat caaggaatca    480
   ttgcagcacg gaatataggt ggccataaag aggaaatcga ttactctgct gtcccttctg    540
   tgatctttac cttccctgaa gtcgcttcag taggcctctc cccaacagca gctcaacaac    600
   a                                                                   601
<210> 23
   <211> 270
   <212> DNA
   <213> Chlamydia trachomatis
<400> 23
   acatctcctt cttcgcttac tttttctgaa aaatttgata cagaagaaga attcctcgca     60
   cacttgcgag gaggagggcg tctggaagac cagttgaatt tagctaagtt ttctgagcgt    120
   tttgattctt tgcgagaatt atccgctaag cttggttacg atagcgatgg agagactggg    180
   gatttcttca acgaggagta cgacgacgaa gaagaggaaa tcaaaccgaa gaaaactacg    240
   aaacgtggac gtaagaagag ccgttcataa                                     270
<210> 24
   <211> 363
   <212> DNA
   <213> Chlamydia trachomatis
<400> 24
   ttacttctct aaaatccaaa tggttgctgt gccaaaaagt agtttgcgtt tccggatagg    60
   gcgtaaatgc gctgcatgaa agattgcttc gagagcggca tcgcgtggga gatcccggat   120
   actttctttc agatacgaat aagcatagct gttcccagaa taaaaacggc cgacgctagg   180
   aacaacaaga tttagataga gcttgtgtag caggtaaact gggttatatg ttgctgggcg   240
   tgttagttct agaataccca agtgtcctcc aggttgtaat actcgataca cttccctaag   300
   agcctctaat ggataggata agttccgtaa tccataggcc atagaagcta aacgaaacgt   360
   att                                                                 363
<210> 25
   <211> 696
   <212> DNA
   <213> Chlamydia trachomatis
<400> 25
   gctcgtgccg gcacgagcaa agaaatccct caaaaaatgg ccattattgg cggtggtgtg    60
   atcggttgcg aattcgcttc cttattccat acgttaggct ccgaagtttc tgtgatcgaa   120
   gcaagctctc aaatccttgc tttgaataat ccagatattt caaaaaccat gttcgataaa   180
   ttcacccgac aaggactccg tttcgtacta gaagcctctg tatcaaatat tgaggatata   240
   ggagatcgcg ttcggttaac tatcaatggg aatgtcgaag aatacgatta cgttctcgta   300
   tctataggac gccgtttgaa tacagaaaat attggcttgg ataaagctgg tgttatttgt   360
   gatgaacgcg gagtcatccc taccgatgcc acaatgcgca caaacgtacc taacatttat   420
   gctattggag atatcacagg aaaatggcaa cttgcccatg tagcttctca tcaaggaatc   480
   attgcagcac ggaatatagg tggccataaa gaggaaatcg attactctgc tgtcccttct   540
   gtgatcttta ccttccctga agtcgcttca gtaggcctct ccccaacagc agctcaacaa   600
   catctccttc ttcgcttact ttttctgaaa aatttgatac agaagaagaa ttcctcgcac    660
   acttgcgagg aggagggcgt ctggaagacc agttga                             696
<210> 26
   <211> 231
   <212> PRT
   <213> Chlamydia trachomatis
<400> 26
   Ala Arg Ala Gly Thr Ser Lys Glu Ile Pro Gln Lys Met Ala Ile Ile
    1               5                  10                  15
   Gly Gly Gly Val Ile Gly Cys Glu Phe Ala Ser Leu Phe His Thr Leu
               20                  25                  30
   Gly Ser Glu Val Ser Val Ile Glu Ala Ser Ser Gln Ile Leu Ala Leu
           35                  40                  45
   Asn Asn Pro Asp Ile Ser Lys Thr Met Phe Asp Lys Phe Thr Arg Gln
       50                  55                  60
   Gly Leu Arg Phe Val Leu Glu Ala Ser Val Ser Asn Ile Glu Asp Ile
   65                  70                  75                  80
   Gly Asp Arg Val Arg Leu Thr Ile Asn Gly Asn Val Glu Glu Tyr Asp
                   85                  90                  95
   Tyr Val Leu Val Ser Ile Gly Arg Arg Leu Asn Thr Glu Asn Ile Gly
               100                 105                 110
   Leu Asp Lys Ala Gly Val Ile Cys Asp Glu Arg Gly Val Ile Pro Thr
           115                 120                 125
   Asp Ala Thr Met Arg Thr Asn Val Pro Asn Ile Tyr Ala Ile Gly Asp
       130                 135                 140
   Ile Thr Gly Lys Trp Gln Leu Ala His Val Ala Ser His Gln Gly Ile
   145                 150                 155                 160
   Ile Ala Ala Arg Asn Ile Gly Gly His Lys Glu Glu Ile Asp Tyr Ser
                   165                 170                 175
   Ala Val Pro Ser Val Ile Phe Thr Phe Pro Glu Val Ala Ser Val Gly
               180                 185                 190
   Leu Ser Pro Thr Ala Ala Gln Gln His Leu Leu Leu Arg Leu Leu Phe
           195                 200                 205
   Leu Lys Asn Leu Ile Gln Lys Lys Asn Ser Ser His Thr Cys Glu Glu
       210                 215                 220
   Glu Gly Val Trp Lys Thr Ser
   225                 230
<210> 27
   <211> 264
   <212> DNA
   <213> Chlamydia pneumoniae
<400> 27
   atgagtcaaa aaaataaaaa ctctgctttt atgcatcccg tgaatatttc cacagattta      60
   gcagttatag ttggcaaggg acctatgccc agaaccgaaa ttgtaaagaa agtttgggaa     120
   tacattaaaa aacacaactg tcaggatcaa aaaaataaac gtaatatcct tcccgatgcg     180
   aatcttgcca aagtctttgg ctctagtgat cctatcgaca tgttccaaat gaccaaagcc     240
   ctttccaaac atattgtaaa ataa                                            264
<210> 28
   <211> 87
   <212> PRT
   <213> Chlamydia pneumoniae
<400> 28
   Met Ser Gln Lys Asn Lys Asn Ser Ala Phe Met His Pro Val Asn Ile
    1               5                  10                  15
   Ser Thr Asp Leu Ala Val Ile Val Gly Lys Gly Pro Met Pro Arg Thr
               20                  25                  30
   Glu Ile Val Lys Lys Val Trp Glu Tyr Ile Lys Lys His Asn Cys Gln
           35                  40                  45
   Asp Gln Lys Asn Lys Arg Asn Ile Leu Pro Asp Ala Asn Leu Ala Lys
       50                  55                  60
   Val Phe Gly Ser Ser Asp Pro Ile Asp Met Phe Gln Met Thr Lys Ala
   65                  70                  75                  80
   Leu Ser Lys His Ile Val Lys
                   85
<210> 29
   <211> 369
   <212> DNA
   <213> Chlamydia pneumoniae
<400> 29
   atgccacgca tcattggaat tgatattcct gcaaagaaaa agttaaaaat aagtctgaca      60
   tatatttatg gaataggatc agctcgttct gatgaaatca ttaaaaagtt gaagttagat     120
   cctgaggcaa gagcctctga attaactgaa gaagaagtag gacgactgaa ctctctgcta     180
   caatcagaat ataccgtaga aggggatttg cgacgtcgtg ttcaatcgga tatcaaaaga     240
   ttgatcgcca tccattctta tcgaggtcag agacatagac tttctttacc agtaagagga     300
   caacgtacaa aaactaattc tcgtactcga aaaggtaaaa gaaaaacagt cgcaggtaag     360
   aagaaataa                                                             369
<210> 30
   <211> 122
   <212> PRT
   <213> Chlamydia pneumoniae

<400> 30
   Met Pro Arg Ile Ile Gly Ile Asp Ile Pro Ala Lys Lys Lys Leu Lys
    1               5                  10                  15
   Ile Ser Leu Thr Tyr Ile Tyr Gly Ile Gly Ser Ala Arg Ser Asp Glu
               20                  25                  30
   Ile Ile Lys Lys Leu Lys Leu Asp Pro Glu Ala Arg Ala Ser Glu Leu
           35                  40                  45
   Thr Glu Glu Glu Val Gly Arg Leu Asn Ser Leu Leu Gln Ser Glu Tyr
       50                  55                  60
   Thr Val Glu Gly Asp Leu Arg Arg Arg Val Gln Ser Asp Ile Lys Arg
   65                  70                  75                  80
   Leu Ile Ala Ile His Ser Tyr Arg Gly Gln Arg His Arg Leu Ser Leu
                   85                  90                  95
   Pro Val Arg Gly Gln Arg Thr Lys Thr Asn Ser Arg Thr Arg Lys Gly
               100                 105                 110
   Lys Arg Lys Thr Val Ala Gly Lys Lys Lys
           115                 120
<210> 31
   <211> 10
   <212> PRT
   <213> Artificial Sequence
<220>
   <223> Made in the lab
<400> 31
   Cys Ser Phe Ile Gly Gly Ile Thr Tyr Leu
   1               5                   10
<210> 32
   <211> 53
   <212> PRT
   <213> Chlamydia trachomatis
<400> 32
   Leu Cys Val Ser His Lys Arg Arg Ala Ala Ala Ala Val Cys Ser Phe
   1               5                   10                  15
   Ile Gly Gly Ile Thr Tyr Leu Ala Thr Phe Gly Ala Ile Arg Pro Ile
               20                  25                  30
   Leu Phe Val Asn Lys Met Leu Ala Gln Pro Phe Leu Ser Ser Gln Thr
           35                  40                  45
   Lys Ala Asn Met Gly
       50
<210> 33
   <211> 161
   <212> DNA
   <213> Chlamydia trachomatis
<400> 33
   atctttgtgt gtctcataag cgcagagcgg ctgcggctgt ctgtagcatc atcggaggaa      60
   ttacctacct cgcgacattc ggagctatcc gtccgattct gtttgtcaac aaaatgctgg     120
   caaaaccgtt tctttcttcc caaactaaag caaatatggg a                         161
<210> 34
   <211> 53
   <212> PRT
   <213> Chlamydia trachomatis
<400> 34
   Leu Cys Val Ser His Lys Arg Arg Ala Ala Ala Ala Val Cys Ser Ile
   1               5                   10                  15
   Ile Gly Gly Ile Thr Tyr Leu Ala Thr Phe Gly Ala Ile Arg Pro Ile
               20                  25                  30
   Leu Phe Val Asn Lys Met Leu Ala Lys Pro Phe Leu Ser Ser Gln Thr
           35                  40                  45
   Lys Ala Asn Met Gly
       50
<210> 35
   <211> 55
   <212> DNA
   <213> Chlamydia pneumoniae
<400> 35
   gatatacata tgcatcacca tcaccatcac atgagtcaaa aaaaataaaa actct 55
<210> 36
   <211> 33
   <212> DNA
   <213> Chlamydia pneumoniae

<400> 36
   ctcgaggaat tcttatttta caatatgttt gga 33
<210> 37
   <211> 53
   <212> DNA
   <213> Chlamydia pneumoniae
<400> 37
   gatatacata tgcatcacca tcaccatcac atgccacgca tcattggaat gat 53
<210> 38
   <211> 30
   <212> DNA
   <213> Chlamydia pneumoniae
<400> 38
   ctcgaggaat tcttatttct tcttacctgc 30
<210> 39
   <211> 16
   <212> PRT
   <213> Artificial Sequence
<220>
   <223> Made in the lab
<400> 39
   Lys Arg Asn Ile Asn Pro Asp Asp Lys Leu Ala Lys Val Phe Gly Thr
   1               5                   10                  15
<210> 40
   <211> 16
   <212> PRT
   <213> Artificial Sequence
<220>
   <223> made in the lab
<400> 40
   Lys Arg Asn Ile Leu Pro Asp Ala Asn Leu Ala Lys Val Phe Gly Ser
   1 5 10 15
<210> 41
   <211> 15
   <212> PRT
   <213> Artificial Sequence
<220>
   <223> made in the lab

<400> 41
   Lys Glu Tyr Ile Asn Gly Asp Lys Tyr Phe Gln Gln Ile Phe Asp
   1 5 10 15
<210> 42
   <211> 16
   <212> PRT
   <213> Artificial Sequence
<220>
   <223> made in the lab
<400> 42
   Lys Lys Ile Ile Ile Pro Asp Ser Lys Leu Gln Gly Val Ile Gly Ala
   1 5 10 15
<210> 43
   <211> 15
   <212> PRT
   <213> Artificial Sequence
<220>
   <223> made in the lab
<400> 43
   Lys Lys Leu Leu Val Pro Asp Asn Asn Leu Ala Thr Ile Ile Gly
   1 5 10 15
<210> 44
   <211> 509
   <212> DNA
   <213> Chlamydia
<400> 44
   ggagctcgaa ttcggcacga gagtgcctat tgttttgcag gctttgtctg atgatagcga 60
   taccgtacgt gagattgctg tacaagtagc tgttatgtat ggttctagtt gcttactgcg 120
   cgccgtgggc gatttagcga aaaatgattc ttctattcaa gtacgcatca ctgcttatcg 180
   tgctgcagcc gtgttggaga tacaagatct tgtgcctcat ttacgagttg tagtccaaaa 240
   tacacaatta gatggaacgg aaagaagaga agcttggaga tctttatgtg ttcttactcg 300
   gcctcatagt ggtgtattaa ctggcataga tcaagcttta atgacctgtg agatgttaaa 360
   ggaatatcct gaaaagtgta cggaagaaca gattcgtaca ttattggctg cagatcatcc 420
   agaagtgcag gtagctactt tacagatcat tctgagagga ggtagagtat tccggtcatc 480
   ttctataatg gaatcggttc tcgtgccgg                                   509
<210> 45
   <211> 481
   <212> DNA
   <213> Chlamydia
<220>
   <221> unsure
   <222> (23)
   <223> n=A,T,C or G

<400> 45
   gatccgaatt cggcacgagg cantatttac tcccaacatt acggttccaa ataagcgata 60
   aggtcttcta ataaggaagt taatgtaaga ggctttttta ttgcttttcg taaggtagta 120
   ttgcaaccgc acgcgattga atgatacgca agccatttcc atcatggaaa agaacccttg 180
   gacaaaaata caaaggaggt tcactcctaa ccagaaaaag ggagagttag tttccatggg 240
   ttttccttat atacacccgt ttcacacaat taggagccgc gtctagtatt tggaatacaa 300
   attgtcccca agcgaatttt gttcctgttt cagggatttc tcctaattgt tctgtcagcc 360
   atccgcctat ggtaacgcaa ttagctgtag taggaagatc aactccaaac aggtcataga 420
   aatcagaaag ctcataggtg cctgcagcaa taacaacatt cttgtctgag tgagcgaatt 480
   g                                                                 481
<210> 46
   <211> 427
   <212> DNA
   <213> Chlamydia
<220>
   <221> unsure
   <222> (20)
   <223> n=A,T,C or G
<400> 46
   gatccgaatt cggcacgagn tttttcctgt tttttcttag tttttagtgt tcccggagca 60
   ataacacaga tcaaagaacg gccattcagt ttaggctctg actcaacaaa acctatgtcc 120
   tctaagccct gacacattct ttgaacaacc ttatgcccgt gttcgggata agccaactct 180
   cgcccccgaa acatacaaga aacctttact ttatttcctt tctcaataaa ggctctagct 240
   tgctttgctt tcgtaagaaa gtcgttatca tcgatattag gcttaagctt aacctctttg 300
   atacgcactt ggtgctgtgc tttcttacta tctttttctt ttttagttat gtcgtaacga 360
   tacttcccgt agtccatgat tttgcacaca ggaggctctg agtttgaagc aacctcgtgc 420
   cgaattc 427
<210> 47
   <211> 600
   <212> DNA
   <213> Chlamydia
<220>
   <221> unsure
   <222> (522)
   <223> n=A,T,C or G
<400> 47
   gatccgaatt cggcacgaga tgcttctatt acaattggtt tggatgcgga aaaagcttac 60
   cagcttattc tagaaaagtt gggagatcaa attcttggtg gaattgctga tactattgtt 120
   gatagtacag tccaagatat tttagacaaa atcacaacag acccttctct aggtttgttg 180
   aaagctttta acaactttcc aatcactaat aaaattcaat gcaacgggtt attcactccc 240
   aggaacattg aaactttatt aggaggaact gaaataggaa aattcacagt cacacccaaa 300
   agctctggga gcatgttctt agtctcagca gatattattg catcaagaat ggaaggcggc 360
   gttgttctag ctttggtacg agaaggtgat tctaagccct acgcgattag ttatggatac 420
   tcatcaggcg ttcctaattt atgtagtcta agaaccagaa ttattaatac aggattgact 480
   ccgacaacgt attcattacg tgtaggcggt ttagaaagcg gngtggtatg ggttaatgcc 540
   ctttctaatg gcaatgatat tttaggaata acaaatcttc taatgtatct tttttggagg 600
<210> 48
   <211> 600
   <212> DNA
   <213> Chlamydia

<400> 48
   ggagctcgaa ttcggcacga gctctatgaa tatccaattc tctaaactgt tcggataaaa 60
   atgatgcagg aattaggtcc acactatctt tttttgtttc gcaaatgatt gattttaaat 120
   cgtttgatgt gtatactatg tcgtgtaagc ctttttggtt acttctgaca ctagccccca 180
   atccagaaga taaattggat tgcgggtcta ggtcagcaag taacactttt ttccctaaaa 240
   attgggccaa gttgcatccc acgtttagag aaagtgttgt ttttccagtt cctcccttaa 300
   aagagcaaaa aactaaggtg tgcaaatcaa ctccaacgtt agagtaagtt atctattcag 360
   ccttggaaaa catgtctttt ctagacaaga taagcataat caaagccttt tttagcttta 420
   aactgttatc ctctaatttt tcaagaacag gagagtctgg gaataatcct aaagagtttt 480
   ctatttgttg aagcagtcct agaattagtg agacactttt atggtagagt tctaagggag 540
   aatttaagaa agttactttt tccttgttta ctcgtatttt taggtctaat tcggggaaat 600
<210> 49
   <211> 600
   <212> DNA
   <213> Chlamydia
<400> 49
   gatccgaatt cggcacgaga tgcttctatt acaattggtt tggatgcgga aaaagcttac 60
   cagcttattc tagaaaagtt gggagatcaa attcttggtg gaattgctga tactattgtt 120
   gatagtacag tccaagatat tttagacaaa atcacaacag acccttctct aggtttgttg 180
   aaagctttta acaactttcc aatcactaat aaaattcaat gcaacgggtt attcactccc 240
   aggaacattg aaactttatt aggaggaact gaaataggaa aattcacagt cacacccaaa 300
   agctctggga gcatgttctt agtctcagca gatattattg catcaagaat ggaaggcggc 360
   gttgttctag ctttggtacg agaaggtgat tctaagccct acgcgattag ttatggatac 420
   tcatcaggcg ttcctaattt atgtagtcta agaaccagaa ttattaatac aggattgact 480
   ccgacaacgt attcattacg tgtaggcggt ttagaaagcg gtgtggtatg ggttaatgcc 540
   ctttctaatg gcaatgatat tttaggaata acaaatactt ctaatgtatc ttttttggag 600
<210> 50
   <211> 406
   <212> DNA
   <213> Chlamydia
<400> 50
   gatccgaatt cggcacgagt tcttagcttg cttaattacg taattaacca aactaaaggg 60
   gctatcaaat agcttattca gtctttcatt agttaaacga tcttttctag ccatgactca 120
   tcctatgttc ttcagctata aaaatacttc ttaaaacttg atatgctgta atcaaatcat 180
   cattaaccac aacataatca aattcgctag cggcagcaat ttcgacagcg ctatgctcta 240
   atctttcttt cttctggaaa tctttctctg aatcccgagc attcaaacgg cgctcaagtt 300
   cttcttgaga gggagcttga ataaaaatgt gactgccggc atttgcttct tcagagccaa 360
   agctccttgt acatcaatca cggctatgca gtctcgtgcc gaattc 406
<210> 51
   <211> 602
   <212> DNA
   <213> Chlamydia
<400> 51
   gatccgaatt cggcacgaga tattttagac aaaatcacaa cagacccttc tctaggtttg 60
   ttgaaagctt ttaacaactt tccaatcact aataaaattc aatgcaacgg gttattcact 120
   cccaggaaca ttgaaacttt attaggagga actgaaatag gaaaattcac agtcacaccc 180
   aaaagctctg ggagcatgtt cttagtctca gcagatatta ttgcatcaag aatggaaggc 240
   ggcgttgttc tagctttggt acgagaaggt gattctaagc cctacgcgat tagttatgga 300
   tactcatcag gcgttcctaa tttatgtagt ctaagaacca gaattattaa tacaggattg 360
   actccgacaa cgtattcatt acgtgtaggc ggtttagaaa gcggtgtggt atgggttaat 420
   gccctttcta atggcaatga tattttagga ataacaaata cttctaatgt atcttttttg 480
   gaggtaatac ctcaaacaaa cgcttaaaca atttttattg gatttttctt ataggtttta 540
   tatttagaga aaaaagttcg aattacgggg tttgttatgc aaaataaact cgtgccgaat 600
   tc 602
<210> 52
   <211> 145
   <212> DNA
   <213> Chlamydia
<400> 52
   gatccgaatt cggcacgagc tcgtgccgat gtgttcaaca gcatccatag gatgggcagt 60
   caaatatact ccaagtaatt ctttttctct tttcaacaac tccttaggag agcgttggat 120
   aacattttca gctcgtgccg aattc                                       145
<210> 53
   <211> 450
   <212> DNA
   <213> Chlamydia
<400> 53
   gatccgaatt cggcacgagg taatcggcac cgcactgctg acactcatct cctcgagctc 60
   gatcaaaccc acacttggga caagtaccta caacataacg gtccgctaaa aacttccctt 120
   cttcctcaga atacagctgt tcggtcacct gattctctac cagtccgcgt tcctgcaagt 180
   ttcgatagaa atcttgcaca atagcaggat gataagcgtt cgtagttctg gaaaagaaat 240
   ctacagaaat tcccaatttc ttgaaggtat ctttatgaag cttatgatac atgtcgacat 300
   attcttgata ccccatgcct gccaactctg cattaagggt aattgcgatt ccgtattcat 360
   cagaaccaca aatatacaaa acctctttgc cttgtagtct ctgaaaacgc gcataaacat 420
   ctgcaggcaa ataagcctcg tgccgaattc                                  450
<210> 54
   <211> 716
   <212> DNA
   <213> Chlamydia
<400> 54
   gatcgaaatt cggcacgagc ggcacgagtt ttctgatagc gatttacaat cctttattca 60
   acttttgcct agagaggcac actatactaa gaagtttctt gggtgtgtgg cacagtcctg 120
   tcgtcagggg attctgctag aggggtaggg gaaaaaaccc ttattactat gaccatgcgc 180
   atgtggaatt acattccata gactttcgca tcattcccaa catttacaca gctctacacc 240
   tcttaagaag aggtgacgtg gattgggtgg ggcagccttg gcaccaaggg attccttttg 300
   agcttcggac tacctctgct ctctacaccc attaccctgt agatggcaca ttctggctta 360
   ttcttaatcc caaagatcct gtactttcct ctctatctaa tcgtcagcga ttgattgctg 420
   ccatccaaaa ggaaaaactg gtgaagcaag ctttaggaac acaatatcga gtagctgaaa 480
   gctctccatc tccagaggga atcatagctc atcaagaagc ttctactcct tttcctggga 540
   aaattacttt gatatatccc aataatatta cgcgctgtca gcgtttggcc gaggtatcca 600
   aaaaatgatc gacaaggagc acgctaaatt tgtacatacc ccaaaatcaa tcagccatct 660
   aggcaaatgg aatatcaaag taaacagtat acaactgggg atctcgtgcc gaattc      716
<210> 55
   <211> 463
   <212> DNA
   <213> Chlamydia trachomatis

<400> 55
   tctcaaatcc ttgctttgaa taatccagat atttcaaaaa ccatgttcga taaattcacc 60
   cgacaaggac tccgtttcgt actagaagcc tctgtatcaa atattgagga tataggagat 120
   cgcgttcggt taactatcaa tgggaatgtc gaagaatacg attacgttct cgtatctata 180
   ggacgccgtt tgaatacaga aaatattggc ttggataaag ctggtgttat ttgtgatgaa 240
   cgcggagtca tccctaccga tgccacaatg cgcacaaacg tacctaacat ttatgctatt 300
   ggagatatca caggaaaatg gcaacttgcc catgtagctt ctcatcaagg aatcattgca 360
   gcacggaata taggtggcca taaagaggaa atcgattact ctgctgtccc ttctgtgatc 420
   tttaccttcc ctgaagtcgc ttcagtaggc ctctccccaa cag 463
<210> 56
   <211> 829
   <212> DNA
   <213> Chlamydia trachomatis
<400> 56
   gtactatggg atcattagtt ggaagacagg ctccggattt ttctggtaaa gccgttgttt 60
   gtggagaaga gaaagaaatc tctctagcag actttcgtgg taagtatgta gtgctcttct 120
   tttatcctaa agattttacc tatgtttgtc ctacagaatt acatgctttt caagatagat 180
   tggtagattt tgaagagcat ggtgcagtcg tccttggttg ctccgttgac gacattgaga 240
   cacattctcg ttggctcact gtagcgagag atgcaggagg gatagaggga acagaatatc 300
   ctctgttagc agacccctct tttaaaatat cagaagcttt tggtgttttg aatcctgaag 360
   gatcgctcgc tttaagagct actttcctta tcgataaaca tggggttatt cgtcatgcgg 420
   ttatcaatga tcttccttta gggcgttcca ttgacgagga attgcgtatt ttagattcat 480
   tgatcttctt tgagaaccac ggaatggttt gtccagctaa ctggcgttct ggagagcgtg 540
   gaatggtgcc ttctgaagag ggattaaaag aatacttcca gacgatggat taagcatctt 600
   tgaaagtaag aaagtcgtac agatcttgat ctgaaaagag aagaaggctt tttaattttc 660
   tgcagagagc cagcgaggct tcaataatgt tgaagtctcc gacaccaggc aatgctaagg 720
   cgacgatatt agttagtgaa gtctgagtat taaggaaatg aaggccaaag aaatagctat 780
   caataaagaa gccttcttcc ttgactctaa agaatagtat gtcgtatcc 829
<210> 57
   <211> 1537
   <212> DNA
   <213> Chlamydia trachomatis
<400> 57
   acatcaagaa atagcggact cgcctttagt gaaaaaagct gaggagcaga ttaatcaagc 60
   acaacaagat attcaaacga tcacacctag tggtttggat attcctatcg ttggtccgag 120
   tgggtcagct gcttccgcag gaagtgcggc aggagcgttg aaatcctcta acaattcagg 180
   aagaatttcc ttgttgcttg atgatgtaga caatgaaatg gcagcgattg caatgcaagg 240
   ttttcgatct atgatcgaac aatttaatgt aaacaatcct gcaacagcta aagagctaca 300
   agctatggag gctcagctga ctgcgatgtc agatcaactg gttggtgcgg atggcgagct 360
   cccagccgaa atacaagcaa tcaaagatgc tcttgcgcaa gctttgaaac aaccatcagc 420
   agatggttta gctacagcta tgggacaagt ggcttttgca gctgccaagg ttggaggagg 480
   ctccgcagga acagctggca ctgtccagat gaatgtaaaa cagctttaca agacagcgtt 540
   ttcttcgact tcttccagct cttatgcagc agcactttcc gatggatatt ctgcttacaa 600
   aacactgaac tctttatatt ccgaaagcag aagcggcgtg cagtcagcta ttagtcaaac 660
   tgcaaatccc gcgctttcca gaagcgtttc tcgttctggc atagaaagtc aaggacgcag 720
   tgcagatgct agccaaagag cagcagaaac tattgtcaga gatagccaaa cgttaggtga 780
   tgtatatagc cgcttacagg ttctggattc tttgatgtct acgattgtga gcaatccgca 840
   agcaaatcaa gaagagatta tgcagaagct cacggcatct attagcaaag ctccacaatt 900
   tgggtatcct gctgttcaga attctgtgga tagcttgcag aagtttgctg cacaattgga 960
   aagagagttt gttgatgggg aacgtagtct cgcagaatct caagagaatg cgtttagaaa 1020
   acagcccgct ttcattcaac aggtgttggt aaacattgct tctctattct ctggttatct 1080
   ttcttaacgt gtgattgaag tttgtgaatt gagggggagc caaaaaagaa tttctttttt 1140
   ggctcttttt tcttttcaaa ggaatctcgt gtctacagaa gtcttttcaa taataagttc 1200
   ttagttccaa aagaagaaaa tatataaaag aaaaaactcc taattcattt aaaaagtgct 1260
   cggcagactt cgtggaaaat gtctgtaaag ctggagggga atcagcagaa agatgcaaga 1320
   tatccgagaa aaaaggctca ggctcgtgcc gaattcggca cgagactacg aaagaaaggt 1380
   cttttctttc ggaatctgtc attggatctg cgtaagactt aaagttcggc aacacaggct 1440
   ctgtcttctc tttaggtttc ttgcgcgaga aaaattttct caagtaacaa gaagatttct 1500
   ttttacagcc ggcatccggc ttctcgcgaa gtataac 1537
<210> 58
   <211> 463
   <212> DNA
   <213> Chlamydia trachomatis
<400> 58
   tctcaaatcc ttgctttgaa taatccagat atttcaaaaa ccatgttcga taaattcacc 60
   cgacaaggac tccgtttcgt actagaagcc tctgtatcaa atattgagga tataggagat 120
   cgcgttcggt taactatcaa tgggaatgtc gaagaatacg attacgttct cgtatctata 180
   ggacgccgtt tgaatacaga aaatattggc ttggataaag ctggtgttat ttgtgatgaa 240
   cgcggagtca tccctaccga tgccacaatg cgcacaaacg tacctaacat ttatgctatt 300
   ggagatatca caggaaaatg gcaacttgcc catgtagctt ctcatcaagg aatcattgca 360
   gcacggaata taggtggcca taaagaggaa atcgattact ctgctgtccc ttctgtgatc 420
   tttaccttcc ctgaagtcgc ttcagtaggc ctctccccaa cag 463
<210> 59
   <211> 552
   <212> DNA
   <213> Chlamydia trachomatis
<400> 59
   acattcctcc tgctcctcgc ggccatccac aaattgaggt aaccttcgat attgatgcca 60
   acggaatttt acacgtttct gctaaagatg ctgctagtgg acgcgaacaa aaaatccgta 120
   ttgaagcaag ctctggatta aaagaagatg aaattcaaca aatgatccgc gatgcagagc 180
   ttcataaaga ggaagacaaa caacgaaaag aagcttctga tgtgaaaaat gaagccgatg 240
   gaatgatctt tagagccgaa aaagctgtga aagattacca cgacaaaatt cctgcagaac 300
   ttgttaaaga aattgaagag catattgaga aagtacgcca agcaatcaaa gaagatgctt 360
   ccacaacagc tatcaaagca gcttctgatg agttgagtac tcgtatgcaa aaaatcggag 420
   aagctatgca ggctcaatcc gcatccgcag cagcatcttc tgcagcgaat gctcaaggag 480
   ggccaaacat taactccgaa gatctgaaaa aacatagttt cagcacacga cctccagcag 540
   gaggaagcgc ct                                                    552
<210> 60
   <211> 1180
   <212> DNA
   <213> Chlamydia trachomatis

<400> 60
   atcctagcgg taaaactgct tactggtcag ataaaatcca tacagaagca acacgtactt 60
   cttttaggag aaaaaatcta taatgctaga aaaatcctga gtaaggatca cttctcctca 120
   acaacttttt catcttggat agagttagtt tttagaacta agtcttctgc ttacaatgct 180
   cttgcatatt acgagctttt tataaacctc cccaaccaaa ctctacaaaa agagtttcaa 240
   tcgatcccct ataaatccgc atatattttg gccgctagaa aaggcgattt aaaaaccaag 300
   gtcgatgtga tagggaaagt atgtggaatc tcgtgccgaa ttcggcacga gcggcacgag 360
   gatgtagagt aattagttaa agagctgcat aattatgaca aagcatggaa aacgcattcg 420
   tggtatccaa gagacttacg atttagctaa gtcgtattct ttgggtgaag cgatagatat 480
   tttaaaacag tgtcctactg tgcgtttcga tcaaacggtt gatgtgtctg ttaaattagg 540
   gatcgatcca agaaagagtg atcagcaaat tcgtggttcg gtttctttac ctcacggtac 600
   aggtaaagtt ttgcgaattt tagtttttgc tgctggagat aaggctgcag aggctattga 660
   agcaggagcg gactttgttg gtagcgacga cttggtagaa aaaatcaaag gtggatgggt 720
   tgacttcgat gttgcggttg ccactcccga tatgatgaga gaggtcggaa agctaggaaa 780
   agttttaggt ccaagaaacc ttatgcctac gcctaaagcc ggaactgtaa caacagatgt 840
   ggttaaaact attgcggaac tgcgaaaagg taaaattgaa tttaaagctg atcgagctgg 900
   tgtatgcaac gtcggagttg cgaagctttc tttcgatagt gcgcaaatca aagaaaatgt 960
   tgaagcgttg tgtgcagcct tagttaaagc taagcccgca actgctaaag gacaatattt 1020
   agttaatttc actatttcct cgaccatggg gccaggggtt accgtggata ctagggagtt 1080
   gattgcgtta taattctaag tttaaagagg aaaaatgaaa gaagagaaaa agttgctgct 1140
   tcgcgaggtt gaagaaaaga taaccgcttc tcggcacgag                       1180
<210> 61
   <211> 1215
   <212> DNA
   <213> Chlamydia trachomatis
<400> 61
<210> 62
   <211> 688
   <212> DNA
   <213> Chlamydia trachomatis
<400> 62
   gtggatccaa aaaagaatct aaaaagccat acaaagattg cgttacttct tgcgatgcct 60
   ctaacacttt atcagcgtca tctttgagaa gcatctcaat gagcgctttt tcttctctag 120
   catgccgcac atccgcttct tcatgttctg tgaaatatgc atagtcttca ggattggaaa 180
   atccaaagta ctcagtcaat ccacgaattt tctctctagc gatacgtgga atttgactct 240
   cataagaata caaagcagcc actcctgcag ctaaagaatc tcctgtacac caccgcatga 300
   aagtagctac tttcgctttt gctgcttcac taggctcatg agcctctaac tcttctggag 360
   taactcctag agcaaacaca aactgcttcc acaaatcaat atgattaggg taaccgttct 420
   cttcatccat caagttatct aacaataact tacgcgcctc taaatcatcg caacgactat 480
   gaatcgcaga taaatattta ggaaaggctt tgatatgtaa ataatagtct ttggcacgag 540
   cctgtaattg ctctttagta agctccccct tcgaccattt cacataaaac gtgtgttcta 600
   gcatatgctt attttgaata attaaatcta actgatctaa aaaattcata aacacctcca 660
   tcatttcttt tcttgactcc acgtaacc                                    688
<210> 63
   <211> 269
   <212> DNA
   <213> Chlamydia trachomatis
<400> 63
   atgttgaaat cacacaagct gttcctaaat atgctacggt aggatctccc tatcctgttg 60
   aaattactgc tacaggtaaa agggattgtg ttgatgttat cattactcag caattaccat 120
   gtgaagcaga gttcgtacgc agtgatccag cgacaactcc tactgctgat ggtaagctag 180
   tttggaaaat tgaccgctta ggacaaggcg aaaagagtaa aattactgta tgggtaaaac 240
   ctcttaaaga aggttgctgc tttacagct                                    269
<210> 64
   <211> 1339
   <212> DNA
   <213> Chlamydia trachomatis
<400> 64
   cttttattat ggcttctggg gatgatgtca acgatatcga cctgctatct cgaggagatt 60
   ttaaaattgt tatacagacg gctccagagg agatgcatgg attagcggac tttttggctc 120
   ccccggcgaa ggatcttggt attctctccg cctgggaagc tggtgagctg cgttacaaac 180
   agctagttaa tccttaggaa acatttctgg acctatgccc atcacattgg ctccgtgatc 240
   cacatagaga gtttctcccg taattgcgct agctagggga gagactaaga aggctgctgc 300
   tgcgcctact tgctcagctt ccattggaga aggtagtgga gcccagtctt ggtagtaatc 360
   caccattctc tcaataaatc caatagcttt tcctgcacgg ctagctaatg gccctgccga 420
   gatagtattc actcggactc cccaacgtcg gccggcttcc caagccagta cttttgtatc 480
   actttctaaa gcagcttttg ctgcgttcat tcctccgcca taccctggaa cagcacgcat 540
   ggaagcaaga taagttagag agatggtgct agctcctgca ttcataattg ggccaaaatg 600
   agagagaagg ctgataaagg agtagctgga tgtacttaag gcggcaagat agcctttacg 660
   agaggtatca agtaatggtt tagcaatttc cggactgttt gctaaagagt gaacaagaat 720
   atcaatgtgt ccaaaatctt ttttcacctg ttctacaact tcggatacag tgtacccaga 780
   aagatctttg taacgtttat tttccaaaat ttcctgagga atatcttctg gggtgtcgaa 840
   actggcatcc atgggataga ttttagcgaa agttagcaat tctccattgg agagttcacg 900
   agatgcattg aatttcccta actcccaaga ttgagagaaa attttataga taggaaccca 960
   ggtccccaca agtatggttg cgcctgcttc tgctaacatt ttggcaatgc cccagccata 1020
   cccgttatca tcgcctatgc cggctatgaa agcaattttt cctgttaaat caattttcaa 1080
   catgagctaa ccccattttg tcttcttgag agaggagagt agcagattct ttattattga 1140
   gaaacgggcc tcataataca taaggagtag attcactggc tggatccagg tttctagagt 1200
   aaagagtttc cttgtcaaat tcttatatgg gtagagttaa tcaactgttt tcaagtgatt 1260
   tatgtttatt ttaaaataat ttgttttaac aactgtttaa tagttttaat ttttaaagtg 1320
   tgaaaaacag gttttatat                                               1339
<210> 65
   <211> 195
   <212> PRT
   <213> Chlamydia trachomatis

<400> 65
   Met Gly Ser Leu Val Gly Arg Gln Ala Pro Asp Phe Ser Gly Lys Ala
                   5                   10                  15
   Val Val Cys Gly Glu Glu Lys Glu Ile Ser Leu Ala Asp Phe Arg Gly
   20 25 30
   Lys Tyr Val Val Leu Phe Phe Tyr Pro Lys Asp Phe Thr Tyr Val Cys
   35 40 45
   Pro Thr Glu Leu His Ala Phe Gln Asp Arg Leu Val Asp Phe Glu Glu
   50 55 60
   His Gly Ala Val Val Leu Gly Cys Ser Val Asp Asp Ile Glu Thr His
   65 70 75 80
   Ser Arg Trp Leu Thr Val Ala Arg Asp Ala Gly Gly Ile Glu Gly Thr
   85 90 95
   Glu Tyr Pro Leu Leu Ala Asp Pro Ser Phe Lys Ile Ser Glu Ala Phe
   100 105 110
   Gly Val Leu Asn Pro Glu Gly Ser Leu Ala Leu Arg Ala Thr Phe Leu
   115 120 125
   Ile Asp Lys His Gly Val Ile Arg His Ala Val Ile Asn Asp Leu Pro
   130 135 140
   Leu Gly Arg Ser Ile Asp Glu Glu Leu Arg Ile Leu Asp Ser Leu Ile
   145 150 155 160
   Phe Phe Glu Asn His Gly Met Val Cys Pro Ala Asn Trp Arg Ser Gly
   165 170 175
   Glu Arg Gly Met Val Pro Ser Glu Glu Gly Leu Lys Glu Tyr Phe Gln
   180 185 190
   Thr Met Asp
   195
<210> 66
   <211> 520
   <212> DNA
   <213> Chlamydia
<400> 66
   gatccgaatt cggcacgagg aggaatggaa gggccctccg attttaaatc tgctaccatg 60
   ccattcacta gaaactccat aacagcggtt ttctctgatg gcgagtaaga agcaagcatt 120
   tgatgtaaat tagcgcaatt agagggggat gaggttactt ggaaatataa ggagcgaagc 180
   gatgaaggag atgtatttgc tctggaagca aaggtttctg aagctaacag aacattgcgt 240
   cctccaacaa tcgcctgagg attctggctc atcagttgat gctttgcctg aatgagagcg 300
   gacttaagtt tcccatcaga gggagctatt tgaattagat aatcaagagc tagatccttt 360
   attgtgggat cagaaaattt acttgtgagc gcatcgagaa tttcgtcaga agaagaatca 420
   tcatcgaacg aatttttcaa tcctcgaaaa tcttctccag agacttcgga aagatcttct 480
   gtgaaacgat cttcaagagg agtatcgcct ttttcctctg 520
<210> 67
   <211> 276
   <212> DNA
   <213> Chlamydia
<400> 67
   gatccgaatt cggcacgagg tattgaagga gaaggatctg actcgatcta tgaaatcatg 60
   atgcctatct atgaagttat gaatatggat ctagaaacac gaagatcttt tgcggtacag 120
   caagggcact atcaggaccc aagagcttca gattatgacc tcccacgtgc tagcgactat 180
   gatttgccta gaagcccata tcctactcca cctttgcctt ctagatatca gctacagaat 240
   atggatgtag aagcagggtt ccgtgaggca gtttat                           276
<210> 68
   <211> 248
   <212> DNA
   <213> Chlamydia
<400> 68
   gatccgaatt cggcacgagg tgttcaagaa tatgtccttc aagaatgggt taaattgaaa 60
   gatctaccgg tagaagagtt gctagaaaaa cgatatcaga aattccgaac gataggtcta 120
   tatgaaactt cttctgaaag cgattctgag gcataagaag catttagttt tattcggttt 180
   ttctctttta tccatattag ggctaacgat aacgtctcaa gcagaaattt tttctctagg 240
   tcttattg                                                          248
<210> 69
   <211> 715
   <212> DNA
   <213> Chlamydia
<220>
   <221> unsure
   <222> (34)
   <223> n=A,T,C or G
<400> 69
   gatccgaatt cggcacgaga aggtagatcc gatntcagca aaagtgctcc taaaggaaga 60
   ttccttcggt atcctgcagc aaataaggtg gcacactcca tctcggacag tttgagcttt 120
   attttcatat agttttcgac ggaactcttt attaaactcc caaaaccgaa tgttagtcgt 180
   gtgggtgatg cctatatggt aagggaggtt tttggcttcg agaatattgg tgatcatttt 240
   ttgtacgaca aaattagcta atgcagggac ctctgggggg aagtatgcat ctgatgttcc 300
   atcttttcgg atgctagcaa cagggacaaa ataatctcct atttggtagt gggatcttaa 360
   gcctccgcac atgcccaaca tgatcgctgc tgtagcattg ggaaggaaag aacacagatc 420
   tacggtaaga gctgctcctg gagagcctaa tttaaaatcg atgattgagg tgtgaatttg 480
   aggcgcatgc gctgccgaaa acatggatcc tcgagaaaca gggacctgat agatttcagc 540
   gaaaacatcc acggtaatac ccmaaattag taagaaggag atagggctgg aactcttgaa 600
   tggtagagcc ggtatagcgc tctagcatgt cacaggcgat tgtttcttcg ctgatttttt 660
   tatgttgatg ggtcataaat cacagatatt ataatggtta gagaatcttt ttttc      715
<210> 70
   <211> 323
   <212> DNA
   <213> Chlamydia

<400> 70
   gatccgaatt cggcacgagc agaacgtaaa cagcacactt aaaccgtgta tgaggtttaa 60
   cactgtttgg caagcaaaca accattcctc tttccacatc gttcttacca atacctctga 120
   ggagcaatcc aacattctct cctgcacgac cttctgggag ttcttttctg aacatttcaa 180
   ccccagtaac aatcgtttct ttagtatctc taagaccgac caactgaact ttatcggaaa 240
   ctttaacaat tccacgctca atacgtccag ttactacagt tcctcgtccg gagatagaga 300
   acacgtcctc aatgggcatt aag                                         323
<210> 71
   <211> 715
   <212> DNA
   <213> Chlamydia
<400> 71
   gatccgaatt cggcacgagg aaaaaaagat tctctaacca ttataatatc tgtgatttat 60
   gacccatcaa cataaaaaaa tcagcgaaga aacaatcgcc tgtgacatgc tagagcggct 120
   ataccggctc taccattcaa gagttccagc cctatctcct tcttactaat tttgggtatt 180
   acgtggatgt tttcgctgaa atctatcagg tccctgtttc tcgaggatcc atgttttcgg 240
   gcagcgcatg cgcctcaaat tcacacctca atcatcgatt ttaaattagg ctctccagga 300
   gcagctctta ccgtagatct gtgttctttc cttcccaatg ctacagcagc gatcatgttg 360
   ggcatgtgcg gaggcttaag atcccactac caaataggag attattttgt ccctgttgct 420
   agcatccgaa aagatggaac atcagatgca tacttccccc cagaggtccc tgcattagct 480
   aattttgtcg tacaaaaaat gatcaccaat attctcgaag ccaaaaacct cccttaccat 540
   ataggcatca cccacacgac taacattcgg ttttgggagt ttaataaaga gttccgtcga 600
   aaactatatg aaaataaagc tcaaactgtc gagatggagt gtgccacctt atttgctgca 660
   ggataccgaa ggaatcttcc tttaggagca cttttgctga tatcggatct acctt      715
<210> 72
   <211> 641
   <212> DNA
   <213> Chlamydia
<220>
   <221> unsure
   <222> (550)
   <223> n=A,T,C or G
   <221> unsure
   <222> (559)
   <223> n=A,T,C or G
   <221> unsure
   <222> (575)
   <223> n=A,T,C or G
   <221> unsure
   <222> (583)
   <223> n=A,T,C or G
   <221> unsure
   <222> (634)
   <223> n=A,T,C or G
   <221> unsure
   <222> (638)
   <223> n=A,T,C or G
<400> 72
   gatccgaatt cggcacgaga tctcctcgag ctcgatcaaa cccacacttg ggacaagtac 60
   ctacaacata acggtccgct aaaaacttcc cttcttcctc agaatacagc tgttcggtca 120
   cctgattctc taccagtccg cgttcctgca agtttcgata gaaatcttgc acaatagcag 180
   gatgataagc gttcgtagtt ctggaaaaga aatctacaga aattcccaat ttcttgaagg 240
   tatctttatg aagcttatga tacatgtcga catattcttg ataccccatg cctgccaact 300
   ctgcattaag ggtaattgcg attccgtatt catcagaacc acaaatatac aaaacctctt 360
   tgccttgtag tctctgaaaa cgcgcataaa catctgcagg caaataagca ccggtaatat 420
   gtccaaaatg caaaggacca tttgcgtaag gcaacgcaga agtaataaga atacgggaag 480
   attccactat ttcacgtcgc tccagttgta cagagaagga tcttttcttc tggatgttcc 540
   gaaaccttgn tctcttcgnc tctctcctgt agcanacaaa tgnctctctc gacatctctt 600
   tcagcgtatt cggactgatg ccctaaagat cccnggangt t 641
<210> 73
   <211> 584
   <212> DNA
   <213> Chlamydia
<220>
   <221> unsure
   <222> (460)
   <223> n=A,T,C or G
   <221> unsure
   <222> (523)
   <223> n=A,T,C or G
   <221> unsure
   <222> (541)
   <223> n=A,T,C or G
   <221> unsure
   <222> (546)
   <223> n=A,T,C or G
<400> 73
   gaattcggca cgagacattt ctagaatgga accggcaaca aacaaaaact ttgtatctga 60
   agatgacttt aagcaatctt tagataggga agattttttg gaatgggtct ttttatttgg 120
   gacttattac ggaacgagta aggcggagat ttctagagtt ctgcaaaagg gtaagcactg 180
   catagccgtg attgatgtac aaggagcttt ggctctgaag aagcaaatgc cggcagtcac 240
   tatttttatt caagctccct ctcaagaaga acttgagcgc cgtttgaatg ctcgggattc 300
   agagaaagat ttccagaaga aagaaagatt agagcatagc gctgtcgaaa ttgctgccgc 360
   tagcgaattt gattatgttg tggttaatga tgatttgatt acagcatatc aagttttaag 420
   aagtattttt atagctgaag aacataggat gagtcatggn tagaaaagat cgtttaacta 480
   atgaaagact gaataagcta tttgatagcc cctttagttt ggntaattac gtaattaagc 540
   nagctnagaa caaaattgct agaggagatg ttcgttcttc taac 584
<210> 74
   <211> 465
   <212> DNA
   <213> Chlamydia
<400> 74
   gatccgaatt cggcacgagc tcgtgccgtt tgggatcgtg taatcgcatc ggagaatggt 60
   taagaaatta ttttcgagtg aaagagctag gcgtaatcat tacagatagc catactactc 120
   caatgcggcg tggagtactg ggtatcgggc tgtgttggta tggattttct ccattacaca 180
   actatatagg atcgctagat tgtttcggtc gtcccttaca gatgacgcaa agtaatcttg 240
   tagatgcctt agcagttgcg gctgttgttt gtatgggaga ggggaatgag caaacaccgt 300
   tagcggtgat agagcaggca cctaatatgg tctaccattc atatcctact tctcgagaag 360
   agtattgttc tttgcgcata gatgaaacag aggacttata cggacctttt ttgcaagcgg 420
   ttaccgtgga gtcaagaaaa gaaatgatgg aggtgtttat gaatt                 465
<210> 75
   <211> 545
   <212> DNA
   <213> Chlamydia

<400> 75
   gaattcggca cgagatgaaa agttagcgtc acaggggatt ctcctaccaa agaattccga 60
   aaagttttct tccaaaaacc tcttcctctc ttgattagtg atccctctgc aactacttta 120
   ctatatgttc tgtgaaatat gcatagtctt caggattgga aaatccaaag tactcagtca 180
   atccacgaat tttctctcta gcgatacgtg gaatttgact ctcataagaa tacaaagcag 240
   ccactcctgc agctaaagaa tctcctgtac accaccgcat gaaagtagct actttcgctt 300
   ttgctgcttc actaggctca tgagcctcta actcttctgg agtaactcct agagcaaaca 360
   caaactgctt ccacaaatca atatgattag ggtaaccgtt ctcttcatcc atcaagttat 420
   ctaacaataa cttacgcgcc tctaaatcat cgcaacgact atgaatcgca gataaatatt 480
   taggaaaggc tttgatatgt aaataatagt ctttggcata cgcctgtaat tgctctttag 540
   taagc                                                             545
<210> 76
   <211> 797
   <212> DNA
   <213> Chlamydia
<220>
   <221> unsure
   <222> (788)
   <223> n=A,T,C or G
   <221> unsure
   <222> (789)
   <223> n=A,T,C or G
<400> 76
   gatccgaatt cggcacgaga tacgctagat gcgataaatg cggataatga ggattatcct 60
   aaaccaggtg acttcccacg atcttccttc tctagtacgc ctcctcatgc tccagtacct 120
   caatctgaga ttccaacgtc acctacctca acacagcctc catcacccta acttgtaaaa 180
   actgtaataa aaagagcgcg cttcctttat gcaaaatcaa tttgaacaac tccttactga 240
   attagggact caaatcaaca gccctcttac tcctgattcc aataatgcct gtatagttcg 300
   ctttggatac aacaatgttg ctgtacaaat tgaagaggat ggtaattcag gatttttagt 360
   tgctggagtc atgcttggaa aacttccaga gaataccttt agacaaaaaa ttttcaaagc 420
   tgctttgtct atcaatggat ctccgcaatc taatattaaa ggcactctag gatacggtga 480
   aatctctaac caactctatc tctgtgatcg gcttaacatg acctatctaa atggagaaaa 540
   gctcgcccgt tacttagttc ttttttcgca gcatgccaat atctggatgc aatctatctc 600
   aaaaggagaa cttccagatt tacatgctct aggtatgtat cacctgtaaa ttatgccgtc 660
   attatcccaa tcccgacgta tcatccagca atcttccatt cgaaagattt ggaatcagat 720
   agatacttct cctaagcatg ggggtatgcg taccggttat ttttctcttc atactcaaaa 780
   aaagttgnng gggaata                                                797
<210> 77
   <211> 399
   <212> DNA
   <213> Chlamydia
<400> 77
   catatgcatc accatcacca tcacatgcca cgcatcattg gaattgatat tcctgcaaag 60
   aaaaagttaa aaataagtct gacatatatt tatggaatag gatcagctcg ttctgatgaa 120
   atcattaaaa agttgaagtt agatcctgag gcaagagcct ctgaattaac tgaagaagaa 180
   gtaggacgac tgaactctct gctacaatca gaatataccg tagaagggga tttgcgacgt 240
   cgtgttcaat cggatatcaa aagattgatc gccatccatt cttatcgagg tcagagacat 300
   agactttctt taccagtaag aggacaacgt acaaaaacta attctcgtac tcgaaaaggt 360
   aaaagaaaaa cagtcgcagg taagaagaaa taagaattc                        399
<210> 78
   <211> 285
   <212> DNA
   <213> Chlamydia
<400> 78
   atgcatcacc atcaccatca catgagtcaa aaaaataaaa actctgcttt tatgcatccc 60
   gtgaatattt ccacagattt agcagttata gttggcaagg gacctatgcc cagaaccgaa 120
   attgtaaaga aagtttggga atacattaaa aaacacaact gtcaggatca aaaaaataaa 180
   cgtaatatcc ttcccgatgc gaatcttgcc aaagtctttg gctctagtga tcctatcgac 240
   atgttccaaa tgaccaaagc cctttccaaa catattgtaa aataa                285
<210> 79
   <211> 950
   <212> DNA
   <213> Chlamydia
<400> 79
   aaattaactc gagcacaaat tacggcaatt gctgagcaaa agatgaagga catggatgtc 60
   gttcttttag agtccgccga gagaatggtt gaagggactg cccgaagcat gggtgtagat 120
   gtagagtaat tagttaaaga gctgcataat tatgacaaag catggaaaac gcattcgtgg 180
   tatccaagag acttacgatt tagctaagtc gtattctttg ggtgaagcga tagatatttt 240
   aaaacagtgt cctactgtgc gtttcgatca aacggttgat gtgtctgtta aattagggat 300
   cgatccaaga aagagtgatc agcaaattcg tggttcggtt tctttacctc acggtacagg 360
   taaagttttg cgaattttag tttttgctgc tggagataag gctgcagagg ctattgaagc 420
   aggagcggac tttgttggta gcgacgactt ggtagaaaaa atcaaaggtg gatgggttga 480
   cttcgatgtt gcggttgcca ctcccgatat gatgagagag gtcggaaagc taggaaaagt 540
   tttaggtcca agaaacctta tgcctacgcc taaagccgga actgtaacaa cagatgtggt 600
   taaaactatt gcggaactgc gaaaaggtaa aattgaattt aaagctgatc gagctggtgt 660
   atgcaacgtc ggagttgcga agctttcttt cgatagtgcg caaatcaaag aaaatgttga 720
   agcgttgtgt gcagccttag ttaaagctaa gcccgcaact gctaaaggac aatatttagt 780
   taatttcact atttcctcga ccatggggcc aggggttacc gtggatacta gggagttgat 840
   tgcgttataa ttctaagttt aaagaggaaa aatgaaagaa gagaaaaagt tgctgcttcg 900
   cgaggttgaa gaaaagataa ccgcttctca aggttttatt ttgttgagat            950
<210> 80
   <211> 395
   <212> DNA
   <213> Chlamydia
<400> 80
   tttcaaggat tttgttttcc cgatcatctt actaaatgca gctccaacaa tcacatcatg 60
   ggctggttta gcatctaagg caacagaagc tcctctgctg taataagtga attcttcaga 120
   agtaggtgtt cctacttgcg atagcatcgt tcctagtcct gatatccaca ggttgttata 180
   gctaacttca tcaaagcgag ctagattcat tttatcgttg agcaagcctt gtttgactgt 240
   gaccattgac atttgagatc ccagaatcga gttcgcatag aaatgattgt ctctaggtac 300
   ataagcccat tgtctataag agtcaaattt ccagagcgct gagatcgttc cattttgtag 360
   ttgatcagga tccagagtga gtgttcctgt atatc                            395
<210> 81
   <211> 2085
   <212> DNA
   <213> Chlamydia

<400> 81
   atttggcgaa ggagtttggg ctacggctat taataaatca ttcgtgttcg ctgcctccaa 60
   gaccagattg tgtactttct tatgaagaat ctcctattga gcaaatgttg cgttggggag 120
   agtctcagtt agaacaattt gctcaagtag gtttagatac aagttggcaa gttgttttcg 180
   atccaggaat aggatttggg aagactcccg ttcagtcgat gttattgatg gatggagtaa 240
   agcagtttaa acgtgtttta gagtgtcctg tattaatagg ccattctaga aaatcgtgtt 300
   tgagtatgtt gggccgattt aatagtgacg atcgtgattg ggaaacgatc ggctgttctg 360
   tatctcttca tgatcgagga gttgattatc tacgtgtgca tcaggttgaa ggtaacagac 420
   gtgccttagc cgctgctgct tgggctggta tgtttgtatg atccaagcaa caggtatcgt 480
   tgctattgat cccagaggag tgatgggagc tttaggcaag ctcccttgga gttatcccga 540
   agatctacgt ttttttgcag aaaccattcg aaatcatccc atcattatgg gacgaaagac 600
   ttgggagtct cttccagaca agtataagca tgggcgggat atcgttgtct tttctcgcag 660
   gatgcatcca ccacaatgca taggagtttc ttcctttgca gagtatggga cactatcttt 720
   gaatcatccg tttttaattg ggggagcgga gctctttgaa agttttttcc aacaaaacct 780
   tctgaaagct tgttttgtca cacatatcaa aaagaaatat tggggcgata ctttcttccc 840
   tatcacgcga ttatcaggat ggaagaagga atgtatttgt aatacagagg atttcagtat 900
   ttattattat gaaaataact ccgatcaaaa cacgtaaagt atttgcacat gattcgcttc 960
   aagagatctt gcaagaggct ttgccgcctc tgcaagaacg gagtgtggta gttgtctctt 1020
   caaagattgt gagtttatgt gaaggcgctg tcgctgatgc aagaatgtgc aaagcagagt 1080
   tgataaaaaa agaagcggat gcttatttgt tttgtgagaa aagcgggata tatctaacga 1140
   aaaaagaagg tattttgatt ccttctgcag ggattgatga atcgaatacg gaccagcctt 1200
   ttgttttata tcctaaagat attttgggat cgtgtaatcg catcggagaa tggttaagaa 1260
   attattttcg agtgaaagag ctaggcgtaa tcattacaga tagccatact actccaatgc 1320
   ggcgtggagt actgggtatc gggctgtgtt ggtatggatt ttctccatta cacaactata 1380
   taggatcgct agattgtttc ggtcgtccct tacagatgac gcaaagtaat cttgtagatg 1440
   ccttagcagt tgcggctgtt gtttgtatgg gagaggggaa tgagcaaaca ccgttagcgg 1500
   tgatagagca ggcacctaat atggtctacc attcatatcc tacttctcga gaagagtatt 1560
   gttctttgcg catagatgaa acagaggact tatacggacc ttttttgcaa gcggttacgt 1620
   ggagtcaaga aaagaaatga tggaggtgtt tatgaatttt ttagatcagt tagatttaat 1680
   tattcaaaat aagcatatgc tagaacacac gttttatgtg aaatggtcga agggggagct 1740
   tactaaagag caattacagg cgtatgccaa agactattat ttacatatca aagcctttcc 1800
   taaatattta tctgcgattc atagtcgttg cgatgattta gaggcgcgta agttattgtt 1860
   agataacttg atggatgaag agaacggtta ccctaatcat attgatttgt ggaagcagtt 1920
   tgtgtttgct ctaggagtta ctccagaaga gttagaggct catgagccta gtgaagcagc 1980
   aaaagcgaaa gtagctactt tcatgcggtg gtgtacagga gattctttag ctgcaggagt 2040
   ggctgctttg tattcttatg agagtcaaat tccacgtatc gcctc                 2085
<210> 82
   <211> 405
   <212> DNA
   <213> Chlamydia
<400> 82
   ttcatcggtc tagttcgcta ttctactctc caatggttcc gcatttttgg gcagagcttc 60
   gcaatcatta tgcaacgagt ggtttgaaaa gcgggtacaa tattgggagt accgatgggt 120
   ttctccctgt cattgggcct gttatatggg agtcggaggg tcttttccgc gcttatattt 180
   cttcggtgac tgatggggat ggtaagagcc ataaagtagg atttctaaga attcctacat 240
   atagttggca ggacatggaa gattttgatc cttcaggacc gcctccttgg gaagaattgt 300
   attggctcca taaagggagg agaaaacttc gatataggga atcgtatcaa ggtgaaagta 360
   gcaaaaaata aattagctcc tccattccga actgcagaat ttgat                 405
<210> 83
   <211> 379
   <212> DNA
   <213> Chlamydia
<400> 83
   tataccattc gtttgaaagt gcctttgacg ggagaaagtg tttttgaaga tcaatgcaaa 60
   ggtcgtgtcg ttttcccttg ggcagatgtt gacgatcaag ttttggttaa atcagacggg 120
   ttccctacgt atcactttgc taatgtagtt gatgatcatt tgatggggat tacccatgtg 180
   ttgcgagggg aagagtggtt aagttctaca cctaaacacc ttcttcttta caaagctttt 240
   gggtgggagc ctccgcagtt tttccatatg ccgcttcttc taaatcctga tggaagtaag 300
   ctttccaaga gaaagaatcc tacttctatt ttttactatc gggatgctgg atacaaaaaa 360
   gaagcgttca tgaatttcc 379
<210> 84
   <211> 715
   <212> DNA
   <213> Chlamydia
<400> 84
   tcaatcctgt attaataatt ctggttctta gactacataa attaggaacg cctgatgagt 60
   atccataact aatcgcgtag ggcttagaat caccttctcg taccaaagct agaacaacgc 120
   cgccttccat tcttgatgca ataatatctg ctgagactaa gaacatgctc ccagagcttt 180
   tgggtgtgac tgtgaatttt cctatttcag ttcctcctaa taaagtttca atgttcctgg 240
   gagtgaataa cccgttgcat tgaattttat tagtgattgg aaagttgtta aaagctttca 300
   acaaacctag agaagggtct gttgtgattt tgtctaaaat atcttggact gtactatcaa 360
   caatagtatc agcaattcca ccaagaattt gatctcccaa cttttctaga ataagctggt 420
   aagctttttc cgcatccaaa ccaattgtaa tagaagcatt ggttgatgga ttattggaga 480
   ctgttaaaga tattccatca gaagctgtca ttttggctgc gacaggtgtt gatgttgtcc 540
   caaggattat ttgctggtcc ttgagcggct ctgtcatttg cccaactttg atattatcag 600
   caaagacgca gttttgagtg ttatacaaat aaaaaccaga atttcccatt ttaaaactct 660
   tttttatttt gagctttaaa taaattaggt ttttagtttc aagtttgcta ttaat 715
<210> 85
   <211> 476
   <212> DNA
   <213> Chlamydia
<400> 85
   ctcgtgccgc tcgtgccgct cgtgccggtc ttttagaaga gcgtgaagct ttaaataatt 60
   cgattacgtt tatcatggat aagcgtaatt ggatagaaac cgagtctgaa caggtacaag 120
   tggttttcag agatagtaca gcttgcttag gaggaggcgc tattgcagct caagaaattg 180
   tttctattca gaacaatcag gctgggattt ccttcgaggg aggtaaggct agtttcggag 240
   gaggtattgc gtgtggatct ttttcttccg caggcggtgc ttctgtttta gggactattg 300
   atatttcgaa gaatttaggc gcgatttcgt tctctcgtac tttatgtacg acctcagatt 360
   taggacaaat ggagtaccag ggaggaggag ctctatttgg tgaaaatatt tctctttctg 420
   agaatgctgg tgtgctcacc tttaaagaca acattgtgaa gacttttgct tcgaat 476
<210> 86
   <211> 1551
   <212> DNA
   <213> Chlamydia
<400> 86
   gcgtatcgat atttcttctg ttacattctt tatagggatt ctgttggctg ttaatgcgct 60
   aacctactct catgtattac gggatttatc tgtgagtatg gatgcgctgt tttctcgtaa 120
   cacgcttgct gttcttttag gtttagtctc tagcgtttta gataatgtgc cattagtcgc 180
   tgcaacaata ggtatgtatg acttacctat gaacgatcct ctttggaaac tcattgccta 240
   tacagcaggc acagggggaa gtattctcat cattggatcc gctgcaggtg ttgcctacat 300
   gggaatggaa aaagtgagtt tcggctggta tgtcaaacac gcttcttgga ttgctttagc 360
   cagttatttt ggaggtctag cagtctattt tctaatggaa aattgtgtga atttgttcgt 420
   ttgaggtagt cagtatggca gagtttcttt aaaaattctt ttaataaaag ggttctctgc 480
   ctattctagg cccctttttg aatggaaaaa tgggtttttg gagaacatcg attatgaaaa 540
   tgaataggat ttggctatta ctgcttacct tttcttctgc catacattct cctgtacgag 600
   gagaaagctt ggtttgcaag aatgctcttc aagatttgag ttttttagag catttattac 660
   aggttaaata tgctcctaaa acatggaaag agcaatactt aggatgggat cttgttcaaa 720
   gctccgtttc tgcacagcag aagcttcgta cacaagaaaa tccatcaaca agtttttgcc 780
   agcaggtcct tgctgatttt atcggaggat taaatgactt tcacgctgga gtaactttct 840
   ttgcgataga aagtgcttac cttccttata ccgtacaaaa aagtagtgac ggccgtttct 900
   actttgtaga tatcatgact ttttcttcag agatccgtgt tggagatgag ttgctagagg 960
   tggatggggc gcctgtccaa gatgtgctcg ctactctata tggaagcaat cacaaaggga 1020
   ctgcagctga agagtcggct gctttaagaa cactattttc tcgcatggcc tctttagggc 1080
   acaaagtacc ttctgggcgc actactttaa agattcgtcg tccttttggt actacgagag 1140
   aagttcgtgt gaaatggcgt tatgttcctg aaggtgtagg agatttggct accatagctc 1200
   cttctatcag ggctccacag ttacagaaat cgatgagaag ctttttccct aagaaagatg 1260
   atgcgtttca tcggtctagt tcgctattct actctccaat ggttccgcat ttttgggcag 1320
   agcttcgcaa tcattatgca acgagtggtt tgaaaagcgg gtacaatatt gggagtaccg 1380
   atgggtttct ccctgtcatt gggcctgtta tatgggagtc ggagggtctt ttccgcgctt 1440
   atatttcttc ggtgactgat ggggatggta agagccataa agtaggattt ctaagaattc 1500
   ctacatatag ttggcaggac atggaagatt ttgatccttc aggaccgcct c          1551
<210> 87
   <211> 3031
   <212> DNA
   <213> Chlamydia

<400> 87
   atgtaggccc tcaagcggtt ttattgttag accaaattcg agatctattc gttgggtcta 60
   aagatagtca ggctgaagga cagtataggt taattgtagg agatccaagt tctttccaag 120
   agaaagatgc agatactctt cccgggaagg tagagcaaag tactttgttc tcagtaacca 180
   atcccgtggt tttccaaggt gtggaccaac aggatcaagt ctcttcccaa gggttaattt 240
   gtagttttac gagcagcaac cttgattctc cccgtgacgg agaatctttt ttaggtattg 300
   cttttgttgg ggatagtagt aaggctggaa tcacattaac tgacgtgaaa gcttctttgt 360
   ctggagcggc tttatattct acagaagatc ttatctttga aaagattaag ggtggattgg 420
   aatttgcatc atgttcttct ctagaacagg ggggagcttg tgcagctcaa agtattttga 480
   ttcatgattg tcaaggattg caggttaaac actgtactac agccgtgaat gctgaggggt 540
   ctagtgcgaa tgatcatctt ggatttggag gaggcgcttt ctttgttacg ggttctcttt 600
   ctggagagaa aagtctctat atgcctgcag gagatatggt agttgcgaat tgtgatgggg 660
   ctatatcttt tgaaggaaac agcgcgaact ttgctaatgg aggagcgatt gctgcctctg 720
   ggaaagtgct ttttgtcgct aatgataaaa agacttcttt tatagagaac cgagctttgt 780
   ctggaggagc gattgcagcc tcttctgata ttgcctttca aaactgcgca gaactagttt 840
   tcaaaggcaa ttgtgcaatt ggaacagagg ataaaggttc tttaggtgga ggggctatat 900
   cttctctagg caccgttctt ttgcaaggga atcacgggat aacttgtgat aataatgagt 960
   ctgcttcgca aggaggcgcc atttttggca aaaattgtca gatttctgac aacgaggggc 1020
   cagtggtttt cagagatagt acagcttgct taggaggagg cgctattgca gctcaagaaa 1080
   ttgtttctat tcagaacaat caggctggga tttccttcga gggaggtaag gctagtttcg 1140
   gaggaggtat tgcgtgtgga tctttttctt ccgcaggcgg tgcttctgtt ttagggacta 1200
   ttgatatttc gaagaattta ggcgcgattt cgttctctcg tactttatgt acgacctcag 1260
   atttaggaca aatggagtac cagggaggag gagctctatt tggtgaaaat atttctcttt 1320
   ctgagaatgc tggtgtgctc acctttaaag acaacattgt gaagactttt gcttcgaatg 1380
   ggaaaattct gggaggagga gcgattttag ctactggtaa ggtggaaatt accaataatt 1440
   ccggaggaat ttcttttaca ggaaatgcga gagctccaca agctcttcca actcaagagg 1500
   agtttccttt attcagcaaa aaagaagggc gaccactctc ttcaggatat tctgggggag 1560
   gagcgatttt aggaagagaa gtagctattc tccacaacgc tgcagtagta tttgagcaaa 1620
   atcgtttgca gtgcagcgaa gaagaagcga cattattagg ttgttgtgga ggaggcgctg 1680
   ttcatgggat ggatagcact tcgattgttg gcaactcttc agtaagattt ggtaataatt 1740
   acgcaatggg acaaggagtc tcaggaggag ctcttttatc taaaacagtg cagttagctg 1800
   gaaatggaag cgtcgatttt tctcgaaata ttgctagttt gggaggacgc aatgttctgt 1860
   tagcttcaga aacctttgct tccagagcaa atacatctcc ttcatcgctt cgctccttat 1920
   atttccaagt aacctcatcc ccctctaatt gcgctaattt acatcaaatg cttgcttctt 1980
   actcgccatc agagaaaacc gctgttatgg agtttctagt gaatggcatg gtagcagatt 2040
   taaaatcgga gggcccttcc attcctcctg caaaattgca agtatatatg acggaactaa 2100
   gcaatctcca agccttacac tctgtagata gcttttttga tagaaatatt gggaacttgg 2160
   aaaatagctt aaagcatgaa ggacatgccc ctattccatc cttaacgaca ggaaatttaa 2220
   ctaaaacctt cttacaatta gtagaagata aattcccttc ctcttccaaa gctcaaaagg 2280
   cattaaatga actggtaggc ccagatactg gtcctcaaac tgaagtttta aacttattct 2340
   tccgcgctct taatggctgt tcgcctagaa tattctctgg agctgaaaaa aaacagcagc 2400
   tggcatcggt tatcacaaat acgctagatg cgataaatgc ggataatgag gattatccta 2460
   aaccaggtga cttcccacga tcttccttct ctagtacgcc tcctcatgct ccagtacctc 2520
   aatctgagat tccaacgtca cctacctcaa cacagcctcc atcaccctaa cttgtaaaaa 2580
   ctgtaataaa aagagcgcgc ttcctttatg caaaatcaat ttgaacaact ccttactgaa 2640
   ttagggactc aaatcaacag ccctcttact cctgattcca ataatgcctg tatagttcgc 2700
   tttggataca acaatgttgc tgtacaaatt gaagaggatg gtaattcagg atttttagtt 2760
   gctggagtca tgcttggaaa acttccagag aataccttta gacaaaaaat tttcaaagct 2820
   gctttgtcta tcaatggatc tccgcaatct aatattaaag gcactctagg atacggtgaa 2880
   atctctaacc aactctatct ctgtgatcgg cttaacatga cctatctaaa tggagaaaag 2940
   ctcgcccgtt acttagttct tttttcgcag catgccaata tctggatgca atctatctca 3000
   aaaggagaac ttccagattt acatgctcta g                                3031
<210> 88
   <211> 976
   <212> DNA
   <213> Chlamydia
<400> 88
   aggtggatgg ggcgcctgtc caagatgtgc tcgctactct atatggaagc aatcacaaag 60
   ggactgcagc tgaagagtcg gctgctttaa gaacactatt ttctcgcatg gcctctttag 120
   ggcacaaagt accttctggg cgcactactt taaagattcg tcgtcctttt ggtactacga 180
   gagaagttcg tgtgaaatgg cgttatgttc ctgaaggtgt aggagatttg gctaccatag 240
   ctccttctat cagggctcca cagttacaga aatcgatgag aagctttttc cctaagaaag 300
   atgatgcgtt tcatcggtct agttcgctat tctactctcc aatggttccg catttttggg 360
   cagagcttcg caatcattat gcaacgagtg gtttgaaaag cgggtacaat attgggagta 420
   ccgatgggtt tctccctgtc attgggcctg ttatatggga gtcggagggt cttttccgcg 480
   cttatatttc ttcggtgact gatggggatg gtaagagcca taaagtagga tttctaagaa 540
   ttcctacata tagttggcag gacatggaag attttgatcc ttcaggaccg cctccttggg 600
   aagaatttgc taagattatt caagtatttt cttctaatac agaagctttg attatcgacc 660
   aaacgaacaa cccaggtggt agtgtccttt atctttatgc actgctttcc atgttgacag 720
   accgtccttt agaacttcct aaacatagaa tgattctgac tcaggatgaa gtggttgatg 780
   ctttagattg gttaaccctg ttggaaaacg tagacacaaa cgtggagtct cgccttgctc 840
   tgggagacaa catggaagga tatactgtgg atctacaggt tgccgagtat ttaaaaagct 900
   ttggacgtca agtattgaat tgttggagta aaggggatat cgagttatca acacctattc 960
   ctctttttgg ttttga                                                 976
<210> 89
   <211> 94
   <212> PRT
   <213> Chlamydia
<400> 89
   Met His His His His His His Met Ser Gln Lys Asn Lys Asn Ser Ala
                       5                   10                  15
   Phe Met His Pro Val Asn Ile Ser Thr Asp Leu Ala Val Ile Val Gly
               20                  25                  30
   Lys Gly Pro Met Pro Arg Thr Glu Ile Val Lys Lys Val Trp Glu Tyr
           35                  40                  45
   Ile Lys Lys His Asn Cys Gln Asp Gln Lys Asn Lys Arg Asn Ile Leu
       50                  55                  60
   Pro Asp Ala Asn Leu Ala Lys Val Phe Gly Ser Ser Asp Pro Ile Asp
   65                  70                  75                  80
   Met Phe Gln Met Thr Lys Ala Leu Ser Lys His Ile Val Lys
                   85                  90
<210> 90
   <211> 474
   <212> PRT
   <213> Chlamydia
<400> 90
   Met Ala Ser His His His His His His Met Asn Glu Ala Phe Asp Cys
                       5                  10                  15
   Val Val Ile Gly Ala Gly Pro Gly Gly Tyr Val Ala Ala Ile Thr Ala
               20                  25                  30
   Ala Gln Ala Gly Leu Lys Thr Ala Leu Ile Glu Lys Arg Glu Ala Gly
           35                  40                  45
   Gly Thr Cys Leu Asn Arg Gly Cys Ile Pro Ser Lys Ala Leu Leu Ala
       50                  55                  60
   Gly Ala Glu Val Val Thr Gln Ile Arg His Ala Asp Gln Phe Gly Ile
   65                  70                  75                  80
   His Val Glu Gly Phe Ser Ile Asn Tyr Pro Ala Met Val Gln Arg Lys
                   85                  90                  95
   Asp Ser Val Val Arg Ser Ile Arg Asp Gly Leu Asn Gly Leu Ile Arg
               100                 105                 110
   Ser Asn Lys Ile Thr Val Phe Ser Gly Arg Gly Ser Leu Ile Ser Ser
           115                 120                 125
   Thr Glu Val Lys Ile Leu Gly Glu Asn Pro Ser Val Ile Lys Ala His
       130                 135                 140
   Ser Ile Ile Leu Ala Thr Gly Ser Glu Pro Arg Ala Phe Pro Gly Ile
   145                 150                 155                 160
   Pro Phe Ser Ala Glu Ser Pro Arg Ile Leu Cys Ser Thr Gly Val Leu
                   165                 170                 175
   Asn Leu Lys Glu Ile Pro Gln Lys Met Ala Ile Ile Gly Gly Gly Val
               180                 185                 190
   Ile Gly Cys Glu Phe Ala Ser Leu Phe His Thr Leu Gly Ser Glu Val
           195                 200                 205
   Ser Val Ile Glu Ala Ser Ser Gln Ile Leu Ala Leu Asn Asn Pro Asp
       210                 215                 220
   Ile Ser Lys Thr Met Phe Asp Lys Phe Thr Arg Gln Gly Leu Arg Phe
   225                 230                 235                 240
   Val Leu Glu Ala Ser Val Ser Asn Ile Glu Asp Ile Gly Asp Arg Val
                   245                 250                 255
   Arg Leu Thr Ile Asn Gly Asn Val Glu Glu Tyr Asp Tyr Val Leu Val
               260                 265                 270
   Ser Ile Gly Arg Arg Leu Asn Thr Glu Asn Ile Gly Leu Asp Lys Ala
           275                 280                 285
   Gly Val Ile Cys Asp Glu Arg Gly Val Ile Pro Thr Asp Ala Thr Met
       290                 295                 300
   Arg Thr Asn Val Pro Asn Ile Tyr Ala Ile Gly Asp Ile Thr Gly Lys
   305                 310                 315                 320
   Trp Gln Leu Ala His Val Ala Ser His Gln Gly Ile Ile Ala Ala Arg
                   325                 330                 335
   Asn Ile Gly Gly His Lys Glu Glu Ile Asp Tyr Ser Ala Val Pro Ser
               340                 345                 350
   Val Ile Phe Thr Phe Pro Glu Val Ala Ser Val Gly Leu Ser Pro Thr
           355                 360                 365
   Ala Ala Gln Gln Gln Lys Ile Pro Val Lys Val Thr Lys Phe Pro Phe
       370                 375                 380
   Arg Ala Ile Gly Lys Ala Val Ala Met Gly Glu Ala Asp Gly Phe Ala
   385                 390                 395                 400
   Ala Ile Ile Ser His Glu Thr Thr Gln Gln Ile Leu Gly Ala Tyr Val
                   405                 410                 415
   Ile Gly Pro His Ala Ser Ser Leu Ile Ser Glu Ile Thr Leu Ala Val
               420                 425                 430
   Arg Asn Glu Leu Thr Leu Pro Cys Ile Tyr Glu Thr Ile His Ala His
           435                 440                 445
   Pro Thr Leu Ala Glu Val Trp Ala Glu Ser Ala Leu Leu Ala Val Asp
   450 455 460
   Thr Pro Leu His Met Pro Pro Ala Lys Lys
   465 470
<210> 91
   <211> 129
   <212> PRT
   <213> Chlamydia
<400> 91
   Met His His His His His His Met Pro Arg Ile Ile Gly Ile Asp Ile
   5 10 15
   Pro Ala Lys Lys Lys Leu Lys Ile Ser Leu Thr Tyr Ile Tyr Gly Ile
   20 25 30
   Gly Ser Ala Arg Ser Asp Glu Ile Ile Lys Lys Leu Lys Leu Asp Pro
   35 40 45
   Glu Ala Arg Ala Ser Glu Leu Thr Glu Glu Glu Val Gly Arg Leu Asn
   50 55 60
   Ser Leu Leu Gln Ser Glu Tyr Thr Val Glu Gly Asp Leu Arg Arg Arg
   65 70 75 80
   Val Gln Ser Asp Ile Lys Arg Leu Ile Ala Ile His Ser Tyr Arg Gly
   85 90 95
   Gln Arg His Arg Leu Ser Leu Pro Val Arg Gly Gln Arg Thr Lys Thr
   100 105 110
   Asn Ser Arg Thr Arg Lys Gly Lys Arg Lys Thr Val Ala Gly Lys Lys
   115 120 125
   Lys
<210> 92
   <211> 202
   <212> PRT
   <213> Chlamydia
<400> 92
   Met His His His His His His Met Gly Ser Leu Val Gly Arg Gln Ala
   5 10 15
   Pro Asp Phe Ser Gly Lys Ala Val Val Cys Gly Glu Glu Lys Glu Ile
   20 25 30
   Ser Leu Ala Asp Phe Arg Gly Lys Tyr Val Val Leu Phe Phe Tyr Pro
   35 40 45
   Lys Asp Phe Thr Tyr Val Cys Pro Thr Glu Leu His Ala Phe Gln Asp
   50 55 60
   Arg Leu Val Asp Phe Glu Glu His Gly Ala Val Val Leu Gly Cys Ser
   65 70 75 80
   Val Asp Asp Ile Glu Thr His Ser Arg Trp Leu Thr Val Ala Arg Asp
   85 90 95
   Ala Gly Gly Ile Glu Gly Thr Glu Tyr Pro Leu Leu Ala Asp Pro Ser
   100 105 110
   Phe Lys Ile Ser Glu Ala Phe Gly Val Leu Asn Pro Glu Gly Ser Leu
   115 120 125
   Ala Leu Arg Ala Thr Phe Leu Ile Asp Lys His Gly Val Ile Arg His
   130 135 140
   Ala Val Ile Asn Asp Leu Pro Leu Gly Arg Ser Ile Asp Glu Glu Leu
   145 150 155 160
   Arg Ile Leu Asp Ser Leu Ile Phe Phe Glu Asn His Gly Met Val Cys
   165 170 175
   Pro Ala Asn Trp Arg Ser Gly Glu Arg Gly Met Val Pro Ser Glu Glu
   180 185 190
   Gly Leu Lys Glu Tyr Phe Gln Thr Met Asp
   195 200
<210> 93
   <211> 19
   <212> PRT
   <213> Artificial Sequence
<220>
   <223> made in a lab
<400> 93
   Glu Asn Ser Leu Gln Asp Pro Thr Asn Lys Arg Asn Ile Asn Pro Asp
   1 5 10 15
   Asp Lys Leu
<210> 94
   <211> 20
   <212> PRT
   <213> Artificial Sequence
<220>
   <223> Made in a lab
<400> 94
   Asp Pro Thr Asn Lys Arg Asn Ile Asn Pro Asp Asp Lys Leu Ala Lys
   1 5 10 15
   Val Phe Gly Thr
   20
<210> 95
   <211> 20
   <212> PRT
   <213> Artificial Sequence
<220>
   <223> Made in a lab
<400> 95
   Lys Arg Asn Ile Asn Pro Asp Asp Lys Leu Ala Lys Val Phe Gly Thr
   1 5 10 15
   Glu Lys Pro Ile
   20
<210> 96
   <211> 20
   <212> PRT
   <213> Artificial Sequence
<220>
   <223> Made in a lab
<400> 96
   Asp Asp Lys Leu Ala Lys Val Phe Gly Thr Glu Lys Pro Ile Asp Met
   1 5 10 15
   Phe Gln Met Thr
   20
<210> 97
   <211> 20
   <212> PRT
   <213> Artificial Sequence
<220>
   <223> Made in a lab
<400> 97
   Lys Val Phe Gly Thr Glu Lys Pro Ile Asp Met Phe Gln Met Thr Lys
    1               5                   10                  15
   Met Val Ser Gln
               20
<210> 98
   <211> 20
   <212> PRT
   <213> Artificial Sequence
<220>
   <223> Made in a lab

<400> 98
   Asn Lys Arg Asn Ile Asn Pro Asp Asp Lys Leu Ala Lys Val Phe Gly
    1               5                   10                  15
   Thr Glu Lys Pro
               20
<210> 99
   <211> 16
   <212> PRT
   <213> Artificial Sequence
<220>
   <223> Made in a lab
<400> 99
   Asn Lys Arg Asn Ile Leu Pro Asp Ala Asn Leu Ala Lys Val Phe Gly
    1               5                   10                  15
<210> 100
   <211> 15
   <212> PRT
   <213> Artificial Sequence
<220>
   <223> Made in a lab
<400> 100
   Lys Met Trp Asp Tyr Ile Lys Glu Asn Ser Leu Gln Asp Pro Thr
    1               5                   10                  15
<210> 101
   <211> 20
   <212> PRT
   <213> Artificial Sequence
<220>
   <223> Made in a lab
<400> 101
   Thr Glu Ile Val Lys Lys Val Trp Glu Tyr Ile Lys Lys His Asn Cys
   1               5                   10                  15
   Gln Asp Gln Lys
               20
<210> 102
   <211> 20
   <212> PRT
   <213> Artificial Sequence
<220>
   <223> Made in a lab
<400> 102
   Lys Val Trp Glu Tyr Ile Lys Lys His Asn Cys Gln Asp Gln Lys Asn
   1               5                   10                  15
   Lys Arg Asn Ile
               20
<210> 103
   <211> 15
   <212> PRT
   <213> Artificial Sequence
<220>
   <223> Made in a lab
<400> 103
   Lys Val Trp Glu Tyr Ile Lys Lys His Asn Cys Gln Asp Gln Lys
   1               5                   10                  15
<210> 104
   <211> 20
   <212> PRT
   <213> Artificial Sequence
<220>
   <223> Made in a lab
<400> 104
   Ala Glu Leu Thr Glu Glu Glu Val Gly Arg Leu Asn Ala Leu Leu Gln
   1               5                   10                  15
   Ser Asp Tyr Val
               20
<210> 105
   <211> 21
   <212> PRT
   <213> Artificial Sequence
<220>
   <223> Made in a lab
<400> 105
   Leu Gln Ser Asp Tyr Val Val Glu Gly Asp Leu Arg Arg Arg Val Gln
   1               5                   10                  15
   Ser Asp Ile Lys Arg
               20
<210> 106
   <211> 20
   <212> PRT
   <213> Artificial Sequence
<220>
   <223> Made in a lab

<400> 106
   Met Pro Arg Ile Ile Gly Ile Asp Ile Pro Ala Lys Lys Lys Leu Lys
   1               5                   10                  15
   Ile Ser Leu Thr
   20
<210> 107
   <211> 20
   <212> PRT
   <213> Artificial Sequence
<220>
   <223> Made in a lab
<400> 107
   Ala Glu Leu Thr Glu Glu Glu Val Gly Arg Leu Asn Ala Leu Leu Gln
   1 5 10 15
   Ser Asp Tyr Val
   20
<210> 108
   <211> 20
   <212> PRT
   <213> Artificial Sequence
<220>
   <223> Made in a lab
<400> 108
   Leu Asn Ala Leu Leu Gln Ser Asp Tyr Val Val Glu Gly Asp Leu Arg
   1 5 10 15
   Arg Arg Val Gln
   20
<210> 109
   <211> 20
   <212> PRT
   <213> Artificial Sequence
<220>
   <223> Made in a lab
<400> 109
   Leu Asn Ser Leu Leu Gln Ser Glu Tyr Thr Val Glu Gly Asp Leu Arg
   1 5 10 15
   Arg Arg Val Gln
   20
<210> 110
   <211> 1461
   <212> DNA
   <213> Chlamydia
<400> 110
   ctatctatga agttatgaat atggatctag aaacacgaag atcttttgcg gtacagcaag 60
   ggcactatca ggacccaaga gcttcagatt atgacctccc acgtgctagc gactatgatt 120
   tgcctagaag cccatatcct actccacctt tgccttctag atatcagcta cagaatatgg 180
   atgtagaagc agggttccgt gaggcagttt atgcttcttt tgtagcagga atgtacaatt 240
   atgtagtgac acagccgcaa gagcgtattc ccaatagtca gcaggtggaa gggattctgc 300
   gtgatatgct taccaacggg tcacagacat ttagcaacct gatgcagcgt tgggatagag 360
   aagtcgatag ggaataaact ggtatctacc ataggtttgt atcaaaaaac taagcccacc 420
   aagaagaaat tctctttggt gggcttcttt ttttattcaa aaaagaaagc cctcttcaag 480
   attatctcgt gccgctcgtg ccgaattcgg cacgagcggc acgaggagct gtaagtaagt 540
   attgccaaga gttggaagaa aaaatattag atttgtgtaa gcgtcatgcc gcaacaattt 600
   gctccattga ggaggatgct aaacaagaaa ttcgtcatca gacagaaagg tttaaacagc 660
   ggttgcaaca aaatcagaac acttgcagtc aattaacagc agagttgtgt aaattgagat 720
   ctgagaataa ggcattatcg gagcggctgc aggtgcaggc atcccgtcgt aaaaaataat 780
   taaagactcc tcagatattg catctgagag ttaggggttc cttttgctta cggcgcttta 840
   gttctgcatg ttgcggattt atagtgattt gcgagtaaag cgccgttctg atacagtttt 900
   tccgctttaa aaataaaaag gtggaaaaat gagtactact attagcggag acgcttcttc 960
   tttaccgttg ccaacagctt cctgcgtaga gacaaaatct acttcgtctt caacaaaagg 1020
   gaatacttgt tccaaaattt tggatatagc tttagctatc gtaggcgctt tagttgttgt 1080
   cgctggggta ttagctttgg ttttgtgcgc tagcaatgtc atatttactg taataggtat 1140
   tcctgcatta attattggat ctgcttgtgt gggtgcggga atatctcgtc ttatgtatcg 1200
   atcctcttat gctagcttag aagcaaaaaa tgttttggct gagcaacgtt tgcgtaatct 1260
   ttcagaagag aaggacgctt tggcctccgt ctctttcatt aataagatgt ttctgcgagg 1320
   tcttacggac gatctccaag ctttggaagc taaggtaatg gaatttgaga ttgattgttt 1380
   ggacagatta gagaaaaatg agcaagcttt attgtccgat gtgcgcttag ttttatctag 1440
   ctacacaaga tggttggata g                                           1461
<210> 111
   <211> 267
   <212> DNA
   <213> Chlamydia
<400> 111
   gtcctcttct tattatagca gaagacattg aaggcgaagc tttagctact ttggtcgtga 60
   acagaattcg tggaggattc cgggtttgcg cagttaaagc tccaggcttt ggagatagaa 120
   gaaaagctat gttggaagac atcgctatct taactggcgg tcaactcatt agcgaagagt 180
   tgggcatgaa attagaaaac gctaacttag ctatgttagg taaagctaaa aaagttatcg 240
   tttctaaaga agacacgacc atcgtcg                                     267
<210> 112
   <211> 698
   <212> DNA
   <213> Chlamydia
<400> 112
   tgataagcaa gcaaccgctc aactagcagc tctaactatt aaaaaaatcc tctgttttga 60
   tgaaaattcc tacgagaagg agctggcatg cttagaaaag aaacgcagta gcgtacaaaa 120
   agatctgagc caactgaaaa aatacacagt tctctacatc aagaagctgc tcgaaaccta 180
   cagacaactc gggcatcgaa agacaaaaat tgcaaaattt gatgacctac ctaccgagag 240
   agtctccgct cataagaaag caaaagaact cgctgcgctc gatcaagaag agaacttcta 300
   aaacgtgact cggcccttga gatccttaaa ctctcgggcc aaaaagacta cagtcttctc 360
   gagaagaaaa acggtgttag aaaatacgcg cgctaagact ttctctaaca atgactcaaa 420
   aagctgtaaa cgtatacgtt taccgctctt ccataatttc taggctgact ttcacattat 480
   ctcgacttgc tacggaaacc aataaagtac ggatagcctt aatagtgcgt ccttctttac 540
   cgataatttt accgatatct cccttagcaa cagtcaattc gtagataatc gtattggttc 600
   cctgcacctc tttcagatgc acttcctctg gcttatcaac aagatttttt acaatgtacg 660
   ctaaaaactc tttcatgcga agcaaatcct acacaagc                         698
<210> 113
   <211> 1142
   <212> DNA
   <213> Chlamydia
<400> 113
   ctcttcaaag attgtgagtt tatgtgaagg cgctgtcgct gatgcaagaa tgtgcaaagc 60
   agagttgata aaaaaagaag cggatgctta tttgttttgt gagaaaagcg ggatatatct 120
   aacgaaaaaa gaaggtattt tgattccttc tgcagggatt gatgaatcga atacggacca 180
   gccttttgtt ttatatccta aagatatttt gggatcgtgt aatcgcatcg gagaatggtt 240
   aagaaattat tttcgagtga aagagctagg cgtaatcatt acagatagcc atactactcc 300
   aatgcggcgt ggagtactgg gtatcgggct gtgttggtat ggattttctc cattacacaa 360
   ctatatagga tcgctagatt gtttcggtcg tcccttacag atgacgcaaa gtaatcttgt 420
   agatgcctta gcagttgcgg ctgttgtttg tatgggagag gggaatgagc aaacaccgtt 480
   agcggtgata gagcaggcac ctaatatggt ctaccattca tatcctactt ctcgagaaga 540
   gtattgttct ttgcgcatag atgaaacaga ggacttatac ggaccttttt tgcaagcggt 600
   tacgtggagt caagaaaaga aatgatggag gtgtttatga attttttaga tcagttagat 660
   ttaattattc aaaataagca tatgctagaa cacacgtttt atgtgaaatg gtcgaagggg 720
   gagcttacta aagagcaatt acaggcgtat gccaaagact attatttaca tatcaaagcc 780
   tttcctaaat atttatctgc gattcatagt cgttgcgatg atttagaggc gcgtaagtta 840
   ttgttagata acttgatgga tgaagagaac ggttacccta atcatattga tttgtggaag 900
   cagtttgtgt ttgctctagg agttactcca gaagagttag aggctcatga gcctagtgaa 960
   gcagcaaaag cgaaagtagc tactttcatg cggtggtgta caggagattc tttagctgca 1020
   ggagtggctg ctttgtattc ttatgagagt caaattccac gtatcgctag agagaaaatt 1080
   cgtggattga ctgagtactt tggattttcc aatcctgaag actatgcata tttcacagaa 1140
   ca                                                                1142
<210> 114
   <211> 976
   <212> DNA
   <213> Chlamydia
<400> 114
   aggtggatgg ggcgcctgtc caagatgtgc tcgctactct atatggaagc aatcacaaag 60
   ggactgcagc tgaagagtcg gctgctttaa gaacactatt ctctcgcatg gcctctttag 120
   ggcacaaagt accttctggg cgcactactt taaagattcg tcgtcctttt ggtactacga 180
   gagaagttcg tgtgaaatgg cgttatgttc ctgaaggtgt aggagatttg gctaccatag 240
   ctccttctat cagggctcca cagttacaga aatcgatgag aagctttttc cctaagaaag 300
   atgatgcgtt tcatcggtct agttcgctat tctactctcc aatggttccg catttttggg 360
   cagagcttcg caatcattat gcaacgagtg gtttgaaaag cgggtacaat attgggagta 420
   ccgatgggtt tctccctgtc attgggcctg ttatatggga gtcggagggt cttttccgcg 480
   cttatatttc ttcggtgact gatggggatg gtaagagcca taaagtagga tttctaagaa 540
   ttcctacata tagttggcag gacatggaag attttgatcc ttcaggaccg cctccttggg 600
   aagaatttgc taagattatt caagtatttt cttctaatac agaagctttg attatcgacc 660
   aaacgaacaa cccaggtggt agtgtccttt atctttatgc actgctttcc atgttgacag 720
   accgtccttt agaacttcct aaacatagaa tgattctgac tcaggatgaa gtggttgatg 780
   ctttagattg gttaaccctg ttggaaaacg tagacacaaa cgtggagtct cgccttgctc 840
   tgggagacaa catggaagga tatactgtgg atctacaggt tgccgagtat ttaaaaagct 900
   ttggacgtca agtattgaat tgttggagta aaggggatat cgagttatca acacctattc 960
   ctctttttgg ttttga                                                 976
<210> 115
   <211> 995
   <212> DNA
   <213> Chlamydia

<400> 115
   ttatcctaga aatttggtgt tcaatatgag cgaaaaaaga aagtctaaca aaattattgg 60
   tatcgaccta gggacgacca actcttgcgt ctctgttatg gaaggtggcc aacctaaagt 120
   tattgcctct tctgaaggaa ctcgtactac tccttctatc gttgctttta aaggtggcga 180
   aactcttgtt ggaattcctg caaaacgtca ggcagtaacc aatcctgaaa aaacattggc 240
   ttctactaag cgattcatcg gtagaaaatt ctctgaagtc gaatctgaaa ttaaaacagt 300
   cccctacaaa gttgctccta actcgaaagg agatgcggtc tttgatgtgg aacaaaaact 360
   gtacactcca gaagaaatcg gcgctcagat cctcatgaag atgaaggaaa ctgctgaggc 420
   ttatctcgga gaaacagtaa cggaagcagt cattaccgta ccagcttact ttaacgattc 480
   tcaaagagct tctacaaaag atgctggacg tatcgcagga ttagatgtta aacgcattat 540
   tcctgaacca acagcggccg ctcttgctta tggtattgat aaggaaggag ataaaaaaat 600
   cgccgtcttc gacttaggag gaggaacttt cgatatttct atcttggaaa tcggtgacgg 660
   agtttttgaa gttctctcaa ccaacgggga tactcacttg ggaggagacg acttcgacgg 720
   agtcatcatc aactggatgc ttgatgaatt caaaaaacaa gaaggcattg atctaagcaa 780
   agataacatg gctttgcaaa gattgaaaga tgctgctgaa aaagcaaaaa tagaattgtc 840
   tggtgtatcg tctactgaaa tcaatcagcc attcatcact atcgacgcta atggacctaa 900
   acatttggct ttaactctaa ctcgcgctca attcgaacac ctagcttcct ctctcattga 960
   gcgaaccaaa caaccttgtg ctcaggcttt aaaag 995
<210> 116
   <211> 437
   <212> DNA
   <213> Chlamydia
<400> 116
   gtcacagcta aaggcggtgg gctttatact gataagaatc tttcgattac taacatcaca 60
   ggaattatcg aaattgcaaa taacaaagcg acagatgttg gaggtggtgc ttacgtaaaa 120
   ggaaccctta cttgtaaaaa ctctcaccgt ctacaatttt tgaaaaactc ttccgataaa 180
   caaggtggag gaatctacgg agaagacaac atcaccctat ctaatttgac agggaagact 240
   ctattccaag agaatactgc caaaaaagag ggcggtggac tcttcataaa aggtacagat 300
   aaagctctta caatgacagg actggatagt ttctgtttaa ttaataacac atcagaaaaa 360
   catggtggtg gagcctttgt taccaaagaa atctctcaga cttacacctc tgatgtggaa 420
   acaattccag gaatcac 437
<210> 117
   <211> 446
   <212> DNA
   <213> Chlamydia
<400> 117
   aagtttacct agaccaaact gaagatgacg aaggaaaagt tgttttatcc agagaaaaag 60
   caacaagaca acgacaatgg gaatacattc ttgctcactg cgaggaaggt tctattgtta 120
   agggacaaat tacccgaaaa gttaagggtg gtttgatcgt agatattggt atggaagcct 180
   tccttccagg atcccaaata gacaataaga agatcaagaa cttagatgat tacgtaggca 240
   aggtttgtga gttcaaaatt ctcaaaatca acgtggatcg tcggaacgtt gttgtatcta 300
   gaagagaact tctcgaagct gaacgcattt ctaagaaagc agagttgatc gagcaaatca 360
   ctatcggtga acgtcgcaaa ggtatcgtta agaatatcac agatttcgga gtattcttgg 420
   atcttgatgg cattgacggc ctactc 446
<210> 118
   <211> 951
   <212> DNA
   <213> Chlamydia
<400> 118
   agtattgcga aatattactg tgagaagcaa tgctgagagc ggttctagta aaagtgaggg 60
   gagagctgtc agaagggatc gctcaggaag cgagacaacg tgtggctgat ttattaggaa 120
   gattccctct ttatcctgaa atcgatctgg aaacgctagt ttagtgggag actctatgcc 180
   tgaaggggaa atgatgcata agttgcaaga tgtcatagat agaaagttgt tggattctcg 240
   tcgtattttc ttctccgaac ctgtaacgga gaaaagtgct gcagaagcca tcaaaaagct 300
   ttggtatttg gaactcacca atcctgggca gccaattgta tttgtcatta atagccctgg 360
   agggtctgtt gatgctgggt ttgctgtttg ggaccaaatt aaaatgatct cttctccttt 420
   gactacagtt gttacaggtt tagcagcatc tatgggatct gtattgagtt tgtgtgctgt 480
   tccaggaaga cgttttgcta cgcctcatgc gcgcattatg attcaccagc cttctattgg 540
   aggaaccatt actggtcaag ccacggactt ggatattcat gctcgtgaaa ttttaaaaac 600
   aaaagcacgc attattgatg tgtatgtcga ggcaactgga caatctccag aggtgataga 660
   gaaagctatc gatcgagata tgtggatgag tgcaaatgaa gcaatggagt ttggactgtt 720
   agatgggatt ctcttctctt ttaacgactt gtagatatct tttatattct ggagcaggaa 780
   acagtttcat tttgggagaa tcgatgcctt ctcttgagga tgttctgttt ttatgccagg 840
   aagagatggt tgatgggttt ttatgtgtag agtcttctga aatagcagat gctaaactca 900
   ctgtttttaa tagtgatgga tctatcgcgt ctatgtgcgg gaatgggttg c 951
<210> 119
   <211> 953
   <212> DNA
   <213> Chlamydia .
<400> 119
   atatcaaagt tgggcaaatg acagagccgc tcaaggacca gcaaataatc cttgggacaa 60
   catcaacacc tgtcgcagcc aaaatgacag cttctgatgg aatatcttta acagtctcca 120
   ataatccatc aaccaatgct tctattacaa ttggtttgga tgcggaaaaa gcttaccagc 180
   ttattctaga aaagttggga gatcaaattc ttggtggaat tgctgatact attgttgata 240
   gtacagtcca agatatttta gacaaaatca caacagaccc ttctctaggt ttgttgaaag 300
   cttttaacaa ctttccaatc actaataaaa ttcaatgcaa cgggttattc actcccagga 360
   acattgaaac tttattagga ggaactgaaa taggaaaatt cacagtcaca cccaaaagct 420
   ctgggagcat gttcttagtc tcagcagata ttattgcatc aagaatggaa ggcggcgttg 480
   ttctagcttt ggtacgagaa ggtgattcta agccctacgc gattagttat ggatactcat 540
   caggcgttcc taatttatgt agtctaagaa ccagaattat taatacagga ttgactccga 600
   caacgtattc attacgtgta ggcggtttag aaagcggtgt ggtatgggtt aatgcccttt 660
   ctaatggcaa tgatatttta ggaataacaa atacttctaa tgtatctttt ttggaggtaa 720
   tacctcaaac aaacgcttaa acaattttta ttggattttt cttataggtt ttatatttag 780
   agaaaaaagt tcgaattacg gggtttgtta tgcaaaataa aagcaaagtg agggacgatt 840
   ttattaaaat tgttaaagat tcctggtatc ggtctgcgat tccgactcgt ccaacatcaa 900
   tacaacctat taatttcccc tcgtcaaaaa taaggttatc aagtgagaaa tca       953
<210> 120
   <211> 897
   <212> DNA
   <213> Chlamydia
<400> 120
   atggcttcta tatgcggacg tttagggtct ggtacaggga atgctctaaa agcttttttt     60
   acacagccca gcaataaaat ggcaagggta gtaaataaga cgaagggaat ggataagact    120
   gttaaggtcg ccaagtctgc tgccgaattg accgcaaata ttttggaaca agctggaggc    180
   gcgggctctt ccgcacacat tacagcttcc caagtgtcca aaggattagg ggatgcgaga    240
   actgttctcg ctttagggaa tgcctttaac ggagcgttgc caggaacagt tcaaagtgcg    300
   caaagcttct tctcttacat gaaagctgct agtcagaaac cgcaagaagg ggatgagggg    360
   ctcgtagcag atctttgtgt gtctcataag cgcanagcgg ctgcggctgt ctgtagcttc    420
   atcggaggaa ttacctacct cgcgacattc ggagctatcc gtccgattct gtttgtcaac    480
   aaaatgctgg cgcaaccgtt tctttcttcc caaattaaag caaatatggg atcttctgtt    540
   agctatatta tggcggctaa ccatgcagcg tttgtggtgg gttctggact cgctatcagt    600
   gcggaaagag cagattgcga agcccgctgc gctcgtattg cgagagaaga gtcgtcactc    660
   gaattgtcgg gagaggaaaa tgcttgcgag aggagagtcg ctggagagaa agccaagacg    720
   ttcacgcgca tcaagtatgc actcctcact atgctcgaga agtttttgga atgcgttgcc    780
   gacgttttca aattggtgcc gttgcctatt acaatgggta ttcgtgcaat tgtggctgcg    840
   ggatgtacgt tcacttctgc agttattgga ttgtggactt tctgcgccag agcataa       897
<210> 121
   <211> 298
   <212> PRT
   <213> Chlamydia
<400> 121
   Met Ala Ser Ile Cys Gly Arg Leu Gly Ser Gly Thr Gly Asn Ala Leu
    1               5                  10                  15
   Lys Ala Phe Phe Thr Gln Pro Ser Asn Lys Met Ala Arg Val Val Asn
               20                  25                  30
   Lys Thr Lys Gly Met Asp Lys Thr Val Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Leu Ala Leu Gly Asn Ala Phe Asn Gly Ala Leu Pro Gly Thr
                   85                  90                  95
   Val Gln Ser Ala Gln Ser Phe Phe Ser Tyr Met Lys Ala Ala Ser Gln
               100                 105                 110
   Lys Pro Gln Glu Gly Asp Glu Gly Leu Val Ala Asp Leu Cys Val Ser
           115                 120                 125
   His Lys Arg Arg Ala Ala Ala Ala Val Cys Ser Phe Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Gln Pro Phe Leu Ser Ser Gln Ile Lys Ala Asn Met
                   165                 170                 175
   Gly Ser Ser Val Ser Tyr Ile Met Ala Ala Asn His Ala Ala Phe Val
               180                 185                 190
   Val Gly Ser Gly Leu Ala Ile Ser Ala Glu Arg Ala Asp Cys Glu Ala
           195                 200                 205
   Arg Cys Ala Arg Ile Ala Arg Glu Glu Ser Ser Leu Glu Leu Ser Gly
       210                 215                 220
   Glu Glu Asn Ala Cys Glu Arg Arg Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Val
           275                 280                 285
   Ile Gly Leu Trp Thr Phe Cys Ala Arg Ala
       290                 295
<210> 122
   <211> 897
   <212> DNA
   <213> Chlamydia
<400> 122
   atggcttcta tatgcggacg tttagggtct ggtacaggga atgctctaaa agcttttttt    60
   acacagccca gcaataaaat ggcaagggta gtaaataaga cgaagggaat ggataagact   120
   gttaaggtcg ccaagtctgc tgccgaattg accgcaaata ttttggaaca agctggaggc   180
   gcgggctctt ccgcacacat tacagcttcc caagtgtcca aaggattagg ggatacgaga   240
   actgttgtcg ctttagggaa tgcctttaac ggagcgttgc caggaacagt tcaaagtgcg   300
   caaagcttct tctctcacat gaaagctgct agtcagaaaa cgcaagaagg ggatgagggg   360
   ctcacagcag atctttgtgt gtctcataag cgcagagcgg ctgcggctgt ctgtggcttc   420
   atcggaggaa ttacctacct cgcgacattc ggagttatcc gtccgattct gtttgtcaac   480
   aaaatgctgg tgaacccgtt tctttcttcc caaactaaag caaatatggg atcttctgtt   540
   agctatatta tggcggctaa ccatgcagcg tctgtggtgg gtgctggact cgctatcagt   600
   gcggaaagag cagattgcga agcccgctgc gctcgtattg cgagagaaga gtcgttactc   660
   gaagtgtcgg gagaggaaaa tgcttgcgag aagagagtcg ctggagagaa agccaagacg   720
   ttcacgcgca tcaagtatgc actcctcact atgctcgaga agtttttgga atgcgttgcc   780
   gacgttttca aattggtgcc gctgcctatt acaatgggta ttcgtgcgat tgtggctgct   840
   ggatgtacgt tcacttctgc aattattgga ttgtgcactt tctgcgccag agcataa      897
<210> 123
   <211> 298
   <212> PRT
   <213> Chlamydia
<400> 123
   Met Ala Ser Ile Cys Gly Arg Leu Gly Ser Gly Thr Gly Asn Ala Leu
   1               5                   10                  15
   Lys Ala Phe Phe Thr Gln Pro Ser Asn Lys Met Ala Arg Val Val Asn
               20                  25                  30
   Lys Thr Lys Gly Met Asp Lys Thr Val Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Thr Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Gly Phe Ile Gly Gly Ile
       130                 135                 140
   Thr Tyr Leu Ala Thr Phe Gly Val Ile Arg Pro Ile Leu Phe Val Asn
   145                 150                 155                 160
   Lys Met Leu Val Asn Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Glu Arg Ala Asp Cys Glu Ala
           195                 200                 205
   Arg Cys Ala Arg Ile Ala Arg Glu Glu Ser Leu Leu Glu Val Ser Gly
       210                 215                 220
   Glu Glu Asn Ala Cys Glu Lys Arg Val Ala Gly Glu Lys Ala Lys Thr
   225                 230                 235                 240
   Phe Thr Arg Ile Lys Tyr Ala Leu Leu Thr Met Leu Glu Lys Phe Leu
                       245                 250                 255
   Glu Cys Val Ala Asp Val Phe Lys Leu Val Pro Leu Pro Ile Thr Met
                   260                 265                 270
   Gly Ile Arg Ala Ile Val Ala Ala Gly Cys Thr Phe Thr Ser Ala Ile
               275                 280                 285
   Ile Gly Leu Cys Thr Phe Cys Ala Arg Ala
       290                 295
<210> 124
   <211> 897
   <212> DNA
   <213> Chlamydia
<400> 124
   atggcttcta tatgcggacg tttagggtct ggtacaggga atgctctaaa agcttttttt    60
   acacagccca acaataaaat ggcaagggta gtaaataaga cgaagggaat ggataagact   120
   attaaggttg ccaagtctgc tgccgaattg accgcaaata ttttggaaca agctggaggc   180
   gcgggctctt ccgcacacat tacagcttcc caagtgtcca aaggattagg ggatgcgaga   240
   actgttgtcg ctttagggaa tgcctttaac ggagcgttgc caggaacagt tcaaagtgcg   300
   caaagcttct tctctcacat gaaagctgct agtcagaaaa cgcaagaagg ggatgagggg   360
   ctcacagcag atctttgtgt gtctcataag cgcagagcgg ctgcggctgt ctgtagcatc   420
   atcggaggaa ttacctacct cgcgacattc ggagctatcc gtccgattct gtttgtcaac   480
   aaaatgctgg caaaaccgtt tctttcttcc caaactaaag caaatatggg atcttctgtt   540
   agctatatta tggcggctaa ccatgcagcg tctgtggtgg gtgctggact cgctatcagt   600
   gcggaaagag cagattgcga agcccgctgc gctcgtattg cgagagaaga gtcgttactc   660
   gaagtgccgg gagaggaaaa tgcttgcgag aagaaagtcg ctggagagaa agccaagacg   720
   ttcacgcgca tcaagtatgc actcctcact atgctcgaga agtttttgga atgcgttgcc   780
   gacgttttca aattggtgcc gctgcctatt acaatgggta ttcgtgcgat tgtggctgct   840
   ggatgtacgt tcacttctgc aattattgga ttgtgcactt tctgcgccag agcataa      897
<210> 125
   <211> 298
   <212> PRT
   <213> Chlamydia

<400> 125
   Met Ala Ser Ile Cys Gly Arg Leu Gly Ser Gly Thr Gly Asn Ala Leu
    1               5                  10                  15
   Lys Ala Phe Phe Thr Gln Pro Asn Asn Lys Met Ala Arg Val Val Asn
               20                  25                  30
   Lys Thr Lys Gly Met Asp Lys Thr Ile Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Ser Ile Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Lys Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Glu Arg Ala Asp Cys Glu Ala
           195                 200                 205
   Arg Cys Ala Arg Ile Ala Arg Glu Glu Ser Leu Leu Glu Val Pro Gly
       210                 215                 220
   Glu Glu Asn Ala Cys Glu Lys Lys Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 126
   <211> 897
   <212> DNA
   <213> Chlamydia
<400> 126
   atggcttcta tatgcggacg tttagggtct ggtacaggga atgctctaaa agcttttttt      60
   acacagccca acaataaaat ggcaagggta gtaaataaga cgaagggaat ggataagact     120
   attaaggttg ccaagtctgc tgccgaattg accgcaaata ttttggaaca agctggaggc     180
   gcgggctctt ccgcacacat tacagcttcc caagtgtcca aaggattagg ggatgcgaga     240
   actgttgtcg ctttagggaa tgcctttaac ggagcgttgc caggaacagt tcaaagtgcg     300
   caaagcttct tctctcacat gaaagctgct agtcagaaaa cgcaagaagg ggatgagggg     360
   ctcacagcag atctttgtgt gtctcataag cgcagagcgg ctgcggctgt ctgtagcatc     420
   atcggaggaa ttacctacct cgcgacattc ggagctatcc gtccgattct gtttgtcaac     480
   aaaatgctgg caaaaccgtt tctttcttcc caaactaaag caaatatggg atcttctgtt     540
   agctatatta tggcggctaa ccatgcagcg tctgtggtgg gtgctggact cgctatcagt     600
   gcggaaagag cagattgcga agcccgctgc gctcgtattg cgagagaaga gtcgttactc     660
   gaagtgccgg gagaggaaaa tgcttgcgag aagaaagtcg ctggagagaa agccaagacg     720
   ttcacgcgca tcaagtatgc actcctcact atgctcgaga agtttttgga atgcgttgcc     780
   gacgttttca aattggtgcc gctgcctatt acaatgggta ttcgtgcgat tgtggctgct     840
   ggatgtacgt tcacttctgc aattattgga ttgtgcactt tctgcgccag agcataa        897
<210> 127
   <211> 298
   <212> PRT
   <213> Chlamydia
<400> 127
   Met Ala Ser Ile Cys Gly Arg Leu Gly Ser Gly Thr Gly Asn Ala Leu
    1               5                  10                  15
   Lys Ala Phe Phe Thr Gln Pro Asn Asn Lys Met Ala Arg Val Val Asn
               20                  25                  30
   Lys Thr Lys Gly Met Asp Lys Thr Ile Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Ser Ile Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Lys Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Glu Arg Ala Asp Cys Glu Ala
           195                 200                 205
   Arg Cys Ala Arg Ile Ala Arg Glu Glu Ser Leu Leu Glu Val Pro Gly
       210                 215                 220
   Glu Glu Asn Ala Cys Glu Lys Lys Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 128
   <211> 897
   <212> DNA
   <213> Chlamydia
<400> 128
   atggcttcta tatgtggacg tttagggtct ggtacaggga atgctctaaa agcttttttt      60
   acacagccca gcaataaaat ggcaagggta gtaaataaga cgaagggaat ggataagact     120
   gttaaggtcg ccaagtctgc tgccgaattg accgcaaata ttttggaaca agctggaggc     180
   gcgggctctt ccgcacacat tacagcttcc caagtgtcca aaggattagg ggatacgaga     240
   actgttgtcg ctttagggaa tgcctttaac ggagcgttgc caggaacagt tcaaagtgcg     300
   caaagcttct tctctcacat gaaagctgct agtcagaaaa cgcaagaagg ggatgagggg     360
   ctcacagcag atctttgtgt gtctcataag cgcagagcgg ctgcggctgt ctgtggcttc     420
   atcggaggaa ttacctacct cgcgacattc ggagttatcc gtccgattct gtttgtcaac     480
   aaaatgctgg tgaacccgtt tctttcttcc caaactaaag caaatatggg atcttctgtt     540
   agctatatta tggcggctaa ccatgcagcg tctgtggtgg gtgctggact cgctatcagt     600
   gcggaaagag cagattgcga agcccgctgc gctcgtattg cgagagaaga gtcgttactc     660
   gaagtgtcgg gagaggaaaa tgcttgcgag aagagagtcg ctggagagaa agccaagacg     720
   ttcacgcgca tcaagtatgc actcctcact atgctcgaga agtttttgga atgcgttgcc     780
   gacgttttca aattggtgcc gctgcctatt acaatgggta ttcgtgcgat tgtggctgct     840
   ggatgtacgt tcacttctgc aattattgga ttgtgcactt tctgcgccag agcataa        897
<210> 129
   <211> 298
   <212> PRT
   <213> Chlamydia
<400> 129
   Met Ala Ser Ile Cys Gly Arg Leu Gly Ser Gly Thr Gly Asn Ala Leu
   1               5                   10                  15
   Lys Ala Phe Phe Thr Gln Pro Ser Asn Lys Met Ala Arg Val Val Asn
               20                  25                  30
   Lys Thr Lys Gly Met Asp Lys Thr Val Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Thr Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Gly Phe Ile Gly Gly Ile
       130                 135                 140
   Thr Tyr Leu Ala Thr Phe Gly Val Ile Arg Pro Ile Leu Phe Val Asn
   145                 150                 155                 160
   Lys Met Leu Val Asn Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Glu Arg Ala Asp Cys Glu Ala
           195                 200                 205
   Arg Cys Ala Arg Ile Ala Arg Glu Glu Ser Leu Leu Glu Val Ser Gly
       210                 215                 220
   Glu Glu Asn Ala Cys Glu Lys Arg Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 130
   <211> 897
   <212> DNA
   <213> Chlamydia
<400> 130
   atggctgcta tatgtggacg tttagggtct ggtacaggga atgctctaaa agcttttttt      60
   acacagccca gcaataaaat ggcaagggta gtaaataaga cgaagggaat ggataagact     120
   gttaaggtcg ccaagtctgc tgccgaattg accgcaaata ttttggaaca agctggaggc     180
   gcgggctctt ccgcacacat tacagcttcc caagtgtcca aaggattagg ggatgcgaga     240
   actgttctcg ctttagggaa tgcctttaac ggagcgttgc caggaacagt tcaaagtgcg     300
   caaagcttct tctcttacat gaaagctgct agtcagaaac cgcaagaagg ggatgagggg     360
   ctcgtagcag atctttgtgt gtctcataag cgcagagcgg ctgcggctgt ctgtagcttc     420
   atcggaggaa ttacctacct cgcgacattc ggagctatcc gtccgattct gtttgtcaac     480
   aaaatgctgg cgcaaccgtt tctttcttcc caaactaaag caaatatggg atcttctgtt     540
   agctatatta tggcggctaa ccatgcagcg tttgtggtgg gttctggact cgctatcagt     600
   gcggaaagag cagattgcga agcccgctgc gctcgtattg cgagagaaga gtcgtcactc     660
   gaattgtcgg gagaggaaaa tgcttgcgag aggggagtcg ctggagagaa agccaagacg     720
   ttcacgcgca tcaagtatgc actcctcact atgctcgaga agtttttgga atgcgttgcc     780
   gacgttttca aattggtgcc gttgcctatt acaatgggta ttcgtgcaat tgtggctgcg     840
   ggatgtacgt tcacttctgc agttattgga ttgtggactt tctgcaacag agtataa        897
<210> 131
   <211> 298
   <212> PRT
   <213> Chlamydia
<400> 131
   Met Ala Ala Ile Cys Gly Arg Leu Gly Ser Gly Thr Gly Asn Ala Leu
   1               5                   10                  15
   Lys Ala Phe Phe Thr Gln Pro Ser Asn Lys Met Ala Arg Val Val Asn
               20                  25                  30
   Lys Thr Lys Gly Met Asp Lys Thr Val Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Leu Ala Leu Gly Asn Ala Phe Asn Gly Ala Leu Pro Gly Thr
                   85                  90                  95
   Val Gln Ser Ala Gln Ser Phe Phe Ser Tyr Met Lys Ala Ala Ser Gln
               100                 105                 110
   Lys Pro Gln Glu Gly Asp Glu Gly Leu Val Ala Asp Leu Cys Val Ser
           115                 120                 125
   His Lys Arg Arg Ala Ala Ala Ala Val Cys Ser Phe Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Gln Pro Phe Leu Ser Ser Gln Thr Lys Ala Asn Met
                   165                 170                 175
   Gly Ser Ser Val Ser Tyr Ile Met Ala Ala Asn His Ala Ala Phe Val
               180                 185                 190
   Val Gly Ser Gly Leu Ala Ile Ser Ala Glu Arg Ala Asp Cys Glu Ala
           195                 200                 205
   Arg Cys Ala Arg Ile Ala Arg Glu Glu Ser Ser Leu Glu Leu Ser Gly
       210                 215                 220
   Glu Glu Asn Ala Cys Glu Arg Gly Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Val
           275                 280                 285
   Ile Gly Leu Trp Thr Phe Cys Asn Arg Val
       290                 295
<210> 132
   <211> 897
   <212> DNA
   <213> Chlamydia
<400> 132
   atggctgcta tatgcggacg tttagggtct ggtacaggga atgctctaaa agcttttttt   60
   acacagccca gcaataaaat ggcaagggta gtaaataaga cgaagggaat ggataagact  120
   gttaaggtcg ccaagtctgc tgccgaattg accgcaaata ttttggaaca agctggaggc  180
   gcgggctctt ccgcacacat tacagcttcc caagtgtcca aaggattagg ggatgcgaga  240
   actgttctcg ctttagggaa tgcctttaac ggagcgttgc caggaacagt tcaaagtgcg  300
   caaagcttct tctcttacat gaaagctgct agtcagaaac cgcaagaagg ggatgagggg  360
   ctcgtagcag atctttgtgt gtctcataag cgcagagcgg ctgcggctgt ctgtagcttc  420
   atcggaggaa ttacctacct cgcgacattc ggagctatcc gtccgattct gtttgtcaac  480
   aaaatgctgg cgcaaccgtt tctttcttcc caaactaaag caaatatggg atcttctgtt  540
   agctatatta tggcggctaa ccatgcagcg tttgtggtgg gttctggact cgctatcagt  600
   gcggaaagag cagattgcga agcccgctgc gctcgtattg cgagagaaga gtcgtcactc  660
   gaattgtcgg gagaggaaaa tgcttgtgag aggagagtcg ctggagagaa agccaagacg  720
   ttcacgcgca tcaagtatgc actcctcact atgctcgaga agtttttgga atgcgttgcc  780
   gacgttttca aattggtgcc gttgcctatt acaatgggta ttcgtgcaat tgtggctgcg  840
   ggatgtacgt tcacttctgc agttattgga ttgtggactt tctgcaacag agtataa     897
<210> 133
   <211> 298
   <212> PRT
   <213> Chlamydia

<400> 133
   Met Ala Ala Ile Cys Gly Arg Leu Gly Ser Gly Thr Gly Asn Ala Leu
    1               5                  10                  15
   Lys Ala Phe Phe Thr Gln Pro Ser Asn Lys Met Ala Arg Val Val Asn
               20                  25                  30
   Lys Thr Lys Gly Met Asp Lys Thr Val Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Leu Ala Leu Gly Asn Ala Phe Asn Gly Ala Leu Pro Gly Thr
                   85                  90                  95
   Val Gln Ser Ala Gln Ser Phe Phe Ser Tyr Met Lys Ala Ala Ser Gln
               100                 105                 110
   Lys Pro Gln Glu Gly Asp Glu Gly Leu Val Ala Asp Leu Cys Val Ser
           115                 120                 125
   His Lys Arg Arg Ala Ala Ala Ala Val Cys Ser Phe Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Gln Pro Phe Leu Ser Ser Gln Thr Lys Ala Asn Met
                   165                 170                 175
   Gly Ser Ser Val Ser Tyr Ile Met Ala Ala Asn His Ala Ala Phe Val
               180                 185                 190
   Val Gly Ser Gly Leu Ala Ile Ser Ala Glu Arg Ala Asp Cys Glu Ala
           195                 200                 205
   Arg Cys Ala Arg Ile Ala Arg Glu Glu Ser Ser Leu Glu Leu Ser Gly
       210                 215                 220
   Glu Glu Asn Ala Cys Glu Arg Arg Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Val
           275                 280                 285
   Ile Gly Leu Trp Thr Phe Cys Asn Arg Val
       290                 295
<210> 134
   <211> 897
   <212> DNA
   <213> Chlamydia
<400> 134
   atggcttcta tatgcggacg tttagggtct ggtacaggga atgctctaaa agcttttttt      60
   acacagccca acaataaaat ggcaagggta gtaaataaga cgaagggaat ggataagact     120
   attaaggttg ccaagtctgc tgccgaattg accgcaaata ttttggaaca agctggaggc     180
   gcgggctctt ccgcacacat tacagcttcc caagtgtcca aaggattagg ggatgcgaga     240
   actgttgtcg ctttagggaa tgcctttaac ggagcgttgc caggaacagt tcaaagtgcg     300
   caaagcttct tctctcacat gaaagctgct agtcagaaaa cgcaagaagg ggatgagggg     360
   ctcacagcag atctttgtgt gtctcataag cgcagagcgg ctgcggctgt ctgtagcatc     420
   atcggaggaa ttacctacct cgcgacattc ggagctatcc gtccgattct gtttgtcaac     480
   aaaatgctgg caaaaccgtt tctttcttcc caaactaaag caaatatggg atcttctgtt     540
   agctatatta tggcggctaa ccatgcagcg tctgtggtgg gtgctggact cgctatcagt     600
   gcggaaagag cagattgcga agcccgctgc gctcgtattg cgagagaaga gtcgttactc     660
   gaaatgccgg gagaggaaaa tgcttgcgag aagaaagtcg ctggagagaa agccaagacg     720
   ttcacgcgca tcaagtatgc actcctcact atgctcgaga agtttttgga atgcgttgcc     780
   gacgttttca aattggtgcc gctgcctatt acaatgggta ttcgtgcgat tgtggctgct     840
   ggatgtacgt tcacttctgc aattattgga ttgtgcactt tctgcgccag agcataa        897
<210> 135
   <211> 298
   <212> PRT
   <213> Chlamydia
<400> 135
   Met Ala Ser Ile Cys Gly Arg Leu Gly Ser Gly Thr Gly Asn Ala Leu
   1 5 10 15
   Lys Ala Phe Phe Thr Gln Pro Asn Asn Lys Met Ala Arg Val Val Asn
   20 25 30
   Lys Thr Lys Gly Met Asp Lys Thr Ile Lys Val Ala Lys Ser Ala Ala
   35 40 45
   Glu Leu Thr Ala Asn Ile Leu Glu Gln Ala Gly Gly Ala Gly Ser Ser
   50 55 60
   Ala His Ile Thr Ala Ser Gln Val Ser Lys Gly Leu Gly Asp Ala Arg
   65 70 75 80
   Thr Val Val Ala Leu Gly Asn Ala Phe Asn Gly Ala Leu Pro Gly Thr
   85 90 95
   Val Gln Ser Ala Gln Ser Phe Phe Ser His Met Lys Ala Ala Ser Gln
   100 105 110
   Lys Thr Gln Glu Gly Asp Glu Gly Leu Thr Ala Asp Leu Cys Val Ser
   115 120 125
   His Lys Arg Arg Ala Ala Ala Ala Val Cys Ser Ile Ile Gly Gly Ile
   130 135 140
   Thr Tyr Leu Ala Thr Phe Gly Ala Ile Arg Pro Ile Leu Phe Val Asn
   145 150 155 160
   Lys Met Leu Ala Lys Pro Phe Leu Ser Ser Gln Thr Lys Ala Asn Met
   165 170 175
   Gly Ser Ser Val Ser Tyr Ile Met Ala Ala Asn His Ala Ala Ser Val
   180 185 190
   Val Gly Ala Gly Leu Ala Ile Ser Ala Glu Arg Ala Asp Cys Glu Ala
   195 200 205
   Arg Cys Ala Arg Ile Ala Arg Glu Glu Ser Leu Leu Glu Met Pro Gly
   210 215 220
   Glu Glu Asn Ala Cys Glu Lys Lys Val Ala Gly Glu Lys Ala Lys Thr
   225 230 235 240
   Phe Thr Arg Ile Lys Tyr Ala Leu Leu Thr Met Leu Glu Lys Phe Leu
   245 250 255
   Glu Cys Val Ala Asp Val Phe Lys Leu Val Pro Leu Pro Ile Thr Met
   260 265 270
   Gly Ile Arg Ala Ile Val Ala Ala Gly Cys Thr Phe Thr Ser Ala Ile
   275 280 285
   Ile Gly Leu Cys Thr Phe Cys Ala Arg Ala
   290 295
<210> 136
   <211> 882
   <212> DNA
   <213> Chlamydia
<400> 136
   atggcttctg tatgtgggcg attaagtgct ggggtgggga acagatttaa cgcatttttc      60
   acgcgtcccg gtaacaagct atcacggttt gtaaatagcg caaaaggatt agacagatca     120
   ataaaggttg ggaagtctgc tgctgaatta acggcgagta ttttagagca aactgggggg     180
   gcagggactg atgcacatgt tacggcggcc aaggtgtcta aagcacttgg ggacgcgcga     240
   acagtaatgg ctctagggaa tgtcttcaat gggtctgtgc cagcaaccat tcaaagtgcg     300
   cgaagctgtc tcgcccattt acgagcggcc ggcaaagaag aagaaacatg ctccaaggtg     360
   aaagatctct gtgtttctca tagacgaaga gctgcggctg aggcttgtaa tgttattgga     420
   ggagcaactt atattacaac tttcggagcg attcgtccga cattactcgt taacaagctt     480
   cttgccaaac cattcctttc ctcccaagcc aaagaagggt tgggagcttc tgttggttat     540
   atcatggcag cgaaccatgc ggcatctgtg cttgggtctg ctttaagtat tagcgcagaa     600
   agagcagact gtgaagagcg gtgtgatcgc attcgatgta gtgaggatgg tgaaatttgc     660
   gaaggcaata aattaacagc tatttcggaa gagaaggcta gatcatggac tctcattaag     720
   tacagattcc ttactatgat agaaaaacta tttgagatgg tggcggatat cttcaagtta     780
   attcctttgc caatttcgca tggaattcgt gctattgttg ctgcgggatg tacgttgact     840
   tctgcagtta ttggcttagg tactttttgg tctagagcat aa                       882
<210> 137
   <211> 293
   <212> PRT
   <213> Chlamydia
<400> 137
   Met Ala Ser Val Cys Gly Arg Leu Ser Ala Gly Val Gly Asn Arg Phe
    1               5                  10                  15
   Asn Ala Phe Phe Thr Arg Pro Gly Asn Lys Leu Ser Arg Phe Val Asn
   20 25 30
   Ser Ala Lys Gly Leu Asp Arg Ser Ile Lys Val Gly Lys Ser Ala Ala
   35 40 45
   Glu Leu Thr Ala Ser Ile Leu Glu Gln Thr Gly Gly Ala Gly Thr Asp
   50 55 60
   Ala His Val Thr Ala Ala Lys Val Ser Lys Ala Leu Gly Asp Ala Arg
   65 70 75 80
   Thr Val Met Ala Leu Gly Asn Val Phe Asn Gly Ser Val Pro Ala Thr
   85 90 95
   Ile Gln Ser Ala Arg Ser Cys Leu Ala His Leu Arg Ala Ala Gly Lys
   100 105 110
   Glu Glu Glu Thr Cys Ser Lys Val Lys Asp Leu Cys Val Ser His Arg
   115 120 125
   Arg Arg Ala Ala Ala Glu Ala Cys Asn Val Ile Gly Gly Ala Thr Tyr
   130 135 140
   Ile Thr Thr Phe Gly Ala Ile Arg Pro Thr Leu Leu Val Asn Lys Leu
   145 150 155 160
   Leu Ala Lys Pro Phe Leu Ser Ser Gln Ala Lys Glu Gly Leu Gly Ala
   165 170 175
   Ser Val Gly Tyr Ile Met Ala Ala Asn His Ala Ala Ser Val Leu Gly
   180 185 190
   Ser Ala Leu Ser Ile Ser Ala Glu Arg Ala Asp Cys Glu Glu Arg Cys
   195 200 205
   Asp Arg Ile Arg Cys Ser Glu Asp Gly Glu Ile Cys Glu Gly Asn Lys
   210 215 220
   Leu Thr Ala Ile Ser Glu Glu Lys Ala Arg Ser Trp Thr Leu Ile Lys
   225 230 235 240
   Tyr Arg Phe Leu Thr Met Ile Glu Lys Leu Phe Glu Met Val Ala Asp
   245 250 255
   Ile Phe Lys Leu Ile Pro Leu Pro Ile Ser His Gly Ile Arg Ala Ile
   260 265 270
   Val Ala Ala Gly Cys Thr Leu Thr Ser Ala Val Ile Gly Leu Gly Thr
   275 280 285
   Phe Trp Ser Arg Ala
   290
<210> 138
   <211> 16
   <212> PRT
   <213> Artificial Sequence
<220>
   <223> Made in a lab
<400> 138
   Asp Leu Cys Val Ser His Lys Arg Arg Ala Ala Ala Ala Val Cys Ser
   1 5 10 15
<210> 139
   <211> 16
   <212> PRT
   <213> Artificial Sequence
<220>
   <223> Made in a lab
<400> 139
   Arg Ala Ala Ala Ala Val Cys Ser Phe Ile Gly Gly Ile Thr Tyr Leu
   1               5                   10                  15
<210> 140
   <211> 18
   <212> PRT
   <213> Artificial Sequence
<220>
   <223> Made in a lab
<400> 140
   Cys Ser Phe Ile Gly Gly Ile Thr Tyr Leu Ala Thr Phe Gly Ala Ile
   1               5                   10                  15
   Arg Pro
<210> 141
   <211> 18
   <212> PRT
   <213> Artificial Sequence
<220>
   <223> Made in a lab
<400> 14
<210> 142
   <211> 18
   <212> PRT
   <213> Artificial Sequence
<220>
   <223> Made in a lab
<400> 142
   Arg Pro Ile Leu Phe Val Asn Lys Met Leu Ala Gln Pro Phe Leu Ser
   1               5                   10                  15
   Ser Gln
<210> 143
   <211> 17
   <212> PRT
   <213> Artificial Sequence
<220>
   <223> Made in a lab

<400> 143
   Met Leu Ala Gln Pro Phe Leu Ser Ser Gln Thr Lys Ala Asn Met Gly
   1 5 10 15
   Ser
<210> 144
   <211> 10
   <212> PRT
   <213> Artificial Sequence
<220>
   <223> Made in a lab
<400> 144
   Cys Ser Phe Ile Gly Gly Ile Thr Tyr Leu
   1 5 10
<210> 145
   <211> 9
   <212> PRT
   <213> Artificial Sequence
<220>
   <223> Made in a lab
<400> 145
   Ser Phe Ile Gly Gly Ile Thr Tyr Leu
   1 5
<210> 146
   <211> 8
   <212> PRT
   <213> Artificial Sequence
<220>
   <223> Made in a lab
<400> 146
   Phe Ile Gly Gly Ile Thr Tyr Leu
   1 5
<210> 147
   <211> 9
   <212> PRT
   <213> Artificial Sequence
<220>
   <223> Made in a lab
<400> 147
   Cys Ser Phe Ile Gly Gly Ile Thr Tyr
   1 5
<210> 148
   <211> 8
   <212> PRT
   <213> Artificial Sequence
<220>
   <223> Made in a lab
<400> 148
   Cys Ser Phe Ile Gly Gly Ile Thr
   1 5
<210> 149
   <211> 10
   <212> PRT
   <213> Artificial Sequence
<220>
   <223> Made in a lab
<400> 149
   Cys Ser Ile Ile Gly Gly Ile Thr Tyr Leu
   1 5 10
<210> 150
   <211> 10
   <212> PRT
   <213> Artificial Sequence
<220>
   <223> Made in a lab
<400> 150
   Cys Gly Phe Ile Gly Gly Ile Thr Tyr Leu
   1 5 10
<210> 151
   <211> 9
   <212> PRT
   <213> Artificial Sequence
<220>
   <223> Made in a lab
<400> 151
   Gly Phe Ile Gly Gly Ile Thr Tyr Leu
   1 5
<210> 152
   <211> 20
   <212> PRT
   <213> Artificial Sequence
<220>
   <223> Made in a lab

<400> 152
   Gln Ile Phe Val Cys Leu Ile Ser Ala Glu Arg Leu Arg Leu Arg Leu
   1 5 10 15
   Ser Val Ala Ser
   20
<210> 153
   <211> 20
   <212> PRT
   <213> Artificial Sequence
<220>
   <223> Made in a lab
<400> 153
   Glu Arg Leu Arg Leu Arg Leu Ser Val Ala Ser Ser Glu Glu Leu Pro
   1 5 10 15
   Thr Ser Arg His
   20
<210> 154
   <211> 20
   <212> PRT
   <213> Artificial Sequence
<220>
   <223> Made in a lab
<400> 154
   Ala Ser Ser Glu Glu Leu Pro Thr Ser Arg His Ser Glu Leu Ser Val
   1 5 10 15
   Arg Phe Cys Leu
   20
<210> 155
   <211> 20
   <212> PRT
   <213> Artificial Sequence
<220>
   <223> Made in a lab
<400> 155
   Arg His Ser Glu Leu Ser Val Arg Phe Cys Leu Ser Thr Lys Cys Trp
   1 5 10 15
   Arg Asn Arg Phe
   20
<210> 156
   <211> 20
   <212> PRT
   <213> Artificial Sequence
<220>
   <223> Made in a lab
<400> 156
   Leu Ser Thr Lys Cys Trp Arg Asn Arg Phe Phe Leu Pro Lys Leu Lys
   1 5 10 15
   Gln Ile Trp Asp
   20
<210> 157
   <211> 53
   <212> PRT
   <213> Artificial Sequence
<220>
   <223> Made in a lab
<400> 157
   Ile Phe Val Cys Leu Ile Ser Ala Glu Arg Leu Arg Leu Ser Val Ala
   1 5 10 15
   Ser Ser Glu Glu Leu Pro Thr Ser Arg His Ser Glu Leu Ser Val Arg
   20 25 30
   Phe Cys Leu Ser Thr Lys Cys Trp Arg Asn Arg Phe Phe Leu Pro Lys
   35 40 45
   Leu Lys Gln Ile Trp
   50
<210> 158
   <211> 52
   <212> PRT
   <213> Artificial Sequence
<220>
   <223> Made in a lab
<400> 158
   Leu Cys Val Ser His Lys Arg Arg Ala Ala Ala Ala Val Cys Ser Phe
    1               5                  10                  15
   Ile Gly Gly Ile Thr Tyr Leu Ala Thr Phe Gly Ala Ile Arg Pro Ile
               20                  25                  30
   Leu Phe Val Asn Lys Met Leu Ala Gln Pro Phe Leu Ser Ser Gln Ile
           35                  40                  45
   Lys Ala Asn Met
       50
<210> 159
   <211> 24
   <212> DNA
   <213> Chlamydia
<400> 159
   ttttgaagca ggtaggtgaa tatg 24
<210> 160
   <211> 24
   <212> DNA
   <213> Chlamydia
<400> 160
   ttaagaaatt taaaaaatcc ctta 24
<210> 161
   <211> 24
   <212> DNA
   <213> Chlamydia
<400> 161
   ggtataatat ctctctaaat tttg 24
<210> 162
   <211> 19
   <212> DNA
   <213> Chlamydia
<400> 162
   agataaaaaa ggctgtttc 19
<210> 163
   <211> 24
   <212> DNA
   <213> Chlamydia
<400> 163
   ttttgaagca ggtaggtgaa tatg 24
<210> 164
   <211> 29
   <212> DNA
   <213> Chlamydia
<400> 164
   tttacaataa gaaaagctaa gcactttgt 29
<210> 165
   <211> 20
   <212> DNA
   <213> Chlamydia
<400> 165
   ccttacacag tcctgctgac 20
<210> 166
   <211> 20
   <212> DNA
   <213> Chlamydia
<400> 166
   gtttccgggc cctcacattg 20
<210> 167
   <211> 9
   <212> PRT
   <213> Artificial Sequence
<220>
   <223> Made in a lab

<400> 167
   Ser Phe Ile Gly Gly Ile Thr Tyr Leu
    1               5
<210> 168
   <211> 9
   <212> PRT
   <213> Artificial Sequence
<220>
   <223> Made in a lab
<400> 168
   Ser Ile Ile Gly Gly Ile Thr Tyr Leu
    1               5
<210> 169
   <211> 2643
   <212> DNA
   <213> Chlamydia
<400> 169
   gcaatcatgc gacctgatca tatgaacttc tgttgtctat gtgctgctat tttgtcatcc 60
   acagcggtcc tctttggcca ggatccctta ggtgaaaccg ccctcctcac taaaaatcct 120
   aatcatgtcg tctgtacatt ttttgaggac tgtaccatgg agagcctctt tcctgctctt 180
   tgtgctcatg catcacaaga cgatcctttg tatgtacttg gaaattccta ctgttggttc 240
   gtatctaaac tccatatcac ggaccccaaa gaggctcttt ttaaagaaaa aggagatctt 300
   tccattcaaa actttcgctt cctttccttc acagattgct cttccaagga aagctctcct 360
   tctattattc atcaaaagaa tggtcagtta tccttgcgca ataatggtag catgagtttc 420
   tgtcgaaatc atgctgaagg ctctggagga gccatctctg cggatgcctt ttctctacag 480
   cacaactatc ttttcacagc ttttgaagag aattcttcta aaggaaatgg cggagccatt 540
   caggctcaaa ccttctcttt atctagaaat gtgtcgccta tttctttcgc ccgtaatcgt 600
   gcggatttaa atggcggcgc tatttgctgt agtaatctta tttgttcagg gaatgtaaac 660
   cctctctttt tcactggaaa ctccgccacg aatggaggcg ctatttgttg tatcagcgat 720
   ctaaacacct cagaaaaagg ctctctctct cttgcttgta accaagaaac gctatttgca 780
   agcaattctg ctaaagaaaa aggcggggct atttatgcca agcacatggt attgcgttat 840
   aacggtcctg tttccttcat taacaacagc gctaaaatag gtggagctat cgccatccag 900
   tccggaggga gtctctctat ccttgcaggt gaaggatctg ttctgttcca gaataactcc 960
   caacgcacct ccgaccaagg tctagtaaga aacgccatct acttaragaa agatgcgatt 1020
   ctttcttcct tagaagctcg caacggagat attcttttct ttgatcctat tgtacaagaa 1080
   agtagcagca aagaatcgcc tcttccctcc tctttgcaag ccagcgtgac ttctcccacc 1140
   ccagccaccg catctccttt agttattcag acaagtgcaa accgttcagt gattttctcg 1200
   agcgaacgtc tttctgaaga agaaaaaact cctgataacc tcacttccca actacagcag 1260
   cctatcgaac tgaaatccgg acgcttagtt ttaaaagatc gcgctgtcct ttccgcgcct 1320
   tctctctctc aggatcctca agctctcctc attatggaag cgggaacttc tttaaaaact    1380
   tcctctgatt tgaagttagc tacgctaagt attccccttc attccttaga tactgaaaaa    1440
   agcgtaacta tccacgcccc taatctttct atccaaaaga tcttcctctc taactctgga 1500
   gatgagaatt tttatgaaaa tgtagagctt ctcagtaaag agcaaaacaa tattcctctc 1560
   cttactctcc ctaaagagca atctcattta catcttcctg atgggaacct ctcttctcac 1620
   tttggatatc aaggagattg gactttttct tggaaagatt ctgatgaagg gcattctctg 1680
   attgctaatt ggacgcctaa aaactatgtg cctcatccag aacgtcaatc tacactcgtt 1740
   gcgaacactc tttggaacac ctattccgat atgcaagctg tgcagtcgat gattaataca 1800
   acagcgcacg gaggagccta tctatttgga acgtggggat ctgctgtttc taatttattc 1860
   tatgttcacg acagctctgg gaaacctatc gataattggc atcatagaag ccttggctac 1920
   ctattcggta tcagtactca cagtttagat gaccattctt tctgcttggc tgcaggacaa 1980
   ttactcggga aatcgtccga ttcctttatt acgtctacag aaacgacctc ctatatagct 2040
   actgtacaag cgcaactcgc tacctctcta atgaaaatct ctgcacaggc atgctacaat 2100
   gaaagtatcc atgagctaaa aacaaaatat cgctccttct ctaaagaagg attcggatcc 2160
   tggcatagcg ttgcagtatc cggagaagtg tgcgcatcga ttcctattgt atccaatggt 2220
   tccggactgt tcagctcctt ctctattttc tctaaactgc aaggattttc aggaacacag 2280
   gacggttttg aggagagttc gggagagatt cggtcctttt ctgccagctc tttcagaaat 2340
   atttcacttc ctataggaat aacatttgaa aaaaaatccc aaaaaacacg aacctactat 2400
   tactttctag gagcctacat ccaagacctg aaacgtgatg tggaatcggg acctgtagtg 2460
   ttactcaaaa atgccgtctc ctgggatgct cctatggcga acttggattc acgagcctac 2520
   atgttccggc ttacgaatca aagagctcta cacagacttc agacgctgtt aaatgtgtct 2580
   tgtgtgctgc gtgggcaaag ccatagttac tccctggatc tggggaccac ttacaggttc 2640
   tag 2643
<210> 170
   <211> 2949
   <212> DNA
   <213> Chlamydia
<400> 170
   atgattcctc aaggaattta cgatggggag acgttaactg tatcatttcc ctatactgtt   60
   ataggagatc cgagtgggac tactgttttt tctgcaggag agttaacatt aaaaaatctt  120
   gacaattcta ttgcagcttt gcctttaagt tgttttggga acttattagg gagttttact  180
   gttttaggga gaggacactc gttgactttc gagaacatac ggacttctac aaatggggca  240
   gctctaagta atagcgctgc tgatggactg tttactattg agggttttaa agaattatcc  300
   ttttccaatt gcaattcatt acttgccgta ctgcctgctg caacgactaa taagggtagc  360
   cagactccga cgacaacatc tacaccgtct aatggtacta tttattctaa aacagatctt 420
   ttgttactca ataatgagaa gttctcattc tatagtaatt tagtctctgg agatggggga 480
   gctatagatg ctaagagctt aacggttcaa ggaattagca agctttgtgt cttccaagaa  540
   aatactgctc aagctgatgg gggagcttgt caagtagtca ccagtttctc tgctatggct  600
   aacgaggctc ctattgcctt tgtagcgaat gttgcaggag taagaggggg agggattgct  660
   gctgttcagg atgggcagca gggagtgtca tcatctactt caacagaaga tccagtagta  720
   agtttttcca gaaatactgc ggtagagttt gatgggaacg tagcccgagt aggaggaggg  780
   atttactcct acgggaacgt tgctttcctg aataatggaa aaaccttgtt tctcaacaat  840
   gttgcttctc ctgtttacat tgctgctaag caaccaacaa gtggacaggc ttctaatacg  900
   agtaataatt acggagatgg aggagctatc ttctgtaaga atggtgcgca agcaggatcc  960
   aataactctg gatcagtttc ctttgatgga gagggagtag ttttctttag tagcaatgta 1020
   gctgctggga aagggggagc tatttatgcc aaaaagctct cggttgctaa ctgtggccct 1080
   gtacaatttt taaggaatat cgctaatgat ggtggagcga tttatttagg agaatctgga 1140
   gagctcagtt tatctgctga ttatggagat attattttcg atgggaatct taaaagaaca 1200
   gccaaagaga atgctgccga tgttaatggc gtaactgtgt cctcacaagc catttcgatg 1260
   ggatcgggag ggaaaataac gacattaaga gctaaagcag ggcatcagat tctctttaat 1320
   gatcccatcg agatggcaaa cggaaataac cagccagcgc agtcttccaa acttctaaaa 1380
   attaacgatg gtgaaggata cacaggggat attgtttttg ctaatggaag cagtactttg 1440
   taccaaaatg ttacgataga gcaaggaagg attgttcttc gtgaaaaggc aaaattatca 1500
   gtgaattctc taagtcagac aggtgggagt ctgtatatgg aagctgggag tacattggat 1560
   tttgtaactc cacaaccacc acaacagcct cctgccgcta atcagttgat cacgctttcc 1620
   aatctgcatt tgtctctttc ttctttgtta gcaaacaatg cagttacgaa tcctcctacc 1680
   aatcctccag cgcaagattc tcatcctgca gtcattggta gcacaactgc tggttctgtt 1740
   acaattagtg ggcctatctt ttttgaggat ttggatgata cagcttatga taggtatgat 1800
   tggctaggtt ctaatcaaaa aatcaatgtc ctgaaattac agttagggac taagccccca 1860
   gctaatgccc catcagattt gactctaggg aatgagatgc ctaagtatgg ctatcaagga 1920
   agctggaagc ttgcgtggga tcctaataca gcaaataatg gtccttatac tctgaaagct 1980
   acatggacta aaactgggta taatcctggg cctgagcgag tagcttcttt ggttccaaat 2040
   agtttatggg gatccatttt agatatacga tctgcgcatt cagcaattca agcaagtgtg 2100
   gatgggcgct cttattgtcg aggattatgg gtttctggag tttcgaattt cttctatcat 2160
   gaccgcgatg ctttaggtca gggatatcgg tatattagtg ggggttattc cttaggagca 2220
   aactcctact ttggatcatc gatgtttggt ctagcattta ccgaagtatt tggtagatct 2280
   aaagattatg tagtgtgtcg ttccaatcat catgcttgca taggatccgt ttatctatct 2340
   acccaacaag ctttatgtgg atcctatttg ttcggagatg cgtttatccg tgctagctac 2400
   gggtttggga atcagcatat gaaaacctca tatacatttg cagaggagag cgatgttcgt 2460
   tgggataata actgtctggc tggagagatt ggagcgggat taccgattgt gattactcca 2520
   tctaagctct atttgaatga gttgcgtcct ttcgtgcaag ctgagttttc ttatgccgat 2580
   catgaatctt ttacagagga aggcgatcaa gctcgggcat tcaagagcgg acatctccta 2640
   aatctatcag ttcctgttgg agtgaagttt gatcgatgtt ctagtacaca tcctaataaa 2700
   tatagcttta tggcggctta tatctgtgat gcttatcgca ccatctctgg tactgagaca 2760
   acgctcctat cccatcaaga gacatggaca acagatgcct ttcatttagc aagacatgga 2820
   gttgtggtta gaggatctat gtatgcttct ctaacaagta atatagaagt atatggccat 2880
   ggaagatatg agtatcgaga tgcttctcga ggctatggtt tgagtgcagg magtaaagtc 2940
   yggttctaa                                                         2949
<210> 171
   <211> 2895
   <212> DNA
   <213> Chlamydia
<400> 171
   atgaaaaaag cgtttttctt tttccttatc ggaaactccc tatcaggact agctagagag 60
   gttccttcta gaatctttct tatgcccaac tcagttccag atcctacgaa agagtcgcta 120
   tcaaataaaa ttagtttgac aggagacact cacaatctca ctaactgcta tctcgataac 180
   ctacgctaca tactggctat tctacaaaaa actcccaatg aaggagctgc tgtcacaata 240
   acagattacc taagcttttt tgatacacaa aaagaaggta tttattttgc aaaaaatctc 300
   acccctgaaa gtggtggtgc gattggttat gcgagtccca attctcctac cgtggagatt 360
   cgtgatacaa taggtcctgt aatctttgaa aataatactt gttgcagact atttacatgg 420
   agaaatcctt atgctgctga taaaataaga gaaggcggag ccattcatgc tcaaaatctt 480
   tacataaatc ataatcatga tgtggtcgga tttatgaaga acttttctta tgtccaagga 540
   ggagccatta gtaccgctaa tacctttgtt gtgagcgaga atcagtcttg ttttctcttt 600
   atggacaaca tctgtattca aactaataca gcaggaaaag gtggcgctat ctatgctgga 660
   acgagcaatt cttttgagag taataactgc gatctcttct tcatcaataa cgcctgttgt 720
   gcaggaggag cgatcttctc ccctatctgt tctctaacag gaaatcgtgg taacatcgtt 780
   ttctataaca atcgctgctt taaaaatgta gaaacagctt cttcagaagc ttctgatgga 840
   ggagcaatta aagtaactac tcgcctagat gttacaggca atcgtggtag gatctttttt 900
   agtgacaata tcacaaaaaa ttatggcgga gctatttacg ctcctgtagt taccctagtg 960
   gataatggcc ctacctactt tataaacaat atcgccaata ataagggggg cgctatctat 1020
   atagacggaa ccagtaactc caaaatttct gccgaccgcc atgctattat ttttaatgaa 1080
   aatattgtga ctaatgtaac taatgcaaat ggtaccagta cgtcagctaa tcctcctaga 1140
   agaaatgcaa taacagtagc aagctcctct ggtgaaattc tattaggagc agggagtagc 1200
   caaaatttaa ttttttatga tcctattgaa gttagcaatg caggggtctc tgtgtccttc 1260
   aataaggaag ctgatcaaac aggctctgta gtattttcag gagctactgt taattctgca 1320
   gattttcatc aacgcaattt acaaacaaaa acacctgcac cccttactct cagtaatggt 1380
   tttctatgta tcgaagatca tgctcagctt acagtgaatc gattcacaca aactgggggt 1440
   gttgtttctc ttgggaatgg agcagttctg agttgctata aaaatggtac aggagattct 1500
   gctagcaatg cctctataac actgaagcat attggattga atctttcttc cattctgaaa 1560
   agtggtgctg agattccttt attgtgggta gagcctacaa ataacagcaa taactataca 1620
   gcagatactg cagctacctt ttcattaagt gatgtaaaac tctcactcat tgatgactac 1680
   gggaactctc cttatgaatc cacagatctg acccatgctc tgtcatcaca gcctatgcta 1740
   tctatttctg aagctagcga taaccagcta caatcagaaa atatagattt ttcgggacta 1800
   aatgtccctc attatggatg gcaaggactt tggacttggg gctgggcaaa aactcaagat 1860
   ccagaaccag catcttcagc aacaatcact gatccacaaa aagccaatag atttcataga 1920
   accttactac taacatggct tcctgccggg tatgttccta gcccaaaaca cagaagtccc 1980
   ctcatagcta acaccttatg ggggaatatg ctgcttgcaa cagaaagctt aaaaaatagt 2040
   gcagagctga cacctagtgg tcatcctttc tggggaatta caggaggagg actaggcatg 2100
   atggtttacc aagatcctcg agaaaatcat cctggattcc atatgcgctc ttccggatac 2160
   tctgcgggga tgatagcagg gcagacacac accttctcat tgaaattcag tcagacctac 2220
   accaaactca atgagcgtta cgcaaaaaac aacgtatctt ctaaaaatta ctcatgccaa 2280
   ggagaaatgc tcttctcatt gcaagaaggt ttcttgctga ctaaattagt tgggctttac 2340
   agctatggag accataactg tcaccatttc tatactcaag gagaaaatct aacatctcaa 2400
   gggacgttcc gcagtcaaac gatgggaggt gctgtctttt ttgatctccc tatgaaaccc 2460
   tttggatcaa cgcatatact gacagctccc tttttaggtg ctcttggtat ttattctagc 2520
   ctgtctcact ttactgaggt gggagcctat ccgcgaagct tttctacaaa gactcctttg 2580
   atcaatgtcc tagtccctat tggagttaaa ggtagcttta tgaatgctac ccacagacct 2640
   caagcctgga ctgtagaatt ggcataccaa cccgttctgt atagacaaga accagggatc 2700
   gcgacccagc tcctagccag taaaggtatt tggtttggta gtggaagccc ctcatcgcgt 2760
   catgccatgt cctataaaat ctcacagcaa acacaacctt tgagttggtt aactctccat 2820
   ttccagtatc atggattcta ctcctcttca accttctgta attatctcaa tggggaaatt 2880
   gctctgcgat tctag 2895
<210> 172
   <211> 4593
   <212> DNA
   <213> Chlamydia
<400> 172
   atgagttccg agaaagatat aaaaagcacc tgttctaagt tttctttgtc tgtagtagca     60
   gctatccttg cctctgttag cgggttagct agttgcgtag atcttcatgc tggaggacag    120
   tctgtaaatg agctggtata tgtaggccct caagcggttt tattgttaga ccaaattcga    180
   gatctattcg ttgggtctaa agatagtcag gctgaaggac agtataggtt aattgtagga    240
   gatccaagtt ctttccaaga gaaagatgca gatactcttc ccgggaaggt agagcaaagt    300
   actttgttct cagtaaccaa tcccgtggtt ttccaaggtg tggaccaaca ggatcaagtc    360
   tcttcccaag ggttaatttg tagttttacg agcagcaacc ttgattctcc ccgtgacgga    420
   gaatcttttt taggtattgc ttttgttggg gatagtagta aggctggaat cacattaact    480
   gacgtgaaag cttctttgtc tggagcggct ttatattcta cagaagatct tatctttgaa    540
   aagattaagg gtggattgga atttgcatca tgttcttctc tagaacaggg gggagcttgt    600
   gcagctcaaa gtattttgat tcatgattgt caaggattgc aggttaaaca ctgtactaca    660
   gccgtgaatg ctgaggggtc tagtgcgaat gatcatcttg gatttggagg aggcgctttc    720
   tttgttacgg gttctctttc tggagagaaa agtctctata tgcctgcagg agatatggta    780
   gttgcgaatt gtgatggggc tatatctttt gaaggaaaca gcgcgaactt tgctaatgga    840
   ggagcgattg ctgcctctgg gaaagtgctt tttgtcgcta atgataaaaa gacttctttt    900
   atagagaacc gagctttgtc tggaggagcg attgcagcct cttctgatat tgcctttcaa    960
   aactgcgcag aactagtttt caaaggcaat tgtgcaattg gaacagagga taaaggttct   1020
   ttaggtggag ggctatatcc ttctctaggc accgttcttt tgcaagggaa tcacgggata   1080
   acttgtgata agaatgagtc tgcttcgcaa ggaggcgcca tttttggcaa aaattgtcag   1140
   atttctgaca acgaggggcc agtggttttc agagatagta cagcttgctt aggaggaggc   1200
   gctattgcag ctcaagaaat tgtttctatt cagaacaatc aggctgggat ttccttcgag   1260
   ggaggtaagg ctagtttcgg aggaggtatt gcgtgtggat ctttttcttc cgcaggcggt   1320
   gcttctgttt tagggactat tgatatttcg aagaatttag gcgcgatttc gttctctcgt   1380
   actttatgta cgacctcaga tttaggacaa atggagtacc agggaggagg agctctattt   1440
   ggtgaaaata tttctctttc tgagaatgct ggtgtgctca cctttaaaga caacattgtg   1500
   aagacttttg cttcgaatgg gaaaattctg ggaggaggag cgattttagc tactggtaag   1560
   gtggaaatta ccaataattc cggaggaatt tcttttacag gaaatgcgag agctccacaa   1620
   gctcttccaa ctcaagagga gtttccttta ttcagcaaaa aagaagggcg accactctct    1680
   tcaggatatt ctgggggagg agcgatttta ggaagagaag tagctattct ccacaacgct    1740
   gcagtagtat ttgagcaaaa tcgtttgcag tgcagcgaag aagaagcgac attattaggt    1800
   tgttgtggag gaggcgctgt tcatgggatg gatagcactt cgattgttgg caactcttca    1860
   gtaagatttg gtaataatta cgcaatggga caaggagtct caggaggagc tcttttatct    1920
   aaaacagtgc agttagctgg aaatggaagc gtcgattttt ctcgaaatat tgctagtttg    1980
   ggaggaggag ctcttcaagc ttctgaagga aattgtgagc tagttgataa cggctatgtg    2040
   ctattcagag ataatcgagg gagggtttat gggggtgcta tttcttgctt acgtggagat    2100
   gtagtcattt ctggaaacaa gggtagagtt gaatttaaag acaacatagc aacacgtctt    2160
   tatgtggaag aaactgtaga aaaggttgaa gaggtagagc cagctcctga gcaaaaagac    2220
   aataatgagc tttctttctt agggagtgta gaacagagtt ttattactgc agctaatcaa    2280
   gctcttttcg catctgaaga tggggattta tcacctgagt catccatttc ttctgaagaa    2340
   cttgcgaaaa gaagagagtg tgctggagga gctatttttg caaaacgggt tcgtattgta    2400
   gataaccaag aggccgttgt attctcgaat aacttctctg atatttatgg cggcgccatt    2460
   tttacaggtt ctcttcgaga agaggataag ttagatgggc aaatccctga agtcttgatc    2520
   tcaggcaatg caggggatgt tgttttttcc ggaaattcct cgaagcgtga tgagcatctt    2580
   cctcatacag gtgggggagc catttgtact caaaatttga cgatttctca gaatacaggg    2640
   aatgttctgt tttataacaa cgtggcctgt tcgggaggag ctgttcgtat agaggatcat    2700
   ggtaatgttc ttttagaagc ttttggagga gatattgttt ttaaaggaaa ttcttctttc    2760
   agagcacaag gatccgatgc tatctatttt gcaggtaaag aatcgcatat tacagccctg    2820
   aatgctacgg aaggacatgc tattgttttc cacgacgcat tagtttttga aaatctaaaa    2880
   gaaaggaaat ctgctgaagt attgttaatc aatagtcgag aaaatccagg ttacactgga    2940
   tctattcgat ttttagaagc agaaagtaaa gttcctcaat gtattcatgt acaacaagga    3000
   agccttgagt tgctaaatgg agctacatta tgtagttatg gttttaaaca agatgctgga    3060
   gctaagttgg tattggctgc tggatctaaa ctgaagattt tagattcagg aactcctgta    3120
   caagggcatg ctatcagtaa acctgaagca gaaatcgagt catcttctga accagagggt    3180
   gcacattctc tttggattgc gaagaatgct caaacaacag ttcctatggt tgatatccat    3240
   actatttctg tagatttagc ctccttctct tctagtcaac aggaggggac agtagaagct    3300
   cctcaggtta ttgttcctgg aggaagttat gttcgatctg gagagcttaa tttggagtta    3360
   gttaacacaa caggtactgg ttatgaaaat catgctttgt tgaagaatga ggctaaagtt    3420
   ccattgatgt ctttcgttgc ttctagtgat gaagcttcag ccgaaatcag taacttgtcg    3480
   gtttctgatt tacagattca tgtagcaact ccagagattg aagaagacac atacggccat    3540
   atgggagatt ggtctgaggc taaaattcaa gatggaactc ttgtcattaa ttggaatcct    3600
   actggatatc gattagatcc tcaaaaagca ggggctttag tatttaatgc attatgggaa    3660
   gaaggggctg tcttgtctgc tctgaaaaat gcacgctttg ctcataatct cactgctcag    3720
   cgtatggaat tcgattattc tacaaatgtg tggggattcg cctttggtgg tttccgaact    3780
   ctatctgcag agaatctggt tgctattgat ggatacaaag gagcttatgg tggtgcttct    3840
   gctggagtcg atattcaatt gatggaagat tttgttctag gagttagtgg agctgctttc    3900
   ctaggtaaaa tggatagtca gaagtttgat gcggaggttt ctcggaaggg agttgttggt    3960
   tctgtatata caggattttt agctggatcc tggttcttca aaggacaata tagccttgga    4020
   gaaacacaga acgatatgaa aacgcgttat ggagtactag gagagtcgag tgcttcttgg    4080
   acatctcgag gagtactggc agatgcttta gttgaatacc gaagtttagt tggtcctgtg    4140
   agacctactt tttatgcttt gcatttcaat ccttatgtcg aagtatctta tgcttctatg    4200
   aaattccctg gctttacaga acaaggaaga gaagcgcgtt cttttgaaga cgcttcccct    4260
   accaatatca ccattccttt agggatgaag tttgaattgg cgttcataaa aggacagttt    4320
   tcagaggtga actctttggg aataagttat gcatgggaag cttatcgaaa agtagaagga    4380
   ggcgcggtgc agcttttaga agctgggttt gattgggagg gagctccaat ggatcttcct    4440
   agacaggagc tgcgtgtcgc tctggaaaat aatacggaat ggagttctta cttcagcaca    4500
   gtcttaggat taacagcttt ttgtggagga tttacttcta cagatagtaa actaggatat    4560
   gaggcgaata ctggattgcg attgatcttt taa                                4593
<210> 173
   <211> 5331
   <212> DNA
   <213> Chlamydia
<400> 173
<210> 174
   <211> 5265
   <212> DNA
   <213> Chlamydia
<400> 174
   gcaatcatga aatggctgtc agctactgcg gtgtttgctg ctgttctccc ctcagtttca 60
   gggttttgct tcccagaacc taaagaatta aatttctctc gcgtagaaac ttcttcctct 120
   accactttta ctgaaacaat tggagaagct ggggcagaat atatcgtctc tggtaacgca 180
   tctttcacaa aatttaccaa cattcctact accgatacaa caactcccac gaactcaaac 240
   tcctctagct ctagcggaga aactgcttcc gtttctgagg atagtgactc tacaacaacg 300
   actcctgatc ctaaaggtgg cggcgccttt tataacgcgc actccggagt tttgtccttt 360
   atgacacgat caggaacaga aggttcctta actctgtctg agataaaaat gactggtgaa 420
   ggcggtgcta tcttctctca aggagagctg ctatttacag atctgacaag tctaaccatc 480
   caaaataact tatcccagct atccggagga gcgatttttg gaggatctac aatctcccta 540
   tcagggatta ctaaagcgac tttctcctgc aactctgcag aagttcctgc tcctgttaag 600
   aaacctacag aacctaaagc tcaaacagca agcgaaacgt cgggttctag tagttctagc 660
   ggaaatgatt cggtgtcttc ccccagttcc agtagagctg aacccgcagc agctaatctt 720
   caaagtcact ttatttgtgc tacagctact cctgctgctc aaaccgatac agaaacatca 780
   actccctctc ataagccagg atctggggga gctatctatg ctaaaggcga ccttactatc 840
   gcagactctc aagaggtact attctcaata aataaagcta ctaaagatgg aggagcgatc 900
   tttgctgaga aagatgtttc tttcgagaat attacatcat taaaagtaca aactaacggt 960
   gctgaagaaa agggaggagc tatctatgct aaaggtgacc tctcaattca atcttctaaa 1020
   cagagtcttt ttaattctaa ctacagtaaa caaggtgggg gggctctata tgttgaagga 1080
   ggtataaact tccaagatct tgaagaaatt cgcattaagt acaataaagc tggaacgttc 1140
   gaaacaaaaa aaatcacttt accttcttta aaagctcaag catctgcagg aaatgcagat 1200
   gcttgggcct cttcctctcc tcaatctggt tctggagcaa ctacagtctc cgactcagga 1260
   gactctagct ctggctcaga ctcggatacc tcagaaacag ttccagtcac agctaaaggc 1320
   ggtgggcttt atactgataa gaatctttcg attactaaca tcacaggaat tatcgaaatt 1380
   gcaaataaca aagcgacaga tgttggaggt ggtgcttacg taaaaggaac ccttacttgt 1440
   gaaaactctc accgtctaca atttttgaaa aactcttccg ataaacaagg tggaggaatc 1500
   tacggagaag acaacatcac cctatctaat ttgacaggga agactctatt ccaagagaat 1560
   actgccaaag aagagggcgg tggactcttc ataaaaggta cagataaagc tcttacaatg 1620
   acaggactgg atagtttctg tttaattaat aacacatcag aaaaacatgg tggtggagcc 1680
   tttgttacca aagaaatctc tcagacttac acctctgatg tggaaacaat tccaggaatc 1740
   acgcctgtac atggtgaaac agtcattact ggcaataaat ctacaggagg taatggtgga 1800
   ggcgtgtgta caaaacgtct tgccttatct aaccttcaaa gcatttctat atccgggaat 1860
   tctgcagcag aaaatggtgg tggagcccac acatgcccag atagcttccc aacggcggat 1920
   actgcagaac agcccgcagc agcttctgcc gcgacgtcta ctcccaaatc tgccccggtc 1980
   tcaactgctc taagcacacc ttcatcttct accgtctctt cattaacctt actagcagcc 2040
   tcttcacaag cctctcctgc aacctctaat aaggaaactc aagatcctaa tgctgataca 2100
   gacttattga tcgattatgt agttgatacg actatcagca aaaacactgc taagaaaggc 2160
   ggtggaatct atgctaaaaa agccaagatg tcccgcatag accaactgaa tatctctgag 2220
   aactccgcta cagagatagg tggaggtatc tgctgtaaag aatctttaga actagatgct 2280
   ctagtctcct tatctgtaac agagaacctt gttgggaaag aaggtggagg cttacatgct 2340
   aaaactgtaa atatttctaa tctgaaatca ggcttctctt tctcgaacaa caaagcaaac 2400
   tcctcatcca caggagtcgc aacaacagct tcagcacctg ctgcagctgc tgcttcccta 2460
   caagcagccg cagcagccgc accatcatct ccagcaacac caacttattc aggtgtagta 2520
   ggaggagcta tctatggaga aaaggttaca ttctctcaat gtagcgggac ttgtcagttc 2580
   tctgggaacc aagctatcga taacaatccc tcccaatcat cgttgaacgt acaaggagga 2640
   gccatctatg ccaaaacctc tttgtctatt ggatcttccg atgctggaac ctcctatatt 2700
   ttctcgggga acagtgtctc cactgggaaa tctcaaacaa cagggcaaat agcgggagga 2760
   gcgatctact cccctactgt tacattgaat tgtcctgcga cattctctaa caatacagcc 2820
   tctatagcta caccgaagac ttcttctgaa gatggatcct caggaaattc tattaaagat 2880
   accattggag gagccattgc agggacagcc attaccctat ctggagtctc tcgattttca 2940
   gggaatacgg ctgatttagg agctgcaata ggaactctag ctaatgcaaa tacacccagt 3000
   gcaactagcg gatctcaaaa tagcattaca gaaaaaatta ctttagaaaa cggttctttt 3060
   atttttgaaa gaaaccaagc taataaacgt ggagcgattt actctcctag cgtttccatt 3120
   aaagggaata atattacctt caatcaaaat acatccactc atgatggaag cgctatctac 3180
   tttacaaaag atgctacgat tgagtcttta ggatctgttc tttttacagg aaataacgtt 3240
   acagctacac aagctagttc tgcaacatct ggacaaaata caaatactgc caactatggg 3300
   gcagccatct ttggagatcc aggaaccact caatcgtctc aaacagatgc cattttaacc 3360
   cttcttgctt cttctggaaa cattactttt agcaacaaca gtttacagaa taaccaaggt 3420
   gatactcccg ctagcaagtt ttgtagtatt gcaggatacg tcaaactctc tctacaagcc 3480
   gctaaaggga agactattag ctttttcgat tgtgtgcaca cctctaccaa aaaaacaggt 3540
   tcaacacaaa acgtttatga aactttagat attaataaag aagagaacag taatccatat 3600
   acaggaacta ttgtgttctc ttctgaatta catgaaaaca aatcttacat cccacagaat 3660
   gcaatccttc acaacggaac tttagttctt aaagagaaaa cagaactcca cgtagtctct 3720
   tttgagcaga aagaagggtc taaattaatt atggaacccg gagctgtgtt atctaaccaa 3780
   aacatagcta acggagctct agctatcaat gggttaacga ttgatctttc cagtatgggg 3840
   actcctcaag caggggaaat cttctctcct ccagaattac gtatcgttgc cacgacctct 3900
   agtgcatccg gaggaagcgg ggtcagcagt agtataccaa caaatcctaa aaggatttct 3960
   gcagcagtgc cttcaggttc tgccgcaact actccaacta tgagcgagaa caaagttttc 4020
   ctaacaggag accttacttt aatagatcct aatggaaact tttaccaaaa ccctatgtta 4080
   ggaagcgatc tagatgtacc actaattaag cttccgacta acacaagtga cgtccaagtc 4140
   tatgatttaa ctttatctgg ggatcttttc cctcagaaag ggtacatggg aacctggaca 4200
   ttagattcta atccacaaac agggaaactt caagccagat ggacattcga tacctatcgt 4260
   cgctgggtat acatacctag ggataatcat ttttatgcga actctatctt aggctcccaa 4320
   aactcaatga ttgttgtgaa gcaagggctt atcaacaaca tgttgaataa tgcccgcttc 4380
   gatgatatcg cttacaataa cttctgggtt tcaggagtag gaactttctt agctcaacaa 4440
   ggaactcctc tttccgaaga attcagttac tacagccgcg gaacttcagt tgccatcgat 4500
   gccaaaccta gacaagattt tatcctagga gctgcattta gtaagatagt ggggaaaacc 4560
   aaagccatca aaaaaatgca taattacttc cataagggct ctgagtactc ttaccaagct 4620
   tctgtctatg gaggtaaatt cctgtatttc ttgctcaata agcaacatgg ttgggcactt 4680
   cctttcctaa tacaaggagt cgtgtcctat ggacatatta aacatgatac aacaacactt     4740
   tacccttcta tccatgaaag aaataaagga gattgggaag atttaggatg gttagcggat     4800
   cttcgtatct ctatggatct taaagaacct tctaaagatt cttctaaacg gatcactgtc 4860
   tatggggaac tcgagtattc cagcattcgc cagaaacagt tcacagaaat cgattacgat 4920
   ccaagacact tcgatgattg tgcttacaga aatctgtcgc ttcctgtggg atgcgctgtc 4980
   gaaggagcta tcatgaactg taatattctt atgtataata agcttgcatt agcctacatg 5040
   ccttctatct acagaaataa tcctgtctgt aaatatcggg tattgtcttc gaatgaagct 5100
   ggtcaagtta tctgcggagt gccaactaga acctctgcta gagcagaata cagtactcaa 5160
   ctatatcttg gtcccttctg gactctctac ggaaactata ctatcgatgt aggcatgtat 5220
   acgctatcgc aaatgactag ctgcggtgct cgcatgatct tctaa 5265
<210> 175
   <211> 880
   <212> PRT
   <213> Chlamydia
<220>
   <221> VARIANT
   <222> (1)...(880)
   <223> Xaa = Any Amino Acid

<400> 175
   Ala Ile Met Arg Pro Asp His Met Asn Phe Cys Cys Leu Cys Ala Ala
   1 5 10 15
   Ile Leu Ser Ser Thr Ala Val Leu Phe Gly Gln Asp Pro Leu Gly Glu
   20 25 30
   Thr Ala Leu Leu Thr Lys Asn Pro Asn His Val Val Cys Thr Phe Phe
   35 40 45
   Glu Asp Cys Thr Met Glu Ser Leu Phe Pro Ala Leu Cys Ala His Ala
   50 55 60
   Ser Gln Asp Asp Pro Leu Tyr Val Leu Gly Asn Ser Tyr Cys Trp Phe
   65 70 75 80
   Val Ser Lys Leu His Ile Thr Asp Pro Lys Glu Ala Leu Phe Lys Glu
   85 90 95
   Lys Gly Asp Leu Ser Ile Gln Asn Phe Arg Phe Leu Ser Phe Thr Asp
   100 105 110
   Cys Ser Ser Lys Glu Ser Ser Pro Ser Ile Ile His Gln Lys Asn Gly
   115 120 125
   Gln Leu Ser Leu Arg Asn Asn Gly Ser Met Ser Phe Cys Arg Asn His
   130 135 140
   Ala Glu Gly Ser Gly Gly Ala Ile Ser Ala Asp Ala Phe Ser Leu Gln
   145                 150                 155                 160
   His Asn Tyr Leu Phe Thr Ala Phe Glu Glu Asn Ser Ser Lys Gly Asn
                   165                 170                 175
   Gly Gly Ala Ile Gln Ala Gln Thr Phe Ser Leu Ser Arg Asn Val Ser
               180                 185                 190
   Pro Ile Ser Phe Ala Arg Asn Arg Ala Asp Leu Asn Gly Gly Ala Ile
           195                 200                 205
   Cys Cys Ser Asn Leu Ile Cys Ser Gly Asn Val Asn Pro Leu Phe Phe
       210                 215                 220
   Thr Gly Asn Ser Ala Thr Asn Gly Gly Ala Ile Cys Cys Ile Ser Asp
   225                 230                 235                 240
   Leu Asn Thr Ser Glu Lys Gly Ser Leu Ser Leu Ala Cys Asn Gln Glu
                   245                 250                 255
   Thr Leu Phe Ala Ser Asn Ser Ala Lys Glu Lys Gly Gly Ala Ile Tyr
               260                 265                 270
   Ala Lys His Met Val Leu Arg Tyr Asn Gly Pro Val Ser Phe Ile Asn
           275                 280                 285
   Asn Ser Ala Lys Ile Gly Gly Ala Ile Ala Ile Gln Ser Gly Gly Ser
       290                 295                 300
   Leu Ser Ile Leu Ala Gly Glu Gly Ser Val Leu Phe Gln Asn Asn Ser
   305                 310                 315                 320
   Gln Arg Thr Ser Asp Gln Gly Leu Val Arg Asn Ala Ile Tyr Leu Xaa
                   325                 330                 335
   Lys Asp Ala Ile Leu Ser Ser Leu Glu Ala Arg Asn Gly Asp Ile Leu
               340                 345                 350
   Phe Phe Asp Pro Ile Val Gln Glu Ser Ser Ser Lys Glu Ser Pro Leu
           355                 360                 365
   Pro Ser Ser Leu Gln Ala Ser Val Thr Ser Pro Thr Pro Ala Thr Ala
       370                 375                 380
   Ser Pro Leu Val Ile Gln Thr Ser Ala Asn Arg Ser Val Ile Phe Ser
   385                 390                 395                 400
   Ser Glu Arg Leu Ser Glu Glu Glu Lys Thr Pro Asp Asn Leu Thr Ser
                   405                 410                 415
   Gln Leu Gln Gln Pro Ile Glu Leu Lys Ser Gly Arg Leu Val Leu Lys
               420                 425                 430
   Asp Arg Ala Val Leu Ser Ala Pro Ser Leu Ser Gln Asp Pro Gln Ala
           435                 440                 445
   Leu Leu Ile Met Glu Ala Gly Thr Ser Leu Lys Thr Ser Ser Asp Leu
       450                 455                 460
   Lys Leu Ala Thr Leu Ser Ile Pro Leu His Ser Leu Asp Thr Glu Lys
   465                 470                 475                 480
   Ser Val Thr Ile His Ala Pro Asn Leu Ser Ile Gln Lys Ile Phe Leu
                   485                 490                 495
   Ser Asn Ser Gly Asp Glu Asn Phe Tyr Glu Asn Val Glu Leu Leu Ser
               500                 505                 510
   Lys Glu Gln Asn Asn Ile Pro Leu Leu Thr Leu Pro Lys Glu Gln Ser
           515                 520                 525
   His Leu His Leu Pro Asp Gly Asn Leu Ser Ser His Phe Gly Tyr Gln
       530                 535                 540
   Gly Asp Trp Thr Phe Ser Trp Lys Asp Ser Asp Glu Gly His Ser Leu
   545                 550                 555                 560
   Ile Ala Asn Trp Thr Pro Lys Asn Tyr Val Pro His Pro Glu Arg Gln
                   565                 570                 575
   Ser Thr Leu Val Ala Asn Thr Leu Trp Asn Thr Tyr Ser Asp Met Gln
           580                     585                     590
   Ala Val Gln Ser Met Ile Asn Thr Thr Ala His Gly Gly Ala Tyr Leu
       595                     600                     605
   Phe Gly Thr Trp Gly Ser Ala Val Ser Asn Leu Phe Tyr Val His Asp
   610                     615                     620
   Ser Ser Gly Lys Pro Ile Asp Asn Trp His His Arg Ser Leu Gly Tyr
   625                     630                     635                     640
   Leu Phe Gly Ile Ser Thr His Ser Leu Asp Asp His Ser Phe Cys Leu
                   645                     650                     655
   Ala Ala Gly Gln Leu Leu Gly Lys Ser Ser Asp Ser Phe Ile Thr Ser
               660                     665                     670
   Thr Glu Thr Thr Ser Tyr Ile Ala Thr Val Gln Ala Gln Leu Ala Thr
           675                     680                     685
   Ser Leu Met Lys Ile Ser Ala Gln Ala Cys Tyr Asn Glu Ser Ile His
       690                     695                     700
   Glu Leu Lys Thr Lys Tyr Arg Ser Phe Ser Lys Glu Gly Phe Gly Ser
   705                     710                     715                     720
   Trp His Ser Val Ala Val Ser Gly Glu Val Cys Ala Ser Ile Pro Ile
                   725                     730                     735
   Val Ser Asn Gly Ser Gly Leu Phe Ser Ser Phe Ser Ile Phe Ser Lys
               740                     745                     750
   Leu Gln Gly Phe Ser Gly Thr Gln Asp Gly Phe Glu Glu Ser Ser Gly
           755                     760                     765
   Glu Ile Arg Ser Phe Ser Ala Ser Ser Phe Arg Asn Ile Ser Leu Pro
       770                     775                     780
   Ile Gly Ile Thr Phe Glu Lys Lys Ser Gln Lys Thr Arg Thr Tyr Tyr
   785                     790                     795                     800
   Tyr Phe Leu Gly Ala Tyr Ile Gln Asp Leu Lys Arg Asp Val Glu Ser
                   805                     810                     815
   Gly Pro Val Val Leu Leu Lys Asn Ala Val Ser Trp Asp Ala Pro Met
               820                     825                     830
   Ala Asn Leu Asp Ser Arg Ala Tyr Met Phe Arg Leu Thr Asn Gln Arg
           835                     840                     845
   Ala Leu His Arg Leu Gln Thr Leu Leu Asn Val Ser Cys Val Leu Arg
       850                     855                     860
   Gly Gln Ser His Ser Tyr Ser Leu Asp Leu Gly Thr Thr Tyr Arg Phe
   865                     870                     875                     880
<210> 176
   <211> 982
   <212> PRT
   <213> Chlamydia
<220>
   <221> VARIANT
   <222> (1)...(982)
   <223> Xaa = Any Amino Acid
<400> 176
   Met Ile Pro Gln Gly Ile Tyr Asp Gly Glu Thr Leu Thr Val Ser Phe
    1               5                  10                  15
   Pro Tyr Thr Val Ile Gly Asp Pro Ser Gly Thr Thr Val Phe Ser Ala
               20                  25                  30
   Gly Glu Leu Thr Leu Lys Asn Leu Asp Asn Ser Ile Ala Ala Leu Pro
           35                  40                  45
   Leu Ser Cys Phe Gly Asn Leu Leu Gly Ser Phe Thr Val Leu Gly Arg
   50 55 60
   Gly His Ser Leu Thr Phe Glu Asn Ile Arg Thr Ser Thr Asn Gly Ala
   65 70 75 80
   Ala Leu Ser Asn Ser Ala Ala Asp Gly Leu Phe Thr Ile Glu Gly Phe
   85 90 95
   Lys Glu Leu Ser Phe Ser Asn Cys Asn Ser Leu Leu Ala Val Leu Pro
   100 105 110
   Ala Ala Thr Thr Asn Lys Gly Ser Gln Thr Pro Thr Thr Thr Ser Thr
   115 120 125
   Pro Ser Asn Gly Thr Ile Tyr Ser Lys Thr Asp Leu Leu Leu Leu Asn
   130 135 140
   Asn Glu Lys Phe Ser Phe Tyr Ser Asn Leu Val Ser Gly Asp Gly Gly
   145 150 155 160
   Ala Ile Asp Ala Lys Ser Leu Thr Val Gln Gly Ile Ser Lys Leu Cys
   165 170 175
   Val Phe Gln Glu Asn Thr Ala Gln Ala Asp Gly Gly Ala Cys Gln Val
   180 185 190
   Val Thr Ser Phe Ser Ala Met Ala Asn Glu Ala Pro Ile Ala Phe Val
   195 200 205
   Ala Asn Val Ala Gly Val Arg Gly Gly Gly Ile Ala Ala Val Gln Asp
   210 215 220
   Gly Gln Gln Gly Val Ser Ser Ser Thr Ser Thr Glu Asp Pro Val Val
   225 230 235 240
   Ser Phe Ser Arg Asn Thr Ala Val Glu Phe Asp Gly Asn Val Ala Arg
   245 250 255
   Val Gly Gly Gly Ile Tyr Ser Tyr Gly Asn Val Ala Phe Leu Asn Asn
   260 265 270
   Gly Lys Thr Leu Phe Leu Asn Asn Val Ala Ser Pro Val Tyr Ile Ala
   275 280 285
   Ala Lys Gln Pro Thr Ser Gly Gln Ala Ser Asn Thr Ser Asn Asn Tyr
   290 295 300
   Gly Asp Gly Gly Ala Ile Phe Cys Lys Asn Gly Ala Gln Ala Gly Ser
   305 310 315 320
   Asn Asn Ser Gly Ser Val Ser Phe Asp Gly Glu Gly Val Val Phe Phe
   325 330 335
   Ser Ser Asn Val Ala Ala Gly Lys Gly Gly Ala Ile Tyr Ala Lys Lys
   340 345 350
   Leu Ser Val Ala Asn Cys Gly Pro Val Gln Phe Leu Arg Asn Ile Ala
   355 360 365
   Asn Asp Gly Gly Ala Ile Tyr Leu Gly Glu Ser Gly Glu Leu Ser Leu
   370 375 380
   Ser Ala Asp Tyr Gly Asp Ile Ile Phe Asp Gly Asn Leu Lys Arg Thr
   385 390 395 400
   Ala Lys Glu Asn Ala Ala Asp Val Asn Gly Val Thr Val Ser Ser Gln
   405 410 415
   Ala Ile Ser Met Gly Ser Gly Gly Lys Ile Thr Thr Leu Arg Ala Lys
   420 425 430
   Ala Gly His Gln Ile Leu Phe Asn Asp Pro Ile Glu Met Ala Asn Gly
   435 440 445
   Asn Asn Gln Pro Ala Gln Ser Ser Lys Leu Leu Lys Ile Asn Asp Gly
   450 455 460
   Glu Gly Tyr Thr Gly Asp Ile Val Phe Ala Asn Gly Ser Ser Thr Leu
   465 470 475 480
   Tyr Gln Asn Val Thr Ile Glu Gln Gly Arg Ile Val Leu Arg Glu Lys
                   485                 490                 495
   Ala Lys Leu Ser Val Asn Ser Leu Ser Gln Thr Gly Gly Ser Leu Tyr
               500                 505                 510
   Met Glu Ala Gly Ser Thr Leu Asp Phe Val Thr Pro Gln Pro Pro Gln
           515                 520                 525
   Gln Pro Pro Ala Ala Asn Gln Leu Ile Thr Leu Ser Asn Leu His Leu
       530                 535                 540
   Ser Leu Ser Ser Leu Leu Ala Asn Asn Ala Val Thr Asn Pro Pro Thr
   545                 550                 555                 560
   Asn Pro Pro Ala Gln Asp Ser His Pro Ala Val Ile Gly Ser Thr Thr
                   565                 570                 575
   Ala Gly Ser Val Thr Ile Ser Gly Pro Ile Phe Phe Glu Asp Leu Asp
               580                 585                 590
   Asp Thr Ala Tyr Asp Arg Tyr Asp Trp Leu Gly Ser Asn Gln Lys Ile
           595                 600                 605
   Asn Val Leu Lys Leu Gln Leu Gly Thr Lys Pro Pro Ala Asn Ala Pro
       610                 615                 620
   Ser Asp Leu Thr Leu Gly Asn Glu Met Pro Lys Tyr Gly Tyr Gln Gly
   625                 630                 635                 640
   Ser Trp Lys Leu Ala Trp Asp Pro Asn Thr Ala Asn Asn Gly Pro Tyr
                   645                 650                 655
   Thr Leu Lys Ala Thr Trp Thr Lys Thr Gly Tyr Asn Pro Gly Pro Glu
               660                 665                 670
   Arg Val Ala Ser Leu Val Pro Asn Ser Leu Trp Gly Ser Ile Leu Asp
           675                 680                 685
   Ile Arg Ser Ala His Ser Ala Ile Gln Ala Ser Val Asp Gly Arg Ser
       690                 695                 700
   Tyr Cys Arg Gly Leu Trp Val Ser Gly Val Ser Asn Phe Phe Tyr His
   705                 710                 715                 720
   Asp Arg Asp Ala Leu Gly Gln Gly Tyr Arg Tyr Ile Ser Gly Gly Tyr
                   725                 730                 735
   Ser Leu Gly Ala Asn Ser Tyr Phe Gly Ser Ser Met Phe Gly Leu Ala
               740                 745                 750
   Phe Thr Glu Val Phe Gly Arg Ser Lys Asp Tyr Val Val Cys Arg Ser
           755                 760                 765
   Asn His His Ala Cys Ile Gly Ser Val Tyr Leu Ser Thr Gln Gln Ala
       770                 775                 780
   Leu Cys Gly Ser Tyr Leu Phe Gly Asp Ala Phe Ile Arg Ala Ser Tyr
   785                 790                 795                 800
   Gly Phe Gly Asn Gln His Met Lys Thr Ser Tyr Thr Phe Ala Glu Glu
                   805                 810                 815
   Ser Asp Val Arg Trp Asp Asn Asn Cys Leu Ala Gly Glu Ile Gly Ala
               820                 825                 830
   Gly Leu Pro Ile Val Ile Thr Pro Ser Lys Leu Tyr Leu Asn Glu Leu
           835                 840                 845
   Arg Pro Phe Val Gln Ala Glu Phe Ser Tyr Ala Asp His Glu Ser Phe
       850                 855                 860
   Thr Glu Glu Gly Asp Gln Ala Arg Ala Phe Lys Ser Gly His Leu Leu
   865                 870                 875                 880
   Asn Leu Ser Val Pro Val Gly Val Lys Phe Asp Arg Cys Ser Ser Thr
                   885                 890                 895
   His Pro Asn Lys Tyr Ser Phe Met Ala Ala Tyr Ile Cys Asp Ala Tyr
               900                 905                 910
   Arg Thr Ile Ser Gly Thr Glu Thr Thr Leu Leu Ser His Gln Glu Thr
           915                 920                 925
   Trp Thr Thr Asp Ala Phe His Leu Ala Arg His Gly Val Val Val Arg
   930 935 940
   Gly Ser Met Tyr Ala Ser Leu Thr Ser Asn Ile Glu Val Tyr Gly His
   945 950 955 960
   Gly Arg Tyr Glu Tyr Arg Asp Ala Ser Arg Gly Tyr Gly Leu Ser Ala
   965 970 975
   Gly Ser Lys Val Xaa Phe
   980
<210> 177
   <211> 964
   <212> PRT
   <213> Chlamydia
<400> 177
   Met Lys Lys Ala Phe Phe Phe Phe Leu Ile Gly Asn Ser Leu Ser Gly
   1               5                   10                  15
   Leu Ala Arg Glu Val Pro Ser Arg Ile Phe Leu Met Pro Asn Ser Val
               20                  25                  30
   Pro Asp Pro Thr Lys Glu Ser Leu Ser Asn Lys Ile Ser Leu Thr Gly
           35                  40                  45
   Asp Thr His Asn Leu Thr Asn Cys Tyr Leu Asp Asn Leu Arg Tyr Ile
       50                  55                  60
   Leu Ala Ile Leu Gln Lys Thr Pro Asn Glu Gly Ala Ala Val Thr Ile
   65                  70                  75                  80
   Thr Asp Tyr Leu Ser Phe Phe Asp Thr Gln Lys Glu Gly Ile Tyr Phe
                   85                  90                  95
   Ala Lys Asn Leu Thr Pro Glu Ser Gly Gly Ala Ile Gly Tyr Ala Ser
               100                 105                 110
   Pro Asn Ser Pro Thr Val Glu Ile Arg Asp Thr Ile Gly Pro Val Ile
           115                 120                 125
   Phe Glu Asn Asn Thr Cys Cys Arg Leu Phe Thr Trp Arg Asn Pro Tyr
       130                 135                 140
   Ala Ala Asp Lys Ile Arg Glu Gly Gly Ala Ile His Ala Gln Asn Leu
   145                 150                 155                 160
   Tyr Ile Asn His Asn His Asp Val Val Gly Phe Met Lys Asn Phe Ser
                   165                 170                 175
   Tyr Val Gln Gly Gly Ala Ile Ser Thr Ala Asn Thr Phe Val Val Ser
               180                 185                 190
   Glu Asn Gln Ser Cys Phe Leu Phe Met Asp Asn Ile Cys Ile Gln Thr
           195                 200                 205
   Asn Thr Ala Gly Lys Gly Gly Ala Ile Tyr Ala Gly Thr Ser Asn Ser
       210                 215                 220
   Phe Glu Ser Asn Asn Cys Asp Leu Phe Phe Ile Asn Asn Ala Cys Cys
   225                 230                 235                 240
   Ala Gly Gly Ala Ile Phe Ser Pro Ile Cys Ser Leu Thr Gly Asn Arg
                   245                 250                 255
   Gly Asn Ile Val Phe Tyr Asn Asn Arg Cys Phe Lys Asn Val Glu Thr
               260                 265                 270
   Ala Ser Ser Glu Ala Ser Asp Gly Gly Ala Ile Lys Val Thr Thr Arg
           275                 280                 285
   Leu Asp Val Thr Gly Asn Arg Gly Arg Ile Phe Phe Ser Asp Asn Ile
       290                 295                 300
   Thr Lys Asn Tyr Gly Gly Ala Ile Tyr Ala Pro Val Val Thr Leu Val
   305                 310                 315                 320
   Asp Asn Gly Pro Thr Tyr Phe Ile Asn Asn Ile Ala Asn Asn Lys Gly
   325 330 335
   Gly Ala Ile Tyr Ile Asp Gly Thr Ser Asn Ser Lys Ile Ser Ala Asp
   340 345 350
   Arg His Ala Ile Ile Phe Asn Glu Asn Ile Val Thr Asn Val Thr Asn
   355 360 365
   Ala Asn Gly Thr Ser Thr Ser Ala Asn Pro Pro Arg Arg Asn Ala Ile
   370 375 380
   Thr Val Ala Ser Ser Ser Gly Glu Ile Leu Leu Gly Ala Gly Ser Ser
   385 390 395 400
   Gln Asn Leu Ile Phe Tyr Asp Pro Ile Glu Val Ser Asn Ala Gly Val
   405 410 415
   Ser Val Ser Phe Asn Lys Glu Ala Asp Gln Thr Gly Ser Val Val Phe
   420 425 430
   Ser Gly Ala Thr Val Asn Ser Ala Asp Phe His Gln Arg Asn Leu Gln
   435 440 445
   Thr Lys Thr Pro Ala Pro Leu Thr Leu Ser Asn Gly Phe Leu Cys Ile
   450 455 460
   Glu Asp His Ala Gln Leu Thr Val Asn Arg Phe Thr Gln Thr Gly Gly
   465 470 475 480
   Val Val Ser Leu Gly Asn Gly Ala Val Leu Ser Cys Tyr Lys Asn Gly
   485 490 495
   Thr Gly Asp Ser Ala Ser Asn Ala Ser Ile Thr Leu Lys His Ile Gly
   500 505 510
   Leu Asn Leu Ser Ser Ile Leu Lys Ser Gly Ala Glu Ile Pro Leu Leu
   515 520 525
   Trp Val Glu Pro Thr Asn Asn Ser Asn Asn Tyr Thr Ala Asp Thr Ala
   530 535 540
   Ala Thr Phe Ser Leu Ser Asp Val Lys Leu Ser Leu Ile Asp Asp Tyr
   545 550 555 560
   Gly Asn Ser Pro Tyr Glu Ser Thr Asp Leu Thr His Ala Leu Ser Ser
   565 570 575
   Gln Pro Met Leu Ser Ile Ser Glu Ala Ser Asp Asn Gln Leu Gln Ser
   580 585 590
   Glu Asn Ile Asp Phe Ser Gly Leu Asn Val Pro His Tyr Gly Trp Gln
   595 600 605
   Gly Leu Trp Thr Trp Gly Trp Ala Lys Thr Gln Asp Pro Glu Pro Ala
   610 615 620
   Ser Ser Ala Thr Ile Thr Asp Pro Gln Lys Ala Asn Arg Phe His Arg
   625 630 635 640
   Thr Leu Leu Leu Thr Trp Leu Pro Ala Gly Tyr Val Pro Ser Pro Lys
   645 650 655
   His Arg Ser Pro Leu Ile Ala Asn Thr Leu Trp Gly Asn Met Leu Leu
   660 665 670
   Ala Thr Glu Ser Leu Lys Asn Ser Ala Glu Leu Thr Pro Ser Gly His
   675 680 685
   Pro Phe Trp Gly Ile Thr Gly Gly Gly Leu Gly Met Met Val Tyr Gln
   690 695 700
   Asp Pro Arg Glu Asn His Pro Gly Phe His Met Arg Ser Ser Gly Tyr
   705 710 715 720
   Ser Ala Gly Met Ile Ala Gly Gln Thr His Thr Phe Ser Leu Lys Phe
   725 730 735
   Ser Gln Thr Tyr Thr Lys Leu Asn Glu Arg Tyr Ala Lys Asn Asn Val
   740 745 750
   Ser Ser Lys Asn Tyr Ser Cys Gln Gly Glu Met Leu Phe Ser Leu Gln
   755 760 765
   Glu Gly Phe Leu Leu Thr Lys Leu Val Gly Leu Tyr Ser Tyr Gly Asp
   770 775 780
   His Asn Cys His His Phe Tyr Thr Gln Gly Glu Asn Leu Thr Ser Gln
   785 790 795 800
   Gly Thr Phe Arg Ser Gln Thr Met Gly Gly Ala Val Phe Phe Asp Leu
   805 810 815
   Pro Met Lys Pro Phe Gly Ser Thr His Ile Leu Thr Ala Pro Phe Leu
   820 825 830
   Gly Ala Leu Gly Ile Tyr Ser Ser Leu Ser His Phe Thr Glu Val Gly
   835 840 845
   Ala Tyr Pro Arg Ser Phe Ser Thr Lys Thr Pro Leu Ile Asn Val Leu
   850 855 860
   Val Pro Ile Gly Val Lys Gly Ser Phe Met Asn Ala Thr His Arg Pro
   865 870 875 880
   Gln Ala Trp Thr Val Glu Leu Ala Tyr Gln Pro Val Leu Tyr Arg Gln
   885 890 895
   Glu Pro Gly Ile Ala Thr Gln Leu Leu Ala Ser Lys Gly Ile Trp Phe
   900 905 910
   Gly Ser Gly Ser Pro Ser Ser Arg His Ala Met Ser Tyr Lys Ile Ser
   915 920 925
   Gln Gln Thr Gln Pro Leu Ser Trp Leu Thr Leu His Phe Gln Tyr His
   930 935 940
   Gly Phe Tyr Ser Ser Ser Thr Phe Cys Asn Tyr Leu Asn Gly Glu Ile
   945 950 955 960
   Ala Leu Arg Phe
<210> 178
   <211> 1530
   <212> PRT
   <213> Chlamydia
<400> 178
   Met Ser Ser Glu Lys Asp Ile Lys Ser Thr Cys Ser Lys Phe Ser Leu
    1               5                  10                  15
   Ser Val Val Ala Ala Ile Leu Ala Ser Val Ser Gly Leu Ala Ser Cys
               20                  25                  30
   Val Asp Leu His Ala Gly Gly Gln Ser Val Asn Glu Leu Val Tyr Val
           35                  40                  45
   Gly Pro Gln Ala Val Leu Leu Leu Asp Gln Ile Arg Asp Leu Phe Val
       50                  55                  60
   Gly Ser Lys Asp Ser Gln Ala Glu Gly Gln Tyr Arg Leu Ile Val Gly
   65                  70                  75                  80
   Asp Pro Ser Ser Phe Gln Glu Lys Asp Ala Asp Thr Leu Pro Gly Lys
                   85                  90                  95
   Val Glu Gln Ser Thr Leu Phe Ser Val Thr Asn Pro Val Val Phe Gln
               100                 105                 110
   Gly Val Asp Gln Gln Asp Gln Val Ser Ser Gln Gly Leu Ile Cys Ser
           115                 120                 125
   Phe Thr Ser Ser Asn Leu Asp Ser Pro Arg Asp Gly Glu Ser Phe Leu
       130                 135                 140
   Gly Ile Ala Phe Val Gly Asp Ser Ser Lys Ala Gly Ile Thr Leu Thr
   145                 150                 155                 160
   Asp Val Lys Ala Ser Leu Ser Gly Ala Ala Leu Tyr Ser Thr Glu Asp
                   165                 170                 175
   Leu Ile Phe Glu Lys Ile Lys Gly Gly Leu Glu Phe Ala Ser Cys Ser
               180                 185                 190
   Ser Leu Glu Gln Gly Gly Ala Cys Ala Ala Gln Ser Ile Leu Ile His
           195                 200                 205
   Asp Cys Gln Gly Leu Gln Val Lys His Cys Thr Thr Ala Val Asn Ala
       210                 215                 220
   Glu Gly Ser Ser Ala Asn Asp His Leu Gly Phe Gly Gly Gly Ala Phe
   225                 230                 235                 240
   Phe Val Thr Gly Ser Leu Ser Gly Glu Lys Ser Leu Tyr Met Pro Ala
                   245                 250                 255
   Gly Asp Met Val Val Ala Asn Cys Asp Gly Ala Ile Ser Phe Glu Gly
               260                 265                 270
   Asn Ser Ala Asn Phe Ala Asn Gly Gly Ala Ile Ala Ala Ser Gly Lys
           275                 280                 285
   Val Leu Phe Val Ala Asn Asp Lys Lys Thr Ser Phe Ile Glu Asn Arg
       290                 295                 300
   Ala Leu Ser Gly Gly Ala Ile Ala Ala Ser Ser Asp Ile Ala Phe Gln
   305                 310                 315                 320
   Asn Cys Ala Glu Leu Val Phe Lys Gly Asn Cys Ala Ile Gly Thr Glu
                   325                 330                 335
   Asp Lys Gly Ser Leu Gly Gly Gly Ala Ile Ser Ser Leu Gly Thr Val
               340                 345                 350
   Leu Leu Gln Gly Asn His Gly Ile Thr Cys Asp Lys Asn Glu Ser Ala
           355                 360                 365
   Ser Gln Gly Gly Ala Ile Phe Gly Lys Asn Cys Gln Ile Ser Asp Asn
       370                 375                 380
   Glu Gly Pro Val Val Phe Arg Asp Ser Thr Ala Cys Leu Gly Gly Gly
   385                 390                 395                 400
   Ala Ile Ala Ala Gln Glu Ile Val Ser Ile Gln Asn Asn Gln Ala Gly
                   405                 410                 415
   Ile Ser Phe Glu Gly Gly Lys Ala Ser Phe Gly Gly Gly Ile Ala Cys
               420                 425                 430
   Gly Ser Phe Ser Ser Ala Gly Gly Ala Ser Val Leu Gly Thr Ile Asp
           435                 440                 445
   Ile Ser Lys Asn Leu Gly Ala Ile Ser Phe Ser Arg Thr Leu Cys Thr
       450                 455                 460
   Thr Ser Asp Leu Gly Gln Met Glu Tyr Gln Gly Gly Gly Ala Leu Phe
   465                 470                 475                 480
   Gly Glu Asn Ile Ser Leu Ser Glu Asn Ala Gly Val Leu Thr Phe Lys
                   485                 490                 495
   Asp Asn Ile Val Lys Thr Phe Ala Ser Asn Gly Lys Ile Leu Gly Gly
               500                 505                 510
   Gly Ala Ile Leu Ala Thr Gly Lys Val Glu Ile Thr Asn Asn Ser Gly
           515                 520                 525
   Gly Ile Ser Phe Thr Gly Asn Ala Arg Ala Pro Gln Ala Leu Pro Thr
       530                 535                 540
   Gln Glu Glu Phe Pro Leu Phe Ser Lys Lys Glu Gly Arg Pro Leu Ser
   545                 550                 555                 560
   Ser Gly Tyr Ser Gly Gly Gly Ala Ile Leu Gly Arg Glu Val Ala Ile
                   565                 570                 575
   Leu His Asn Ala Ala Val Val Phe Glu Gln Asn Arg Leu Gln Cys Ser
               580                 585                 590
   Glu Glu Glu Ala Thr Leu Leu Gly Cys Cys Gly Gly Gly Ala Val His
           595                 600                 605
   Gly Met Asp Ser Thr Ser Ile Val Gly Asn Ser Ser Val Arg Phe Gly
   610 615 620
   Asn Asn Tyr Ala Met Gly Gln Gly Val Ser Gly Gly Ala Leu Leu Ser
   625 630 635 640
   Lys Thr Val Gln Leu Ala Gly Asn Gly Ser Val Asp Phe Ser Arg Asn
   645 650 655
   Ile Ala Ser Leu Gly Gly Gly Ala Leu Gln Ala Ser Glu Gly Asn Cys
   660 665 670
   Glu Leu Val Asp Asn Gly Tyr Val Leu Phe Arg Asp Asn Arg Gly Arg
   675 680 685
   Val Tyr Gly Gly Ala Ile Ser Cys Leu Arg Gly Asp Val Val Ile Ser
   690 695 700
   Gly Asn Lys Gly Arg Val Glu Phe Lys Asp Asn Ile Ala Thr Arg Leu
   705 710 715 720
   Tyr Val Glu Glu Thr Val Glu Lys Val Glu Glu Val Glu Pro Ala Pro
   725 730 735
   Glu Gln Lys Asp Asn Asn Glu Leu Ser Phe Leu Gly Ser Val Glu Gln
   740 745 750
   Ser Phe Ile Thr Ala Ala Asn Gln Ala Leu Phe Ala Ser Glu Asp Gly
   755 760 765
   Asp Leu Ser Pro Glu Ser Ser Ile Ser Ser Glu Glu Leu Ala Lys Arg
   770 775 780
   Arg Glu Cys Ala Gly Gly Ala Ile Phe Ala Lys Arg Val Arg Ile Val
   785 790 795 800
   Asp Asn Gln Glu Ala Val Val Phe Ser Asn Asn Phe Ser Asp Ile Tyr
   805 810 815
   Gly Gly Ala Ile Phe Thr Gly Ser Leu Arg Glu Glu Asp Lys Leu Asp
   820 825 830
   Gly Gln Ile Pro Glu Val Leu Ile Ser Gly Asn Ala Gly Asp Val Val
   835 840 845
   Phe Ser Gly Asn Ser Ser Lys Arg Asp Glu His Leu Pro His Thr Gly
   850 855 860
   Gly Gly Ala Ile Cys Thr Gln Asn Leu Thr Ile Ser Gln Asn Thr Gly
   865 870 875 880
   Asn Val Leu Phe Tyr Asn Asn Val Ala Cys Ser Gly Gly Ala Val Arg
   885 890 895
   Ile Glu Asp His Gly Asn Val Leu Leu Glu Ala Phe Gly Gly Asp Ile
   900 905 910
   Val Phe Lys Gly Asn Ser Ser Phe Arg Ala Gln Gly Ser Asp Ala Ile
   915 920 925
   Tyr Phe Ala Gly Lys Glu Ser His Ile Thr Ala Leu Asn Ala Thr Glu
   930 935 940
   Gly His Ala Ile Val Phe His Asp Ala Leu Val Phe Glu Asn Leu Lys
   945 950 955 960
   Glu Arg Lys Ser Ala Glu Val Leu Leu Ile Asn Ser Arg Glu Asn Pro
   965 970 975
   Gly Tyr Thr Gly Ser Ile Arg Phe Leu Glu Ala Glu Ser Lys Val Pro
   980 985 990
   Gln Cys Ile His Val Gln Gln Gly Ser Leu Glu Leu Leu Asn Gly Ala
   995 1000 1005
   Thr Leu Cys Ser Tyr Gly Phe Lys Gln Asp Ala Gly Ala Lys Leu Val
   1010 1015 1020
   Leu Ala Ala Gly Ser Lys Leu Lys Ile Leu Asp Ser Gly Thr Pro Val
   1025 1030 1035 1040
   Gln Gly His Ala Ile Ser Lys Pro Glu Ala Glu Ile Glu Ser Ser Ser
   1045 1050 1055
   Glu Pro Glu Gly Ala His Ser Leu Trp Ile Ala Lys Asn Ala Gln Thr
   1060 1065 1070
   Thr Val Pro Met Val Asp Ile His Thr Ile Ser Val Asp Leu Ala Ser
   1075 1080 1085
   Phe Ser Ser Ser Gln Gln Glu Gly Thr Val Glu Ala Pro Gln Val Ile
   1090 1095 1100
   Val Pro Gly Gly Ser Tyr Val Arg Ser Gly Glu Leu Asn Leu Glu Leu
   1105 1110 1115 1120
   Val Asn Thr Thr Gly Thr Gly Tyr Glu Asn His Ala Leu Leu Lys Asn
   1125 1130 1135
   Glu Ala Lys Val Pro Leu Met Ser Phe Val Ala Ser Ser Asp Glu Ala
   1140 1145 1150
   Ser Ala Glu Ile Ser Asn Leu Ser Val Ser Asp Leu Gln Ile His Val
   1155 1160 1165
   Ala Thr Pro Glu Ile Glu Glu Asp Thr Tyr Gly His Met Gly Asp Trp
   1170 1175 1180
   Ser Glu Ala Lys Ile Gln Asp Gly Thr Leu Val Ile Asn Trp Asn Pro
   1185 1190 1195 1200
   Thr Gly Tyr Arg Leu Asp Pro Gln Lys Ala Gly Ala Leu Val Phe Asn
   1205 1210 1215
   Ala Leu Trp Glu Glu Gly Ala Val Leu Ser Ala Leu Lys Asn Ala Arg
   1220 1225 1230
   Phe Ala His Asn Leu Thr Ala Gln Arg Met Glu Phe Asp Tyr Ser Thr
   1235 1240 1245
   Asn Val Trp Gly Phe Ala Phe Gly Gly Phe Arg Thr Leu Ser Ala Glu
   1250 1255 1260
   Asn Leu Val Ala Ile Asp Gly Tyr Lys Gly Ala Tyr Gly Gly Ala Ser
   1265 1270 1275 1280
   Ala Gly Val Asp Ile Gln Leu Met Glu Asp Phe Val Leu Gly Val Ser
   1285 1290 1295
   Gly Ala Ala Phe Leu Gly Lys Met Asp Ser Gln Lys Phe Asp Ala Glu
   1300 1305 1310
   Val Ser Arg Lys Gly Val Val Gly Ser Val Tyr Thr Gly Phe Leu Ala
   1315 1320 1325
   Gly Ser Trp Phe Phe Lys Gly Gln Tyr Ser Leu Gly Glu Thr Gln Asn
   1330 1335 1340
   Asp Met Lys Thr Arg Tyr Gly Val Leu Gly Glu Ser Ser Ala Ser Trp
   1345 1350 1355 1360
   Thr Ser Arg Gly Val Leu Ala Asp Ala Leu Val Glu Tyr Arg Ser Leu
   1365 1370 1375
   Val Gly Pro Val Arg Pro Thr Phe Tyr Ala Leu His Phe Asn Pro Tyr
   1380 1385 1390
   Val Glu Val Ser Tyr Ala Ser Met Lys Phe Pro Gly Phe Thr Glu Gln
   1395 1400 1405
   Gly Arg Glu Ala Arg Ser Phe Glu Asp Ala Ser Leu Thr Asn Ile Thr
   1410 1415 1420
   Ile Pro Leu Gly Met Lys Phe Glu Leu Ala Phe Ile Lys Gly Gln Phe
   1425 1430 1435 1440
   Ser Glu Val Asn Ser Leu Gly Ile Ser Tyr Ala Trp Glu Ala Tyr Arg
   1445 1450 1455
   Lys Val Glu Gly Gly Ala Val Gln Leu Leu Glu Ala Gly Phe Asp Trp
   1460 1465 1470
   Glu Gly Ala Pro Met Asp Leu Pro Arg Gln Glu Leu Arg Val Ala Leu
   1475 1480 1485
   Glu Asn Asn Thr Glu Trp Ser Ser Tyr Phe Ser Thr Val Leu Gly Leu
   1490 1495 1500
   Thr Ala Phe Cys Gly Gly Phe Thr Ser Thr Asp Ser Lys Leu Gly Tyr
   1505 1510 1515 1520
   Glu Ala Asn Thr Gly Leu Arg Leu Ile Phe
   1525 1530
<210> 179
   <211> 1776
   <212> PRT
   <213> Chlamydia
<400> 179
   Ala Ile Met Lys Phe Met Ser Ala Thr Ala Val Phe Ala Ala Val Leu
   1               5                   10                  15
   Ser Ser Val Thr Glu Ala Ser Ser Ile Gln Asp Gln Ile Lys Asn Thr
               20                  25                  30
   Asp Cys Asn Val Ser Lys Val Gly Tyr Ser Thr Ser Gln Ala Phe Thr
           35                  40                  45
   Asp Met Met Leu Ala Asp Asn Thr Glu Tyr Arg Ala Ala Asp Ser Val
       50                  55                  60
   Ser Phe Tyr Asp Phe Ser Thr Ser Ser Gly Leu Pro Arg Lys His Leu
   65                  70                  75                  80
   Ser Ser Ser Ser Glu Ala Ser Pro Thr Thr Glu Gly Val Ser Ser Ser
                   85                  90                  95
   Ser Ser Gly Glu Asn Thr Glu Asn Ser Gln Asp Ser Ala Pro Ser Ser
               100                 105                 110
   Gly Glu Thr Asp Lys Lys Thr Glu Glu Glu Leu Asp Asn Gly Gly Ile
           115                 120                 125
   Ile Tyr Ala Arg Glu Lys Leu Thr Ile Ser Glu Ser Gln Asp Ser Leu
       130                 135                 140
   Ser Asn Pro Ser Ile Glu Leu His Asp Asn Ser Phe Phe Phe Gly Glu
   145                 150                 155                 160
   Gly Glu Val Ile Phe Asp His Arg Val Ala Leu Lys Asn Gly Gly Ala
                   165                 170                 175
   Ile Tyr Gly Glu Lys Glu Val Val Phe Glu Asn Ile Lys Ser Leu Leu
               180                 185                 190
   Val Glu Val Asn Ile Ser Val Glu Lys Gly Gly Ser Val Tyr Ala Lys
           195                 200                 205
   Glu Arg Val Ser Leu Glu Asn Val Thr Glu Ala Thr Phe Ser Ser Asn
       210                 215                 220
   Gly Gly Glu Gln Gly Gly Gly Gly Ile Tyr Ser Glu Gln Asp Met Leu
   225                 230                 235                 240
   Ile Ser Asp Cys Asn Asn Val His Phe Gln Gly Asn Ala Ala Gly Ala
                   245                 250                 255
   Thr Ala Val Lys Gln Cys Leu Asp Glu Glu Met Ile Val Leu Leu Thr
               260                 265                 270
   Glu Cys Val Asp Ser Leu Ser Glu Asp Thr Leu Asp Ser Thr Pro Glu
           275                 280                 285
   Thr Glu Gln Thr Lys Ser Asn Gly Asn Gln Asp Gly Ser Ser Glu Thr
       290                 295                 300
   Lys Asp Thr Gln Val Ser Glu Ser Pro Glu Ser Thr Pro Ser Pro Asp
   305                 310                 315                 320
   Asp Val Leu Gly Lys Gly Gly Gly Ile Tyr Thr Glu Lys Ser Leu Thr
                   325                 330                 335
   Ile Thr Gly Ile Thr Gly Thr Ile Asp Phe Val Ser Asn Ile Ala Thr
   340 345 350
   Asp Ser Gly Ala Gly Val Phe Thr Lys Glu Asn Leu Ser Cys Thr Asn
   355 360 365
   Thr Asn Ser Leu Gln Phe Leu Lys Asn Ser Ala Gly Gln His Gly Gly
   370 375 380
   Gly Ala Tyr Val Thr Gln Thr Met Ser Val Thr Asn Thr Thr Ser Glu
   385 390 395 400
   Ser Ile Thr Thr Pro Pro Leu Val Gly Glu Val Ile Phe Ser Glu Asn
   405 410 415
   Thr Ala Lys Gly His Gly Gly Gly Ile Cys Thr Asn Lys Leu Ser Leu
   420 425 430
   Ser Asn Leu Lys Thr Val Thr Leu Thr Lys Asn Ser Ala Lys Glu Ser
   435 440 445
   Gly Gly Ala Ile Phe Thr Asp Leu Ala Ser Ile Pro Thr Thr Asp Thr
   450 455 460
   Pro Glu Ser Ser Thr Pro Ser Ser Ser Ser Pro Ala Ser Thr Pro Glu
   465 470 475 480
   Val Val Ala Ser Ala Lys Ile Asn Arg Phe Phe Ala Ser Thr Ala Glu
   485 490 495
   Pro Ala Ala Pro Ser Leu Thr Glu Ala Glu Ser Asp Gln Thr Asp Gln
   500 505 510
   Thr Glu Thr Ser Asp Thr Asn Ser Asp Ile Asp Val Ser Ile Glu Asn
   515 520 525
   Ile Leu Asn Val Ala Ile Asn Gln Asn Thr Ser Ala Lys Lys Gly Gly
   530 535 540
   Ala Ile Tyr Gly Lys Lys Ala Lys Leu Ser Arg Ile Asn Asn Leu Glu
   545 550 555 560
   Leu Ser Gly Asn Ser Ser Gln Asp Val Gly Gly Gly Leu Cys Leu Thr
   565 570 575
   Glu Ser Val Glu Phe Asp Ala Ile Gly Ser Leu Leu Ser His Tyr Asn
   580 585 590
   Ser Ala Ala Lys Glu Gly Gly Val Ile His Ser Lys Thr Val Thr Leu
   595 600 605
   Ser Asn Leu Lys Ser Thr Phe Thr Phe Ala Asp Asn Thr Val Lys Ala
   610 615 620
   Ile Val Glu Ser Thr Pro Glu Ala Pro Glu Glu Ile Pro Pro Val Glu
   625 630 635 640
   Gly Glu Glu Ser Thr Ala Thr Glu Asn Pro Asn Ser Asn Thr Glu Gly
   645 650 655
   Ser Ser Ala Asn Thr Asn Leu Glu Gly Ser Gln Gly Asp Thr Ala Asp
   660 665 670
   Thr Gly Thr Gly Val Val Asn Asn Glu Ser Gln Asp Thr Ser Asp Thr
   675 680 685
   Gly Asn Ala Glu Ser Gly Glu Gln Leu Gln Asp Ser Thr Gln Ser Asn
   690 695 700
   Glu Glu Asn Thr Leu Pro Asn Ser Ser Ile Asp Gln Ser Asn Glu Asn
   705 710 715 720
   Thr Asp Glu Ser Ser Asp Ser His Thr Glu Glu Ile Thr Asp Glu Ser
   725 730 735
   Val Ser Ser Ser Ser Lys Ser Gly Ser Ser Thr Pro Gln Asp Gly Gly
   740 745 750
   Ala Ala Ser Ser Gly Ala Pro Ser Gly Asp Gln Ser Ile Ser Ala Asn
   755 760 765
   Ala Cys Leu Ala Lys Ser Tyr Ala Ala Ser Thr Asp Ser Ser Pro Val
       770                 775                 780
   Ser Asn Ser Ser Gly Ser Asp Val Thr Ala Ser Ser Asp Asn Pro Asp
   785                 790                 795                 800
   Ser Ser Ser Ser Gly Asp Ser Ala Gly Asp Ser Glu Gly Pro Thr Glu
                   805                 810                 815
   Pro Glu Ala Gly Ser Thr Thr Glu Thr Pro Thr Leu Ile Gly Gly Gly
               820                 825                 830
   Ala Ile Tyr Gly Glu Thr Val Lys Ile Glu Asn Phe Ser Gly Gln Gly
           835                 840                 845
   Ile Phe Ser Gly Asn Lys Ala Ile Asp Asn Thr Thr Glu Gly Ser Ser
       850                 855                 860
   Ser Lys Ser Asn Val Leu Gly Gly Ala Val Tyr Ala Lys Thr Leu Phe
   865                 870                 875                 880
   Asn Leu Asp Ser Gly Ser Ser Arg Arg Thr Val Thr Phe Ser Gly Asn
                   885                 890                 895
   Thr Val Ser Ser Gln Ser Thr Thr Gly Gln Val Ala Gly Gly Ala Ile
               900                 905                 910
   Tyr Ser Pro Thr Val Thr Ile Ala Thr Pro Val Val Phe Ser Lys Asn
           915                 920                 925
   Ser Ala Thr Asn Asn Ala Asn Asn Ala Thr Asp Thr Gln Arg Lys Asp
       930                 935                 940
   Thr Phe Gly Gly Ala Ile Gly Ala Thr Ser Ala Val Ser Leu Ser Gly
   945                 950                 955                 960
   Gly Ala His Phe Leu Glu Asn Val Ala Asp Leu Gly Ser Ala Ile Gly
                   965                 970                 975
   Leu Val Pro Asp Thr Gln Asn Thr Glu Thr Val Lys Leu Glu Ser Gly
               980                 985                 990
   Ser Tyr Tyr Phe Glu Lys Asn Lys Ala Leu Lys Arg Ala Thr Ile Tyr
           995                 1000                1005
   Ala Pro Val Val Ser Ile Lys Ala Tyr Thr Ala Thr Phe Asn Gln Asn
       1010                1015                1020
   Arg Ser Leu Glu Glu Gly Ser Ala Ile Tyr Phe Thr Lys Glu Ala Ser
   1025                1030                1035                1040
   Ile Glu Ser Leu Gly Ser Val Leu Phe Thr Gly Asn Leu Val Thr Pro
                   1045                1050                1055
   Thr Leu Ser Thr Thr Thr Glu Gly Thr Pro Ala Thr Thr Ser Gly Asp
               1060                1065                1070
   Val Thr Lys Tyr Gly Ala Ala Ile Phe Gly Gln Ile Ala Ser Ser Asn
           1075                1080                1085
   Gly Ser Gln Thr Asp Asn Leu Pro Leu Lys Leu Ile Ala Ser Gly Gly
       1090                1095                1100
   Asn Ile Cys Phe Arg Asn Asn Glu Tyr Arg Pro Thr Ser Ser Asp Thr
   1105                1110                1115                1120
   Gly Thr Ser Thr Phe Cys Ser Ile Ala Gly Asp Val Lys Leu Thr Met
                   1125                1130                1135
   Gln Ala Ala Lys Gly Lys Thr Ile Ser Phe Phe Asp Ala Ile Arg Thr
               1140                1145                1150
   Ser Thr Lys Lys Thr Gly Thr Gln Ala Thr Ala Tyr Asp Thr Leu Asp
           1155                1160                1165
   Ile Asn Lys Ser Glu Asp Ser Glu Thr Val Asn Ser Ala Phe Thr Gly
       1170                1175                1180
   Thr Ile Leu Phe Ser Ser Glu Leu His Glu Asn Lys Ser Tyr Ile Pro
   1185                1190                1195                1200
   Gln Asn Val Val Leu His Ser Gly Ser Leu Val Leu Lys Pro Asn Thr
                   1205                1210                1215
   Glu Leu His Val Ile Ser Phe Glu Gln Lys Glu Gly Ser Ser Leu Val
   1220 1225 1230
   Met Thr Pro Gly Ser Val Leu Ser Asn Gln Thr Val Ala Asp Gly Ala
   1235 1240 1245
   Leu Val Ile Asn Asn Met Thr Ile Asp Leu Ser Ser Val Glu Lys Asn
   1250 1255 1260
   Gly Ile Ala Glu Gly Asn Ile Phe Thr Pro Pro Glu Leu Arg Ile Ile
   1265 1270 1275 1280
   Asp Thr Thr Thr Ser Gly Ser Gly Gly Thr Pro Ser Thr Asp Ser Glu
   1285 1290 1295
   Ser Asn Gln Asn Ser Asp Asp Thr Lys Glu Gln Asn Asn Asn Asp Ala
   1300 1305 1310
   Ser Asn Gln Gly Glu Ser Ala Asn Gly Ser Ser Ser Pro Ala Val Ala
   1315 1320 1325
   Ala Ala His Thr Ser Arg Thr Arg Asn Phe Ala Ala Ala Ala Thr Ala
   1330 1335 1340
   Thr Pro Thr Thr Thr Pro Thr Ala Thr Thr Thr Thr Ser Asn Gln Val
   1345 1350 1355 1360
   Ile Leu Gly Gly Glu Ile Lys Leu Ile Asp Pro Asn Gly Thr Phe Phe
   1365 1370 1375
   Gln Asn Pro Ala Leu Arg Ser Asp Gln Gln Ile Ser Leu Leu Val Leu
   1380 1385 1390
   Pro Thr Asp Ser Ser Lys Met Gln Ala Gln Lys Ile Val Leu Thr Gly
   1395 1400 1405
   Asp Ile Ala Pro Gln Lys Gly Tyr Thr Gly Thr Leu Thr Leu Asp Pro
   1410 1415 1420
   Asp Gln Leu Gln Asn Gly Thr Ile Ser Ala Leu Trp Lys Phe Asp Ser
   1425 1430 1435 1440
   Tyr Arg Gln Trp Ala Tyr Val Pro Arg Asp Asn His Phe Tyr Ala Asn
   1445 1450 1455
   Ser Ile Leu Gly Ser Gln Met Ser Met Val Thr Val Lys Gln Gly Leu
   1460 1465 1470
   Leu Asn Asp Lys Met Asn Leu Ala Arg Phe Asp Glu Val Ser Tyr Asn
   1475 1480 1485
   Asn Leu Trp Ile Ser Gly Leu Gly Thr Met Leu Ser Gln Val Gly Thr
   1490 1495 1500
   Pro Thr Ser Glu Glu Phe Thr Tyr Tyr Ser Arg Gly Ala Ser Val Ala
   1505 1510 1515 1520
   Leu Asp Ala Lys Pro Ala His Asp Val Ile Val Gly Ala Ala Phe Ser
   1525 1530 1535
   Lys Met Ile Gly Lys Thr Lys Ser Leu Lys Arg Glu Asn Asn Tyr Thr
   1540 1545 1550
   His Lys Gly Ser Glu Tyr Ser Tyr Gln Ala Ser Val Tyr Gly Gly Lys
   1555 1560 1565
   Pro Phe His Phe Val Ile Asn Lys Lys Thr Glu Lys Ser Leu Pro Leu
   1570 1575 1580
   Leu Leu Gln Gly Val Ile Ser Tyr Gly Tyr Ile Lys His Asp Thr Val
   1585 1590 1595 1600
   Thr His Tyr Pro Thr Ile Arg Glu Arg Asn Gln Gly Glu Trp Glu Asp
   1605 1610 1615
   Leu Gly Trp Leu Thr Ala Leu Arg Val Ser Ser Val Leu Arg Thr Pro
   1620 1625 1630
   Ala Gln Gly Asp Thr Lys Arg Ile Thr Val Tyr Gly Glu Leu Glu Tyr
   1635 1640 1645
   Ser Ser Ile Arg Gln Lys Gln Phe Thr Glu Thr Glu Tyr Asp Pro Arg
   1650 1655 1660
   Tyr Phe Asp Asn Cys Thr Tyr Arg Asn Leu Ala Ile Pro Met Gly Leu
   1665 1670 1675 1680
   Ala Phe Glu Gly Glu Leu Ser Gly Asn Asp Ile Leu Met Tyr Asn Arg
   1685 1690 1695
   Phe Ser Val Ala Tyr Met Pro Ser Ile Tyr Arg Asn Ser Pro Thr Cys
   1700 1705 1710
   Lys Tyr Gln Val Leu Ser Ser Gly Glu Gly Gly Glu Ile Ile Cys Gly
   1715 1720 1725
   Val Pro Thr Arg Asn Ser Ala Arg Gly Glu Tyr Ser Thr Gln Leu Tyr
   1730 1735 1740
   Pro Gly Pro Leu Trp Thr Leu Tyr Gly Ser Tyr Thr Ile Glu Ala Asp
   1745 1750 1755 1760
   Ala His Thr Leu Ala His Met Met Asn Cys Gly Ala Arg Met Thr Phe
   1765 1770 1775
<210> 180
   <211> 1752
   <212> PRT
   <213> Chlamydia
<400> 180
   Met Lys Trp Leu Ser Ala Thr Ala Val Phe Ala Ala Val Leu Pro Ser
   1 5 10 15
   Val Ser Gly Phe Cys Phe Pro Glu Pro Lys Glu Leu Asn Phe Ser Arg
   20 25 30
   Val Glu Thr Ser Ser Ser Thr Thr Phe Thr Glu Thr Ile Gly Glu Ala
   35 40 45
   Gly Ala Glu Tyr Ile Val Ser Gly Asn Ala Ser Phe Thr Lys Phe Thr
   50 55 60
   Asn Ile Pro Thr Thr Asp Thr Thr Thr Pro Thr Asn Ser Asn Ser Ser
   65 70 75 80
   Ser Ser Ser Gly Glu Thr Ala Ser Val Ser Glu Asp Ser Asp Ser Thr
   85 90 95
   Thr Thr Thr Pro Asp Pro Lys Gly Gly Gly Ala Phe Tyr Asn Ala His
   100 105 110
   Ser Gly Val Leu Ser Phe Met Thr Arg Ser Gly Thr Glu Gly Ser Leu
   115 120 125
   Thr Leu Ser Glu Ile Lys Met Thr Gly Glu Gly Gly Ala Ile Phe Ser
   130 135 140
   Gln Gly Glu Leu Leu Phe Thr Asp Leu Thr Ser Leu Thr Ile Gln Asn
   145 150 155 160
   Asn Leu Ser Gln Leu Ser Gly Gly Ala Ile Phe Gly Gly Ser Thr Ile
   165 170 175
   Ser Leu Ser Gly Ile Thr Lys Ala Thr Phe Ser Cys Asn Ser Ala Glu
   180 185 190
   Val Pro Ala Pro Val Lys Lys Pro Thr Glu Pro Lys Ala Gln Thr Ala
   195 200 205
   Ser Glu Thr Ser Gly Ser Ser Ser Ser Ser Gly Asn Asp Ser Val Ser
   210 215 220
   Ser Pro Ser Ser Ser Arg Ala Glu Pro Ala Ala Ala Asn Leu Gln Ser
   225 230 235 240
   His Phe Ile Cys Ala Thr Ala Thr Pro Ala Ala Gln Thr Asp Thr Glu
   245 250 255
   Thr Ser Thr Pro Ser His Lys Pro Gly Ser Gly Gly Ala Ile Tyr Ala
                   260                 265                 270
   Lys Gly Asp Leu Thr Ile Ala Asp Ser Gln Glu Val Leu Phe Ser Ile
               275                 280                 285
   Asn Lys Ala Thr Lys Asp Gly Gly Ala Ile Phe Ala Glu Lys Asp Val
       290                 295                 300
   Ser Phe Glu Asn Ile Thr Ser Leu Lys Val Gln Thr Asn Gly Ala Glu
   305                 310                 315                 320
   Glu Lys Gly Gly Ala Ile Tyr Ala Lys Gly Asp Leu Ser Ile Gln Ser
                   325                 330                 335
   Ser Lys Gln Ser Leu Phe Asn Ser Asn Tyr Ser Lys Gln Gly Gly Gly
               340                 345                 350
   Ala Leu Tyr Val Glu Gly Gly Ile Asn Phe Gln Asp Leu Glu Glu Ile
           355                 360                 365
   Arg Ile Lys Tyr Asn Lys Ala Gly Thr Phe Glu Thr Lys Lys Ile Thr
       370                 375                 380
   Leu Pro Ser Leu Lys Ala Gln Ala Ser Ala Gly Asn Ala Asp Ala Trp
   385                 390                 395                 400
   Ala Ser Ser Ser Pro Gln Ser Gly Ser Gly Ala Thr Thr Val Ser Asp
                   405                 410                 415
   Ser Gly Asp Ser Ser Ser Gly Ser Asp Ser Asp Thr Ser Glu Thr Val
               420                 425                 430
   Pro Val Thr Ala Lys Gly Gly Gly Leu Tyr Thr Asp Lys Asn Leu Ser
           435                 440                 445
   Ile Thr Asn Ile Thr Gly Ile Ile Glu Ile Ala Asn Asn Lys Ala Thr
       450                 455                 460
   Asp Val Gly Gly Gly Ala Tyr Val Lys Gly Thr Leu Thr Cys Glu Asn
   465                 470                 475                 480
   Ser His Arg Leu Gln Phe Leu Lys Asn Ser Ser Asp Lys Gln Gly Gly
                   485                 490                 495
   Gly Ile Tyr Gly Glu Asp Asn Ile Thr Leu Ser Asn Leu Thr Gly Lys
               500                 505                 510
   Thr Leu Phe Gln Glu Asn Thr Ala Lys Glu Glu Gly Gly Gly Leu Phe
           515                 520                 525
   Ile Lys Gly Thr Asp Lys Ala Leu Thr Met Thr Gly Leu Asp Ser Phe
       530                 535                 540
   Cys Leu Ile Asn Asn Thr Ser Glu Lys His Gly Gly Gly Ala Phe Val
   545                 550                 555                 560
   Thr Lys Glu Ile Ser Gln Thr Tyr Thr Ser Asp Val Glu Thr Ile Pro
                   565                 570                 575
   Gly Ile Thr Pro Val His Gly Glu Thr Val Ile Thr Gly Asn Lys Ser
               580                 585                 590
   Thr Gly Gly Asn Gly Gly Gly Val Cys Thr Lys Arg Leu Ala Leu Ser
           595                 600                 605
   Asn Leu Gln Ser Ile Ser Ile Ser Gly Asn Ser Ala Ala Glu Asn Gly
       610                 615                 620
   Gly Gly Ala His Thr Cys Pro Asp Ser Phe Pro Thr Ala Asp Thr Ala
   625                 630                 635                 640
   Glu Gln Pro Ala Ala Ala Ser Ala Ala Thr Ser Thr Pro Lys Ser Ala
                   645                 650                 655
   Pro Val Ser Thr Ala Leu Ser Thr Pro Ser Ser Ser Thr Val Ser Ser
               660                 665                 670
   Leu Thr Leu Leu Ala Ala Ser Ser Gln Ala Ser Pro Ala Thr Ser Asn
           675                 680                 685
   Lys Glu Thr Gln Asp Pro Asn Ala Asp Thr Asp Leu Leu Ile Asp Tyr
       690                 695                 700
   Val Val Asp Thr Thr Ile Ser Lys Asn Thr Ala Lys Lys Gly Gly Gly
   705                 710                 715                 720
   Ile Tyr Ala Lys Lys Ala Lys Met Ser Arg Ile Asp Gln Leu Asn Ile
                   725                 730                 735
   Ser Glu Asn Ser Ala Thr Glu Ile Gly Gly Gly Ile Cys Cys Lys Glu
               740                 745                 750
   Ser Leu Glu Leu Asp Ala Leu Val Ser Leu Ser Val Thr Glu Asn Leu
           755                 760                 765
   Val Gly Lys Glu Gly Gly Gly Leu His Ala Lys Thr Val Asn Ile Ser
       770                 775                 780
   Asn Leu Lys Ser Gly Phe Ser Phe Ser Asn Asn Lys Ala Asn Ser Ser
   785                 790                 795                 800
   Ser Thr Gly Val Ala Thr Thr Ala Ser Ala Pro Ala Ala Ala Ala Ala
                   805                 810                 815
   Ser Leu Gln Ala Ala Ala Ala Ala Ala Pro Ser Ser Pro Ala Thr Pro
               820                 825                 830
   Thr Tyr Ser Gly Val Val Gly Gly Ala Ile Tyr Gly Glu Lys Val Thr
           835                 840                 845
   Phe Ser Gln Cys Ser Gly Thr Cys Gln Phe Ser Gly Asn Gln Ala Ile
       850                 855                 860
   Asp Asn Asn Pro Ser Gln Ser Ser Leu Asn Val Gln Gly Gly Ala Ile
   865                 870                 875                 880
   Tyr Ala Lys Thr Ser Leu Ser Ile Gly Ser Ser Asp Ala Gly Thr Ser
                   885                 890                 895
   Tyr Ile Phe Ser Gly Asn Ser Val Ser Thr Gly Lys Ser Gln Thr Thr
               900                 905                 910
   Gly Gln Ile Ala Gly Gly Ala Ile Tyr Ser Pro Thr Val Thr Leu Asn
           915                 920                 925
   Cys Pro Ala Thr Phe Ser Asn Asn Thr Ala Ser Ile Ala Thr Pro Lys
       930                 935                 940
   Thr Ser Ser Glu Asp Gly Ser Ser Gly Asn Ser Ile Lys Asp Thr Ile
   945                 950                 955                 960
   Gly Gly Ala Ile Ala Gly Thr Ala Ile Thr Leu Ser Gly Val Ser Arg
                   965                 970                 975
   Phe Ser Gly Asn Thr Ala Asp Leu Gly Ala Ala Ile Gly Thr Leu Ala
               980                 985                 990
   Asn Ala Asn Thr Pro Ser Ala Thr Ser Gly Ser Gln Asn Ser Ile Thr
           995                 1000                1005
   Glu Lys Ile Thr Leu Glu Asn Gly Ser Phe Ile Phe Glu Arg Asn Gln
       1010                1015                1020
   Ala Asn Lys Arg Gly Ala Ile Tyr Ser Pro Ser Val Ser Ile Lys Gly
   1025                1030                1035                1040
   Asn Asn Ile Thr Phe Asn Gln Asn Thr Ser Thr His Asp Gly Ser Ala
                   1045                1050                1055
   Ile Tyr Phe Thr Lys Asp Ala Thr Ile Glu Ser Leu Gly Ser Val Leu
               1060                1065                1070
   Phe Thr Gly Asn Asn Val Thr Ala Thr Gln Ala Ser Ser Ala Thr Ser
           1075                1080                1085
   Gly Gln Asn Thr Asn Thr Ala Asn Tyr Gly Ala Ala Ile Phe Gly Asp
       1090                1095                1100
   Pro Gly Thr Thr Gln Ser Ser Gln Thr Asp Ala Ile Leu Thr Leu Leu
   1105                1110                1115                1120
   Ala Ser Ser Gly Asn Ile Thr Phe Ser Asn Asn Ser Leu Gln Asn Asn
                   1125                1130                1135
   Gln Gly Asp Thr Pro Ala Ser Lys Phe Cys Ser Ile Ala Gly Tyr Val
   1140 1145 1150
   Lys Leu Ser Leu Gln Ala Ala Lys Gly Lys Thr Ile Ser Phe Phe Asp
   1155 1160 1165
   Cys Val His Thr Ser Thr Lys Lys Thr Gly Ser Thr Gln Asn Val Tyr
   1170 1175 1180
   Glu Thr Leu Asp Ile Asn Lys Glu Glu Asn Ser Asn Pro Tyr Thr Gly
   1185 1190 1195 1200
   Thr Ile Val Phe Ser Ser Glu Leu His Glu Asn Lys Ser Tyr Ile Pro
   1205 1210 1215
   Gln Asn Ala Ile Leu His Asn Gly Thr Leu Val Leu Lys Glu Lys Thr
   1220 1225 1230
   Glu Leu His Val Val Ser Phe Glu Gln Lys Glu Gly Ser Lys Leu Ile
   1235 1240 1245
   Met Glu Pro Gly Ala Val Leu Ser Asn Gln Asn Ile Ala Asn Gly Ala
   1250 1255 1260
   Leu Ala Ile Asn Gly Leu Thr Ile Asp Leu Ser Ser Met Gly Thr Pro
   1265 1270 1275 1280
   Gln Ala Gly Glu Ile Phe Ser Pro Pro Glu Leu Arg Ile Val Ala Thr
   1285 1290 1295
   Thr Ser Ser Ala Ser Gly Gly Ser Gly Val Ser Ser Ser Ile Pro Thr
   1300 1305 1310
   Asn Pro Lys Arg Ile Ser Ala Ala Val Pro Ser Gly Ser Ala Ala Thr
   1315 1320 1325
   Thr Pro Thr Met Ser Glu Asn Lys Val Phe Leu Thr Gly Asp Leu Thr
   1330 1335 1340
   Leu Ile Asp Pro Asn Gly Asn Phe Tyr Gln Asn Pro Met Leu Gly Ser
   1345 1350 1355 1360
   Asp Leu Asp Val Pro Leu Ile Lys Leu Pro Thr Asn Thr Ser Asp Val
   1365 1370 1375
   Gln Val Tyr Asp Leu Thr Leu Ser Gly Asp Leu Phe Pro Gln Lys Gly
   1380 1385 1390
   Tyr Met Gly Thr Trp Thr Leu Asp Ser Asn Pro Gln Thr Gly Lys Leu
   1395 1400 1405
   Gln Ala Arg Trp Thr Phe Asp Thr Tyr Arg Arg Trp Val Tyr Ile Pro
   1410 1415 1420
   Arg Asp Asn His Phe Tyr Ala Asn Ser Ile Leu Gly Ser Gln Asn Ser
   1425 1430 1435 1440
   Met Ile Val Val Lys Gln Gly Leu Ile Asn Asn Met Leu Asn Asn Ala
   1445 1450 1455
   Arg Phe Asp Asp Ile Ala Tyr Asn Asn Phe Trp Val Ser Gly Val Gly
   1460 1465 1470
   Thr Phe Leu Ala Gln Gln Gly Thr Pro Leu Ser Glu Glu Phe Ser Tyr
   1475 1480 1485
   Tyr Ser Arg Gly Thr Ser Val Ala Ile Asp Ala Lys Pro Arg Gln Asp
   1490 1495 1500
   Phe Ile Leu Gly Ala Ala Phe Ser Lys Ile Val Gly Lys Thr Lys Ala
   1505 1510 1515 1520
   Ile Lys Lys Met His Asn Tyr Phe His Lys Gly Ser Glu Tyr Ser Tyr
   1525 1530 1535
   Gln Ala Ser Val Tyr Gly Gly Lys Phe Leu Tyr Phe Leu Leu Asn Lys
   1540 1545 1550
   Gln His Gly Trp Ala Leu Pro Phe Leu Ile Gln Gly Val Val Ser Tyr
   1555 1560 1565
   Gly His Ile Lys His Asp Thr Thr Thr Leu Tyr Pro Ser Ile His Glu
   1570 1575 1580
   Arg Asn Lys Gly Asp Trp Glu Asp Leu Gly Trp Leu Ala Asp Leu Arg
   1585 1590 1595 1600
   Ile Ser Met Asp Leu Lys Glu Pro Ser Lys Asp Ser Ser Lys Arg Ile
   1605 1610 1615
   Thr Val Tyr Gly Glu Leu Glu Tyr Ser Ser Ile Arg Gln Lys Gln Phe
   1620 1625 1630
   Thr Glu Ile Asp Tyr Asp Pro Arg His Phe Asp Asp Cys Ala Tyr Arg
   1635 1640 1645
   Asn Leu Ser Leu Pro Val Gly Cys Ala Val Glu Gly Ala Ile Met Asn
   1650 1655 1660
   Cys Asn Ile Leu Met Tyr Asn Lys Leu Ala Leu Ala Tyr Met Pro Ser
   1665 1670 1675 1680
   Ile Tyr Arg Asn Asn Pro Val Cys Lys Tyr Arg Val Leu Ser Ser Asn
   1685 1690 1695
   Glu Ala Gly Gln Val Ile Cys Gly Val Pro Thr Arg Thr Ser Ala Arg
   1700 1705 1710
   Ala Glu Tyr Ser Thr Gln Leu Tyr Leu Gly Pro Phe Trp Thr Leu Tyr
   1715 1720 1725
   Gly Asn Tyr Thr Ile Asp Val Gly Met Tyr Thr Leu Ser Gln Met Thr
   1730 1735 1740
   Ser Cys Gly Ala Arg Met Ile Phe
   1745 1750
<210> 181
   <211> 2601
   <212> DNA
   <213> Chlamydia
<400> 181
   atggctagcc atcaccatca ccatcacctc tttggccagg atcccttagg tgaaaccgcc 60
   ctcctcacta aaaatcctaa tcatgtcgtc tgtacatttt ttgaggactg taccatggag 120
   agcctctttc ctgctctttg tgctcatgca tcacaagacg atcctttgta tgtacttgga 180
   aattcctact gttggttcgt atctaaactc catatcacgg accccaaaga ggctcttttt 240
   aaagaaaaag gagatctttc cattcaaaac tttcgcttcc tttccttcac agattgctct 300
   tccaaggaaa gctctccttc tattattcat caaaagaatg gtcagttatc cttgcgcaat 360
   aatggtagca tgagtttctg tcgaaatcat gctgaaggct ctggaggagc catctctgcg 420
   gatgcctttt ctctacagca caactatctt ttcacagctt ttgaagagaa ttcttctaaa 480
   ggaaatggcg gagccattca ggctcaaacc ttctctttat ctagaaatgt gtcgcctatt 540
   tctttcgccc gtaatcgtgc ggatttaaat ggcggcgcta tttgctgtag taatcttatt 600
   tgttcaggga atgtaaaccc tctctttttc actggaaact ccgccacraa tggaggcsct 660
   atttgttgta tcagcgatct aaacacctca gaaaaaggct ctctctctct tgcttgtaac 720
   caaraaacgc tatttgcaag caattctgct aaagaaaaag gcggggctat ttatgccaag 780
   cacatggtat tgcgttataa cggtcctgtt tccttcatta acaacagcgc taaaataggt 840
   ggagctatcg ccatccagtc cggagggagt ctctctatcc ttgcaggtga aggatctgtt 900
   ctgttccaga ataactccca acgcacctcc gaccaaggtc tagtaagaaa cgccatctac 960
   ttagagaaag atgcgattct ttcttcctta gaagctcgca acggagatat tcttttcttt 1020
   gatcctattg tacaagaaag tagcagcaaa gaatcgcctc ttccctcctc tttgcaagcc 1080
   agcgtgactt ctcccacccc agccaccgca tctcctttag ttattcagac aagtgcaaac 1140
   cgttcagtga ttttctcgag cgaacgtctt tctgaagaag aaaaaactcc tgataacctc 1200
   acttcccaac tacagcagcc tatcgaactg aaatccggac gcttagtttt aaaagatcgc 1260
   gctgtccttt ccgsgccttc tctctctcag gatcctcaag ctctcctcat tatggaagcg 1320
   ggaacttctt taaaaacttc ctytgatttg aagttagsta cgstaagtat tccccttcat 1380
   tccttagata ctgaaaaaag cgtaactatc cacgccccta atctttctat ccaaaagatc 1440
   ttcctctcta actctggaga tgagaatttt tatgaaaatg tagagcttct cagtaaagag 1500
   caaaacaata ttcctctcct tactctccct aaagagcaat ctcatttaca tcttcctgat 1560
   gggaacctct cttctcactt tggatatcaa ggagattgga ctttttcttg gaaagattct     1620
   gatgaagggc attctctgat tgctaattgg acgcctaaaa actatgtgcc tcatccagaa     1680
   cgtcaatcta cactcgttgc gaacactctt tggaacacct attccgatat gcaagctgtg     1740
   cagtcgatga ttaatacaac agcgcacgga ggagcctatc tatttggaac gtggggatct     1800
   gctgtttcta atttattcta tgttcacgac agctctggga aacctatcga taattggcat     1860
   catagaagcc ttggctacct attcggtatc agtactcaca gtttagatga ccattctttc     1920
   tgcttggctg caggacaatt actcgggaaa tcgtccgatt cctttattac gtctacagaa     1980
   acgacctcct atatagctac tgtacaagcg caactcgcta cctctctaat gaaaatctct     2040
   gcacaggcat gctacaatga aagtatccat gagctaaaaa caaaatatcg ctccttctct     2100
   aaagaaggat tcggatcctg gcatagcgtt gcagtatccg gagaagtgtg cgcatcgatt     2160
   cctattgtat ccaatggttc cggactgttc agctccttct ctattttctc taaactgcaa     2220
   ggattttcag gaacacagga cggttttgag gagagttcgg gagagattcg gtccttttct     2280
   gccagctctt tcagaaatat ttcacttcct ataggaataa catttgaaaa aaaatcccaa     2340
   aaaacacgaa cctactatta ctttctagga gcctacatcc aagacctgaa acgtgatgtg     2400
   gaatcgggac ctgtagtgtt actcaaaaat gccgtctcct gggatgctcc tatggcgaac     2460
   ttggattcac gagcctacat gttccggctt acgaatcaaa gagctctaca cagacttcag     2520
   acgctgttaa atgtgtcttg tgtgctgcgt gggcaaagcc atagttactc cctggatctg     2580
   gggaccactt acaggttcta g                                              2601
<210> 182
   <211> 3021
   <212> DNA
   <213> Chlamydia
<400> 182
   atggctagca tgactggtgg acagcaaatg ggtcgggatt caagcttggt accgcatcac     60
   catcaccatc acatgattcc tcaaggaatt tacgatgggg agacgttaac tgtatcattt    120
   ccctatactg ttataggaga tccgagtggg actactgttt tttctgcagg agagttaaca    180
   ttaaaaaatc ttgacaattc tattgcagct ttgcctttaa gttgttttgg gaacttatta    240
   gggagtttta ctgttttagg gagaggacac tcgttgactt tcgagaacat acggacttct    300
   acaaatgggg cagctctaag taatagcgct gctgatggac tgtttactat tgagggtttt    360
   aaagaattat ccttttccaa ttgcaattca ttacttgccg tactgcctgc tgcaacgact    420
   aataagggta gccagactcc gacgacaaca tctacaccgt ctaatggtac tatttattct    480
   aaaacagatc ttttgttact caataatgag aagttctcat tctatagtaa tttagtctct    540
   ggagatgggg gagctataga tgctaagagc ttaacggttc aaggaattag caagctttgt    600
   gtcttccaag aaaatactgc tcaagctgat gggggagctt gtcaagtagt caccagtttc    660
   tctgctatgg ctaacgaggc tcctattgcc tttgtagcga atgttgcagg agtaagaggg    720
   ggagggattg ctgctgttca ggatgggcag cagggagtgt catcatctac ttcaacagaa    780
   gatccagtag taagtttttc cagaaatact gcggtagagt ttgatgggaa cgtagcccga    840
   gtaggaggag ggatttactc ctacgggaac gttgctttcc tgaataatgg aaaaaccttg    900
   tttctcaaca atgttgcttc tcctgtttac attgctgcta agcaaccaac aagtggacag    960
   gcttctaata cgagtaataa ttacggagat ggaggagcta tcttctgtaa gaatggtgcg   1020
   caagcaggat ccaataactc tggatcagtt tcctttgatg gagagggagt agtttctttt   1080
   agtagcaatg tagctgctgg gaaaggggga gctatttatg ccaaaaagct ctcggttgct   1140
   aactgtggcc ctgtacaatt tttaaggaat atcgctaatg atggtggagc gatttattta   1200
   ggagaatctg gagagctcag tttatctgct gattatggag atattatttt cgatgggaat   1260
   cttaaaagaa cagccaaaga gaatgctgcc gatgttaatg gcgtaactgt gtcctcacaa   1320
   gccatttcga tgggatcggg agggaaaata acgacattaa gagctaaagc agggcatcag   1380
   attctcttta atgatcccat cgagatggca aacggaaata accagccagc gcagtcttcc   1440
   aaacttctaa aaattaacga tggtgaagga tacacagggg atattgtttt tgctaatgga   1500
   agcagtactt tgtaccaaaa tgttacgata gagcaaggaa ggattgttct tcgtgaaaag   1560
   gcaaaattat cagtgaattc tctaagtcag acaggtggga gtctgtatat ggaagctggg   1620
   agtacattgg attttgtaac tccacaacca ccacaacagc ctcctgccgc taatcagttg   1680
   atcacgcttt ccaatctgca tttgtctctt tcttctttgt tagcaaacaa tgcagttacg   1740
   aatcctccta ccaatcctcc agcgcaagat tctcatcctg cagtcattgg tagcacaact   1800
   gctggttctg ttacaattag tgggcctatc ttttttgagg atttggatga tacagcttat    1860
   gataggtatg attggctagg ttctaatcaa aaaatcaatg tcctgaaatt acagttaggg   1920
   actaagcccc cagctaatgc cccatcagat ttgactctag ggaatgagat gcctaagtat   1980
   ggctatcaag gaagctggaa gcttgcgtgg gatcctaata cagcaaataa tggtccttat   2040
   actctgaaag ctacatggac taaaactggg tataatcctg ggcctgagcg agtagcttct   2100
   ttggttccaa atagtttatg ggatccattt ttagatatac gatctgcgca ttcagcaatt   2160
   caagcaagtg tggatgggcg ctcttattgt cgaggattat gggtttctgg agtttcgaat   2220
   ttcttctatc atgaccgcga tgctttaggt cagggatatc ggtatattag tgggggttat   2280
   tccttaggag caaactccta ctttggatca tcgatgtttg gtctagcatt taccgaagta   2340
   tttggtagat ctaaagatta tgtagtgtgt cgttccaatc atcatgcttg cataggatcc   2400
   gtttatctat ctacccaaca agctttatgt ggatcctatt tgttcggaga tgcgtttatc   2460
   cgtgctagct acgggtttgg gaatcagcat atgaaaacct catatacatt tgcagaggag   2520
   agcgatgttc gttgggataa taactgtctg gctggagaga ttggagcggg attaccgatt   2580
   gtgattactc catctaagct ctatttgaat gagttgcgtc ctttcgtgca agctgagttt   2640
   tcttatgccg atcatgaatc ttttacagag gaaggcgatc aagctcgggc attcaagagc   2700
   ggacatctcc taaatctatc agttcctgtt ggagtgaagt ttgatcgatg ttctagtaca   2760
   catcctaata aatatagctt tatggcggct tatatctgtg atgcttatcg caccatctct   2820
   ggtactgaga caacgctcct atcccatcaa gagacatgga caacagatgc ctttcattta   2880
   gcaagacatg gagttgtggt tagaggatct atgtatgctt ctctaacaag taatatagaa   2940
   gtatatggcc atggaagata tgagtatcga gatgcttctc gaggctatgg tttgagtgca   3000
   ggaagtaaag tccggttcta a                                             3021
<210> 183
   <211> 2934
   <212> DNA
   <213> Chlamydia

<400> 183
   atggctagca tgactggtgg acagcaaatg ggtcgggatt caagcttggt accgagctcg    60
   gatccacatc accatcacca tcacggacta gctagagagg ttccttctag aatctttctt   120
   atgcccaact cagttccaga tcctacgaaa gagtcgctat caaataaaat tagtttgaca   180
   ggagacactc acaatctcac taactgctat ctcgataacc tacgctacat actggctatt   240
   ctacaaaaaa ctcccaatga aggagctgct gtcacaataa cagattacct aagctttttt   300
   gatacacaaa aagaaggtat ttattttgca aaaaatctca cccctgaaag tggtggtgcg   360
   attggttatg cgagtcccaa ttctcctacc gtggagattc gtgatacaat aggtcctgta   420
   atctttgaaa ataatacttg ttgcagacta tttacatgga gaaatcctta tgctgctgat   480
   aaaataagag aaggcggagc cattcatgct caaaatcttt acataaatca taatcatgat   540
   gtggtcggat ttatgaagaa cttttcttat gtccaaggag gagccattag taccgctaat   600
   acctttgttg tgagcgagaa tcagtcttgt ttctctttta tggacaacat ctgtattcaa   660
   actaatacag caggaaaagg tggcgctatc tatgctggaa cgagcaattc ttttgagagt   720
   aataactgcg atctcttctt catcaataac gcctgttgtg caggaggagc gatcttctcc   780
   cctatctgtt ctctaacagg aaatcgtggt aacatcgttt tctataacaa tcgctgcttt   840
   aaaaatgtag aaacagcttc ttcagaagct tctgatggag gagcaattaa agtaactact   900
   cgcctagatg ttacaggcaa tcgtggtagg atctttttta gtgacaatat cacaaaaaat   960
   tatggcggag ctatttacgc tcctgtagtt accctagtgg ataatggccc tacctacttt  1020
   ataaacaata tcgccaataa taaggggggc gctatctata tagacggaac cagtaactcc  1080
   aaaatttctg ccgaccgcca tgctattatt tttaatgaaa atattgtgac taatgtaact  1140
   aatgcaaatg gtaccagtac gtcagctaat cctcctagaa gaaatgcaat aacagtagca  1200
   agctcctctg gtgaaattct attaggagca gggagtagcc aaaatttaat tttttatgat  1260
   cctattgaag ttagcaatgc aggggtctct gtgtccttca ataaggaagc tgatcaaaca  1320
   ggctctgtag tattttcagg agctactgtt aattctgcag attttcatca acgcaattta  1380
   caaacaaaaa cacctgcacc ccttactctc agtaatggtt ttctatgtat cgaagatcat  1440
   gctcagctta cagtgaatcg attcacacaa actgggggtg ttgtttctct tgggaatgga  1500
   gcagttctga gttgctataa aaatggtaca ggagattctg ctagcaatgc ctctataaca  1560
   ctgaagcata ttggattgaa tctttcttcc attctgaaaa gtggtgctga gattccttta  1620
   ttgtgggtag agcctacaaa taacagcaat aactatacag cagatactgc agctaccttt  1680
   tcattaagtg atgtaaaact ctcactcatt gatgactacg ggaactctcc ttatgaatcc  1740
   acagatctga cccatgctct gtcatcacag cctatgctat ctatttctga agctagcgat  1800
   aaccagctac aatcagaaaa tatagatttt tcgggactaa atgtccctca ttatggatgg  1860
   caaggacttt ggacttgggg ctgggcaaaa actcaagatc cagaaccagc atcttcagca  1920
   acaatcactg atccacaaaa agccaataga tttcatagaa ccttactact aacatggctt  1980
   cctgccgggt atgttcctag cccaaaacac agaagtcccc tcatagctaa caccttatgg  2040
   gggaatatgc tgcttgcaac agaaagctta aaaaatagtg cagagctgac acctagtggt  2100
   catcctttct ggggaattac aggaggagga ctaggcatga tggtttacca agatcctcga  2160
   gaaaatcatc ctggattcca tatgcgctct tccggatact ctgcggggat gatagcaggg  2220
   cagacacaca ccttctcatt gaaattcagt cagacctaca ccaaactcaa tgagcgttac  2280
   gcaaaaaaca acgtatcttc taaaaattac tcatgccaag gagaaatgct cttctcattg  2340
   caagaaggtt tcttgctgac taaattagtt gggctttaca gctatggaga ccataactgt  2400
   caccatttct atactcaagg agaaaatcta acatctcaag ggacgttccg cagtcaaacg  2460
   atgggaggtg ctgtcttttt tgatctccct atgaaaccct ttggatcaac gcatatactg  2520
   acagctccct ttttaggtgc tcttggtatt tattctagcc tgtctcactt tactgaggtg  2580
   ggagcctatc cgcgaagctt ttctacaaag actcctttga tcaatgtcct agtccctatt  2640
   ggagttaaag gtagctttat gaatgctacc cacagacctc aagcctggac tgtagaattg  2700
   gcataccaac ccgttctgta tagacaagaa ccagggatcg cgacccagct cctagccagt  2760
   aaaggtattt ggtttggtag tggaagcccc tcatcgcgtc atgccatgtc ctataaaatc  2820
   tcacagcaaa cacaaccttt gagttggtta actctccatt tccagtatca tggattctac  2880
   tcctcttcaa ccttctgtaa ttatctcaat ggggaaattg ctctgcgatt ctag        2934
<210> 184
   <211> 2547
   <212> DNA
   <213> Chlamydia
<400> 184
   atggctagcc atcaccatca ccatcacggt gctatttctt gcttacgtgg agatgtagtc      60
   atttctggaa acaagggtag agttgaattt aaagacaaca tagcaacacg tctttatgtg     120
   gaagaaactg tagaaaaggt tgaagaggta gagccagctc ctgagcaaaa agacaataat     180
   gagctttctt tcttagggag tgtagaacag agttttatta ctgcagctaa tcaagctctt     240
   ttcgcatctg aagatgggga tttatcacct gagtcatcca tttcttctga agaacttgcg     300
   aaaagaagag agtgtgctgg aggagctatt tttgcaaaac gggttcgtat tgtagataac     360
   caagaggccg ttgtattctc gaataacttc tctgatattt atggcggcgc catttttaca     420
   ggttctcttc gagaagagga taagttagat gggcaaatcc ctgaagtctt gatctcaggc     480
   aatgcagggg atgttgtttt ttccggaaat tcctcgaagc gtgatgagca tcttcctcat     540
   acaggtgggg gagccatttg tactcaaaat ttgacgattt ctcagaatac agggaatgtt     600
   ctgttttata acaacgtggc ctgttcggga ggagctgttc gtatagagga tcatggtaat     660
   gttcttttag aagcttttgg aggagatatt gtttttaaag gaaattcttc tttcagagca     720
   caaggatccg atgctatcta ttttgcaggt aaagaatcgc atattacagc cctgaatgct     780
   acggaaggac atgctattgt tttccacgac gcattagttt ttgaaaatct aaaagaaagg     840
   aaatctgctg aagtattgtt aatcaatagt cgagaaaatc caggttacac tggatctatt     900
   cgatttttag aagcagaaag taaagttcct caatgtattc atgtacaaca aggaagcctt     960
   gagttgctaa atggagctac attatgtagt tatggtttta aacaagatgc tggagctaag    1020
   ttggtattgg ctgctggatc taaactgaag attttagatt caggaactcc tgtacaaggg    1080
   catgctatca gtaaacctga agcagaaatc gagtcatctt ctgaaccaga gggtgcacat    1140
   tctctttgga ttgcgaagaa tgctcaaaca acagttccta tggttgatat ccatactatt    1200
   tctgtagatt tagcctcctt ctcttctagt caacaggagg ggacagtaga agctcctcag    1260
   gttattgttc ctggaggaag ttatgttcga tctggagagc ttaatttgga gttagttaac    1320
   acaacaggta ctggttatga aaatcatgct ttgttgaaga atgaggctaa agttccattg    1380
   atgtctttcg ttgcttctag tgatgaagct tcagccgaaa tcagtaactt gtcggtttct    1440
   gatttacaga ttcatgtagc aactccagag attgaagaag acacatacgg ccatatggga    1500
   gattggtctg aggctaaaat tcaagatgga actcttgtca ttaattggaa tcctactgga    1560
   tatcgattag atcctcaaaa agcaggggct ttagtattta atgcattatg ggaagaaggg    1620
   gctgtcttgt ctgctctgaa aaatgcacgc tttgctcata atctcactgc tcagcgtatg    1680
   gaattcgatt attctacaaa tgtgtgggga ttcgcctttg gtggtttccg aactctatct    1740
   gcagagaatc tggttgctat tgatggatac aaaggagctt atggtggtgc ttctgctgga    1800
   gtcgatattc aattgatgga agattttgtt ctaggagtta gtggagctgc tttcctaggt     1860
   aaaatggata gtcagaagtt tgatgcggag gtttctcgga agggagttgt tggttctgta    1920
   tatacaggat ttttagctgg atcctggttc ttcaaaggac aatatagcct tggagaaaca    1980
   cagaacgata tgaaaacgcg ttatggagta ctaggagagt cgagtgcttc ttggacatct    2040
   cgaggagtac tggcagatgc tttagttgaa taccgaagtt tagttggtcc tgtgagacct    2100
   actttttatg ctttgcattt caatccttat gtcgaagtat cttatgcttc tatgaaattc    2160
   cctggcttta cagaacaagg aagagaagcg cgttcttttg aagacgcttc ccttaccaat    2220
   atcaccattc ctttagggat gaagtttgaa ttggcgttca taaaaggaca gttttcagag    2280
   gtgaactctt tgggaataag ttatgcatgg gaagcttatc gaaaagtaga aggaggcgcg    2340
   gtgcagcttt tagaagctgg gtttgattgg gagggagctc caatggatct tcctagacag    2400
   gagctgcgtg tcgctctgga aaataatacg gaatggagtt cttacttcag cacagtctta    2460
   ggattaacag ctttttgtgg aggatttact tctacagata gtaaactagg atatgaggcg    2520
   aatactggat tgcgattgat cttttaa                                       2547
<210> 185
   <211> 2337
   <212> DNA
   <213> Chlamydia
<400> 185
   atgcatcacc atcaccatca cgggttagct agttgcgtag atcttcatgc tggaggacag      60
   tctgtaaatg agctggtata tgtaggccct caagcggttt tattgttaga ccaaattcga     120
   gatctattcg ttgggtctaa agatagtcag gctgaaggac agtataggtt aattgtagga     180
   gatccaagtt ctttccaaga gaaagatgca gatactcttc ccgggaaggt agagcaaagt     240
   actttgttct cagtaaccaa tcccgtggtt ttccaaggtg tggaccaaca ggatcaagtc     300
   tcttcccaag ggttaatttg tagttttacg agcagcaacc ttgattctcc ccgtgacgga     360
   gaatcttttt taggtattgc ttttgttggg gatagtagta aggctggaat cacattaact     420
   gacgtgaaag cttctttgtc tggagcggct ttatattcta cagaagatct tatctttgaa     480
   aagattaagg gtggattgga atttgcatca tgttcttctc tagaacaggg gggagcttgt     540
   gcagctcaaa gtattttgat tcatgattgt caaggattgc aggttaaaca ctgtactaca     600
   gccgtgaatg ctgaggggtc tagtgcgaat gatcatcttg gatttggagg aggcgctttc     660
   tttgttacgg gttctctttc tggagagaaa agtctctata tgcctgcagg agatatggta     720
   gttgcgaatt gtgatggggc tatatctttt gaaggaaaca gcgcgaactt tgctaatgga     780
   ggagcgattg ctgcctctgg gaaagtgctt tttgtcgcta atgataaaaa gacttctttt     840
   atagagaacc gagctttgtc tggaggagcg attgcagcct cttctgatat tgcctttcaa     900
   aactgcgcag aactagtttt caaaggcaat tgtgcaattg gaacagagga taaaggttct     960
   ttaggtggag gggctatatc ttctctaggc accgttcttt tgcaagggaa tcacgggata    1020
   acttgtgata agaatgagtc tgcttcgcaa ggaggcgcca tttttggcaa aaattgtcag    1080
   atttctgaca acgaggggcc agtggttttc agagatagta cagcttgctt aggaggaggc    1140
   gctattgcag ctcaagaaat tgtttctatt cagaacaatc aggctgggat ttccttcgag    1200
   ggaggtaagg ctagtttcgg aggaggtatt gcgtgtggat ctttttcttc cgcaggcggt    1260
   gcttctgttt tagggactat tgatatttcg aagaatttag gcgcgatttc gttctctcgt    1320
   actttatgta cgacctcaga tttaggacaa atggagtacc agggaggagg agctctattt    1380
   ggtgaaaata tttctctttc tgagaatgct ggtgtgctca cctttaaaga caacattgtg    1440
   aagacttttg cttcgaatgg gaaaattctg ggaggaggag cgattttagc tactggtaag    1500
   gtggaaatta ccaataattc cggaggaatt tcttttacag gaaatgcgag agctccacaa    1560
   gctcttccaa ctcaagagga gtttccttta ttcagcaaaa aagaagggcg accactctct    1620
   tcaggatatt ctgggggagg agcgatttta ggaagagaag tagctattct ccacaacgct    1680
   gcagtagtat ttgagcaaaa tcgtttgcag tgcagcgaag aagaagcgac attattaggt    1740
   tgttgtggag gaggcgctgt tcatgggatg gatagcactt cgattgttgg caactcttca    1800
   gtaagatttg gtaataatta cgcaatggga caaggagtct caggaggagc tcttttatct    1860
   aaaacagtgc agttagctgg aaatggaagc gtcgattttt ctcgaaatat tgctagtttg    1920
   ggaggaggag ctcttcaagc ttctgaagga aattgtgagc tagttgataa cggctatgtg    1980
   ctattcagag ataatcgagg gagggtttat gggggtgcta tttcttgctt acgtggagat    2040
   gtagtcattt ctggaaacaa gggtagagtt gaatttaaag acaacatagc aacacgtctt    2100
   tatgtggaag aaactgtaga aaaggttgaa gaggtagagc cagctcctga gcaaaaagac    2160
   aataatgagc tttctttctt agggagtgta gaacagagtt ttattactgc agctaatcaa    2220
   gctcttttcg catctgaaga tggggattta tcacctgagt catccatttc ttctgaagaa    2280
   cttgcgaaaa gaagagagtg tgctggagga gctgactcga gcagatccgg ctgctaa      2337
<210> 186
   <211> 2847
   <212> DNA
   <213> Chlamydia
<400> 186
   atggctagca tgcatcacca tcaccatcac gttaagattg agaacttctc tggccaagga    60
   atattttctg gaaacaaagc tatcgataac accacagaag gctcctcttc caaatctaac    120
   gtcctcggag gtgcggtcta tgctaaaaca ttgtttaatc tcgatagcgg gagctctaga    180
   cgaactgtca ccttctccgg gaatactgtc tcttctcaat ctacaacagg tcaggttgct    240
   ggaggagcta tctactctcc tactgtaacc attgctactc ctgtagtatt ttctaaaaac    300
   tctgcaacaa acaatgctaa taacgctaca gatactcaga gaaaagacac ctttggagga    360
   gctatcggag ctacttctgc tgtttctcta tcaggagggg ctcatttctt agaaaacgtt    420
   gctgacctcg gatctgctat tgggttggtg ccagacacac aaaatacaga aacagtgaaa    480
   ttagagtctg gctcctacta ctttgaaaaa aataaagctt taaaacgagc tactatttac    540
   gcacctgtcg tttccattaa agcctatact gcgacattta accaaaacag atctctagaa    600
   gaaggaagcg cgatttactt tacaaaagaa gcatctattg agtctttagg ctctgttctc    660
   ttcacaggaa acttagtaac cccaacgcta agcacaacta cagaaggcac accagccaca    720
   acctcaggag atgtaacaaa atatggtgct gctatctttg gacaaatagc aagctcaaac    780
   ggatctcaga cggataacct tcccctgaaa ctcattgctt caggaggaaa tatttgtttc    840
   cgaaacaatg aataccgtcc tacttcttct gataccggaa cctctacttt ctgtagtatt    900
   gcgggagatg ttaaattaac catgcaagct gcaaaaggga aaacgatcag tttctttgat    960
   gcaatccgga cctctactaa gaaaacaggt acacaggcaa ctgcctacga tactctcgat   1020
   attaataaat ctgaggattc agaaactgta aactctgcgt ttacaggaac gattctgttc   1080
   tcctctgaat tacatgaaaa taaatcctat attccacaaa acgtagttct acacagtgga   1140
   tctcttgtat tgaagccaaa taccgagctt catgtcattt cttttgagca gaaagaaggc   1200
   tcttctctcg ttatgacacc tggatctgtt ctttcgaacc agactgttgc tgatggagct   1260
   ttggtcataa ataacatgac cattgattta tccagcgtag agaaaaatgg tattgctgaa   1320
   ggaaatatct ttactcctcc agaattgaga atcatagaca ctactacaag tggaagcggt   1380
   ggaaccccat ctacagatag tgaaagtaac cagaatagtg atgataccaa ggagcaaaat   1440
   aataatgacg cctcgaatca aggagaaagc gcgaatggat cgtcttctcc tgcagtagct   1500
   gctgcacaca catctcgtac aagaaacttt gccgctgcag ctacagccac acctacgaca   1560
   acaccaacgg ctacaactac aacaagcaac caagtaatcc taggaggaga aatcaaactc   1620
   atcgatccta atgggacctt cttccagaac cctgcattaa gatccgacca acaaatctcc   1680
   ttgttagtgc tccctacaga ctcatcaaaa atgcaagctc agaaaatagt actgacgggt   1740
   gatattgctc ctcagaaagg atatacagga acactcactc tggatcctga tcaactacaa   1800
   aatggaacga tctcagcgct ctggaaattt gactcttata gacaatgggc ttatgtacct   1860
   agagacaatc atttctatgc gaactcgatt ctgggatctc aaatgtcaat ggtcacagtc   1920
   aaacaaggct tgctcaacga taaaatgaat ctagctcgct ttgatgaagt tagctataac   1980
   aacctgtgga tatcaggact aggaacgatg ctatcgcaag taggaacacc tacttctgaa   2040
   gaattcactt attacagcag aggagcttct gttgccttag atgctaaacc agcccatgat   2100
   gtgattgttg gagctgcatt tagtaagatg atcgggaaaa caaaatcctt gaaaagagag   2160
   aataactaca ctcacaaagg atccgaatat tcttaccaag catcggtata cggaggcaaa   2220
   ccattccact ttgtaatcaa taaaaaaacg gaaaaatcgc taccgctatt gttacaagga   2280
   gtcatctctt acggatatat caaacatgat acagtgactc actatccaac gatccgtgaa   2340
   cgaaaccaag gagaatggga agacttagga tggctgacag ctctccgtgt ctcctctgtc   2400
   ttaagaactc ctgcacaagg ggatactaaa cgtatcactg tttacggaga attggaatac   2460
   tccagtatcc gtcagaaaca attcacagaa acagaatacg atcctcgtta cttcgacaac   2520
   tgcacctata gaaacttagc aattcctatg gggttagcat tcgaaggaga gctctctggt   2580
   aacgatattt tgatgtacaa cagattctct gtagcataca tgccatcaat ctatcgaaat   2640
   tctccaacat gcaaatacca agtgctctct tcaggagaag gcggagaaat tatttgtgga   2700
   gtaccgacaa gaaactcagc tcgcggagaa tacagcacgc agctgtaccc gggacctttg   2760
   tggactctgt atggatccta cacgatagaa gcagacgcac atacactagc tcatatgatg   2820
   aactgcggtg ctcgtatgac attctaa                                       2847
<210> 187
   <211> 2466
   <212> DNA
   <213> Chlamydia
<400> 187
<210> 188
   <211> 1578
   <212> DNA
   <213> Chlamydia
<400> 188
   atgcatcacc atcaccatca cacggccgcg tccgataact tccagctgtc ccagggtggg      60
   cagggattcg ccattccgat cgggcaggcg atggcgatcg cgggccagat caagcttccc     120
   accgttcata tcgggcctac cgccttcctc ggcttgggtg ttgtcgacaa caacggcaac     180
   ggcgcacgag tccaacgcgt ggtcgggagc gctccggcgg caagtctcgg catctccacc     240
   ggcgacgtga tcaccgcggt cgacggcgct ccgatcaact cggccaccgc gatggcggac     300
   gcgcttaacg ggcatcatcc cggtgacgtc atctcggtga cctggcaaac caagtcgggc     360
   ggcacgcgta cagggaacgt gacattggcc gagggacccc cggccgaatt cccgctagta     420
   cctagaggtt caccgctgcc tgtggggaat ccagctgaac caagtttatt aatcgatggc     480
   actatgtggg aaggtgcttc aggagatcct tgcgatcctt gcgctacttg gtgtgacgcc     540
   attagcatcc gcgcaggata ctacggagat tatgttttcg atcgtgtatt aaaagttgat     600
   gtgaataaaa cttttagcgg catggctgca actcctacgc aggctatagg taacgcaagt     660
   aatactaatc agccagaagc aaatggcaga ccgaacatcg cttacggaag gcatatgcaa     720
   gatgcagagt ggttttcaaa tgcagccttc ctagccttaa acatttggga tcgcttcgac     780
   attttctgca  ccttaggggc atccaatgga tacttcaaag caagttcggc tgcattcaac     840
   ttggttgggt taatagggtt ttcagctgca agctcaatct ctaccgatct tccaatgcaa     900
   cttcctaacg taggcattac ccaaggtgtt gtggaatttt atacagacac atcattttct     960
   tggagcgtag gtgcacgtgg agctttatgg gaatgtggtt gtgcaacttt aggagctgag    1020
   ttccaatacg ctcaatctaa tcctaagatt gagatgctca acgtcacttc aagcccagca    1080
   caatttgtga ttcacaaacc aagaggctat aaaggagcta gctcgaattt tcctttacct    1140
   ataacggctg gaacaacaga agctacagac accaaatcag ctacaattaa ataccatgaa    1200
   tggcaagtag gcctcgccct gtcttacaga ttgaatatgc ttgttccata tattggcgta    1260
   aactggtcaa gagcaacttt tgatgctgat actatccgca ttgctcaacc taaattaaaa    1320
   tcggagattc ttaacattac tacatggaac ccaagcctta taggatcaac cactgctttg    1380
   cccaataata gtggtaagga tgttctatct gatgtcttgc aaattgcttc gattcagatc    1440
   aacaaaatga agtctagaaa agcttgtggt gtagctgttg gtgcaacgtt aatcgacgct    1500
   gacaaatggt caatcactgg tgaagcacgc ttaatcaatg aaagagctgc tcacatgaat    1560
   gcacaattcc gcttctaa                                                  1578
<210> 189
   <211> 866
   <212> PRT
   <213> Chlamydia
<220>
   <221> VARIANT
   <222> (1)...(866)
   <223> Xaa = Any Amino Acid

<400> 189
   Met Ala Ser His His His His His His Leu Phe Gly Gln Asp Pro Leu
    1               5                  10                  15
   Gly Glu Thr Ala Leu Leu Thr Lys Asn Pro Asn His Val Val Cys Thr
               20                  25                  30
   Phe Phe Glu Asp Cys Thr Met Glu Ser Leu Phe Pro Ala Leu Cys Ala
           35                  40                  45
   His Ala Ser Gln Asp Asp Pro Leu Tyr Val Leu Gly Asn Ser Tyr Cys
       50                  55                  60
   Trp Phe Val Ser Lys Leu His Ile Thr Asp Pro Lys Glu Ala Leu Phe
   65                  70                  75                  80
   Lys Glu Lys Gly Asp Leu Ser Ile Gln Asn Phe Arg Phe Leu Ser Phe
                   85                  90                  95
   Thr Asp Cys Ser Ser Lys Glu Ser Ser Pro Ser Ile Ile His Gln Lys
               100                 105                 110
   Asn Gly Gln Leu Ser Leu Arg Asn Asn Gly Ser Met Ser Phe Cys Arg
           115                 120                 125
   Asn His Ala Glu Gly Ser Gly Gly Ala Ile Ser Ala Asp Ala Phe Ser
       130                 135                 140
   Leu Gln His Asn Tyr Leu Phe Thr Ala Phe Glu Glu Asn Ser Ser Lys
   145                 150                 155                 160
   Gly Asn Gly Gly Ala Ile Gln Ala Gln Thr Phe Ser Leu Ser Arg Asn
                   165                 170                 175
   Val Ser Pro Ile Ser Phe Ala Arg Asn Arg Ala Asp Leu Asn Gly Gly
               180                 185                 190
   Ala Ile Cys Cys Ser Asn Leu Ile Cys Ser Gly Asn Val Asn Pro Leu
           195                 200                 205
   Phe Phe Thr Gly Asn Ser Ala Thr Asn Gly Gly Xaa Ile Cys Cys Ile
       210                 215                 220
   Ser Asp Leu Asn Thr Ser Glu Lys Gly Ser Leu Ser Leu Ala Cys Asn
   225                 230                 235                 240
   Gln Xaa Thr Leu Phe Ala Ser Asn Ser Ala Lys Glu Lys Gly Gly Ala
                   245                 250                 255
   Ile Tyr Ala Lys His Met Val Leu Arg Tyr Asn Gly Pro Val Ser Phe
               260                 265                 270
   Ile Asn Asn Ser Ala Lys Ile Gly Gly Ala Ile Ala Ile Gln Ser Gly
           275                 280                 285
   Gly Ser Leu Ser Ile Leu Ala Gly Glu Gly Ser Val Leu Phe Gln Asn
       290                 295                 300
   Asn Ser Gln Arg Thr Ser Asp Gln Gly Leu Val Arg Asn Ala Ile Tyr
   305                 310                 315                 320
   Leu Glu Lys Asp Ala Ile Leu Ser Ser Leu Glu Ala Arg Asn Gly Asp
                   325                 330                 335
   Ile Leu Phe Phe Asp Pro Ile Val Gln Glu Ser Ser Ser Lys Glu Ser
               340                 345                 350
   Pro Leu Pro Ser Ser Leu Gln Ala Ser Val Thr Ser Pro Thr Pro Ala
           355                 360                 365
   Thr Ala Ser Pro Leu Val Ile Gln Thr Ser Ala Asn Arg Ser Val Ile
       370                 375                 380
   Phe Ser Ser Glu Arg Leu Ser Glu Glu Glu Lys Thr Pro Asp Asn Leu
   385                 390                 395                 400
   Thr Ser Gln Leu Gln Gln Pro Ile Glu Leu Lys Ser Gly Arg Leu Val
                   405                 410                 415
   Leu Lys Asp Arg Ala Val Leu Ser Xaa Pro Ser Leu Ser Gln Asp Pro
               420                 425                 430
   Gln Ala Leu Leu Ile Met Glu Ala Gly Thr Ser Leu Lys Thr Ser Xaa
           435                 440                 445
   Asp Leu Lys Leu Xaa Thr Xaa Ser Ile Pro Leu His Ser Leu Asp Thr
       450                 455                 460
   Glu Lys Ser Val Thr Ile His Ala Pro Asn Leu Ser Ile Gln Lys Ile
   465                 470                 475                 480
   Phe Leu Ser Asn Ser Gly Asp Glu Asn Phe Tyr Glu Asn Val Glu Leu
                   485                 490                 495
   Leu Ser Lys Glu Gln Asn Asn Ile Pro Leu Leu Thr Leu Pro Lys Glu
   500 505 510
   Gln Ser His Leu His Leu Pro Asp Gly Asn Leu Ser Ser His Phe Gly
   515 520 525
   Tyr Gln Gly Asp Trp Thr Phe Ser Trp Lys Asp Ser Asp Glu Gly His
   530 535 540
   Ser Leu Ile Ala Asn Trp Thr Pro Lys Asn Tyr Val Pro His Pro Glu
   545 550 555 560
   Arg Gln Ser Thr Leu Val Ala Asn Thr Leu Trp Asn Thr Tyr Ser Asp
   565 570 575
   Met Gln Ala Val Gln Ser Met Ile Asn Thr Thr Ala His Gly Gly Ala
   580 585 590
   Tyr Leu Phe Gly Thr Trp Gly Ser Ala Val Ser Asn Leu Phe Tyr Val
   595 600 605
   His Asp Ser Ser Gly Lys Pro Ile Asp Asn Trp His His Arg Ser Leu
   610 615 620
   Gly Tyr Leu Phe Gly Ile Ser Thr His Ser Leu Asp Asp His Ser Phe
   625 630 635 640
   Cys Leu Ala Ala Gly Gln Leu Leu Gly Lys Ser Ser Asp Ser Phe Ile
   645 650 655
   Thr Ser Thr Glu Thr Thr Ser Tyr Ile Ala Thr Val Gln Ala Gln Leu
   660 665 670
   Ala Thr Ser Leu Met Lys Ile Ser Ala Gln Ala Cys Tyr Asn Glu Ser
   675 680 685
   Ile His Glu Leu Lys Thr Lys Tyr Arg Ser Phe Ser Lys Glu Gly Phe
   690 695 700
   Gly Ser Trp His Ser Val Ala Val Ser Gly Glu Val Cys Ala Ser Ile
   705 710 715 720
   Pro Ile Val Ser Asn Gly Ser Gly Leu Phe Ser Ser Phe Ser Ile Phe
   725 730 735
   Ser Lys Leu Gln Gly Phe Ser Gly Thr Gln Asp Gly Phe Glu Glu Ser
   740 745 750
   Ser Gly Glu Ile Arg Ser Phe Ser Ala Ser Ser Phe Arg Asn Ile Ser
   755 760 765
   Leu Pro Ile Gly Ile Thr Phe Glu Lys Lys Ser Gln Lys Thr Arg Thr
   770 775 780
   Tyr Tyr Tyr Phe Leu Gly Ala Tyr Ile Gln Asp Leu Lys Arg Asp Val
   785 790 795 800
   Glu Ser Gly Pro Val Val Leu Leu Lys Asn Ala Val Ser Trp Asp Ala
   805 810 815
   Pro Met Ala Asn Leu Asp Ser Arg Ala Tyr Met Phe Arg Leu Thr Asn
   820 825 830
   Gln Arg Ala Leu His Arg Leu Gln Thr Leu Leu Asn Val Ser Cys Val
   835 840 845
   Leu Arg Gly Gln Ser His Ser Tyr Ser Leu Asp Leu Gly Thr Thr Tyr
   850 855 860
   Arg Phe
   865
<210> 190
   <211> 1006
   <212> PRT
   <213> Chlamydia
<400> 190
   Met Ala Ser Met Thr Gly Gly Gln Gln Met Gly Arg Asp Ser Ser Leu
   1 5 10 15
   Val Pro His His His His His His Met Ile Pro Gln Gly Ile Tyr Asp
   20 25 30
   Gly Glu Thr Leu Thr Val Ser Phe Pro Tyr Thr Val Ile Gly Asp Pro
   35 40 45
   Ser Gly Thr Thr Val Phe Ser Ala Gly Glu Leu Thr Leu Lys Asn Leu
   50 55 60
   Asp Asn Ser Ile Ala Ala Leu Pro Leu Ser Cys Phe Gly Asn Leu Leu
   65 70 75 80
   Gly Ser Phe Thr Val Leu Gly Arg Gly His Ser Leu Thr Phe Glu Asn
   85 90 95
   Ile Arg Thr Ser Thr Asn Gly Ala Ala Leu Ser Asn Ser Ala Ala Asp
   100 105 110
   Gly Leu Phe Thr Ile Glu Gly Phe Lys Glu Leu Ser Phe Ser Asn Cys
   115 120 125
   Asn Ser Leu Leu Ala Val Leu Pro Ala Ala Thr Thr Asn Lys Gly Ser
   130 135 140
   Gln Thr Pro Thr Thr Thr Ser Thr Pro Ser Asn Gly Thr Ile Tyr Ser
   145 150 155 160
   Lys Thr Asp Leu Leu Leu Leu Asn Asn Glu Lys Phe Ser Phe Tyr Ser
   165 170 175
   Asn Leu Val Ser Gly Asp Gly Gly Ala Ile Asp Ala Lys Ser Leu Thr
   180 185 190
   Val Gln Gly Ile Ser Lys Leu Cys Val Phe Gln Glu Asn Thr Ala Gln
   195 200 205
   Ala Asp Gly Gly Ala Cys Gln Val Val Thr Ser Phe Ser Ala Met Ala
   210 215 220
   Asn Glu Ala Pro Ile Ala Phe Val Ala Asn Val Ala Gly Val Arg Gly
   225 230 235 240
   Gly Gly Ile Ala Ala Val Gln Asp Gly Gln Gln Gly Val Ser Ser Ser
   245 250 255
   Thr Ser Thr Glu Asp Pro Val Val Ser Phe Ser Arg Asn Thr Ala Val
   260 265 270
   Glu Phe Asp Gly Asn Val Ala Arg Val Gly Gly Gly Ile Tyr Ser Tyr
   275 280 285
   Gly Asn Val Ala Phe Leu Asn Asn Gly Lys Thr Leu Phe Leu Asn Asn
   290 295 300
   Val Ala Ser Pro Val Tyr Ile Ala Ala Lys Gln Pro Thr Ser Gly Gln
   305 310 315 320
   Ala Ser Asn Thr Ser Asn Asn Tyr Gly Asp Gly Gly Ala Ile Phe Cys
   325 330 335
   Lys Asn Gly Ala Gln Ala Gly Ser Asn Asn Ser Gly Ser Val Ser Phe
   340 345 350
   Asp Gly Glu Gly Val Val Phe Phe Ser Ser Asn Val Ala Ala Gly Lys
   355 360 365
   Gly Gly Ala Ile Tyr Ala Lys Lys Leu Ser Val Ala Asn Cys Gly Pro
   370 375 380
   Val Gln Phe Leu Arg Asn Ile Ala Asn Asp Gly Gly Ala Ile Tyr Leu
   385 390 395 400
   Gly Glu Ser Gly Glu Leu Ser Leu Ser Ala Asp Tyr Gly Asp Ile Ile
   405 410 415
   Phe Asp Gly Asn Leu Lys Arg Thr Ala Lys Glu Asn Ala Ala Asp Val
   420 425 430
   Asn Gly Val Thr Val Ser Ser Gln Ala Ile Ser Met Gly Ser Gly Gly
   435 440 445
   Lys Ile Thr Thr Leu Arg Ala Lys Ala Gly His Gln Ile Leu Phe Asn
   450 455 460
   Asp Pro Ile Glu Met Ala Asn Gly Asn Asn Gln Pro Ala Gln Ser Ser
   465 470 475 480
   Lys Leu Leu Lys Ile Asn Asp Gly Glu Gly Tyr Thr Gly Asp Ile Val
   485 490 495
   Phe Ala Asn Gly Ser Ser Thr Leu Tyr Gln Asn Val Thr Ile Glu Gln
   500 505 510
   Gly Arg Ile Val Leu Arg Glu Lys Ala Lys Leu Ser Val Asn Ser Leu
   515 520 525
   Ser Gln Thr Gly Gly Ser Leu Tyr Met Glu Ala Gly Ser Thr Leu Asp
   530 535 540
   Phe Val Thr Pro Gln Pro Pro Gln Gln Pro Pro Ala Ala Asn Gln Leu
   545 550 555 560
   Ile Thr Leu Ser Asn Leu His Leu Ser Leu Ser Ser Leu Leu Ala Asn
   565 570 575
   Asn Ala Val Thr Asn Pro Pro Thr Asn Pro Pro Ala Gln Asp Ser His
   580 585 590
   Pro Ala Val Ile Gly Ser Thr Thr Ala Gly Ser Val Thr Ile Ser Gly
   595 600 605
   Pro Ile Phe Phe Glu Asp Leu Asp Asp Thr Ala Tyr Asp Arg Tyr Asp
   610 615 620
   Trp Leu Gly Ser Asn Gln Lys Ile Asn Val Leu Lys Leu Gln Leu Gly
   625 630 635 640
   Thr Lys Pro Pro Ala Asn Ala Pro Ser Asp Leu Thr Leu Gly Asn Glu
   645 650 655
   Met Pro Lys Tyr Gly Tyr Gln Gly Ser Trp Lys Leu Ala Trp Asp Pro
   660 665 670
   Asn Thr Ala Asn Asn Gly Pro Tyr Thr Leu Lys Ala Thr Trp Thr Lys
   675 680 685
   Thr Gly Tyr Asn Pro Gly Pro Glu Arg Val Ala Ser Leu Val Pro Asn
   690 695 700
   Ser Leu Trp Gly Ser Ile Leu Asp Ile Arg Ser Ala His Ser Ala Ile
   705 710 715 720
   Gln Ala Ser Val Asp Gly Arg Ser Tyr Cys Arg Gly Leu Trp Val Ser
   725 730 735
   Gly Val Ser Asn Phe Phe Tyr His Asp Arg Asp Ala Leu Gly Gln Gly
   740 745 750
   Tyr Arg Tyr Ile Ser Gly Gly Tyr Ser Leu Gly Ala Asn Ser Tyr Phe
   755 760 765
   Gly Ser Ser Met Phe Gly Leu Ala Phe Thr Glu Val Phe Gly Arg Ser
   770 775 780
   Lys Asp Tyr Val Val Cys Arg Ser Asn His His Ala Cys Ile Gly Ser
   785 790 795 800
   Val Tyr Leu Ser Thr Gln Gln Ala Leu Cys Gly Ser Tyr Leu Phe Gly
   805 810 815
   Asp Ala Phe Ile Arg Ala Ser Tyr Gly Phe Gly Asn Gln His Met Lys
   820 825 830
   Thr Ser Tyr Thr Phe Ala Glu Glu Ser Asp Val Arg Trp Asp Asn Asn
   835 840 845
   Cys Leu Ala Gly Glu Ile Gly Ala Gly Leu Pro Ile Val Ile Thr Pro
   850 855 860
   Ser Lys Leu Tyr Leu Asn Glu Leu Arg Pro Phe Val Gln Ala Glu Phe
   865 870 875 880
   Ser Tyr Ala Asp His Glu Ser Phe Thr Glu Glu Gly Asp Gln Ala Arg
   885 890 895
   Ala Phe Lys Ser Gly His Leu Leu Asn Leu Ser Val Pro Val Gly Val
   900 905 910
   Lys Phe Asp Arg Cys Ser Ser Thr His Pro Asn Lys Tyr Ser Phe Met
   915 920 925
   Ala Ala Tyr Ile Cys Asp Ala Tyr Arg Thr Ile Ser Gly Thr Glu Thr
   930 935 940
   Thr Leu Leu Ser His Gln Glu Thr Trp Thr Thr Asp Ala Phe His Leu
   945 950 955 960
   Ala Arg His Gly Val Val Val Arg Gly Ser Met Tyr Ala Ser Leu Thr
   965 970 975
   Ser Asn Ile Glu Val Tyr Gly His Gly Arg Tyr Glu Tyr Arg Asp Ala
   980 985 990
   Ser Arg Gly Tyr Gly Leu Ser Ala Gly Ser Lys Val Arg Phe
   995 1000 1005
<210> 191
   <211> 977
   <212> PRT
   <213> Chlamydia
<400> 191
   Met Ala Ser Met Thr Gly Gly Gln Gln Met Gly Arg Asp Ser Ser Leu
    1               5                  10                  15
   Val Pro Ser Ser Asp Pro His His His His His His Gly Leu Ala Arg
               20                  25                  30
   Glu Val Pro Ser Arg Ile Phe Leu Met Pro Asn Ser Val Pro Asp Pro
           35                  40                  45
   Thr Lys Glu Ser Leu Ser Asn Lys Ile Ser Leu Thr Gly Asp Thr His
       50                  55                  60
   Asn Leu Thr Asn Cys Tyr Leu Asp Asn Leu Arg Tyr Ile Leu Ala Ile
   65                  70                  75                  80
   Leu Gln Lys Thr Pro Asn Glu Gly Ala Ala Val Thr Ile Thr Asp Tyr
                   85                  90                  95
   Leu Ser Phe Phe Asp Thr Gln Lys Glu Gly Ile Tyr Phe Ala Lys Asn
               100                 105                 110
   Leu Thr Pro Glu Ser Gly Gly Ala Ile Gly Tyr Ala Ser Pro Asn Ser
           115                 120                 125
   Pro Thr Val Glu Ile Arg Asp Thr Ile Gly Pro Val Ile Phe Glu Asn
       130                 135                 140
   Asn Thr Cys Cys Arg Leu Phe Thr Trp Arg Asn Pro Tyr Ala Ala Asp
   145                 150                 155                 160
   Lys Ile Arg Glu Gly Gly Ala Ile His Ala Gln Asn Leu Tyr Ile Asn
                   165                 170                 175
   His Asn His Asp Val Val Gly Phe Met Lys Asn Phe Ser Tyr Val Gln
               180                 185                 190
   Gly Gly Ala Ile Ser Thr Ala Asn Thr Phe Val Val Ser Glu Asn Gln
           195                 200                 205
   Ser Cys Phe Leu Phe Met Asp Asn Ile Cys Ile Gln Thr Asn Thr Ala
       210                 215                 220
   Gly Lys Gly Gly Ala Ile Tyr Ala Gly Thr Ser Asn Ser Phe Glu Ser
   225                 230                 235                 240
   Asn Asn Cys Asp Leu Phe Phe Ile Asn Asn Ala Cys Cys Ala Gly Gly
                   245                 250                 255
   Ala Ile Phe Ser Pro Ile Cys Ser Leu Thr Gly Asn Arg Gly Asn Ile
               260                 265                 270
   Val Phe Tyr Asn Asn Arg Cys Phe Lys Asn Val Glu Thr Ala Ser Ser
           275                 280                 285
   Glu Ala Ser Asp Gly Gly Ala Ile Lys Val Thr Thr Arg Leu Asp Val
       290                 295                 300
   Thr Gly Asn Arg Gly Arg Ile Phe Phe Ser Asp Asn Ile Thr Lys Asn
   305                 310                 315                 320
   Tyr Gly Gly Ala Ile Tyr Ala Pro Val Val Thr Leu Val Asp Asn Gly
                   325                 330                 335
   Pro Thr Tyr Phe Ile Asn Asn Ile Ala Asn Asn Lys Gly Gly Ala Ile
               340                 345                 350
   Tyr Ile Asp Gly Thr Ser Asn Ser Lys Ile Ser Ala Asp Arg His Ala
           355                 360                 365
   Ile Ile Phe Asn Glu Asn Ile Val Thr Asn Val Thr Asn Ala Asn Gly
       370                 375                 380
   Thr Ser Thr Ser Ala Asn Pro Pro Arg Arg Asn Ala Ile Thr Val Ala
   385                 390                 395                 400
   Ser Ser Ser Gly Glu Ile Leu Leu Gly Ala Gly Ser Ser Gln Asn Leu
                   405                 410                 415
   Ile Phe Tyr Asp Pro Ile Glu Val Ser Asn Ala Gly Val Ser Val Ser
               420                 425                 430
   Phe Asn Lys Glu Ala Asp Gln Thr Gly Ser Val Val Phe Ser Gly Ala
           435                 440                 445
   Thr Val Asn Ser Ala Asp Phe His Gln Arg Asn Leu Gln Thr Lys Thr
       450                 455                 460
   Pro Ala Pro Leu Thr Leu Ser Asn Gly Phe Leu Cys Ile Glu Asp His
   465                 470                 475                 480
   Ala Gln Leu Thr Val Asn Arg Phe Thr Gln Thr Gly Gly Val Val Ser
                   485                 490                 495
   Leu Gly Asn Gly Ala Val Leu Ser Cys Tyr Lys Asn Gly Thr Gly Asp
               500                 505                 510
   Ser Ala Ser Asn Ala Ser Ile Thr Leu Lys His Ile Gly Leu Asn Leu
           515                 520                 525
   Ser Ser Ile Leu Lys Ser Gly Ala Glu Ile Pro Leu Leu Trp Val Glu
       530                 535                 540
   Pro Thr Asn Asn Ser Asn Asn Tyr Thr Ala Asp Thr Ala Ala Thr Phe
   545                 550                 555                 560
   Ser Leu Ser Asp Val Lys Leu Ser Leu Ile Asp Asp Tyr Gly Asn Ser
                   565                 570                 575
   Pro Tyr Glu Ser Thr Asp Leu Thr His Ala Leu Ser Ser Gln Pro Met
               580                 585                 590
   Leu Ser Ile Ser Glu Ala Ser Asp Asn Gln Leu Gln Ser Glu Asn Ile
           595                 600                 605
   Asp Phe Ser Gly Leu Asn Val Pro His Tyr Gly Trp Gln Gly Leu Trp
       610                 615                 620
   Thr Trp Gly Trp Ala Lys Thr Gln Asp Pro Glu Pro Ala Ser Ser Ala
   625                 630                 635                 640
   Thr Ile Thr Asp Pro Gln Lys Ala Asn Arg Phe His Arg Thr Leu Leu
                   645                 650                 655
   Leu Thr Trp Leu Pro Ala Gly Tyr Val Pro Ser Pro Lys His Arg Ser
               660                 665                 670
   Pro Leu Ile Ala Asn Thr Leu Trp Gly Asn Met Leu Leu Ala Thr Glu
           675                 680                 685
   Ser Leu Lys Asn Ser Ala Glu Leu Thr Pro Ser Gly His Pro Phe Trp
       690                 695                 700
   Gly Ile Thr Gly Gly Gly Leu Gly Met Met Val Tyr Gln Asp Pro Arg
   705                 710                 715                 720
   Glu Asn His Pro Gly Phe His Met Arg Ser Ser Gly Tyr Ser Ala Gly
                   725                 730                 735
   Met Ile Ala Gly Gln Thr His Thr Phe Ser Leu Lys Phe Ser Gln Thr
               740                 745                 750
   Tyr Thr Lys Leu Asn Glu Arg Tyr Ala Lys Asn Asn Val Ser Ser Lys
           755                 760                 765
   Asn Tyr Ser Cys Gln Gly Glu Met Leu Phe Ser Leu Gln Glu Gly Phe
       770                 775                 780
   Leu Leu Thr Lys Leu Val Gly Leu Tyr Ser Tyr Gly Asp His Asn Cys
   785                 790                 795                 800
   His His Phe Tyr Thr Gln Gly Glu Asn Leu Thr Ser Gln Gly Thr Phe
                   805                 810                 815
   Arg Ser Gln Thr Met Gly Gly Ala Val Phe Phe Asp Leu Pro Met Lys
               820                 825                 830
   Pro Phe Gly Ser Thr His Ile Leu Thr Ala Pro Phe Leu Gly Ala Leu
           835                 840                 845
   Gly Ile Tyr Ser Ser Leu Ser His Phe Thr Glu Val Gly Ala Tyr Pro
       850                 855                 860
   Arg Ser Phe Ser Thr Lys Thr Pro Leu Ile Asn Val Leu Val Pro Ile
   865                 870                 875                 880
   Gly Val Lys Gly Ser Phe Met Asn Ala Thr His Arg Pro Gln Ala Trp
                   885                 890                 895
   Thr Val Glu Leu Ala Tyr Gln Pro Val Leu Tyr Arg Gln Glu Pro Gly
               900                 905                 910
   Ile Ala Thr Gln Leu Leu Ala Ser Lys Gly Ile Trp Phe Gly Ser Gly
           915                 920                 925
   Ser Pro Ser Ser Arg His Ala Met Ser Tyr Lys Ile Ser Gln Gln Thr
       930                 935                 940
   Gln Pro Leu Ser Trp Leu Thr Leu His Phe Gln Tyr His Gly Phe Tyr
   945                 950                 955                 960
   Ser Ser Ser Thr Phe Cys Asn Tyr Leu Asn Gly Glu Ile Ala Leu Arg
                   965                 970                 975
   Phe
<210> 192
   <211> 848
   <212> PRT
   <213> Chlamydia
<400> 192
   Met Ala Ser His His His His His His Gly Ala Ile Ser Cys Leu Arg
    1               5                  10                  15
   Gly Asp Val Val Ile Ser Gly Asn Lys Gly Arg Val Glu Phe Lys Asp
               20                  25                  30
   Asn Ile Ala Thr Arg Leu Tyr Val Glu Glu Thr Val Glu Lys Val Glu
           35                  40                  45
   Glu Val Glu Pro Ala Pro Glu Gln Lys Asp Asn Asn Glu Leu Ser Phe
       50                  55                  60
   Leu Gly Ser Val Glu Gln Ser Phe Ile Thr Ala Ala Asn Gln Ala Leu
   65                  70                  75                  80
   Phe Ala Ser Glu Asp Gly Asp Leu Ser Pro Glu Ser Ser Ile Ser Ser
                   85                  90                  95
   Glu Glu Leu Ala Lys Arg Arg Glu Cys Ala Gly Gly Ala Ile Phe Ala
               100                 105                 110
   Lys Arg Val Arg Ile Val Asp Asn Gln Glu Ala Val Val Phe Ser Asn
           115                 120                 125
   Asn Phe Ser Asp Ile Tyr Gly Gly Ala Ile Phe Thr Gly Ser Leu Arg
       130                 135                 140
   Glu Glu Asp Lys Leu Asp Gly Gln Ile Pro Glu Val Leu Ile Ser Gly
   145                 150                 155                 160
   Asn Ala Gly Asp Val Val Phe Ser Gly Asn Ser Ser Lys Arg Asp Glu
                   165                 170                 175
   His Leu Pro His Thr Gly Gly Gly Ala Ile Cys Thr Gln Asn Leu Thr
               180                 185                 190
   Ile Ser Gln Asn Thr Gly Asn Val Leu Phe Tyr Asn Asn Val Ala Cys
           195                 200                 205
   Ser Gly Gly Ala Val Arg Ile Glu Asp His Gly Asn Val Leu Leu Glu
       210                 215                 220
   Ala Phe Gly Gly Asp Ile Val Phe Lys Gly Asn Ser Ser Phe Arg Ala
   225                 230                 235                 240
   Gln Gly Ser Asp Ala Ile Tyr Phe Ala Gly Lys Glu Ser His Ile Thr
                   245                 250                 255
   Ala Leu Asn Ala Thr Glu Gly His Ala Ile Val Phe His Asp Ala Leu
               260                 265                 270
   Val Phe Glu Asn Leu Lys Glu Arg Lys Ser Ala Glu Val Leu Leu Ile
           275                 280                 285
   Asn Ser Arg Glu Asn Pro Gly Tyr Thr Gly Ser Ile Arg Phe Leu Glu
       290                 295                 300
   Ala Glu Ser Lys Val Pro Gln Cys Ile His Val Gln Gln Gly Ser Leu
   305                 310                 315                 320
   Glu Leu Leu Asn Gly Ala Thr Leu Cys Ser Tyr Gly Phe Lys Gln Asp
                   325                 330                 335
   Ala Gly Ala Lys Leu Val Leu Ala Ala Gly Ser Lys Leu Lys Ile Leu
               340                 345                 350
   Asp Ser Gly Thr Pro Val Gln Gly His Ala Ile Ser Lys Pro Glu Ala
           355                 360                 365
   Glu Ile Glu Ser Ser Ser Glu Pro Glu Gly Ala His Ser Leu Trp Ile
       370                 375                 380
   Ala Lys Asn Ala Gln Thr Thr Val Pro Met Val Asp Ile His Thr Ile
   385                 390                 395                 400
   Ser Val Asp Leu Ala Ser Phe Ser Ser Ser Gln Gln Glu Gly Thr Val
                   405                 410                 415
   Glu Ala Pro Gln Val Ile Val Pro Gly Gly Ser Tyr Val Arg Ser Gly
               420                 425                 430
   Glu Leu Asn Leu Glu Leu Val Asn Thr Thr Gly Thr Gly Tyr Glu Asn
           435                 440                 445
   His Ala Leu Leu Lys Asn Glu Ala Lys Val Pro Leu Met Ser Phe Val
       450                 455                 460
   Ala Ser Ser Asp Glu Ala Ser Ala Glu Ile Ser Asn Leu Ser Val Ser
   465                 470                 475                 480
   Asp Leu Gln Ile His Val Ala Thr Pro Glu Ile Glu Glu Asp Thr Tyr
                   485                 490                 495
   Gly His Met Gly Asp Trp Ser Glu Ala Lys Ile Gln Asp Gly Thr Leu
               500                 505                 510
   Val Ile Asn Trp Asn Pro Thr Gly Tyr Arg Leu Asp Pro Gln Lys Ala
           515                 520                 525
   Gly Ala Leu Val Phe Asn Ala Leu Trp Glu Glu Gly Ala Val Leu Ser
   530 535 540
   Ala Leu Lys Asn Ala Arg Phe Ala His Asn Leu Thr Ala Gln Arg Met
   545 550 555 560
   Glu Phe Asp Tyr Ser Thr Asn Val Trp Gly Phe Ala Phe Gly Gly Phe
   565 570 575
   Arg Thr Leu Ser Ala Glu Asn Leu Val Ala Ile Asp Gly Tyr Lys Gly
   580 585 590
   Ala Tyr Gly Gly Ala Ser Ala Gly Val Asp Ile Gln Leu Met Glu Asp
   595 600 605
   Phe Val Leu Gly Val Ser Gly Ala Ala Phe Leu Gly Lys Met Asp Ser
   610 615 620
   Gln Lys Phe Asp Ala Glu Val Ser Arg Lys Gly Val Val Gly Ser Val
   625 630 635 640
   Tyr Thr Gly Phe Leu Ala Gly Ser Trp Phe Phe Lys Gly Gln Tyr Ser
   645 650 655
   Leu Gly Glu Thr Gln Asn Asp Met Lys Thr Arg Tyr Gly Val Leu Gly
   660 665 670
   Glu Ser Ser Ala Ser Trp Thr Ser Arg Gly Val Leu Ala Asp Ala Leu
   675 680 685
   Val Glu Tyr Arg Ser Leu Val Gly Pro Val Arg Pro Thr Phe Tyr Ala
   690 695 700
   Leu His Phe Asn Pro Tyr Val Glu Val Ser Tyr Ala Ser Met Lys Phe
   705 710 715 720
   Pro Gly Phe Thr Glu Gln Gly Arg Glu Ala Arg Ser Phe Glu Asp Ala
   725 730 735
   Ser Leu Thr Asn Ile Thr Ile Pro Leu Gly Met Lys Phe Glu Leu Ala
   740 745 750
   Phe Ile Lys Gly Gln Phe Ser Glu Val Asn Ser Leu Gly Ile Ser Tyr
   755 760 765
   Ala Trp Glu Ala Tyr Arg Lys Val Glu Gly Gly Ala Val Gln Leu Leu
   770 775 780
   Glu Ala Gly Phe Asp Trp Glu Gly Ala Pro Met Asp Leu Pro Arg Gln
   785 790 795 800
   Glu Leu Arg Val Ala Leu Glu Asn Asn Thr Glu Trp Ser Ser Tyr Phe
   805 810 815
   Ser Thr Val Leu Gly Leu Thr Ala Phe Cys Gly Gly Phe Thr Ser Thr
   820 825 830
   Asp Ser Lys Leu Gly Tyr Glu Ala Asn Thr Gly Leu Arg Leu Ile Phe
   835 840 845
<210> 193
   <211> 778
   <212> PRT
   <213> Chlamydia
<400> 193
   Met His His His His His His Gly Leu Ala Ser Cys Val Asp Leu His
    1               5                  10                  15
   Ala Gly Gly Gln Ser Val Asn Glu Leu Val Tyr Val Gly Pro Gln Ala
               20                  25                  30
   Val Leu Leu Leu Asp Gln Ile Arg Asp Leu Phe Val Gly Ser Lys Asp
           35                  40                  45
   Ser Gln Ala Glu Gly Gln Tyr Arg Leu Ile Val Gly Asp Pro Ser Ser
       50                  55                  60
   Phe Gln Glu Lys Asp Ala Asp Thr Leu Pro Gly Lys Val Glu Gln Ser
   65 70 75 80
   Thr Leu Phe Ser Val Thr Asn Pro Val Val Phe Gln Gly Val Asp Gln
   85 90 95
   Gln Asp Gln Val Ser Ser Gln Gly Leu Ile Cys Ser Phe Thr Ser Ser
   100 105 110
   Asn Leu Asp Ser Pro Arg Asp Gly Glu Ser Phe Leu Gly Ile Ala Phe
   115 120 125
   Val Gly Asp Ser Ser Lys Ala Gly Ile Thr Leu Thr Asp Val Lys Ala
   130 135 140
   Ser Leu Ser Gly Ala Ala Leu Tyr Ser Thr Glu Asp Leu Ile Phe Glu
   145 150 155 160
   Lys Ile Lys Gly Gly Leu Glu Phe Ala Ser Cys Ser Ser Leu Glu Gln
   165 170 175
   Gly Gly Ala Cys Ala Ala Gln Ser Ile Leu Ile His Asp Cys Gln Gly
   180 185 190
   Leu Gln Val Lys His Cys Thr Thr Ala Val Asn Ala Glu Gly Ser Ser
   195 200 205
   Ala Asn Asp His Leu Gly Phe Gly Gly Gly Ala Phe Phe Val Thr Gly
   210 215 220
   Ser Leu Ser Gly Glu Lys Ser Leu Tyr Met Pro Ala Gly Asp Met Val
   225 230 235 240
   Val Ala Asn Cys Asp Gly Ala Ile Ser Phe Glu Gly Asn Ser Ala Asn
   245 250 255
   Phe Ala Asn Gly Gly Ala Ile Ala Ala Ser Gly Lys Val Leu Phe Val
   260 265 270
   Ala Asn Asp Lys Lys Thr Ser Phe Ile Glu Asn Arg Ala Leu Ser Gly
   275 280 285
   Gly Ala Ile Ala Ala Ser Ser Asp Ile Ala Phe Gln Asn Cys Ala Glu
   290 295 300
   Leu Val Phe Lys Gly Asn Cys Ala Ile Gly Thr Glu Asp Lys Gly Ser
   305 310 315 320
   Leu Gly Gly Gly Ala Ile Ser Ser Leu Gly Thr Val Leu Leu Gln Gly
   325 330 335
   Asn His Gly Ile Thr Cys Asp Lys Asn Glu Ser Ala Ser Gln Gly Gly
   340 345 350
   Ala Ile Phe Gly Lys Asn Cys Gln Ile Ser Asp Asn Glu Gly Pro Val
   355 360 365
   Val Phe Arg Asp Ser Thr Ala Cys Leu Gly Gly Gly Ala Ile Ala Ala
   370 375 380
   Gln Glu Ile Val Ser Ile Gln Asn Asn Gln Ala Gly Ile Ser Phe Glu
   385 390 395 400
   Gly Gly Lys Ala Ser Phe Gly Gly Gly Ile Ala Cys Gly Ser Phe Ser
   405 410 415
   Ser Ala Gly Gly Ala Ser Val Leu Gly Thr Ile Asp Ile Ser Lys Asn
   420 425 430
   Leu Gly Ala Ile Ser Phe Ser Arg Thr Leu Cys Thr Thr Ser Asp Leu
   435 440 445
   Gly Gln Met Glu Tyr Gln Gly Gly Gly Ala Leu Phe Gly Glu Asn Ile
   450 455 460
   Ser Leu Ser Glu Asn Ala Gly Val Leu Thr Phe Lys Asp Asn Ile Val
   465 470 475 480
   Lys Thr Phe Ala Ser Asn Gly Lys Ile Leu Gly Gly Gly Ala Ile Leu
   485 490 495
   Ala Thr Gly Lys Val Glu Ile Thr Asn Asn Ser Gly Gly Ile Ser Phe
                   500                     505                     510
   Thr Gly Asn Ala Arg Ala Pro Gln Ala Leu Pro Thr Gln Glu Glu Phe
               515                     520                     525
   Pro Leu Phe Ser Lys Lys Glu Gly Arg Pro Leu Ser Ser Gly Tyr Ser
           530                     535                     540
   Gly Gly Gly Ala Ile Leu Gly Arg Glu Val Ala Ile Leu His Asn Ala
   545                     550                     555                     560
   Ala Val Val Phe Glu Gln Asn Arg Leu Gln Cys Ser Glu Glu Glu Ala
                   565                     570                     575
   Thr Leu Leu Gly Cys Cys Gly Gly Gly Ala Val His Gly Met Asp Ser
               580                     585                     590
   Thr Ser Ile Val Gly Asn Ser Ser Val Arg Phe Gly Asn Asn Tyr Ala
           595                     600                     605
   Met Gly Gln Gly Val Ser Gly Gly Ala Leu Leu Ser Lys Thr Val Gln
       610                     615                     620
   Leu Ala Gly Asn Gly Ser Val Asp Phe Ser Arg Asn Ile Ala Ser Leu
   625                     630                     635                     640
   Gly Gly Gly Ala Leu Gln Ala Ser Glu Gly Asn Cys Glu Leu Val Asp
                   645                     650                     655
   Asn Gly Tyr Val Leu Phe Arg Asp Asn Arg Gly Arg Val Tyr Gly Gly
               660                     665                     670
   Ala Ile Ser Cys Leu Arg Gly Asp Val Val Ile Ser Gly Asn Lys Gly
           675                     680                     685
   Arg Val Glu Phe Lys Asp Asn Ile Ala Thr Arg Leu Tyr Val Glu Glu
       690                     695                     700
   Thr Val Glu Lys Val Glu Glu Val Glu Pro Ala Pro Glu Gln Lys Asp
   705                     710                     715                     720
   Asn Asn Glu Leu Ser Phe Leu Gly Ser Val Glu Gln Ser Phe Ile Thr
                   725                     730                     735
   Ala Ala Asn Gln Ala Leu Phe Ala Ser Glu Asp Gly Asp Leu Ser Pro
               740                     745                     750
   Glu Ser Ser Ile Ser Ser Glu Glu Leu Ala Lys Arg Arg Glu Cys Ala
           755                     760                     765
   Gly Gly Ala Asp Ser Ser Arg Ser Gly Cys
       770                     775
<210> 194
   <211> 948
   <212> PRT
   <213> Chlamydia

<400> 194
   Met Ala Ser Met His His His His His His Val Lys Ile Glu Asn Phe
    1               5                  10                  15
   Ser Gly Gln Gly Ile Phe Ser Gly Asn Lys Ala Ile Asp Asn Thr Thr
               20                  25                  30
   Glu Gly Ser Ser Ser Lys Ser Asn Val Leu Gly Gly Ala Val Tyr Ala
           35                  40                  45
   Lys Thr Leu Phe Asn Leu Asp Ser Gly Ser Ser Arg Arg Thr Val Thr
       50                  55                  60
   Phe Ser Gly Asn Thr Val Ser Ser Gln Ser Thr Thr Gly Gln Val Ala
   65                  70                  75                  80
   Gly Gly Ala Ile Tyr Ser Pro Thr Val Thr Ile Ala Thr Pro Val Val
                   85                  90                  95
   Phe Ser Lys Asn Ser Ala Thr Asn Asn Ala Asn Asn Ala Thr Asp Thr
                   100                 105                 110
   Gln Arg Lys Asp Thr Phe Gly Gly Ala Ile Gly Ala Thr Ser Ala Val
               115                 120                 125
   Ser Leu Ser Gly Gly Ala His Phe Leu Glu Asn Val Ala Asp Leu Gly
       130                 135                 140
   Ser Ala Ile Gly Leu Val Pro Asp Thr Gln Asn Thr Glu Thr Val Lys
   145                 150                 155                 160
   Leu Glu Ser Gly Ser Tyr Tyr Phe Glu Lys Asn Lys Ala Leu Lys Arg
                   165                 170                 175
   Ala Thr Ile Tyr Ala Pro Val Val Ser Ile Lys Ala Tyr Thr Ala Thr
               180                 185                 190
   Phe Asn Gln Asn Arg Ser Leu Glu Glu Gly Ser Ala Ile Tyr Phe Thr
           195                 200                 205
   Lys Glu Ala Ser Ile Glu Ser Leu Gly Ser Val Leu Phe Thr Gly Asn
       210                 215                 220
   Leu Val Thr Pro Thr Leu Ser Thr Thr Thr Glu Gly Thr Pro Ala Thr
   225                 230                 235                 240
   Thr Ser Gly Asp Val Thr Lys Tyr Gly Ala Ala Ile Phe Gly Gln Ile
                   245                 250                 255
   Ala Ser Ser Asn Gly Ser Gln Thr Asp Asn Leu Pro Leu Lys Leu Ile
               260                 265                 270
   Ala Ser Gly Gly Asn Ile Cys Phe Arg Asn Asn Glu Tyr Arg Pro Thr
           275                 280                 285
   Ser Ser Asp Thr Gly Thr Ser Thr Phe Cys Ser Ile Ala Gly Asp Val
       290                 295                 300
   Lys Leu Thr Met Gln Ala Ala Lys Gly Lys Thr Ile Ser Phe Phe Asp
   305                 310                 315                 320
   Ala Ile Arg Thr Ser Thr Lys Lys Thr Gly Thr Gln Ala Thr Ala Tyr
                   325                 330                 335
   Asp Thr Leu Asp Ile Asn Lys Ser Glu Asp Ser Glu Thr Val Asn Ser
               340                 345                 350
   Ala Phe Thr Gly Thr Ile Leu Phe Ser Ser Glu Leu His Glu Asn Lys
           355                 360                 365
   Ser Tyr Ile Pro Gln Asn Val Val Leu His Ser Gly Ser Leu Val Leu
       370                 375                 380
   Lys Pro Asn Thr Glu Leu His Val Ile Ser Phe Glu Gln Lys Glu Gly
   385                 390                 395                 400
   Ser Ser Leu Val Met Thr Pro Gly Ser Val Leu Ser Asn Gln Thr Val
                   405                 410                 415
   Ala Asp Gly Ala Leu Val Ile Asn Asn Met Thr Ile Asp Leu Ser Ser
               420                 425                 430
   Val Glu Lys Asn Gly Ile Ala Glu Gly Asn Ile Phe Thr Pro Pro Glu
           435                 440                 445
   Leu Arg Ile Ile Asp Thr Thr Thr Ser Gly Ser Gly Gly Thr Pro Ser
       450                 455                 460
   Thr Asp Ser Glu Ser Asn Gln Asn Ser Asp Asp Thr Lys Glu Gln Asn
   465                 470                 475                 480
   Asn Asn Asp Ala Ser Asn Gln Gly Glu Ser Ala Asn Gly Ser Ser Ser
                   485                 490                 495
   Pro Ala Val Ala Ala Ala His Thr Ser Arg Thr Arg Asn Phe Ala Ala
               500                 505                 510
   Ala Ala Thr Ala Thr Pro Thr Thr Thr Pro Thr Ala Thr Thr Thr Thr
           515                 520                 525
   Ser Asn Gln Val Ile Leu Gly Gly Glu Ile Lys Leu Ile Asp Pro Asn
       530                 535                 540
   Gly Thr Phe Phe Gln Asn Pro Ala Leu Arg Ser Asp Gln Gln Ile Ser
   545 550 555 560
   Leu Leu Val Leu Pro Thr Asp Ser Ser Lys Met Gln Ala Gln Lys Ile
   565 570 575
   Val Leu Thr Gly Asp Ile Ala Pro Gln Lys Gly Tyr Thr Gly Thr Leu
   580 585 590
   Thr Leu Asp Pro Asp Gln Leu Gln Asn Gly Thr Ile Ser Ala Leu Trp
   595 600 605
   Lys Phe Asp Ser Tyr Arg Gln Trp Ala Tyr Val Pro Arg Asp Asn His
   610 615 620
   Phe Tyr Ala Asn Ser Ile Leu Gly Ser Gln Met Ser Met Val Thr Val
   625 630 635 640
   Lys Gln Gly Leu Leu Asn Asp Lys Met Asn Leu Ala Arg Phe Asp Glu
   645 650 655
   Val Ser Tyr Asn Asn Leu Trp Ile Ser Gly Leu Gly Thr Met Leu Ser
   660 665 670
   Gln Val Gly Thr Pro Thr Ser Glu Glu Phe Thr Tyr Tyr Ser Arg Gly
   675 680 685
   Ala Ser Val Ala Leu Asp Ala Lys Pro Ala His Asp Val Ile Val Gly
   690 695 700
   Ala Ala Phe Ser Lys Met Ile Gly Lys Thr Lys Ser Leu Lys Arg Glu
   705 710 715 720
   Asn Asn Tyr Thr His Lys Gly Ser Glu Tyr Ser Tyr Gln Ala Ser Val
   725 730 735
   Tyr Gly Gly Lys Pro Phe His Phe Val Ile Asn Lys Lys Thr Glu Lys
   740 745 750
   Ser Leu Pro Leu Leu Leu Gln Gly Val Ile Ser Tyr Gly Tyr Ile Lys
   755 760 765
   His Asp Thr Val Thr His Tyr Pro Thr Ile Arg Glu Arg Asn Gln Gly
   770 775 780
   Glu Trp Glu Asp Leu Gly Trp Leu Thr Ala Leu Arg Val Ser Ser Val
   785 790 795 800
   Leu Arg Thr Pro Ala Gln Gly Asp Thr Lys Arg Ile Thr Val Tyr Gly
   805 810 815
   Glu Leu Glu Tyr Ser Ser Ile Arg Gln Lys Gln Phe Thr Glu Thr Glu
   820 825 830
   Tyr Asp Pro Arg Tyr Phe Asp Asn Cys Thr Tyr Arg Asn Leu Ala Ile
   835 840 845
   Pro Met Gly Leu Ala Phe Glu Gly Glu Leu Ser Gly Asn Asp Ile Leu
   850 855 860
   Met Tyr Asn Arg Phe Ser Val Ala Tyr Met Pro Ser Ile Tyr Arg Asn
   865 870 875 880
   Ser Pro Thr Cys Lys Tyr Gln Val Leu Ser Ser Gly Glu Gly Gly Glu
   885 890 895
   Ile Ile Cys Gly Val Pro Thr Arg Asn Ser Ala Arg Gly Glu Tyr Ser
   900 905 910
   Thr Gln Leu Tyr Pro Gly Pro Leu Trp Thr Leu Tyr Gly Ser Tyr Thr
   915 920 925
   Ile Glu Ala Asp Ala His Thr Leu Ala His Met Met Asn Cys Gly Ala
   930 935 940
   Arg Met Thr Phe
   945
<210> 195
   <211> 821
   <212> PRT
   <213> Chlamydia
<400> 195
   Met His His His His His His Glu Ala Ser Ser Ile Gln Asp Gln Ile
   1 5 10 15
   Lys Asn Thr Asp Cys Asn Val Ser Lys Val Gly Tyr Ser Thr Ser Gln
   20 25 30
   Ala Phe Thr Asp Met Met Leu Ala Asp Asn Thr Glu Tyr Arg Ala Ala
   35 40 45
   Asp Ser Val Ser Phe Tyr Asp Phe Ser Thr Ser Ser Gly Leu Pro Arg
   50 55 60
   Lys His Leu Ser Ser Ser Ser Glu Ala Ser Pro Thr Thr Glu Gly Val
   65 70 75 80
   Ser Ser Ser Ser Ser Gly Glu Asn Thr Glu Asn Ser Gln Asp Ser Ala
   85 90 95
   Pro Ser Ser Gly Glu Thr Asp Lys Lys Thr Glu Glu Glu Leu Asp Asn
   100 105 110
   Gly Gly Ile Ile Tyr Ala Arg Glu Lys Leu Thr Ile Ser Glu Ser Gln
   115 120 125
   Asp Ser Leu Ser Asn Pro Ser Ile Glu Leu His Asp Asn Ser Phe Phe
   130 135 140
   Phe Gly Glu Gly Glu Val Ile Phe Asp His Arg Val Ala Leu Lys Asn
   145 150 155 160
   Gly Gly Ala Ile Tyr Gly Glu Lys Glu Val Val Phe Glu Asn Ile Lys
   165 170 175
   Ser Leu Leu Val Glu Val Asn Ile Ser Val Glu Lys Gly Gly Ser Val
   180 185 190
   Tyr Ala Lys Glu Arg Val Ser Leu Glu Asn Val Thr Glu Ala Thr Phe
   195 200 205
   Ser Ser Asn Gly Gly Glu Gln Gly Gly Gly Gly Ile Tyr Ser Glu Gln
   210 215 220
   Asp Met Leu Ile Ser Asp Cys Asn Asn Val His Phe Gln Gly Asn Ala
   225 230 235 240
   Ala Gly Ala Thr Ala Val Lys Gln Cys Leu Asp Glu Glu Met Ile Val
   245 250 255
   Leu Leu Thr Glu Cys Val Asp Ser Leu Ser Glu Asp Thr Leu Asp Ser
   260 265 270
   Thr Pro Glu Thr Glu Gln Thr Lys Ser Asn Gly Asn Gln Asp Gly Ser
   275 280 285
   Ser Glu Thr Lys Asp Thr Gln Val Ser Glu Ser Pro Glu Ser Thr Pro
   290 295 300
   Ser Pro Asp Asp Val Leu Gly Lys Gly Gly Gly Ile Tyr Thr Glu Lys
   305 310 315 320
   Ser Leu Thr Ile Thr Gly Ile Thr Gly Thr Ile Asp Phe Val Ser Asn
   325 330 335
   Ile Ala Thr Asp Ser Gly Ala Gly Val Phe Thr Lys Glu Asn Leu Ser
   340 345 350
   Cys Thr Asn Thr Asn Ser Leu Gln Phe Leu Lys Asn Ser Ala Gly Gln
   355 360 365
   His Gly Gly Gly Ala Tyr Val Thr Gln Thr Met Ser Val Thr Asn Thr
   370 375 380
   Thr Ser Glu Ser Ile Thr Thr Pro Pro Leu Val Gly Glu Val Ile Phe
   385 390 395 400
   Ser Glu Asn Thr Ala Lys Gly His Gly Gly Gly Ile Cys Thr Asn Lys
   405 410 415
   Leu Ser Leu Ser Asn Leu Lys Thr Val Thr Leu Thr Lys Asn Ser Ala
   420 425 430
   Lys Glu Ser Gly Gly Ala Ile Phe Thr Asp Leu Ala Ser Ile Pro Thr
   435 440 445
   Thr Asp Thr Pro Glu Ser Ser Thr Pro Ser Ser Ser Ser Pro Ala Ser
   450 455 460
   Thr Pro Glu Val Val Ala Ser Ala Lys Ile Asn Arg Phe Phe Ala Ser
   465 470 475 480
   Thr Ala Glu Pro Ala Ala Pro Ser Leu Thr Glu Ala Glu Ser Asp Gln
   485 490 495
   Thr Asp Gln Thr Glu Thr Ser Asp Thr Asn Ser Asp Ile Asp Val Ser
   500 505 510
   Ile Glu Asn Ile Leu Asn Val Ala Ile Asn Gln Asn Thr Ser Ala Lys
   515 520 525
   Lys Gly Gly Ala Ile Tyr Gly Lys Lys Ala Lys Leu Ser Arg Ile Asn
   530 535 540
   Asn Leu Glu Leu Ser Gly Asn Ser Ser Gln Asp Val Gly Gly Gly Leu
   545 550 555 560
   Cys Leu Thr Glu Ser Val Glu Phe Asp Ala Ile Gly Ser Leu Leu Ser
   565 570 575
   His Tyr Asn Ser Ala Ala Lys Glu Gly Gly Val Ile His Ser Lys Thr
   580 585 590
   Val Thr Leu Ser Asn Leu Lys Ser Thr Phe Thr Phe Ala Asp Asn Thr
   595 600 605
   Val Lys Ala Ile Val Glu Ser Thr Pro Glu Ala Pro Glu Glu Ile Pro
   610 615 620
   Pro Val Glu Gly Glu Glu Ser Thr Ala Thr Glu Asn Pro Asn Ser Asn
   625 630 635 640
   Thr Glu Gly Ser Ser Ala Asn Thr Asn Leu Glu Gly Ser Gln Gly Asp
   645 650 655
   Thr Ala Asp Thr Gly Thr Gly Val Val Asn Asn Glu Ser Gln Asp Thr
   660 665 670
   Ser Asp Thr Gly Asn Ala Glu Ser Gly Glu Gln Leu Gln Asp Ser Thr
   675 680 685
   Gln Ser Asn Glu Glu Asn Thr Leu Pro Asn Ser Ser Ile Asp Gln Ser
   690 695 700
   Asn Glu Asn Thr Asp Glu Ser Ser Asp Ser His Thr Glu Glu Ile Thr
   705 710 715 720
   Asp Glu Ser Val Ser Ser Ser Ser Lys Ser Gly Ser Ser Thr Pro Gln
   725 730 735
   Asp Gly Gly Ala Ala Ser Ser Gly Ala Pro Ser Gly Asp Gln Ser Ile
   740 745 750
   Ser Ala Asn Ala Cys Leu Ala Lys Ser Tyr Ala Ala Ser Thr Asp Ser
   755 760 765
   Ser Pro Val Ser Asn Ser Ser Gly Ser Asp Val Thr Ala Ser Ser Asp
   770 775 780
   Asn Pro Asp Ser Ser Ser Ser Gly Asp Ser Ala Gly Asp Ser Glu Gly
   785 790 795 800
   Pro Thr Glu Pro Glu Ala Gly Ser Thr Thr Glu Thr Pro Thr Leu Ile
   805 810 815
   Gly Gly Gly Ala Ile
   820
<210> 196
   <211> 525
   <212> PRT
   <213> Chlamydia
<400> 196
   Met His His His His His His Thr Ala Ala Ser Asp Asn Phe Gln Leu
   1 5 10 15
   Ser Gln Gly Gly Gln Gly Phe Ala Ile Pro Ile Gly Gln Ala Met Ala
   20 25 30
   Ile Ala Gly Gln Ile Lys Leu Pro Thr Val His Ile Gly Pro Thr Ala
   35 40 45
   Phe Leu Gly Leu Gly Val Val Asp Asn Asn Gly Asn Gly Ala Arg Val
   50 55 60
   Gln Arg Val Val Gly Ser Ala Pro Ala Ala Ser Leu Gly Ile Ser Thr
   65 70 75 80
   Gly Asp Val Ile Thr Ala Val Asp Gly Ala Pro Ile Asn Ser Ala Thr
   85 90 95
   Ala Met Ala Asp Ala Leu Asn Gly His His Pro Gly Asp Val Ile Ser
   100 105 110
   Val Thr Trp Gln Thr Lys Ser Gly Gly Thr Arg Thr Gly Asn Val Thr
   115 120 125
   Leu Ala Glu Gly Pro Pro Ala Glu Phe Pro Leu Val Pro Arg Gly Ser
   130 135 140
   Pro Leu Pro Val Gly Asn Pro Ala Glu Pro Ser Leu Leu Ile Asp Gly
   145 150 155 160
   Thr Met Trp Glu Gly Ala Ser Gly Asp Pro Cys Asp Pro Cys Ala Thr
   165 170 175
   Trp Cys Asp Ala Ile Ser Ile Arg Ala Gly Tyr Tyr Gly Asp Tyr Val
   180 185 190
   Phe Asp Arg Val Leu Lys Val Asp Val Asn Lys Thr Phe Ser Gly Met
   195 200 205
   Ala Ala Thr Pro Thr Gln Ala Ile Gly Asn Ala Ser Asn Thr Asn Gln
   210 215 220
   Pro Glu Ala Asn Gly Arg Pro Asn Ile Ala Tyr Gly Arg His Met Gln
   225 230 235 240
   Asp Ala Glu Trp Phe Ser Asn Ala Ala Phe Leu Ala Leu Asn Ile Trp
   245 250 255
   Asp Arg Phe Asp Ile Phe Cys Thr Leu Gly Ala Ser Asn Gly Tyr Phe
   260 265 270
   Lys Ala Ser Ser Ala Ala Phe Asn Leu Val Gly Leu Ile Gly Phe Ser
   275 280 285
   Ala Ala Ser Ser Ile Ser Thr Asp Leu Pro Met Gln Leu Pro Asn Val
   290 295 300
   Gly Ile Thr Gln Gly Val Val Glu Phe Tyr Thr Asp Thr Ser Phe Ser
   305 310 315 320
   Trp Ser Val Gly Ala Arg Gly Ala Leu Trp Glu Cys Gly Cys Ala Thr
   325 330 335
   Leu Gly Ala Glu Phe Gln Tyr Ala Gln Ser Asn Pro Lys Ile Glu Met
   340 345 350
   Leu Asn Val Thr Ser Ser Pro Ala Gln Phe Val Ile His Lys Pro Arg
   355 360 365
   Gly Tyr Lys Gly Ala Ser Ser Asn Phe Pro Leu Pro Ile Thr Ala Gly
   370 375 380
   Thr Thr Glu Ala Thr Asp Thr Lys Ser Ala Thr Ile Lys Tyr His Glu
   385 390 395 400
   Trp Gln Val Gly Leu Ala Leu Ser Tyr Arg Leu Asn Met Leu Val Pro
                   405                 410                 415
   Tyr Ile Gly Val Asn Trp Ser Arg Ala Thr Phe Asp Ala Asp Thr Ile
               420                 425                 430
   Arg Ile Ala Gln Pro Lys Leu Lys Ser Glu Ile Leu Asn Ile Thr Thr
           435                 440                 445
   Trp Asn Pro Ser Leu Ile Gly Ser Thr Thr Ala Leu Pro Asn Asn Ser
       450                 455                 460
   Gly Lys Asp Val Leu Ser Asp Val Leu Gln Ile Ala Ser Ile Gln Ile
   465                 470                 475                 480
   Asn Lys Met Lys Ser Arg Lys Ala Cys Gly Val Ala Val Gly Ala Thr
                   485                 490                 495
   Leu Ile Asp Ala Asp Lys Trp Ser Ile Thr Gly Glu Ala Arg Leu Ile
               500                 505                 510
   Asn Glu Arg Ala Ala His Met Asn Ala Gln Phe Arg Phe
           515                 520                 525
<210> 197
   <211> 43
   <212> DNA
   <213> Chlamydia
<400> 197
   gataggcgcg ccgcaatcat gaaatttatg tcagctactg ctg 43
<210> 198
   <211> 34
   <212> DNA
   <213> Chlamydia
<400> 198
   cagaacgcgt ttagaatgtc atacgagcac cgca 34
<210> 199
   <211> 6
   <212> DNA
   <213> Chlamydia
<400> 199
   gcaatc 6
<210> 200
   <211> 34
   <212> DNA
   <213> Chlamydia
<400> 200
   tgcaatcatg agttcgcaga aagatataaa aagc 34
<210> 201
   <211> 38
   <212> DNA
   <213> Chlamydia

<400> 201
   cagagctagc ttaaaagatc aatcgcaatc cagtattc 38
<210> 202
   <211> 5
   <212> DNA
   <213> Chlamydia
<400> 202
   caatc 5
<210> 203
   <211> 31
   <212> DNA
   <213> Chlamydia
<400> 203
   tgcaatcatg aaaaaagcgt ttttcttttt c 31
<210> 204
   <211> 31
   <212> DNA
   <213> Chlamydia
<400> 204
   cagaacgcgt ctagaatcgc agagcaattt c 31
<210> 205
   <211> 30
   <212> DNA
   <213> Chlamydia
<400> 205
   gtgcaatcat gattcctcaa ggaatttacg 30
<210> 206
   <211> 31
   <212> DNA
   <213> Chlamydia
<400> 206
   cagaacgcgt ttagaaccgg actttacttc c 31
<210> 207
   <211> 50
   <212> DNA
   <213> Chlamydia
<400> 207
   cagacatatg catcaccatc accatcacga ggcgagctcg atccaagatc 50
<210> 208
   <211> 40
   <212> DNA
   <213> Chlamydia

<400> 208
   cagaggtacc tcagatagca ctctctccta ttaaagtagg 40
<210> 209
   <211> 55
   <212> DNA
   <213> Chlamydia
<400> 209
   cagagctagc atgcatcacc atcaccatca cgttaagatt gagaacttct ctggc 55
<210> 210
   <211> 35
   <212> DNA
   <213> Chlamydia
<400> 210
   cagaggtacc ttagaatgtc atacgagcac cgcag 35
<210> 211
   <211> 36
   <212> DNA
   <213> Chlamydia
<400> 211
   cagacatatg catcaccatc accatcacgg gttagc 36
<210> 212
   <211> 35
   <212> DNA
   <213> Chlamydia
<400> 212
   cagaggtacc tcagctcctc cagcacactc tcttc 35
<210> 213
   <211> 51
   <212> DNA
   <213> Chlamydia
<400> 213
   cagagctagc catcaccatc accatcacgg tgctatttct tgcttacgtg g 51
<210> 214
   <211> 38
   <212> DNA
   <213> Chlamydia
<400> 214
   cagaggtact taaaagatca atcgcaatcc agtattcg 38
<210> 215
   <211> 48
   <212> DNA
   <213> Chlamydia

<400> 215
   cagaggatcc acatcaccat caccatcacg gactagctag agaggttc 48
<210> 216
   <211> 31
   <212> DNA
   <213> Chlamydia
<400> 216
   cagagaattc ctagaatcgc agagcaattt c 31
<210> 217
   <211> 7
   <212> DNA
   <213> Chlamydia
<400> 217
   tgcaatc 7
<210> 218
   <211> 22
   <212> PRT
   <213> Chlamydia
<400> 218
   Met Ala Ser Met Thr Gly Gly Gln Gln Met Gly Arg Asp Ser Ser Leu
    1               5                  10                  15
   Val Pro Ser Ser Asp Pro
               20
<210> 219
   <211> 51
   <212> DNA
   <213> Chlamydia
<400> 219
   cagaggtacc gcatcaccat caccatcaca tgattcctca aggaatttac g 51
<210> 220
   <211> 33
   <212> DNA
   <213> Chlamydia
<400> 220
   cagagcggcc gcttagaacc ggactttact tcc 33
<210> 221
   <211> 24
   <212> PRT
   <213> Chlamydia
<400> 221
   Met Ala Ser Met Thr Gly Gly Gln Gln Asn Gly Arg Asp Ser Ser Leu
    1               5                  10                  15
   Val Pro His His His His His His
               20
<210> 222
   <211> 46
   <212> DNA
   <213> Chlamydia
<400> 222
   cagagctagc catcaccatc accatcacct ctttggccag gatccc 46
<210> 223
   <211> 30
   <212> DNA
   <213> Chlamydial
<400> 223
   cagaactagt ctagaacctg taagtggtcc 30
<210> 224
   <211> 20
   <212> PRT
   <213> Artificial Sequence
<220>
   <223> Made in a lab
<400> 224
   Met Ser Gln Lys Asn Lys Asn Ser Ala Phe Met His Pro Val Asn Ile
    1               5                  10                  15
   Ser Thr Asp Leu
               20
<210> 225
   <211> 20
   <212> PRT
   <213> Artificial Sequence
<220>
   <223> Made in a lab
<400> 225
   Lys Asn Ser Ala Phe Met His Pro Val Asn Ile Ser Thr Asp Leu Ala
    1               5                  10                  15
   Val Ile Val Gly
               20
<210> 226
   <211> 20
   <212> PRT
   <213> Artificial Sequence
<220>
   <223> Made in a lab

<400> 226
   His Pro Val Asn Ile Ser Thr Asp Leu Ala Val Ile Val Gly Lys Gly
   1 5 10 15
   Pro Met Pro Arg
   20
<210> 227
   <211> 20
   <212> PRT
   <213> Artificial Sequence
<220>
   <223> Made in a lab
<400> 227
   Ser Thr Asp Leu Ala Val Ile Val Gly Lys Gly Pro Met Pro Arg Thr
   1 5 10 15
   Glu Ile Val Lys
   20
<210> 228
   <211> 20
   <212> PRT
   <213> Artificial Sequence
<220>
   <223> Made in a lab
<400> 228
   Val Ile Val Gly Lys Gly Pro Met Pro Arg Thr Glu Ile Val Lys Lys
   1 5 10 15
   Val Trp Glu Tyr
   20
<210> 229
   <211> 20
   <212> PRT
   <213> Artificial Sequence
<220>
   <223> Made in a lab
<400> 229
   Gly Pro Met Pro Arg Thr Glu Ile Val Lys Lys Val Trp Glu Tyr Ile
   1 5 10 15
   Lys Lys His Asn
   20
<210> 230
   <211> 20
   <212> PRT
   <213> Artificial Sequence
<220>
   <223> Made in a lab
<400> 230
   Ile Lys Lys His Asn Cys Gln Asp Gln Lys Asn Lys Arg Asn Ile Leu
   1 5 10 15
   Pro Asp Ala Asn
   20
<210> 231
   <211> 20
   <212> PRT
   <213> Artificial Sequence
<220>
   <223> Made in a lab
<400> 231
   Asn Cys Gln Asp Gln Lys Asn Lys Arg Asn Ile Leu Pro Asp Ala Asn
   1 5 10 15
   Leu Ala Lys Val
   20
<210> 232
   <211> 20
   <212> PRT
   <213> Artificial Sequence
<220>
   <223> Made in a lab
<400> 232
   Lys Asn Lys Arg Asn Ile Leu Pro Asp Ala Asn Leu Ala Lys Val Phe
   1 5 10 15
   Gly Ser Ser Asp
   20
<210> 233
   <211> 20
   <212> PRT
   <213> Artificial Sequence
<220>
   <223> Made in a lab
<400> 233
   Ile Leu Pro Asp Ala Asn Leu Ala Lys Val Phe Gly Ser Ser Asp Pro
   1 5 10 15
   Ile Asp Met Phe
   20
<210> 234
   <211> 20
   <212> PRT
   <213> Artificial Sequence
<220>
   <223> Made in a lab

<400> 234
   Asn Leu Ala Lys Val Phe Gly Ser Ser Asp Pro Ile Asp Met Phe Gln
    1               5                  10                  15
   Met Thr Lys Ala
               20
<210> 235
   <211> 22
   <212> PRT
   <213> Artificial Sequence
<220>
   <223> Made in a lab
<400> 235
   Phe Gly Ser Ser Asp Pro Ile Asp Met Phe Gln Met Thr Lys Ala Leu
    1               5                  10                  15
   Ser Lys His Ile Val Lys
               20
<210> 236
   <211> 20
   <212> PRT
   <213> Artificial Sequence
<220>
   <223> Made in a lab
<400> 236
   Val Glu Ile Thr Gln Ala Val Pro Lys Tyr Ala Thr Val Gly Ser Pro
    1               5                  10                  15
   Tyr Pro Val Glu
               20
<210> 237
   <211> 20
   <212> PRT
   <213> Artificial Sequence
<220>
   <223> Made in a lab
<400> 237
   Ala Val Pro Lys Tyr Ala Thr Val Gly Ser Pro Tyr Pro Val Glu Ile
   1 5 10 15
   Thr Ala Thr Gly
   20
<210> 238
   <211> 20
   <212> PRT
   <213> Artificial Sequence
<220>
   <223> Made in a lab
<400> 238
   Ala Thr Val Gly Ser Pro Tyr Pro Val Glu Ile Thr Ala Thr Gly Lys
   1 5 10 15
   Arg Asp Cys Val
   20
<210> 239
   <211> 20
   <212> PRT
   <213> Artificial Sequence
<220>
   <223> Made in a lab
<400> 239
   Pro Tyr Pro Val Glu Ile Thr Ala Thr Gly Lys Arg Asp Cys Val Asp
   1 5 10 15
   Val Ile Ile Thr
   20
<210> 240
   <211> 21
   <212> PRT
   <213> Artificial Sequence
<220>
   <223> Made in a lab
<400> 240
   Ile Thr Ala Thr Gly Lys Arg Asp Cys Val Asp Val Ile Ile Thr Gln
   1 5 10 15
   Gln Leu Pro Cys Glu
   20
<210> 241
   <211> 20
   <212> PRT
   <213> Artificial Sequence
<220>
   <223> Made in a lab
<400> 241
   Lys Arg Asp Cys Val Asp Val Ile Ile Thr Gln Gln Leu Pro Cys Glu
    1              5                   10                  15
   Ala Glu Phe Val
               20
<210> 242
   <211> 20
   <212> PRT
   <213> Artificial Sequence
<220>
   <223> Made in a lab

<400> 242
   Asp Val Ile Ile Thr Gln Gln Leu Pro Cys Glu Ala Glu Phe Val Arg
    1              5                   10                  15
   Ser Asp Pro Ala
               20
<210> 243
   <211> 20
   <212> PRT
   <213> Artificial Sequence
<220>
   <223> Made in a lab
<400> 243
   Thr Gln Gln Leu Pro Cys Glu Ala Glu Phe Val Arg Ser Asp Pro Ala
    1              5                   10                  15
   Thr Thr Pro Thr
               20
<210> 244
   <211> 20
   <212> PRT
   <213> Artificial Sequence
<220>
   <223> Made in a lab
<400> 244
   Cys Glu Ala Glu Phe Val Arg Ser Asp Pro Ala Thr Thr Pro Thr Ala
    1              5                   10                  15
   Asp Gly Lys Leu
               20
<210> 245
   <211> 20
   <212> PRT
   <213> Artificial Sequence
<220>
   <223> Made in a lab
<400> 245
   Val Arg Ser Asp Pro Ala Thr Thr Pro Thr Ala Asp Gly Lys Leu Val
   1               5                   10                  15
   Trp Lys Ile Asp
               20
<210> 246
   <211> 20
   <212> PRT
   <213> Artificial Sequence
<220>
   <223> Made in a lab
<400> 246
   Ala Thr Thr Pro Thr Ala Asp Gly Lys Leu Val Trp Lys Ile Asp Arg
   1               5                   10                  15
   Leu Gly Gln Gly
               20
<210> 247
   <211> 20
   <212> PRT
   <223> Artificial Sequence
<220>
   <223> Made in a lab
<400> 247
   Ala Asp Gly Lys Leu Val Trp Lys Ile Asp Arg Leu Gly Gln Gly Glu
   1               5                   10                  15
   Lys Ser Lys Ile
               20
<210> 248
   <211> 20
   <212> PRT
   <213> Artificial Sequence
<220>
   <223> Made in a lab
<400> 248
   Val Trp Lys Ile Asp Arg Leu Gly Gln Gly Glu Lys Ser Lys Ile Thr
   1 5 10 15
   Val Trp Val Lys
   20
<210> 249
   <211> 20
   <212> PRT
   <213> Artificial Sequence
<220>
   <223> Made in a lab
<400> 249
   Arg Leu Gly Gln Gly Glu Lys Ser Lys Ile Thr Val Trp Val Lys Pro
   1 5 10 15
   Leu Lys Glu Gly
   20
<210> 250
   <211> 20
   <212> PRT
   <213> Artificial Sequence
<220>
   <223> Made in a lab
<400> 250
   Gly Glu Lys Ser Lys Ile Thr Val Trp Val Lys Pro Leu Lys Glu Gly
   1 5 10 15
   Cys Cys Phe Thr
   20
<210> 251
   <211> 16
   <212> PRT
   <213> Artificial Sequence
<220>
   <223> Made in a lab

<400> 251
   Gly Glu Lys Ser Lys Ile Thr Val Trp Val Lys Pro Leu Lys Glu Gly
   1 5 10 15
<210> 252
   <211> 12
   <212> PRT
   <213> Artificial Sequence
<220>
   <223> Made in a lab
<400> 252
   Lys Ile Thr Val Trp Val Lys Pro Leu Lys Glu Gly
    1               5                   10
<210> 253
   <211> 16
   <212> PRT
   <213> Artificial Sequence
<220>
   <223> Made in a lab
<400> 253
   Gly Asp Lys Cys Lys Ile Thr Val Trp Val Lys Pro Leu Lys Glu Gly
    1               5                   10                  15
<210> 254
   <211> 20
   <212> PRT
   <213> Artificial Sequence
<220>
   <223> Made in a lab
<400> 254
   Thr Glu Tyr Pro Leu Leu Ala Asp Pro Ser Phe Lys Ile Ser Glu Ala
    1               5                   10                  15
   Phe Gly Val Leu
               20
<210> 255
   <211> 20
   <212> PRT
   <213> Artificial Sequence
<220>
   <223> Made in a lab
<400> 255
   Leu Ala Asp Pro Ser Phe Lys Ile Ser Glu Ala Phe Gly Val Leu Asn
    1               5                   10                  15
   Pro Glu Gly Ser
               20
<210> 256
   <211> 20
   <212> PRT
   <213> Artificial Sequence
<220>
   <223> Made in a lab
<400> 256
   Phe Lys Ile Ser Glu Ala Phe Gly Val Leu Asn Pro Glu Gly Ser Leu
   1               5                   10                  15
   Ala Leu Arg Ala
               20
<210> 257
   <211> 20
   <212> PRT
   <213> Artificial Sequence
<220>
   <223> Made in a lab
<400> 257
   Ala Phe Gly Val Leu Asn Pro Glu Gly Ser Leu Ala Leu Arg Ala Thr
   1               5                   10                  15
   Phe Leu Ile Asp
               20
<210> 258
   <211> 20
   <212> PRT
   <213> Artificial Sequence
<220>
   <223> Made in a lab
<400> 258
   Asn Pro Glu Gly Ser Leu Ala Leu Arg Ala Thr Phe Leu Ile Asp Lys
   1               5                   10                  15
   His Gly Val Ile
               20
<210> 259
   <211> 20
   <212> PRT
   <213> Artificial Sequence
<220>
   <223> Made in a lab
<400> 259
   Leu Ala Leu Arg Ala Thr Phe Leu Ile Asp Lys His Gly Val Ile Arg
   1               5                   10                  15
   His Ala Val Ile
               20
<210> 260
   <211> 20
   <212> PRT
   <213> Artificial Sequence
<220>
   <223> Made in a lab
<400> 260
   Thr Phe Leu Ile Asp Lys His Gly Val Ile Arg His Ala Val Ile Asn
   1               5                   10                  15
   Asp Leu Pro Leu
               20
<210> 261
   <211> 20
   <212> PRT
   <213> Artificial Sequence
<220>
   <223> Made in a lab
<400> 261
   Lys His Gly Val Ile Arg His Ala Val Ile Asn Asp Leu Pro Leu Gly
   1               5                   10                  15
   Arg Ser Ile Asp
               20
<210> 262
   <211> 20
   <212> PRT
   <213> Artificial Sequence
<220>
   <223> Made in a lab
<400> 262
   Arg His Ala Val Ile Asn Asp Leu Pro Leu Gly Arg Ser Ile Asp Glu
   1               5                   10                  15
   Glu Leu Arg Ile
               20
<210> 263
   <211> 897
   <212> DNA
   <213> Chlamydia
<220>
   <221> misc_feature
   <222> (1)...(897)
   <223> n = A,T,C or G
<400> 263
   atggcttcta tatgcggacg tttagggtct ggtacaggga atgctctaaa agcttttttt      60
   acacagccca acaataaaat ggcaagggta gtaaataaga cgaagggagt ggataagact     120
   attaaggttg ccaagtctgc tgccgaattg accgcaaata ttttggaaca agctggaggc     180
   gcgggctctt ccgcacacat tacagcttcc caagtgtcca aaggattagg ggatgcgaga     240
   actgttgtcg ctttagggaa tgcctttaac ggagcgttgc caggaacagt tcaaagtgcg     300
   caaagcttct tctctcacat gaaagctgct agtcagaaaa cgcaagaagg ggatgagggg     360
   ctcacagcag atctttgtgt gtctcataag cgcagagcgg ctgcggctgt ctgtagcatc     420
   atcggaggaa ttacctacct cgcgacattc ggagctatcc gtccgattct gtttgtcaac     480
   aaaatgctgg caaaaccgtt tctttcttcc caaactaaag caaatatggg atcttctgtt     540
   agctatatta tggcggctaa ccatgcagcg tctgtggtgg gtgctggact cgctatcagt     600
   gcgnaaagag cagattgcga agcccgctgc gctcgtattg cgagagaaga gtcgttactc     660
   gaagtgccgg gagaggaaaa tgcttgcgag aagaaagtcg ctggagagaa agccaagacg     720
   ttcacgcgca tcaagtatgc actcctcact atgctcgaga agtttttgga atgcgttgcc     780
   gacgttttca aattggtgcc gctgcctatt acaatgggta ttcgtgcgat tgtggctgct     840
   ggatgtacgt tcacttctgc aattattgga ttgtgcactt tctgcgccag agcataa        897
<210> 264
   <211> 298
   <212> PRT
   <213> Chlamydia
<220>
   <221> VARIANT
   <222> (1)...(298)
   <223> Xaa = Any Amino Acid

<400> 264
   Met Ala Ser Ile Cys Gly Arg Leu Gly Ser Gly Thr Gly Asn Ala Leu
    1               5                  10                  15
   Lys Ala Phe Phe Thr Gln Pro Asn Asn Lys Met Ala Arg Val Val Asn
               20                  25                  30
   Lys Thr Lys Gly Val Asp Lys Thr Ile Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Ser Ile Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Lys Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Xaa Arg Ala Asp Cys Glu Ala
           195                 200                 205
   Arg Cys Ala Arg Ile Ala Arg Glu Glu Ser Leu Leu Glu Val Pro Gly
       210                 215                 220
   Glu Glu Asn Ala Cys Glu Lys Lys Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 265
   <211> 897
   <212> DNA
   <213> Chlamydia
<220>
   <221> misc_feature
   <222> (1)...(897)
   <223> n = A,T,C or G
<400> 265
   atggcttcta tatgcggacg tttagggtct ggtacaggga atgctctaaa agcttttttt      60
   acacagccca acaataaaat ggcaagggta gtaaataaga cgaagggaat ggataagact     120
   attaaggttg ccaagtctgc tgccgaattg accgcaaata ttttggaaca agctggaggc     180
   gcgggctctt ccgcacacat tacagcttcc caagtgtcca aaggattagg ggatgcgaga     240
   actgttgtcg ctttagggaa tgcctttaac ggagcgttgc caggaacagt tcaaagtgcg     300
   caaagcttct tctctcacat gaaagctgct agtcagaaaa cgcaagaagg ggatgagggg     360
   ctcacagcag atctttgtgt gtctcataag cgcagagcgg ctgcggctgt ctgtagcatc     420
   atcggaggaa ttacctacct cgcgacattc ggagctatcc gtccgattct gtttgtcaac     480
   aaaatgctgg caaaaccgtt tctttcttcc caaactaaag caaatatggg atcttctgtt     540
   agctatatta tggcggctaa ccatgcagcg tctgtggtgg gtgctggact cgctatcagt     600
   gcgnaaagag cagattgcga agcccgctgc gctcgtattg cgagagaaga gtcgttactc     660
   gaagtgccgg gagaggaaaa tgcttgcgag aagaaagtcg ctggagagaa agccaagacg     720
   ttcacgcgca tcaagtatgc actcctcact atgctcgaga agtttttgga atgcgttgcc     780
   gacgttttca aattggtgcc gctgcctatt acaatgggta ttcgtgcgat tgtggctgct     840
   ggatgtacgt tcacttctgc aattattgga ttgtgcactt tctgcgccag agcataa        897
<210> 266
   <211> 298
   <212> PRT
   <213> Chlamydia
<220>
   <221> VARIANT
   <222> (1)...(298)
   <223> Xaa = Any Amino Acid
<400> 266
   Met Ala Ser Ile Cys Gly Arg Leu Gly Ser Gly Thr Gly Asn Ala Leu
    1               5                  10                  15
   Lys Ala Phe Phe Thr Gln Pro Asn Asn Lys Met Ala Arg Val Val Asn
               20                  25                  30
   Lys Thr Lys Gly Met Asp Lys Thr Ile Lys Val Ala Lys Ser Ala Ala
           35                  40                  45
   Glu Leu Thr Ala Asn Ile Leu Glu Gln Ala Gly Gly Ala Gly Ser Ser
       50                  55                  60
   Ala His Ile Thr Ala Ser Gln Val Ser Lys Gly Leu Gly Asp Ala Arg
   65                  70                  75                  80
   Thr Val Val Ala Leu Gly Asn Ala Phe Asn Gly Ala Leu Pro Gly Thr
                   85                  90                  95
   Val Gln Ser Ala Gln Ser Phe Phe Ser His Met Lys Ala Ala Ser Gln
               100                 105                 110
   Lys Thr Gln Glu Gly Asp Glu Gly Leu Thr Ala Asp Leu Cys Val Ser
           115                 120                 125
   His Lys Arg Arg Ala Ala Ala Ala Val Cys Ser Ile Ile Gly Gly Ile
       130                 135                 140
   Thr Tyr Leu Ala Thr Phe Gly Ala Ile Arg Pro Ile Leu Phe Val Asn
   145                 150                 155                 160
   Lys Met Leu Ala Lys Pro Phe Leu Ser Ser Gln Thr Lys Ala Asn Met
                   165                 170                 175
   Gly Ser Ser Val Ser Tyr Ile Met Ala Ala Asn His Ala Ala Ser Val
               180                 185                 190
   Val Gly Ala Gly Leu Ala Ile Ser Ala Xaa Arg Ala Asp Cys Glu Ala
           195                 200                 205
   Arg Cys Ala Arg Ile Ala Arg Glu Glu Ser Leu Leu Glu Val Pro Gly
       210                 215                 220
   Glu Glu Asn Ala Cys Glu Lys Lys Val Ala Gly Glu Lys Ala Lys Thr
   225                 230                 235                 240
   Phe Thr Arg Ile Lys Tyr Ala Leu Leu Thr Met Leu Glu Lys Phe Leu
                   245                 250                 255
   Glu Cys Val Ala Asp Val Phe Lys Leu Val Pro Leu Pro Ile Thr Met
               260                 265                 270
   Gly Ile Arg Ala Ile Val Ala Ala Gly Cys Thr Phe Thr Ser Ala Ile
           275                 280                 285
   Ile Gly Leu Cys Thr Phe Cys Ala Arg Ala
       290                 295
<210> 267
   <211> 680
   <212> DNA
   <213> Chlamydia
<400> 267
   tctatatcca tattgatagg aaaaaacgtc gcagaaagat tttagctatg acgtttatcc      60
   gagctttagg atattcaaca gatgcagata ttattgaaga gttcttttct gtagaggagc     120
   gttccttacg ttcagagaag gattttgtcg cgttagttgg taaagtttta gctgataacg     180
   tagttgatgc ggattcttca ttagtttacg ggaaagctgg agagaagcta agtactgcta     240
   tgctaaaacg catcttagat acgggagtcc aatctttgaa gattgctgtt ggcgcagatg     300
   aaaatcaccc aattattaag atgctcgcaa aagatcctac ggattcttac gaagctgctc     360
   ttaaagattt ttatcgcaga ttacgaccag gagagcctgc aactttagct aatgctcgat     420
   ccacaattat gcgtttattc ttcgatgcta aacgttataa tttaggccgc gttggacgtt     480
   ataaattaaa taaaaaatta ggcttcccat tagacgacga aacattatct caagtgactt     540
   tgagaaaaga agatgttatc ggcgcgttga aatatttgat tcgtttgcga atgggcgatg     600
   agaagacatc tatcgatgat attgaccatt tggcaaaccg acgagttcgc tctgttggag     660
   aactaattca gaatcactgt                                                 680
<210> 268
   <211> 359
   <212> DNA
   <213> Chlamydia
<400> 268
   cttatgttct ggagaatgtt gcaacaacat attaatcgaa ccagctcctc ctagtaacat     60
   agaaaccaag cccttttgag aaaaaacctg tacttcgcat cctttagcca tttgttgaat     120
   agctcctaac aaagagctaa ttttttcctc ttccttgttt ttctgaggcg ctgtggactc     180
   taaatatagc aagtgctctt ggaacacctc atcaacaatc gcttgtccta gattaggtat     240
   agagactgtc tctccatcaa ttaaatggag tttcaaagta atatcccctt ccgtccctcc     300
   atcacaagac tctatgaaag ctatctgatt ccatcgagca gaaatgtatg gggaaatac      359
<210> 269
   <211> 124
   <212> DNA
   <213> Chlamydia
<400> 269
   gatcgaatca attgagggag ctcattaaca agaatagctg cagtttcttt gcgttcttct     60
   ggaataacaa gaaataggta atcggtacca ttgatagaac gaacacgaca aatcgcagaa    120
   ggtt                                                                 124
<210> 270
   <211> 219
   <212> DNA
   <213> Chlamydia
<400> 270
   gatcctgttg ggcctagtaa taatacgttg gatttcccat aactcacttg tttatcctgc     60
   ataagagcac ggatacgctt atagtggtta tagacggcaa ccgaaatcgt ttttttcgcg     120
   cgctcttgtc caatgacata agagtcgatg tggcgtttga tttctttagg ggttaacact    180
   ctcagacttg ttggagagct tgtggaagat gttgcgatc                           219
<210> 271
   <211> 511
   <212> DNA
   <213> Chlamydia
<220>
   <221> misc_feature
   <222> (1)...(511)
   <223> n = A,T,C or G
<400> 271
   ggatccgaat tcggcacgag gagaaaatat aggaggttcc akcatcggaa gatctaatag    60
   acaaagaggt tttggcatag atggctcctc cttgtacgtt caacgatgat tgggagggat   120
   tgttatcgat agcttggttc ccagagaact gacaagtccc gctacattga gagaatgtaa   180
   cctgttctcc atagatagct cctcctacta cacctgaata agttggtgtt gctggagatg   240
   atggtgcggc tgctgcggct gcttgtaggg aagcagcagc tgcagcaggt gctgaagctg   300
   ttgttgcgac tcctgtggat gaggagtttg ctttgttgtt cgagaaagag aagcctgatt   360
   tcagattaga aatatttaca gttttagcat gtaagcctcc accttctttc ccaacaaggt   420
   tctctgttac agataaggag actagangca tctagtttta aagatttttt acagcagata   480
   cctccaccta tctctgtagc ggagttctca g                                  511
<210> 272
   <211> 598
   <212> DNA
   <213> Chlamydia
<400> 272
   ctcttcctct cctcaatcta gttctggagc aactacagtc tccgactcag gagactctag    60
   ctctggctca aactcggata cctcaaaaac agttccagtc acagctaaag gcggtgggct   120
   ttatactgat aagaatcttt cgattactaa catcacagga attatcgaaa ttgcaaataa   180
   caaagcgaca gatgttggag gtggtgctta cgtaaaagga acccttactt gtaaaaactc   240
   tcaccgtcta caatttttga aaaactcttc cgataaacaa ggtggaggaa tctacggaga   300
   agacaacatc accctatcta atttgacagg gaagactcta ttccaagaga atactgccaa   360
   aaaagagggc ggtggactct tcataaaagg tacagataaa gctcttacaa tgacaggact   420
   ggatagtttc tgtttaatta ataacacatc agaaaaacat ggtggtggga gcctttgtta   480
   ccaaagaaat ctctcagact tacacctctt gatgtggaaa caattccagg aatcacgcct   540
   gtacatggtg aaacagtcat tactggcaat aaatctacag gaggtaatgg tggagggc     598
<210> 273
   <211> 126
   <212> DNA
   <213> Chlamydia
<400> 273
   ggatccgaat tcggcacgag atgagcctta tagtttaaca aaagcttctc acattccttc    60
   gatagctttt tattagccgt ttttagcatc ctaatgagat ctcctcgttc gtaacaaata   120
   cgagag                                                              126
<210> 274
   <211> 264
   <212> DNA
   <213> Chlamydia

<400> 274
   ggatccgaat tcggcacgag ctcttttaaa tcttaattac aaaaagacaa attaattcaa    60
   tttttcaaaa aagaatttaa acattaattg ttgtaaaaaa acaatattta ttctaaaata   120
   ataaccatag ttacggggga atctctttca tggtttattt tagagctcat caacctaggc   180
   atacgcctaa aacatttcct ttgaaagttc accattcgtt ctccgataag catcctcaaa   240
   ttgctaaagc tatgtggatt acgg                                          264
<210> 275
   <211> 359
   <212> DNA
   <213> Chlamydia
<400> 275
   ggatccgaat tcggcacgag ataaaacctg aaccacaaca aagatctaaa acttcttgat      60
   tttcagctgc aaattctttt agataaatat caaccatttc ttcagtttca tatcttggaa     120
   ttaaaacttg ttctcttaaa ttaattctag tatttaagta ttcaacatag cccattatta     180
   attgaattgg ataattttgc cttaataatt cacattcttt ttcagtaatt ttaggttcta     240
   aaccgtaccg ctttttttct aaaattaatg tttcttcatt attcatttta taagccactt     300
   tcctttattt tttgattttg ttcttctgtt agtaatgctt caataatagt taataattt      359
<210> 276
   <211> 357
   <212> DNA
   <213> Chlamydia
<400> 276
   aaaacaattg atataatttt ttttttcata acttccagac tcctttctag aaaagtcttt      60
   atgggtagta gtgactctaa cgttttttat tattaagacg atccccggag atccttttaa     120
   tgatgaaaac ggaaacatcc tttcgccaga aactttagca ctattaaaga atcgttacgg     180
   gttagataag cctttattca cccagtatct tatctatttg aaatgtctgc taacactaga     240
   tttcggggaa tctcttatct acaaagatcg aaatctcagc attattgctg ccgctcttcc     300
   atcttccgct attcttggac ttgaaagctt gtgtttactc gtgccgaatt cggatcc        357
<210> 277
   <211> 505
   <212> DNA
   <213> Chlamydia
<400> 277
   ggatccgaat tcggcacgag ctcgtgccga ttgcttgctt cagtcacccc atcggtatag      60
   agcactaaaa gagactcctc ttcaagaacg agagtgtaag cagggtgagg aggaacttca     120
   ggtaaaaatc ctaaggccat accaggatgc gacaggaaag agatatctcc attaggagct     180
   cggagacacg ctgggttgtg gccacaagaa tagtattcta gttctcgtgt tgcgtaatga     240
   taacaataaa tgcatagtgt tacaaacatc ccagattcag ctgtctgttg atagaagaga     300
   gcagctgttt gttgaacggc ttcttgaata gaggagagct cactcaaaaa ggtatgtaac     360
   atgtttttca ggaataagga gtaggcgcac gcattgactc ctttcccgga agcatcagca     420
   acgattagaa agagtttagc ttggggacct tcgcctataa caaagatatc aaagaaatct     480
   cctcctaccg taactgcagg aatat                                           505
<210> 278
   <211> 407
   <212> DNA
   <213> Chlamydia
<400> 278
   ggatccgaat tcggcacgag aactactgag caaattgggt atccaacttc ctctttacga     60
   aagaaaaaca gaaggcattc tccataccaa gatttgttgc atcgacaata aaactccaat    120
   ctttggctct gctaactgga gcggtgctgg tatgattaaa aactttgaag acctattcat    180
   ccttcgccca attacagaga cacagcttca ggcctttatg gacgtctggt ctcttctaga    240
   aacaaatagc tcctatctgt ccccagagag cgtgcttacg gcccctactc cttcaagtag    300
   acctactcaa caagatacag attctgatga cgaacaaccg agtaccagcc agcaagctat    360
   ccgtatgaga aaataggatt agggaaacaa aacgacagca aaccaca                  407
<210> 279
   <211> 351
   <212> DNA
   <213> Chlamydia
<400> 279
   ctcgtgccgc ttacaggagg cttgtatcct ttaaaataga gtttttctta tgaccccatg     60
   tggcgatagg ccgggtctag cgccgatagt agaaatatcg gttggttttt gtccttgagg    120
   ggatcgtata ctttttcaaa gtatggtccc cgtatcgatt atctggaggc tcttatgtct    180
   ttttttcata ctagaaaata taagcttatc ctcagaggac tcttgtgttt agcaggctgt    240
   ttcttaatga acagctgttc ctctagtcga ggaaatcaac ccgctgatga gagcatctat    300
   gtcttgtcta tgaatcgcat gatttgtgat tctcgtgccg aattcggatc c             351
<210> 280
   <211> 522
   <212> DNA
   <213> Chlamydia
<400> 280
   ggatccgaat tcggcacgag cagaggaaaa aggcgatact cctcttgaag atcgtttcac     60
   agaagatctt tccgaagtct ctggagaaga ttttcgagga ttgaaaaatt cgttcgatga    120
   tgattcttct tctgacgaaa ttctcgatgc gctcacaagt aaattttctg atcccacaat    180
   aaaggatcta gctcttgatt atctaattca aatagctccc tctgatggga aacttaagtc    240
   cgctctcatt caggcaaagc atcaactgat gagccagaat cctcaggcga ttgttggagg    300
   acgcaatgtt ctgttagctt cagaaacctt tgcttccaga gcaaatacat ctccttcatc    360
   gcttcgctcc ttatatttcc aagtaacctc atccccctct aattgcgcta atttacatca    420
   aatgcttgct tcttactcgc catcagagaa aaccgctgtt atggagtttc tagtgaatgg    480
   catggtagca gatttaaaat cggagggccc ttccattcct cc                       522
<210> 281
   <211> 577
   <212> DNA
   <213> Chlamydia
<400> 281
   ggatccgaat tcggcacgag atgcttctat tacaattggt ttggatgcgg aaaaagctta 60
   ccagcttatt ctagaaaagt tgggagatca aattcttggt ggaattgctg atactattgt 120
   tgatagtaca gtccaagata ttttagacaa aatcacaaca gacccttctc taggtttgtt 180
   gaaagctttt aacaactttc caatcactaa taaaattcaa tgcaacgggt tattcactcc 240
   caggaacatt gaaactttat taggaggaac tgaaatagga aaattcacag tcacacccaa 300
   aagctctggg agcatgttct tagtctcagc agatattatt gcatcaagaa tggaaggcgg 360
   cgttgttcta gctttggtac gagaaggtga ttctaagccc tacgcgatta gttatggata 420
   ctcatcaggc gttcctaatt tatgtagtct aagaaccaga attattaata caggattgac 480
   tccgacaacg tattcattac gtgtaggcgg tttagaaagc ggtgtggtat gggttaatgc 540
   cctttctaat ggcaatgata ttttaggaat aacaaat 577
<210> 282
   <211> 607
   <212> DNA
   <213> Chlamydia
<400> 282
   actmatcttc cccgggctcg agtgcggccg caagcttgtc gacggagctc gatacaaaaa 60
   tgtgtgcgtg tgaaccgctt cttcaaaagc ttgtcttaaa agatattgtc tcgcttccgg 120
   attagttaca tgtttaaaaa ttgctagaac aatattattc ccaaccaagc tctctgcggt 180
   gctgaaaaaa cctaaattca aaagaatgac tcgccgctca tcttcagaaa gacgatccga 240
   cttccataat tcgatgtctt tccccatggg gatctctgta gggagccagt tatttgcgca 300
   gccattcaaa taatgttccc aagcccattt gtacttaata ggaacaagtt ggttgacatc 360
   gacctggttg cagttcacta gacgcttgct atttagatta acgcgtttct gttttccatc 420
   taaaatatct gcttgcataa gaaccgttaa ttttattgtt aatttatatg attaattact 480
   gacatgcttc acacccttct tccaaagaac agacaggtgc tttcttcgct ctttcaacaa 540
   taattcctgc cgaagcagac ttattcttca tccaacgagg ctgaattcct ctcttattaa 600
   tatctac 607
<210> 283
   <211> 1077
   <212> DNA
   <213> Chlamydia
<400> 283
   ggatccgaat tcggcacgag aagttaacga tgacgatttg ttcctttggt agagaaggag     60
   caatcgaaac taaatgtgcg agagcatgtg aagactccaa tgcaggaata atcccctcat    120
   ttctagtaag caggaaaaaa gctcgtaacg cctcttcatc ggtggctaat gtataaaagg    180
   ctcgtcctga ctcatgcatt tcggcatgat ctggcccaac tgaaggataa tctaatccag    240
   cggaaatgga gtgagtttgt aatacttgtc catcgtcatc ttgaagaaga tacgaataaa    300
   atccgtggaa tactccaggt cgccctgttg caaaacgtgc tgcatgtttt cctgaagaaa    360
   tgcccagtcc tcccccttcc actccaatta attggacttt tggattcggg ataaaatgat    420
   ggaaaaatcc aatagcgttg gagccacctc cgatacatgc aatcagaata tcaggatctc    480
   ttcctgcaac tgcatggatt tgctctttca cttcagcgct tataacagac tgaaaaaatc    540
   gaacgatatc gggataaggt aaaggtccta aggccgatcc taagcaatag tgagtaaatg    600
   agtgtgttgt tgcccaatct tgtagagctt gattaactgc atctttgagt ccacaagatc    660
   cttttgttac agaaacgact tcagcaccta aaaagcgcat ttctctacat tttggtttct     720
   gtcgttccac atcttttgct cccatgtata ctacacaatc taatcctaga taagcacacg    780
   ctgttgctgt tgctactcca tgttgtcccg cacctgtttc agctacaaca cgtgttttcc    840
   caagatattt agcaagcaaa cactgaccaa gagcattatt cagtttatgt gctcctgtat    900
   gcaaaagatc ttcgcgttta agaaatactc tagggccatc aatagctcga gcaaaattct    960
   taacttcagt cagaggagtt tgtctccccg catagttttt caaaatacaa tctagttcag   1020
   ataaaaaact ttgctgagtt ttgagaatct cccattccgc ttttagattc tgtatag      1077
<210> 284
   <211> 407
   <212> DNA
   <213> Chlamydia
<400> 284
   ggatccgaat tcggcacgag aactactgag caaattgggt atccaacttc ctctttacga     60
   aagaaaaaca gaaggcattc tccataccaa gatttgttgc atcgacaata aaactccaat    120
   ctttggctct gctaactgga gcggtgctgg tatgattaaa aactttgaag acctattcat    180
   ccttcgccca attacagaga cacagcttca ggcctttatg gacgtctggt ctcttctaga    240
   aacaaatagc tcctatctgt ccccagagag cgtgcttacg gcccctactc cttcaagtag    300
   acctactcaa caagatacag attctgatga cgaacaaccg agtaccagcc agcaagctat    360
   ccgtatgaga aaataggatt agggaaacaa aacgacagca aaccaca                  407
<210> 285
   <211> 802
   <212> DNA
   <213> Chlamydia

<400> 285
   ggatccgaat tcggcacgag ttagcttaat gtctttgtca tctctaccta catttgcagc     60
   taattctaca ggcacaattg gaatcgttaa tttacgtcgc tgcctagaag agtctgctct    120
   tgggaaaaaa gaatctgctg aattcgaaaa gatgaaaaac caattctcta acagcatggg    180
   gaagatggag gaagaactgt cttctatcta ttccaagctc caagacgacg attacatgga    240
   aggtctatcc gagaccgcag ctgccgaatt aagaaaaaaa ttcgaagatc tatctgcaga    300
   atacaacaca gctcaagggc agtattacca aatattaaac caaagtaatc tcaagcgcat    360
   gcaaaagatt atggaagaag tgaaaaaagc ttctgaaact gtgcgtattc aagaaggctt    420
   gtcagtcctt cttaacgaag atattgtctt atctatcgat agttcggcag ataaaaccga    480
   tgctgttatt aaagttcttg atgattcttt tcaaaataat taacatgcga agctagccga    540
   ggagtgccgt atgtctcaat ccacttattc tcttgaacaa ttagctgatt ttttgaaagt    600
   cgagtttcaa ggaaatggag ctactcttct ttccggagtt gaagagatcg aggaagcaaa    660
   aacggcacac atcacattct tagataatga aaaatatgct aaacatttaa aatcatcgga    720
   agctggcgct atcatcatat ctcgaacaca gtttcaaaaa tatcgagact tgaataaaaa    780
   ctttcttatc acttctgagt ct                                             802
<210> 286
   <211> 588
   <212> DNA
   <213> Chlamydia
<400> 286
   ggatccgaat tcggcacgag gcaatattta ctcccaacat tacggttcca aataagcgat      60
   aaggtcttct aataaggaag ttaatgtaag aggctttttt attgcttttc gtaaggtagt     120
   attgcaaccg cacgcgattg aatgatacgc aagccatttc catcatggaa aagaaccctt     180
   ggacaaaaat acaaaggagg ttcactccta accagaaaaa gggagagtta gtttccatgg     240
   gttttcctta tatacacccg tttcacacaa ttaggagccg cgtctagtat ttggaataca     300
   aattgtcccc aagcgaattt tgttcctgtt tcagggattt ctcctaattg ttctgtcagc     360
   catccgccta tggtaacgca attagctgta gtaggaagat caactccaaa caggtcatag     420
   aaatcagaaa gctcataggt gcctgcagca ataacaacat tcttgtctga gtgagcgaat     480
   tgtttaaaag atgggcgatt atgagctacc tcatcagaga ctattttaaa tagatcattt     540
   tgggtaatca atccttctat agacccatat tcatcaatga taatctcg                  588
<210> 287
   <211> 489
   <212> DNA
   <213> Chlamydia
<220>
   <221> misc_feature
   <222> (1)...(489)
   <223> n = A,T,C or G
<400> 287
   agtgcctatt gttttgcagg ctttgtctga tgatagcgat accgtacgtg agattgctgt      60
   acaagtagct gttatgtatg gttctagttg cttactgcgc gccgtgggcg atttagcgaa     120
   aaatgattct tctattcaag tacgcatcac tgcttatcgt gctgcagccg tgttggagat     180
   acaagatctt gtgcctcatt tacgagttgt agtccaaaat acacaattag atggaacgga     240
   aagaagagaa gcttggagat ctttatgtgt tcttactcgg cctcatagtg gtgtattaac     300
   tggcatagat caagctttaa tgacctgtga gatgttaaag gaatatcctg aaaagtgtac     360
   ggaagaacag attcgtacat tattggctgc agatcatcca gaagtgcagg tagctacttt     420
   acagatcatt ctgagaggag gtagagtatt ccggtcatct tctataatgg aatcggttct     480
   cgtgccgnt                                                             489
<210> 288
   <211> 191
   <212> DNA
   <213> Chlamydia
<400> 288
   ggatccgaat tcaggatatg ctgttgggtt atcaataaaa agggttttgc cattttttaa     60
   gacgactttg tagataacgc taggagctgt agcaataata tcgagatcaa attctctaga     120
   gattctctca aagatgattt ctaagtgcag cagtcctaaa aatccacagc ggaacccaaa     180
   tccgagagag t                                                          191
<210> 289
   <211> 515
   <212> DNA
   <213> Chlamydia
<400> 289
   ggatccgaat tcggcacgag gagcgacgtg aaatagtgga atcttcccgt attcttatta 60
   cttctgcgtt gccttacgca aatggtcctt tgcattttgg acatattacc ggtgcttatt 120
   tgcctgcaga tgtttatgcg cgttttcaga gactacaagg caaagaggtt ttgtatattt 180
   gtggttctga tgaatacgga atcgcaatta cccttaatgc agagttggca ggcatggggt 240
   atcaagaata tgtcgacatg tatcataagc ttcataaaga taccttcaag aaattgggaa 300
   tttctgtaga tttcttttcc agaactacga acgcttatca tcctgctatt gtgcaagatt 360
   tctatcgaaa cttgcaggaa cgcggactgg tagagaatca ggtgaccgaa cagctgtatt 420
   ctgaggaaga agggaagttt ttagcggacc gttatgttgt aggtacttgt cccaagtgtg 480
   ggtttgatcg agctcgagga gatgagtgtc agcag 515
<210> 290
   <211> 522
   <212> DNA
   <213> Chlamydia
<400> 290
   ggatccgaat tcggcacgag ggaggaatgg aagggccctc cgattktama tctgctacca 60
   tgccattcac tagaaactcc ataacagcgg ttttctctga tggcgagtaa gaagcaagca 120
   tttgatgtaa attagcgcaa ttagaggggg atgaggttac ttggaaatat aaggagcgaa 180
   gcgatgaagg agatgtattt gctctggaag caaaggtttc tgaagctaac agaacattgc 240
   gtcctccaac aatcgcctga ggattctggc tcatcagttg atgctttgcc tgaatgagag 300
   cggacttaag tttcccatca gagggagcta tttgaattag ataatcaaga gctagatcct 360
   ttattgtggg atcagaaaat ttacttgtga gcgcatcgag aatttcgtca gaagaagaat 420
   catcatcgaa cgaatttttc aatcctcgaa aatcttctcc agagacttcg gaaagatctt 480
   ctgtgaaacg atcttcaaga ggagtatcgc ctttttccyc tg 522
<210> 291
   <211> 1002
   <212> DNA
   <213> Chlamydia
<400> 291
   atggcgacta acgcaattag atcggcagga agtgcagcaa gtaagatgct gctgccagtt 60
   gccaaagaac cagcggctgt cagctccttt gctcagaaag ggatttattg tattcaacaa 120
   ttttttacaa accctgggaa taagttagca aagtttgtag gggcaacaaa aagtttagat 180
   aaatgcttta agctaagtaa ggcggtttct gactgtgtcg taggatcgct ggaagaggcg 240
   ggatgcacag gggacgcatt gacctccgcg agaaacgccc agggtatgtt aaaaacaact 300
   cgagaagttg ttgccttagc taatgtgctc aatggagctg ttccatctat cgttaactcg 360
   actcagaggt gttaccaata cacacgtcaa gccttcgagt taggaagcaa gacaaaagaa 420
   agaaaaacgc ctggggagta tagtaaaatg ctattaactc gaggtgatta cctattggca      480
   gcttccaggg aagcttgtac ggcagtcggt gcaacgactt actcagcgac attcggtgtt      540
   ttacgtccgt taatgttaat caataaactc acagcaaaac cattcttaga caaagcgact      600
   gtaggcaatt ttggcacggc tgttgctgga attatgacca ttaatcatat ggcaggagtt      660
   gctggtgctg ttggcggaat cgcattagaa caaaagctgt tcaaacgtgc gaaggaatcc      720
   ctatacaatg agagatgtgc cttagaaaac caacaatctc agttgagtgg ggacgtgatt      780
   ctaagcgcgg aaagggcatt acgtaaagaa cacgttgcta ctctaaaaag aaatgtttta      840
   actcttcttg aaaaagcttt agagttggta gtggatggag tcaaactcat tcctttaccg      900
   attacagtgg cttgctccgc tgcaatttct ggagccttga cggcagcatc cgcaggaatt      960
   ggcttatata gcatatggca gaaaacaaag tctggcaaat aa                        1002
<210> 292
   <211> 333
   <212> PRT
   <213> Chlamydia
<400> 292
   Met Ala Thr Asn Ala Ile Arg Ser Ala Gly Ser Ala Ala Ser Lys Met
    1               5                  10                  15
   Leu Leu Pro Val Ala Lys Glu Pro Ala Ala Val Ser Ser Phe Ala Gln
               20                  25                  30
   Lys Gly Ile Tyr Cys Ile Gln Gln Phe Phe Thr Asn Pro Gly Asn Lys
           35                  40                  45
   Leu Ala Lys Phe Val Gly Ala Thr Lys Ser Leu Asp Lys Cys Phe Lys
       50                  55                  60
   Leu Ser Lys Ala Val Ser Asp Cys Val Val Gly Ser Leu Glu Glu Ala
   65                  70                  75                  80
   Gly Cys Thr Gly Asp Ala Leu Thr Ser Ala Arg Asn Ala Gln Gly Met
                   85                  90                  95
   Leu Lys Thr Thr Arg Glu Val Val Ala Leu Ala Asn Val Leu Asn Gly
               100                 105                 110
   Ala Val Pro Ser Ile Val Asn Ser Thr Gln Arg Cys Tyr Gln Tyr Thr
           115                 120                 125
   Arg Gln Ala Phe Glu Leu Gly Ser Lys Thr Lys Glu Arg Lys Thr Pro
       130                 135                 140
   Gly Glu Tyr Ser Lys Met Leu Leu Thr Arg Gly Asp Tyr Leu Leu Ala
   145                 150                 155                 160
   Ala Ser Arg Glu Ala Cys Thr Ala Val Gly Ala Thr Thr Tyr Ser Ala
                   165                 170                 175
   Thr Phe Gly Val Leu Arg Pro Leu Met Leu Ile Asn Lys Leu Thr Ala
               180                 185                 190
   Lys Pro Phe Leu Asp Lys Ala Thr Val Gly Asn Phe Gly Thr Ala Val
           195                 200                 205
   Ala Gly Ile Met Thr Ile Asn His Met Ala Gly Val Ala Gly Ala Val
       210                 215                 220
   Gly Gly Ile Ala Leu Glu Gln Lys Leu Phe Lys Arg Ala Lys Glu Ser
   225                 230                 235                 240
   Leu Tyr Asn Glu Arg Cys Ala Leu Glu Asn Gln Gln Ser Gln Leu Ser
                   245                 250                 255
   Gly Asp Val Ile Leu Ser Ala Glu Arg Ala Leu Arg Lys Glu His Val
               260                 265                 270
   Ala Thr Leu Lys Arg Asn Val Leu Thr Leu Leu Glu Lys Ala Leu Glu
           275                 280                 285
   Leu Val Val Asp Gly Val Lys Leu Ile Pro Leu Pro Ile Thr Val Ala
       290                 295                 300
   Cys Ser Ala Ala Ile Ser Gly Ala Leu Thr Ala Ala Ser Ala Gly Ile
   305                 310                 315                 320
   Gly Leu Tyr Ser Ile Trp Gln Lys Thr Lys Ser Gly Lys
                   325                 330
<210> 293
   <211> 7
   <212> DNA
   <213> Chlamydia
<400> 293
   tgcaatc 7
<210> 294
   <211> 196
   <212> PRT
   <213> Chlamydia

<400> 294
   Thr Met Gly Ser Leu Val Gly Arg Gln Ala Pro Asp Phe Ser Gly Lys
   5 10 15
   Ala Val Val Cys Gly Glu Glu Lys Glu Ile Ser Leu Ala Asp Phe Arg
   20 25 30
   Gly Lys Tyr Val Val Leu Phe Phe Tyr Pro Lys Asp Phe Thr Tyr Val
   35 40 45
   Cys Pro Thr Glu Leu His Ala Phe Gln Asp Arg Leu Val Asp Phe Glu
   50 55 60
   Glu His Gly Ala Val Val Leu Gly Cys Ser Val Asp Asp Ile Glu Thr
   65 70 75 80
   His Ser Arg Trp Leu Thr Val Ala Arg Asp Ala Gly Gly Ile Glu Gly
   85 90 95
   Thr Glu Tyr Pro Leu Leu Ala Asp Pro Ser Phe Lys Ile Ser Glu Ala
   100 105 110
   Phe Gly Val Leu Asn Pro Glu Gly Ser Leu Ala Leu Arg Ala Thr Phe
   115 120 125
   Leu Ile Asp Lys His Gly Val Ile Arg His Ala Val Ile Asn Asp Leu
   130 135 140
   Pro Leu Gly Arg Ser Ile Asp Glu Glu Leu Arg Ile Leu Asp Ser Leu
   145 150 155 160
   Ile Phe Phe Glu Asn His Gly Met Val Cys Pro Ala Asn Trp Arg Ser
   165 170 175
   Gly Glu Arg Gly Met Val Pro Ser Glu Glu Gly Leu Lys Glu Tyr Phe
   180 185 190
   Gln Thr Met Asp
   195
<210> 295
   <211> 181
   <212> PRT
   <213> Chlamydia
<400> 295
   Lys Gly Gly Lys Met Ser Thr Thr Ile Ser Gly Asp Ala Ser Ser Leu
   5 10 15
   Pro Leu Pro Thr Ala Ser Cys Val Glu Thr Lys Ser Thr Ser Ser Ser
   20 25 30
   Thr Lys Gly Asn Thr Cys Ser Lys Ile Leu Asp Ile Ala Leu Ala Ile
   35 40 45
   Val Gly Ala Leu Val Val Val Ala Gly Val Leu Ala Leu Val Leu Cys
   50 55 60
   Ala Ser Asn Val Ile Phe Thr Val Ile Gly Ile Pro Ala Leu Ile Ile
   65 70 75 80
   Gly Ser Ala Cys Val Gly Ala Gly Ile Ser Arg Leu Met Tyr Arg Ser
   85 90 95
   Ser Tyr Ala Ser Leu Glu Ala Lys Asn Val Leu Ala Glu Gln Arg Leu
   100 105 110
   Arg Asn Leu Ser Glu Glu Lys Asp Ala Leu Ala Ser Val Ser Phe Ile
   115 120 125
   Asn Lys Met Phe Leu Arg Gly Leu Thr Asp Asp Leu Gln Ala Leu Glu
   130 135 140
   Ala Lys Val Met Glu Phe Glu Ile Asp Cys Leu Asp Arg Leu Glu Lys
   145 150 155 160
   Asn Glu Gln Ala Leu Leu Ser Asp Val Arg Leu Val Leu Ser Ser Tyr
   165 170 175
   Thr Arg Trp Leu Asp
   180
<210> 296
   <211> 124
   <212> PRT
   <213> Chlamydia
<400> 296
   Ile Tyr Glu Val Met Asn Met Asp Leu Glu Thr Arg Arg Ser Phe Ala
   5 10 15
   Val Gln Gln Gly His Tyr Gln Asp Pro Arg Ala Ser Asp Tyr Asp Leu
   20 25 30
   Pro Arg Ala Ser Asp Tyr Asp Leu Pro Arg Ser Pro Tyr Pro Thr Pro
   35 40 45
   Pro Leu Pro Ser Arg Tyr Gln Leu Gln Asn Met Asp Val Glu Ala Gly
   50 55 60
   Phe Arg Glu Ala Val Tyr Ala Ser Phe Val Ala Gly Met Tyr Asn Tyr
   65 70 75 80
   Val Val Thr Gln Pro Gln Glu Arg Ile Pro Asn Ser Gln Gln Val Glu
   85 90 95
   Gly Ile Leu Arg Asp Met Leu Thr Asn Gly Ser Gln Thr Phe Ser Asn
   100 105 110
   Leu Met Gln Arg Trp Asp Arg Glu Val Asp Arg Glu
   115 120
<210> 297
   <211> 488
   <212> PRT
   <213> Chlamydia
<400> 297
   Lys Gly Ser Leu Pro Ile Leu Gly Pro Phe Leu Asn Gly Lys Met Gly
                       5                  10                  15
   Phe Trp Arg Thr Ser Ile Met Lys Met Asn Arg Ile Trp Leu Leu Leu
                   20                  25                  30
   Leu Thr Phe Ser Ser Ala Ile His Ser Pro Val Arg Gly Glu Ser Leu
               35                  40                  45
   Val Cys Lys Asn Ala Leu Gln Asp Leu Ser Phe Leu Glu His Leu Leu
       50                  55                  60
   Gln Val Lys Tyr Ala Pro Lys Thr Trp Lys Glu Gln Tyr Leu Gly Trp
   65                  70                  75                  80
   Asp Leu Val Gln Ser Ser Val Ser Ala Gln Gln Lys Leu Arg Thr Gln
                       85                  90                  95
   Glu Asn Pro Ser Thr Ser Phe Cys Gln Gln Val Leu Ala Asp Phe Ile
                   100                 105                 110
   Gly Gly Leu Asn Asp Phe His Ala Gly Val Thr Phe Phe Ala Ile Glu
               115                 120                 125
   Ser Ala Tyr Leu Pro Tyr Thr Val Gln Lys Ser Ser Asp Gly Arg Phe
       130                 135                 140
   Tyr Phe Val Asp Ile Met Thr Phe Ser Ser Glu Ile Arg Val Gly Asp
   145 150 155 160
   Glu Leu Leu Glu Val Asp Gly Ala Pro Val Gln Asp Val Leu Ala Thr
   165 170 175
   Leu Tyr Gly Ser Asn His Lys Gly Thr Ala Ala Glu Glu Ser Ala Ala
   180 185 190
   Leu Arg Thr Leu Phe Ser Arg Met Ala Ser Leu Gly His Lys Val Pro
   195 200 205
   Ser Gly Arg Thr Thr Leu Lys Ile Arg Arg Pro Phe Gly Thr Thr Arg
   210 215 220
   Glu Val Arg Val Lys Trp Arg Tyr Val Pro Glu Gly Val Gly Asp Leu
   225 230 235 240
   Ala Thr Ile Ala Pro Ser Ile Arg Ala Pro Gln Leu Gln Lys Ser Met
   245 250 255
   Arg Ser Phe Phe Pro Lys Lys Asp Asp Ala Phe His Arg Ser Ser Ser
   260 265 270
   Leu Phe Tyr Ser Pro Met Val Pro His Phe Trp Ala Glu Leu Arg Asn
   275 280 285
   His Tyr Ala Thr Ser Gly Leu Lys Ser Gly Tyr Asn Ile Gly Ser Thr
   290 295 300
   Asp Gly Phe Leu Pro Val Ile Gly Pro Val Ile Trp Glu Ser Glu Gly
   305 310 315 320
   Leu Phe Arg Ala Tyr Ile Ser Ser Val Thr Asp Gly Asp Gly Lys Ser
   325 330 335
   His Lys Val Gly Phe Leu Arg Ile Pro Thr Tyr Ser Trp Gln Asp Met
   340 345 350
   Glu Asp Phe Asp Pro Ser Gly Pro Pro Pro Trp Glu Glu Phe Ala Lys
   355 360 365
   Ile Ile Gln Val Phe Ser Ser Asn Thr Glu Ala Leu Ile Ile Asp Gln
   370 375 380
   Thr Asn Asn Pro Gly Gly Ser Val Leu Tyr Leu Tyr Ala Leu Leu Ser
   385 390 395 400
   Met Leu Thr Asp Arg Pro Leu Glu Leu Pro Lys His Arg Met Ile Leu
   405 410 415
   Thr Gln Asp Glu Val Val Asp Ala Leu Asp Trp Leu Thr Leu Leu Glu
   420 425 430
   Asn Val Asp Thr Asn Val Glu Ser Arg Leu Ala Leu Gly Asp Asn Met
   435 440 445
   Glu Gly Tyr Thr Val Asp Leu Gln Val Ala Glu Tyr Leu Lys Ser Phe
   450 455 460
   Gly Arg Gln Val Leu Asn Cys Trp Ser Lys Gly Asp Ile Glu Leu Ser
   465 470 475 480
   Thr Pro Ile Pro Leu Phe Gly Phe
   485
<210> 298
   <211> 140
   <212> PRT
   <213> Chlamydia
<400> 298
   Arg Ile Asp Ile Ser Ser Val Thr Phe Phe Ile Gly Ile Leu Leu Ala
   5 10 15
   Val Asn Ala Leu Thr Tyr Ser His Val Leu Arg Asp Leu Ser Val Ser
   20 25 30
   Met Asp Ala Leu Phe Ser Arg Asn Thr Leu Ala Val Leu Leu Gly Leu
   35 40 45
   Val Ser Ser Val Leu Asp Asn Val Pro Leu Val Ala Ala Thr Ile Gly
   50 55 60
   Met Tyr Asp Leu Pro Met Asn Asp Pro Leu Trp Lys Leu Ile Ala Tyr
   65 70 75 80
   Thr Ala Gly Thr Gly Gly Ser Ile Leu Ile Ile Gly Ser Ala Ala Gly
   85 90 95
   Val Ala Tyr Met Gly Met Glu Lys Val Ser Phe Gly Trp Tyr Val Lys
   100 105 110
   His Ala Ser Trp Ile Ala Leu Ala Ser Tyr Phe Gly Gly Leu Ala Val
   115 120 125
   Tyr Phe Leu Met Glu Asn Cys Val Asn Leu Phe Val
   130 135 140
<210> 299
   <211> 361
   <212> PRT
   <213> Chlamydia
<400> 299
   His Gln Glu Ile Ala Asp Ser Pro Leu Val Lys Lys Ala Glu Glu Gln
   5 10 15
   Ile Asn Gln Ala Gln Gln Asp Ile Gln Thr Ile Thr Pro Ser Gly Leu
   20 25 30
   Asp Ile Pro Ile Val Gly Pro Ser Gly Ser Ala Ala Ser Ala Gly Ser
   35 40 45
   Ala Ala Gly Ala Leu Lys Ser Ser Asn Asn Ser Gly Arg Ile Ser Leu
   50 55 60
   Leu Leu Asp Asp Val Asp Asn Glu Met Ala Ala Ile Ala Met Gln Gly
   65 70 75 80
   Phe Arg Ser Met Ile Glu Gln Phe Asn Val Asn Asn Pro Ala Thr Ala
   85 90 95
   Lys Glu Leu Gln Ala Met Glu Ala Gln Leu Thr Ala Met Ser Asp Gln
   100 105 110
   Leu Val Gly Ala Asp Gly Glu Leu Pro Ala Glu Ile Gln Ala Ile Lys
   115 120 125
   Asp Ala Leu Ala Gln Ala Leu Lys Gln Pro Ser Ala Asp Gly Leu Ala
   130 135 140
   Thr Ala Met Gly Gln Val Ala Phe Ala Ala Ala Lys Val Gly Gly Gly
   145 150 155 160
   Ser Ala Gly Thr Ala Gly Thr Val Gln Met Asn Val Lys Gln Leu Tyr
   165 170 175
   Lys Thr Ala Phe Ser Ser Thr Ser Ser Ser Ser Tyr Ala Ala Ala Leu
   180 185 190
   Ser Asp Gly Tyr Ser Ala Tyr Lys Thr Leu Asn Ser Leu Tyr Ser Glu
   195 200 205
   Ser Arg Ser Gly Val Gln Ser Ala Ile Ser Gln Thr Ala Asn Pro Ala
   210 215 220
   Leu Ser Arg Ser Val Ser Arg Ser Gly Ile Glu Ser Gln Gly Arg Ser
   225 230 235 240
   Ala Asp Ala Ser Gln Arg Ala Ala Glu Thr Ile Val Arg Asp Ser Gln
   245 250 255
   Thr Leu Gly Asp Val Tyr Ser Arg Leu Gln Val Leu Asp Ser Leu Met
   260 265 270
   Ser Thr Ile Val Ser Asn Pro Gln Ala Asn Gln Glu Glu Ile Met Gln
   275 280 285
   Lys Leu Thr Ala Ser Ile Ser Lys Ala Pro Gln Phe Gly Tyr Pro Ala
   290 295 300
   Val Gln Asn Ser Val Asp Ser Leu Gln Lys Phe Ala Ala Gln Leu Glu
   305 310 315 320
   Arg Glu Phe Val Asp Gly Glu Arg Ser Leu Ala Glu Ser Gln Glu Asn
   325 330 335
   Ala Phe Arg Lys Gln Pro Ala Phe Ile Gln Gln Val Leu Val Asn Ile
   340 345 350
   Ala Ser Leu Phe Ser Gly Tyr Leu Ser
   355 360
<210> 300
   <211> 207
   <212> PRT
   <213> Chlamydia
<400> 300
   Ser Ser Lys Ile Val Ser Leu Cys Glu Gly Ala Val Ala Asp Ala Arg
                   5                   10                  15
   Met Cys Lys Ala Glu Leu Ile Lys Lys Glu Ala Asp Ala Tyr Leu Phe
               20                  25                  30
   Cys Glu Lys Ser Gly Ile Tyr Leu Thr Lys Lys Glu Gly Ile Leu Ile
           35                  40                  45
   Pro Ser Ala Gly Ile Asp Glu Ser Asn Thr Asp Gln Pro Phe Val Leu
       50                  55                  60
   Tyr Pro Lys Asp Ile Leu Gly Ser Cys Asn Arg Ile Gly Glu Trp Leu
   65                  70                  75                  80
   Arg Asn Tyr Phe Arg Val Lys Glu Leu Gly Val Ile Ile Thr Asp Ser
                   85                  90                  95
   His Thr Thr Pro Met Arg Arg Gly Val Leu Gly Ile Gly Leu Cys Trp
               100                 105                 110
   Tyr Gly Phe Ser Pro Leu His Asn Tyr Ile Gly Ser Leu Asp Cys Phe
           115                 120                 125
   Gly Arg Pro Leu Gln Met Thr Gln Ser Asn Leu Val Asp Ala Leu Ala
       130                 135                 140
   Val Ala Ala Val Val Cys Met Gly Glu Gly Asn Glu Gln Thr Pro Leu
   145                 150                 155                 160
   Ala Val Ile Glu Gln Ala Pro Asn Met Val Tyr His Ser Tyr Pro Thr
                   165                 170                 175
   Ser Arg Glu Glu Tyr Cys Ser Leu Arg Ile Asp Glu Thr Glu Asp Leu
               180                 185                 190
   Tyr Gly Pro Phe Leu Gln Ala Val Thr Trp Ser Gln Glu Lys Lys
           195                 200                 205
<210> 301
   <211> 183
   <212> PRT
   <213> Chlamydia
<400> 301
   Ile Pro Pro Ala Pro Arg Gly His Pro Gln Ile Glu Val Thr Phe Asp
   5 10 15
   Ile Asp Ala Asn Gly Ile Leu His Val Ser Ala Lys Asp Ala Ala Ser
   20 25 30
   Gly Arg Glu Gln Lys Ile Arg Ile Glu Ala Ser Ser Gly Leu Lys Glu
   35 40 45
   Asp Glu Ile Gln Gln Met Ile Arg Asp Ala Glu Leu His Lys Glu Glu
   50 55 60
   Asp Lys Gln Arg Lys Glu Ala Ser Asp Val Lys Asn Glu Ala Asp Gly
   65 70 75 80
   Met Ile Phe Arg Ala Glu Lys Ala Val Lys Asp Tyr His Asp Lys Ile
   85 90 95
   Pro Ala Glu Leu Val Lys Glu Ile Glu Glu His Ile Glu Lys Val Arg
   100 105 110
   Gln Ala Ile Lys Glu Asp Ala Ser Thr Thr Ala Ile Lys Ala Ala Ser
   115 120 125
   Asp Glu Leu Ser Thr Arg Met Gln Lys Ile Gly Glu Ala Met Gln Ala
   130 135 140
   Gln Ser Ala Ser Ala Ala Ala Ser Ser Ala Ala Asn Ala Gln Gly Gly
   145 150 155 160
   Pro Asn Ile Asn Ser Glu Asp Leu Lys Lys His Ser Phe Ser Thr Arg
   165 170 175
   Pro Pro Ala Gly Gly Ser Ala
   180
<210> 302
   <211> 232
   <212> PRT
   <213> Chlamydia
<400> 302
   Met Thr Lys His Gly Lys Arg Ile Arg Gly Ile Gln Glu Thr Tyr Asp
   5 10 15
   Leu Ala Lys Ser Tyr Ser Leu Gly Glu Ala Ile Asp Ile Leu Lys Gln
               20                  25                  30
   Cys Pro Thr Val Arg Phe Asp Gln Thr Val Asp Val Ser Val Lys Leu
           35                  40                  45
   Gly Ile Asp Pro Arg Lys Ser Asp Gln Gln Ile Arg Gly Ser Val Ser
       50                  55                  60
   Leu Pro His Gly Thr Gly Lys Val Leu Arg Ile Leu Val Phe Ala Ala
   65                  70                  75                  80
   Gly Asp Lys Ala Ala Glu Ala Ile Glu Ala Gly Ala Asp Phe Val Gly
                   85                  90                  95
   Ser Asp Asp Leu Val Glu Lys Ile Lys Gly Gly Trp Val Asp Phe Asp
               100                 105                 110
   Val Ala Val Ala Thr Pro Asp Met Met Arg Glu Val Gly Lys Leu Gly
           115                 120                 125
   Lys Val Leu Gly Pro Arg Asn Leu Met Pro Thr Pro Lys Ala Gly Thr
       130                 135                 140
   Val Thr Thr Asp Val Val Lys Thr Ile Ala Glu Leu Arg Lys Gly Lys
   145                 150                 155                 160
   Ile Glu Phe Lys Ala Asp Arg Ala Gly Val Cys Asn Val Gly Val Ala
                   165                 170                 175
   Lys Leu Ser Phe Asp Ser Ala Gln Ile Lys Glu Asn Val Glu Ala Leu
               180                 185                 190
   Cys Ala Ala Leu Val Lys Ala Lys Pro Ala Thr Ala Lys Gly Gln Tyr
           195                 200                 205
   Leu Val Asn Phe Thr Ile Ser Ser Thr Met Gly Pro Gly Val Thr Val
       210                 215                 220
   Asp Thr Arg Glu Leu Ile Ala Leu
   225                 230
<210> 303
   <211> 238
   <212> PRT
   <213> chlamydia
<400> 303
   Ile Asn Ser Lys Leu Glu Thr Lys Asn Leu Ile Tyr Leu Lys Leu Lys
                   5                   10                  15
   Ile Lys Lys Ser Phe Lys Met Gly Asn Ser Gly Phe Tyr Leu Tyr Asn
               20                  25                  30
   Thr Gln Asn Cys Val Phe Ala Asp Asn Ile Lys Val Gly Gln Met Thr
           35                  40                  45
   Glu Pro Leu Lys Asp Gln Gln Ile Ile Leu Gly Thr Thr Ser Thr Pro
       50                  55                  60
   Val Ala Ala Lys Met Thr Ala Ser Asp Gly Ile Ser Leu Thr Val Ser
   65                  70                  75                  80
   Asn Asn Pro Ser Thr Asn Ala Ser Ile Thr Ile Gly Leu Asp Ala Glu
                   85                  90                  95
   Lys Ala Tyr Gln Leu Ile Leu Glu Lys Leu Gly Asp Gln Ile Leu Gly
               100                 105                 110
   Gly Ile Ala Asp Thr Ile Val Asp Ser Thr Val Gln Asp Ile Leu Asp
           115                 120                 125
   Lys Ile Thr Thr Asp Pro Ser Leu Gly Leu Leu Lys Ala Phe Asn Asn
       130                 135                 140
   Phe Pro Ile Thr Asn Lys Ile Gln Cys Asn Gly Leu Phe Thr Pro Arg
   145                 150                 155                 160
   Asn Ile Glu Thr Leu Leu Gly Gly Thr Glu Ile Gly Lys Phe Thr Val
                   165                 170                 175
   Thr Pro Lys Ser Ser Gly Ser Met Phe Leu Val Ser Ala Asp Ile Ile
               180                 185                 190
   Ala Ser Arg Met Glu Gly Gly Val Val Leu Ala Leu Val Arg Glu Gly
           195                 200                 205
   Asp Ser Lys Pro Tyr Ala Ile Ser Tyr Gly Tyr Ser Ser Gly Val Pro
       210                 215                 220
   Asn Leu Cys Ser Leu Arg Thr Arg Ile Ile Asn Thr Gly Leu
   225                 230                 235

## Description

### TECHNICAL FIELD

The present invention relates generally to the detection and treatment of Chlamydial infection. In particular, the invention is related to polypeptides comprising a *Chlamydia* antigen and the use of such polypeptides in the manufacture of a medicament for treatment of Chlamydial infection.

### BACKGROUND OF THE INVENTION

Chlamydiae are intracellular bacterial pathogens that are responsible for a wide variety of important human and animal infections. *Chlamydia trachomatis* is one of the most common causes of sexually transmitted diseases and can lead to pelvic inflammatory disease (PID), resulting in tubal obstruction and infertility. *Chlamydia trachomatis* may also play a role in male infertility. In 1990, the cost of treating PID in the US was estimated to be $4 billion. Trachoma, due to ocular infection with *Chlamydia trachomatis,* is the leading cause of preventable blindness worldwide. *Chlamydia pneumonia* is a major cause of acute respiratory tract infections in humans and is also believed to play a role in the pathogenesis of atherosclerosis and, in particular, coronary heart disease. Individuals with a high titer of antibodies to *Chlamydia pneumonia* have been shown to be at least twice as likely to suffer from coronary heart disease as seronegative individuals. Chlamydial infections thus constitute a significant health problem both in the US and worldwide.

Chlamydial infection is often asymptomatic. For example, by the time a woman seeks medical attention for PID, irreversible damage may have already occurred resulting in infertility. There thus remains a need in the art for improved vaccines and pharmaceutical compositions for the prevention and treatment of *Chlamydia* infections. The present invention fulfills this need and further provides other related advantages.

### SUMMARY OF THE INVENTION

The present invention provides pharmaceutical compositions and vaccines for use in therapy of *Chlamydia* infection. In one aspect, the present invention provides pharmaceutical compositions and vaccines comprising polypeptides, wherein the polypeptides comprise an immunogenic portion of a *Chlamydia* antigen, or a variant of such an antigen. Certain portions and other variants are immunogenic, such that the ability of the variant to react with antigen-specific antisera is not substantially diminished. Within certain embodiments" the polypeptide comprises an amino acid sequence set forth in SEQ ID NO. 5 or a variant thereof having at least 90% identity thereto. In other embodiments the polypeptide comprises an amino acid sequence set forth in any one of SEQ ID NO.5 6, 39, 94, 95, 96 or 98, or a variant thereof having at least 90% identity thereto.

The present invention further provides pharmaceutical compositions and vaccines comprising polynucleotides that encode a polypeptide as described above, or a portion thereof (such as a portion encoding at least 15 amino acid residues of a *Chlamydial* protein).

In another aspect, the present invention provides pharmaceutical compositions and vaccines comprising fusion proteins comprising an a polypeptide, as described above. The pharmaceutical compositions comprise a physiologically acceptable carrier. The vaccines comprise an immunostimulant.

In yet another aspect, methods of manufacturing a medicament for inducing protective, immunity in a patient, are provided, wherein the medicament may be administered to a patient an effective amount of one or more of the above pharmaceutical compositions or vaccines.

These and other aspects of the present invention will become apparent upon reference to the following detailed description.

### SEQUENCE IDENTIFIERS

SEQ ID NO: 1 is the determined DNA sequence for the *C. trachomatis* clone 1-B1-66.
SEQ ID NO: 2 is the determined DNA sequence for the *C. trachomatis* clone 4-D7-28.
SEQ ID NO: 3 is the determined DNA sequence for the *C. trachomatis* clone 3-G3-10.
SEQ ID NO: 4 is the determined DNA sequence for the *C. trachomatis* clone 10-C10-31.
SEQ ID NO: 5 is the predicted amino acid sequence for 1-B1-66.
SEQ ID NO: 6 is the predicted amino acid sequence for 4-D7-28.
SEQ ID NO: 7 is a first predicted amino acid sequence for 3-G3-10.
SEQ ID NO: 8 is a second predicted amino acid sequence for 3-G3-10.
SEQ ID NO: 9 is a third predicted amino acid sequence for 3-G3-10.
SEQ ID NO: 10 is a fourth predicted amino acid sequence for 3-G3-10.
SEQ ID NO: 11 is a fifth predicted amino acid sequence for 3-G3-10.
SEQ ID NO: 12 is the predicted amino acid sequence for 10-C10-31.
SEQ ID NO: 13 is the amino acid sequence of the synthetic peptide 1-B1-66/48-67.
SEQ ID NO: 14 is the amino acid sequence of the synthetic peptide 1-B1-66/58-77.
SEQ ID NO: 15 is the determined DNA sequence for the *C. trachomatis* serovar LGV II clone 2C7-8
SEQ ID NO: 16 is the determined DNA sequence for a first putative open reading frame from *C. trachomatis* serovar D
SEQ ID NO: 17 is the predicted amino acid sequence encoded by the first putative open reading frame from *C. trachomatis* serovar D
SEQ ID NO: 18 is the amino acid sequence of the synthetic peptide CtC7.8-12
SEQ ID NO: 19 is the amino acid sequence of the synthetic peptide CtC7.8-13
SEQ ID NO: 20 is the predicted amino acid sequence encoded by a second putative open reading from *C. trachomatis* serovar D
SEQ ID NO: 21 is the determined DNA sequence for clone 4C9-18 from *C. trachomatis* LGV II
SEQ ID NO: 22 is the determined DNA sequence homologous to Lipoamide Dehydrogenase from *C. trachomatis* LGV II
SEQ ID NO: 23 is the determined DNA sequence homologous to Hypothetical protein from *C. trachomatis* LGV II
SEQ ID NO: 24 is the determined DNA sequence homologous to Ubiquinone Mehtyltransferase from *C. trachomatis* LGV II
SEQ ID NO: 25 is the determined DNA sequence for clone 4C9-18#2 BL21 pLysS from *C. trachomatis* LGV II
SEQ ID NO: 26 is the predicted amino acid sequence for 4C9-18#2 from *C. trachomatis* LGV II
SEQ ID NO: 27 is the determined DNA sequence for Cp-SWIB from *C. pneumonia* strain TWAR
SEQ ID NO: 28 is the predicted amino acid sequence for Cp-S WIB from *C. pneumonia* strain TWAR
SEQ ID NO: 29 is the determined DNA sequence for Cp-S13 from *C. pneumonia* strain TWAR
SEQ ID NO: 30 is the predicted amino acid sequence for Cp-S 13 from *C. pneumonia* strain TWAR
SEQ ID NO: 31 is the amino acid sequence for a 10mer consensus peptide from CtC7.8-12 and CtC7.8-13
SEQ ID NO: 32 is the predicted amino acid sequence for clone 2C7-8 from *C. trachomatis* LGV II
SEQ ID NO: 33 is the determined DNA sequence of a clone from *C. trachomatis* serovar D which shows homology to clone 2C7-8
SEQ ID NO: 34 is the predicted amino acid sequence encoded by the sequence of SEQ ID NO: 33
SEQ ID NO: 35 is the DNA sequence for C.p. SWIB Nde (5' primer) from *C. pneumonia*
SEQ ID NO: 36 is the DNA sequence for C.p. SWIB EcoRI (3' primer) from *C pneumonia*
SEQ ID NO : 37 is the DNA sequence for C.p. S13 Nde (5' primer) from *C. pneumonia*
SEQ ID NO: 38 is the DNA sequence for C.p. S13 EcoRI (3' primer) from *C. pneumonia*
SEQ ID NO: 39 is the amino acid sequence for CtSwib 52-67 peptide from *C. trachomatis* LGV II
SEQ ID NO: 40 is the amino acid sequence for CpSwib 53-68 peptide from *C. pneumonia*
SEQ ID NO: 41 is the amino acid sequence for HuSwib 288-302 peptide from Human SWI domain
SEQ ID NO: 42 is the amino acid sequence for CtSWI-T 822-837 peptide from the topoisomerase-SWIB fusion of *C. trachomatis*
SEQ ID NO: 43 is the amino acid sequence for CpSWI-T 828-842 peptide from the topoisomerase-SWIB fusion of *C. pneumonia*
SEQ ID NO: 44 is a first determined DNA sequence for the *C. trachomatis* LGV II clone 19783.3,jen.seq(1>509)CTL2#11-3', representing the 3' end.
SEQ ID NO: 45 is a second determined DNA sequence for the *C. trachomatis* LGV II clone 19783.4,jen.seq(1>481)CTL2#11-5', representing the 5' end.
SEQ ID NO: 46 is the determined DNA sequence for the *C. trachomatis* LGV II clone 19784CTL2-12consensus.seq(1>427)CTL2#12.
SEQ ID NO: 47 is the determined DNA sequence for the *C. trachomatis* LGV II clone 19785.4,jen.seq(1>600)CTL2#16-5', representing the 5' end.
SEQ ID NO: 48 is a first determined DNA sequence for the *C. trachomatis* LGV II clone 19786.3 jen.seq(1>600)CTL2# 18-3', representing the 3' end.
SEQ ID NO: 49 is a second determined DNA sequence for the *C. trachomatis* LGV II clone 19786.4jen.seq(1>600)CTL2#18-5', representing the 5' end.
SEQ ID NO: 50 is the determined DNA sequence for the *C. trachomatis* LGV II clone 19788CTL2_21consensus.seq(1>406)CTL2#21.
SEQ ID NO: 51 is the determined DNA sequence for the *C. trachomatis* LGV II clone 19790CTL2_23consensus.seq(1>602)CTL2#23.
SEQ ID NO: 52 is the determined DNA sequence for the *C. trachomatis* LGV II clone 19791CTL2 24consensus.seq(1>145)CTL2#24.
SEQ ID NO: 53 is the determined DNA sequence for the *C. trachomatis* LGV II clone CTL2#4.
SEQ ID NO: 54 is the determined DNA sequence for the *C. trachomatis* LGV II clone CTL2#8b.
SEQ ID NO: 55 is the determined DNA sequence for the *C. trachomatis* LGV II clone15-G1-89, sharing homology to the lipoamide dehydrogenase gene CT557.
SEQ ID NO: 56 is the determined DNA sequence for the *C. trachomatis* LGV II clone 14-H1-4, sharing homology to the thiol specific antioxidant gene CT603.
SEQ ID NO: 57 is the determined DNA sequence for the *C. trachomatis* LGV II clone 12-G3-83, sharing homology to the hypothetical protein CT622.
SEQ ID NO: 58 is the determined DNA sequence for the *C. trachomatis* LGV II clone 12-B3-95, sharing homology to the lipoamide dehydrogenase gene CT557.
SEQ ID NO: 59 is the determined DNA sequence for the *C. trachomatis* LGV II clone 11-H4-28, sharing homology to the dnaK gene CT396.
SEQ ID NO: 60 is the determined DNA sequence for the *C. trachomatis* LGV II clone 11-H3-68, sharing partial homology to the PGP6-D virulence protein and L1 ribosomal gene CT318.
SEQ ID NO: 61 is the determined DNA sequence for the *C*. *trachomatis* LGV II clone 11-G1-34, sharing partial homology to the malate dehydrogenase gene CT3 76 and to the glycogen hydrolase gene CT042.
SEQ ID NO: 62 is the determined DNA sequence for the *C. trachomatis* LGV II clone 11-G10-46, sharing homology to the hypothetical protein CT610.
SEQ ID NO: 63 is the determined DNA sequence for the *C. trachomatis* LGV II clone 11-C12-91, sharing homology to the OMP2 gene CT443.
SEQ ID NO: 64 is the determined DNA sequence for the *C. trachomatis* LGV II clone 11-A3-93, sharing homology to the HAD superfamily gene CT103.
SEQ ID NO: 65 is the determined amino acid sequence for the *C. trachomatis* LGV II clone 14-H1-4, sharing homology to the thiol specific antioxidant gene CT603.
SEQ ID NO: 66 is the determined DNA sequence for the *C. trachomatis* LGV II clone CtL2#9.
SEQ ID NO: 67 is the determined DNA sequence for the *C*. *trachomatis* LGV II clone CtL2#7.
SEQ ID NO: 68 is the determined DNA sequence for the *C*. *trachomatis* LGV II clone CtL2#6.
SEQ ID NO: 69 is the determined DNA sequence for the *C*. *trachomatis* LGV II clone CtL2#5.
SEQ ID NO: 70 is the determined DNA sequence for the *C*. *trachomatis* LGV II clone CtL2#2.
SEQ ID NO: 71 is the determined DNA sequence for the *C*. *trachomatis* LGV II clone CtL2#1.
SEQ ID NO: 72 is a first determined DNA sequence for the *C*. *trachomatis* LGV II clone 23509.2CtL2#3-5', representing the 5' end.
SEQ ID NO: 73 is a second determined DNA sequence for the *C*. *trachomatis* LGV II clone 23509.1CtL2#3-3', representing the 3' end.
SEQ ID NO: 74 is a first determined DNA sequence for the *C*. *trachomatis* LGVII clone 22121.2CtL2#10-5', representing the 5' end.
SEQ ID NO: 75 is a second determined DNA sequence for the *C*. *trachomatis* LGV II clone 22121.1CtL2#10-3', representing the 3' end.
SEQ ID NO: 76 is the determined DNA sequence for the *C*. *trachomatis* LGV II clone 19787.6CtL2#19-5', representing the 5' end.
SEQ ID NO: 77 is the determined DNA sequence for the *C*. *pneumoniae* LGV II clone CpS13-His.
SEQ ID NO: 78 is the determined DNA sequence for the *C*. *pneumoniae* LGV II clone Cp_SWIB-His.
SEQ ID NO: 79 is the determined DNA sequence for the *C*. *trachomatis* LGV II clone 23-G7-68, sharing partial homology to the L11, L10 and L1 ribosomal protein.
SEQ ID NO: 80 is the determined DNA sequence for the *C*. *trachomatis* LGV II clone 22-F8-91, sharing homology to the pmpC gene.
SEQ ID NO: 81 is the determined DNA sequence for the *C*. *trachomatis* LGV II clone 21-E8-95, sharing homology to the CT610-CT613 genes.
SEQ ID NO: 82 is the determined DNA sequence for the *C*. *trachomatis* LGV II clone 19-F12-57, sharing homology to the CT858 and recA genes.
SEQ ID NO: 83 is the determined DNA sequence for the *C*. *trachomatis* LGV II clone 19-F12-53, sharing homology to the CT445 gene encoding glutamyl tRNA synthetase.
SEQ ID NO: 84 is the determined DNA sequence for the *C*. *trachomatis* LGV II clone 19-A5-54, sharing homology to the cryptic plasmid gene.
SEQ ID NO: 85 is the determined DNA sequence for the *C*. *trachomatis* LGV II clone 17-E11-72, sharing partial homology to the OppC_2 and pmpD genes.
SEQ ID NO: 86 is the determined DNA sequence for the *C*. *trachomatis* LGV II clone 17-C1-77, sharing partial homology to the CT857 and CT858 open reading frames.
SEQ ID NO: 87 is the determined DNA sequence for the *C*. *trachomatis* LGV II clone 15-H2-76, sharing partial homology to the pmpD and SycE genes, and to the CT089 ORF.
SEQ ID NO: 88 is the determined DNA sequence for the *C*. *trachomatis* LGV II clone 15-A3-26, sharing homology to the CT858 ORF.
SEQ ID NO: 89 is the determined amino acid sequence for the *C*. *pnuemoniae* clone Cp_SWIB-His.
SEQ ID NO: 90 is the determined amino acid sequence for the *C*. *trachomatis* LGV II clone CtL2_LPDA_FL.
SEQ ID NO: 91 is the determined amino acid sequence for the *C pnuemoniae* clone CpS 13-His.
SEQ ID NO: 92 is the determined amino acid sequence for the *C*. *trachomatis* LGV II clone CtL2_TSA_FL.
SEQ ID NO: 93 is the amino acid sequence for Ct-Swib 43-61 peptide from *C*. *trachomatis* LGV II.
SEQ ID NO: 94 is the amino acid sequence for Ct-Swib 48-67 peptide from *C*. *trachomatis* LGV II.
SEQ ID NO: 95 is the amino acid sequence for Ct-Swib 52-71 peptide from *C*. *trachomatis* LGV II.
SEQ ID NO: 96 is the amino acid sequence for Ct-Swib 58-77 peptide from *C*. *trachomatis* LGV II.
SEQ ID NO: 97 is the amino acid sequence for Ct-Swib 63-82 peptide from *C*. *trachomatis* LGV II.
SEQ ID NO: 98 is the amino acid sequence for Ct-Swib 51-66 peptide from *C*. *trachomatis* LGV II.
SEQ ID NO: 99 is the amino acid sequence for Cp-Swib 52-67 peptide from *C. pneumonia.*
SEQ ID NO: 100 is the amino acid sequence for Cp-Swib 37-51 peptide from *C. pneumonia.*
SEQ ID NO: 101 is the amino acid sequence for Cp-Swib 32-51 peptide from *C. pneumonia.*
SEQ ID NO: 102 is the amino acid sequence for Cp-Swib 37-56 peptide from *C. pneumonia.*
SEQ ID NO: 103 is the amino acid sequence for Ct-Swib 36-50 peptide from *C. trachomatis.*
SEQ ID NO: 104 is the amino acid sequence for Ct-S 13 46-65 peptide from *C*. *trachomatis.*
SEQ ID NO: 105 is the amino acid sequence for Ct-S13 60-80 peptide from *C*. *trachomatis.*
SEQ ID NO: 106 is the amino acid sequence for Ct-S13 1-20 peptide from *C*. *trachomatis.*
SEQ ID NO: 107 is the amino acid sequence for Ct-S 13 46-65 peptide from *C*. *trachomatis.*
SEQ ID NO: 108 is the amino acid sequence for Ct-S13 56-75 peptide from *C*. *trachomatis.*
SEQ ID NO: 109 is the amino acid sequence for Cp-S 13 56-75 peptide from *C. pneumoniae.*
SEQ ID NO: 110 is the determined DNA sequence for the *C*. *trachomatis* LGV II clone 21-G12-60, containing partial open reading frames for hypothetical proteins CT875, CT229 and CT228.
SEQ ID NO: 111 is the determined DNA sequence for the *C*. *trachomatis* LGV II clone 22-B3-53, sharing homology to the CT110 ORF of GroEL.
SEQ ID NO: 112 is the determined DNA sequence for the *C*. *trachomatis* LGV II clone 22-A1-49, sharing partial homology to the CT660 and CT659 ORFs.
SEQ ID NO: 113 is the determined DNA sequence for the *C*. *trachomatis* LGV II clone 17-E2-9, sharing partial homology to the CT611 and CT 610 ORFs.
SEQ ID NO: 114 is the determined DNA sequence for the *C*. *trachomatis* LGV II clone 17-C10-31, sharing partial homology to the CT858 ORF.
SEQ ID NO: 115 is the determined DNA sequence for the *C*. *trachomatis* LGV II clone 21-C7-66, sharing homology to the dnaK-like gene.
SEQ ID NO: 116 is the determined DNA sequence for the *C*. *trachomatis* LGV II clone 20-G3-45, containing part of the pmpB gene CT413.
SEQ ID NO: 117 is the determined DNA sequence for the *C*. *trachomatis* LGV II clone 18-C5-2, sharing homology to the S 1 ribosomal protein ORF.
SEQ ID NO: 118 is the determined DNA sequence for the *C*. *trachomatis* LGV II clone 17-C5-19, containing part of the ORFs for CT431 and CT430.
SEQ ID NO: 119 is the determined DNA sequence for the *C*. *trachomatis* LGV II clone 16-D4-22, contains partial sequences of ORF3 and ORF4 of the plasmid for growth within mammalian cells.
SEQ ID NO: 120 is the determined full-length DNA sequence for the *C*. *trachomatis* serovar LGV II Cap gene CT529.
SEQ ID NO: 121 is the predicted full-length amino acid sequence for the *C*. *trachomatis* serovar LGV II Cap1 gene CT529.
SEQ ID NO: 122 is the determined full-length DNA sequence for the *C*. *trachomatis* serovar E Cap 1 gene CT529.
SEQ ID NO: 123 is the predicted full-length amino acid sequence for the *C*. *trachomatis* serovar E Cap 1 gene CT529.
SEQ ID NO: 124 is the determined full-length DNA sequence for the *C*. *trachomatis* serovar 1A Cap 1 gene CT529.
SEQ ID NO: 125 is the predicted full-length amino acid sequence for the *C*. *trachomatis* serovar 1A Cap 1 gene CT529.
SEQ ID NO: 126 is the determined full-length DNA sequence for the *C*. *trachomatis* serovar G Cap1 gene CT529.
SEQ ID NO: 127 is the predicted full-length amino acid sequence for the *C*. *trachomatis* serovar G Cap1 gene CT529.
SEQ ID NO: 128 is the determined full-length DNA sequence for the *C*. *trachomatis* serovar F1 NII Cap 1 gene CT529.
SEQ ID NO: 129 is the predicted full-length amino acid sequence for the *C*. *trachomatis* serovar F1 NII Cap 1 gene CT529.
SEQ ID NO: 130 is the determined full-length DNA sequence for the *C*. *trachomatis* serovar L1 Cap1 gene CT529.
SEQ ID NO: 131 is the predicted full-length amino acid sequence for the *C*. *trachomatis* serovar L1 Cap 1 gene CT529.
SEQ ID NO: 132 is the determined full-length DNA sequence for the *C*. *trachomatis* serovar L3 Cap1 gene CT529.
SEQ ID NO: 133 is the predicted full-length amino acid sequence for the *C*. *trachomatis* serovar L3 Cap1 gene CT529.
SEQ ID NO: 134 is the determined full-length DNA sequence for the *C*. *trachomatis* serovar Ba Cap 1 gene CT529.
SEQ ID NO: 135 is the predicted full-length amino acid sequence for the *C*. *trachomatis* serovar Ba Cap1 gene CT529.
SEQ ID NO: 136 is the determined full-length DNA sequence for the *C*. *trachomatis* serovar MOPN Cap1 gene CT529.
SEQ ID NO: 137 is the predicted full-length amino acid sequence for the *C*. *trachomatis* serovar MOPN Cap1 gene CT529.
SEQ ID NO: 138 is the determined amino acid sequence for the Cap1 CT529 ORF peptide #124-139 of *C*. *trachomatis* serovar L2.
SEQ ID NO: 139 is the determined amino acid sequence for the Cap1 CT529 ORF peptide #132-147 of *C*. *trachomatis* serovar L2.
SEQ ID NO: 140 is the determined amino acid sequence for the Cap 1 CT529 ORF peptide #138-155 of *C*. *trachomatis* serovar L2.
SEQ ID NO: 141 is the determined amino acid sequence for the Cap 1 CT529 ORF peptide #146-163 of *C*. *trachomatis* serovar L2.
SEQ ID NO: 142 is the determined amino acid sequence for the Cap 1 CT529 ORF peptide #154-171 of *C*. *trachomatis* serovar L2.
SEQ ID NO: 143 is the determined amino acid sequence for the Cap1 CT529 ORF peptide #162-178 of *C*. *trachomatis* serovar L2.
SEQ ID NO: 144 is the determined amino acid sequence for the Cap1 CT529 ORF peptide # 138-147 of *C. trachomatis* serovar L2.
SEQ ID NO: 145 is the determined amino acid sequence for the Cap1 CT529 ORF peptide #139-147 of *C. trachomatis* serovar L2.
SEQ ID NO: 146 is the determined amino acid sequence for the Cap1 CT529 ORF peptide #140-147 of *C. trachomatis* serovar L2.
SEQ ID NO: 147 is the determined amino acid sequence for the Cap1 CT529 ORF peptide #138-146 of *C. trachomatis* serovar L2.
SEQ ID NO: 148 is the determined amino acid sequence for the Cap1 CT529 ORF peptide # 138-145 of *C. trachomatis* serovar L2.
SEQ ID NO: 149 is the determined amino acid sequence for the Cap1 CT529 ORF peptide # F140->I of *C. trachomatis* serovar L2.
SEQ ID NO: 150 is the determined amino acid sequence for the Cap1 CT529 ORF peptide # #S139>Ga of *C. trachomatis* serovar L2.
SEQ ID NO: 151 is the determined amino acid sequence for the Cap1 CT529 ORF peptide # #S139>Gb of *C. trachomatis* serovar L2.
SEQ ID NO: 152 is the determined amino acid sequence for the peptide # 2 C7.8-6 of the 216aa ORF of *C. trachomatis* serovar L2.
SEQ ID NO: 153 is the determined amino acid sequence for the peptide # 2 C7.8-7 of the 216aa ORF of *C. trachomatis* serovar L2.
SEQ ID NO: 154 is the determined amino acid sequence for the peptide # 2 C7.8-8 of the 216aa ORF of *C. trachomatis* serovar L2.
SEQ ID NO: 155 is the determined amino acid sequence for the peptide # 2 C7.8-9 of the 216aa ORF of *C. trachomatis* serovar L2.
SEQ ID NO: 156 is the determined amino acid sequence for the peptide # 2 C7.8-10 of the 216aa ORF of *C. trachomatis* serovar L2.
SEQ ID NO: 157 is the determined amino acid sequence for the 53 amino acid residue peptide of the 216aa ORF within clone 2C7.8 of *C. trachomatis* serovar L2.
SEQ ID NO: 158 is the determined amino acid sequence for the 52 amino acid residue peptide of the CT529 ORF within clone 2C7.8 of *C. trachomatis* serovar L2.
SEQ ID NO: 159 is the determined DNA sequence for the 5' (forward) primer for cloning full-length CT529 serovar L2.
SEQ ID NO: 160 is the determined DNA sequence for the 5' (reverse) primer for cloning full-length CT529 serovar L2.
SEQ ID NO: 161 is the determined DNA sequence for the 5' (forward) primer for cloning full-length CT529 for serovars other than L2 and MOPN.
SEQ ID NO: 162 is the determined DNA sequence for the 5' (reverse) primer for cloning full-length CT529 serovars other than L2 and MOPN.
SEQ ID NO: 163 is the determined DNA sequence for the 5' (forward) primer for cloning full-length CT529 serovar MOPN.
SEQ ID NO: 164 is the determined DNA sequence for the 5' (reverse) primer for cloning full-length CT529 serovar MOPN.
SEQ ID NO: 165 is the determined DNA sequence for the 5' (forward) primer for pBIB-KS.
SEQ ID NO: 166 is the determined DNA sequence for the 5' (reverse) primer for pBIB-KS.
SEQ ID NO: 167 is the determined amino acid sequence for the 9-mer epitope peptide Cap1#139-147 from serovar L2.
SEQ ID NO: 168 is the determined amino acid sequence for the 9-mer epitope peptide Cap1#139-147 from serovar D.
SEQ ID NO: 169 is the determined full-length DNA sequence for the *C. trachomatis* pmpI gene.
SEQ ID NO: 170 is the determined full-length DNA sequence for the *C. trachomatis* pmpG gene.
SEQ ID NO: 171 is the determined full-length DNA sequence for the *C. trachomatis* pmpE gene.
SEQ ID NO: 172 is the determined full-length DNA sequence for the *C. trachomatis* pmpD gene.
SEQ ID NO: 173 is the determined full-length DNA sequence for the *C. trachomatis* pmpC gene.
SEQ ID NO: 174 is the determined full-length DNA sequence for the *C. trachomatis* pmpB gene.
SEQ ID NO: 175 is the predicted full-length amino acid sequence for the *C. trachomatis* pmpI gene.
SEQ ID NO: 176 is the predicted full-length amino acid sequence for the *C. trachomatis* pmpG gene.
SEQ ID NO: 177 is the predicted full-length amino acid sequence for the *C. trachomatis* pmpE gene.
SEQ ID NO: 178 is the predicted full-length amino acid sequence for the *C. trachomatis* pmpD gene.
SEQ ID NO: 179 is the predicted full-length amino acid sequence for the *C. trachomatis* pmpC gene.
SEQ ID NO: 180 is the predicted full-length amino acid sequence for the *C. trachomatis* pmpB gene.
SEQ ID NO: 181 is the determined DNA sequence minus the signal sequence for the *C. trachomatis* pmpI gene.
SEQ ID NO: 182 is a subsequently determined full-length DNA sequence for the *C. trachomatis* pmpG gene.
SEQ ID NO: 183 is the determined DNA sequence minus the signal sequence for the *C. trachomatis* pmpE gene.
SEQ ID NO: 184 is a first determined DNA sequence representing the carboxy terminus for the *C. trachomatis* pmpD gene.
SEQ ID NO: 185 is a second determined DNA sequence representing the amino terminus minus the signal sequnce for the *C. trachomatis* pmpD gene.
SEQ ID NO: 186 is a first determined DNA sequence representing the carboxy terminus for the *C. trachomatis* pmpC gene.
SEQ ID NO: 187 is a second determined DNA sequence representing the amino terminus minus the signal sequence for the *C. trachomatis* pmpC gene.
SEQ ID NO: 188 is the determined DNA sequence representing the *C. pneumoniae* serovar MOMPS pmp gene in a fusion molecule with Ra12.
SEQ ID NO: 189 is the predicted amino acid sequence minus the signal sequence for the *C. trachomatis* pmpI gene.
SEQ ID NO: 190 is subsequently predicted amino acid sequence for the *C. trachomatis* pmpG gene.
SEQ ID NO: 191 is the predicted amino acid sequence minus the signal sequence for the *C. trachomatis* pmpE gene.
SEQ ID NO: 192 is a first predicted amino acid sequence representing the carboxy terminus for the *C. trachomatis* pmpD gene.
SEQ ID NO: 193 is a second predicted amino acid sequence representing the Amino terminus minus the signal sequence for the *C. trachomatis* pmpD gene.
SEQ ID NO: 194 is a first predicted amino acid sequence representing the Carboxy terminus for the *C. trachomatis* pmpc gene.
SEQ ID NO: 195 is a second predicted amino acid sequence representing the Amino terminus for the *C. trachomatis* pmpC gene.
SEQ ID NO: 196 is the predicted amino acid sequence representing the *C. pneumoniae* serovar MOMPS pmp gene in a fusion molecule with Ra12.
SEQ ID NO: 197 is the determined DNA sequence for the 5' oligo primer for cloning the *C. trachomatis* pmpC gene in the SKB vaccine vector.
SEQ ID NO: 198 is the determined DNA sequence for the 3' oligo primer for cloning the *C. trachomatis* pmpC gene in the SKB vaccine vector.
SEQ ID NO: 199 is the determined DNA sequence for the insertion sequence for cloning the *C*. *trachomatis* pmpC gene in the SKB vaccine vector.
SEQ ID NO: 200 is the determined DNA sequence for the 5' oligo primer for cloning the *C. trachomatis* pmpD gene in the SKB vaccine vector.
SEQ ID NO: 201 is the determined DNA sequence for the 3' oligo primer for cloning the *C. trachomatis* pmpD gene in the SKB vaccine vector.
SEQ ID NO: 202 is the determined DNA sequence for the insertion sequence for cloning the *C. trachomatis* pmpD gene in the SKB vaccine vector.
SEQ ID NO: 203 is the determined DNA sequence for the 5' oligo primer for cloning the *C. trachomatis* pmpE gene in the SKB vaccine vector.
SEQ ID NO: 204 is the determined DNA sequence for the 3' oligo primer for cloning the *C. trachomatis* pmpE gene in the SKB vaccine vector.
SEQ ID NO: 205 is the determined DNA sequence for the 5' oligo primer for cloning the *C. trachomatis* pmpG gene in the SKB vaccine vector.
SEQ ID NO: 206 is the determined DNA sequence for the 3' oligo primer for cloning the *C. trachomatis* pmpG gene in the SKB vaccine vector.
SEQ ID NO: 207 is the determined DNA sequence for the 5' oligo primer for cloning the amino terminus portion of the *C*. *trachomatis* pmpC gene in the pET17b vector.
SEQ ID NO: 208 is the determined DNA sequence for the 3' oligo primer for cloning the amino terminus portion of the *C*. *trachomatis* pmpC gene in the pET17b vector.
SEQ ID NO: 209 is the determined DNA sequence for the 5' oligo primer for cloning the carboxy terminus portion of the *C*. *trachomatis* pmpC gene in the pET17b vector.
SEQ ID NO: 210 is the determined DNA sequence for the 3' oligo primer for cloning the carboxy terminus portion of the *C*. *trachomatis* pmpC gene in the pET17b vector.
SEQ ID NO: 211 is the determined DNA sequence for the 5' oligo primer for cloning the amino terminus portion of the *C*. *trachomatis* pmpD gene in the pET17b vector.
SEQ ID NO: 212 is the determined DNA sequence for the 3' oligo primer for cloning the amino terminus portion of the *C*. *trachomatis* pmpD gene in the pET17b vector.
SEQ ID NO: 213 is the determined DNA sequence for the 5' oligo primer for cloning the carboxy terminus portion of the *C*. *trachomatis* pmpD gene in the pET17b vector.
SEQ ID NO: 214 is the determined DNA sequence for the 3' oligo primer for cloning the carboxy terminus portion of the *C*. *trachomatis* pmpD gene in the pET17b vector.
SEQ ID NO: 215 is the determined DNA sequence for the 5' oligo primer for cloning the *C*. *trachomatis* pmpE gene in the pET17b vector.
SEQ ID NO: 216 is the determined DNA sequence for the 3' oligo primer for cloning the *C. trachomatis* pmpE gene in the pET17b vector.
SEQ ID NO: 217 is the determined DNA sequence for the insertion sequence for cloning the *C. trachomatis* pmpE gene in the pET17b vector.
SEQ ID NO: 218 is the amino acid sequence for the insertion sequence for cloning the *C. trachomatis* pmpE gene in the pET17b vector.
SEQ ID NO: 219 is the determined DNA sequence for the 5' oligo primer for cloning the *C. trachomatis* pmpG gene in the pET17b vector.
SEQ ID NO: 220 is the determined DNA sequence for the 3' oligo primer for cloning the *C. trachomatis* pmpG gene in the pET17b vector.
SEQ ID NO: 221 is the amino acid sequence for the insertion sequence for cloning the *C. trachomatis* pmpG gene in the pET17b vector.
SEQ ID NO: 222 is the determined DNA sequence for the 5' oligo primer for cloning the *C. trachomatis* pmpI gene in the pET17b vector.
SEQ ID NO: 223 is the determined DNA sequence for the 3' oligo primer for cloning the *C. trachomatis* pmpI gene in the pET17b vector.
SEQ ID NO: 224 is the determined amino acid sequence for the *C. pneumoniae* Swib peptide 1-20.
SEQ ID NO: 225 is the determined amino acid sequence for the *C. pneumoniae* Swib peptide 6-25.
SEQ ID NO: 226 is the determined amino acid sequence for the *C*. *pneumoniae* Swib peptide 12-31.
SEQ ID NO: 227 is the determined amino acid sequence for the *C*. *pneumoniae* Swib peptide 17-36.
SEQ ID NO: 228 is the determined amino acid sequence for the *C*. *pneumoniae* Swib peptide 22-41.
SEQ ID NO: 229 is the determined amino acid sequence for the *C*. *pneumoniae* Swib peptide 27-46.
SEQ ID NO: 230 is the determined amino acid sequence for the *C*. *pneumoniae* Swib peptide 42-61.
SEQ ID NO: 231 is the determined amino acid sequence for the *C*. *pneumoniae* Swib peptide 46-65.
SEQ ID NO: 232 is the determined amino acid sequence for the *C*. *pneumoniae* Swib peptide 51-70.
SEQ ID NO: 233 is the determined amino acid sequence for the *C*. *pneumoniae* Swib peptide 56-75.
SEQ ID NO: 234 is the determined amino acid sequence for the *C*. *pneumoniae* Swib peptide 61-80.
SEQ ID NO: 235 is the determined amino acid sequence for the *C*. *pneumoniae* Swib peptide 66-87.
SEQ ID NO: 236 is the determined amino acid sequence for the *C*. *trachomatis* OMCB peptide 103-122.
SEQ ID NO: 237 is the determined amino acid sequence for the *C*. *trachomatis* OMCB peptide 108-127.
SEQ ID NO: 238 is the determined amino acid sequence for the *C*. *trachomatis* OMCB peptide 113-132.
SEQ ID NO: 239 is the determined amino acid sequence for the *C*. *trachomatis* OMCB peptide 118-137.
SEQ ID NO: 240 is the determined amino acid sequence for the *C*. *trachomatis* OMCB peptide 123-143.
SEQ ID NO: 241 is the determined amino acid sequence for the *C*. *trachomatis* OMCB peptide 128-147.
SEQ ID NO: 242 is the determined amino acid sequence for the *C*. *trachomatis* OMCB peptide 133-152.
SEQ ID NO: 243 is the determined amino acid sequence for the *C*. *trachomatis* OMCB peptide 137-156.
SEQ ID NO: 244 is the determined amino acid sequence for the *C*. *trachomatis* OMCB peptide 142-161.
SEQ ID NO: 245 is the determined amino acid sequence for the *C*. *trachomatis* OMCB peptide 147-166.
SEQ ID NO: 246 is the determined amino acid sequence for the *C*. *trachomatis* OMCB peptide 152-171.
SEQ ID NO: 247 is the determined amino acid sequence for the *C*. *trachomatis* OMCB peptide 157-176.
SEQ ID NO: 248 is the determined amino acid sequence for the *C*. *trachomatis* OMCB peptide 162-181.
SEQ ID NO: 249 is the determined amino acid sequence for the *C*. *trachomatis* OMCB peptide 167-186.
SEQ ID NO: 250 is the determined amino acid sequence for the *C*. *trachomatis* OMCB peptide 171-190.
SEQ ID NO: 251 is the determined amino acid sequence for the *C*. *trachomatis* OMCB peptide 171-186.
SEQ ID NO: 252 is the determined amino acid sequence for the *C*. *trachomatis* OMCB peptide 175-186.
SEQ ID NO: 252 is the determined amino acid sequence for the *C*. *trachomatis* OMCB peptide 175-186.
SEQ ID NO: 253 is the determined amino acid sequence for the *C*. *pneumoniae* OMCB peptide 185-198.
SEQ ID NO: 254 is the determined amino acid sequence for the *C*. *trachomatis* TSA peptide 96-115.
SEQ ID NO: 255 is the determined amino acid sequence for the *C*. *trachomatis* TSA peptide 101-120.
SEQ ID NO: 256 is the determined amino acid sequence for the *C*. *trachomatis* TSA peptide 106-125.
SEQ ID NO: 257 is the determined amino acid sequence for the *C*. *trachomatis* TSA peptide 111-130.
SEQ ID NO: 258 is the determined amino acid sequence for the *C*. *trachomatis* TSA peptide 116-135.
SEQ ID NO: 259 is the determined amino acid sequence for the *C*. *trachomatis* TSA peptide 121-140.
SEQ ID NO: 260 is the determined amino acid sequence for the *C*. *trachomatis* TSA peptide 126-145.
SEQ ID NO: 261 is the determined amino acid sequence for the *C*. *trachomatis* TSA peptide 131-150.
SEQ ID NO: 262 is the determined amino acid sequence for the *C*. *trachomatis* TSA peptide 136-155.
SEQ ID NO: 263 is the determined full-length DNA sequence for the *C*. *trachomatis* CT529/Cap 1 gene serovar I.
SEQ ID NO: 264 is the predicted full-length amino sequence for the *C*. *trachomatis* CT529/Cap 1 gene serovar I.
SEQ ID NO: 265 is the determined full-length DNA sequence for the *C. trachomatis* CT529/Cap 1 gene serovar K.
SEQ ID NO: 266 is the predicted full-length amino sequence for the *C. trachomatis* CT529/Cap 1 gene serovar K.
SEQ ID NO: 267 is the determined DNA sequence for the *C. trachomatis* clone 17-G4-36 sharing homology to part of the ORF of DNA-dirrected RNA polymerase beta subunit- CT315 in serD.
SEQ ID NO: 268 is the determined DNA sequence for the partial sequence of the *C. trachomatis* CT016 gene in clone 2E10.
SEQ ID NO: 269 is the determined DNA sequence for the partial sequence of the *C. trachomatis* tRNA syntase gene in clone 2E10.
SEQ ID NO: 270 is the determined DNA sequence for the partial sequence for the *C. trachomatis* clpX gene in clone 2E10.
SEQ ID NO: 271 is a first determined DNA sequence for the *C. trachomatis* clone CtL2gam-30 representing the 5'end.
SEQ ID NO: 272 is a second determined DNA sequence for the *C. trachomatis* clone CtL2gam-30 representing the 3'end.
SEQ ID NO: 273 is the determined DNA sequence for the *C. trachomatis* clone CtL2gam-28.
SEQ ID NO: 274 is the determined DNA sequence for the *C*. *trachomatis* clone CtL2gam-27.
SEQ ID NO: 275 is the determined DNA sequence for the *C*. *trachomatis* clone CtL2gam-26.
SEQ ID NO: 276 is the determined DNA sequence for the *C*. *trachomatis* clone CtL2gam-24.
SEQ ID NO: 277 is the determined DNA sequence for the *C*. *trachomatis* clone CtL2gam-23.
SEQ ID NO: 278 is the determined DNA sequence for the *C*. *trachomatis* clone CtL2gam-21.
SEQ ID NO: 279 is the determined DNA sequence for the *C*. *trachomatis* clone CtL2gam-18.
SEQ ID NO: 280 is the determined DNA sequence for the *C. trachomatis* clone CtL2gam-17.
SEQ ID NO: 281 is a first determined DNA sequence for the *C. trachomatis* clone CtL2gam-15 representing the 5' end.
SEQ ID NO: 282 is a second determined DNA sequence for the *C. trachomatis* clone CtL2gam-15 representing the 3' end.
SEQ ID NO: 283 is the determined DNA sequence for the *C. trachomatis* clone CtL2gam-13.
SEQ ID NO: 284 is the determined DNA sequence for the *C*. *trachomatis* clone CtL2gam-10.
SEQ ID NO: 285 is the determined DNA sequence for the *C*. *trachomatis* clone CtL2gam-8.
SEQ ID NO: 286 is a first determined DNA sequence for the *C*. *trachomatis* clone CtL2gam-6 representing the 5' end.
SEQ ID NO: 287 is a second determined DNA sequence for the *C*. *trachomatis* clone CtL2gam-6 representing the 3' end.
SEQ ID NO: 288 is the determined DNA sequence for the *C*. *trachomatis* clone CtL2gam-5.
SEQ ID NO: 289 is the determined DNA sequence for the *C*. *trachomatis* clone CtL2gam-2.
SEQ ID NO: 290 is the determined DNA sequence for the *C*. *trachomatis* clone CtL2gam-1.
SEQ ID NO: 291 is the determined full-length DNA sequence for the *C*. *pneumoniae* homologue of the CT529 gene.
SEQ ID NO: 292 is the predicted full-length amino acid sequence for the *C*. *pneumoniae* homologue of the CT529 gene.
SEQ ID NO: 293 is the determined DNA sequence for the insertion sequence for cloning the *C. trachomatis* pmpG gene in the SKB vaccine vector.

### DESCRIPTION OF THE FIGURES

Fig. 1 illustrates induction of INF-γ from a *Chlamydia-specific* T cell line activated by target cells expressing clone 4C9-18#2.
Fig. 2 illustrates retroviral vectors pBIB-KS1,2,3 modified to contain a Kosak translation initiation site and stop codons.
Fig. 3 shows specific lysis in a chromium release assay of P815 cells pulsed with *Chlamydia* peptides CtC7.8-12 (SEQ ID NO: 18) and CtC7.8-13 (SEQ ID NO: 19).
Fig. 4 shows antibody isotype titers in C57B1/6 mice immunized with *C. trachomatis* SWIB protein.
Fig, 5 shows *Chlamydia-specific* T-cell proliferative responses in splenocytes from C3H mice immunized with *C. trachomatis* SWIB protein.
Fig. 6 illustrates the 5' and 3' primer sequences designed from *C. pneumoniae* which were used to isolate the SWIB and S13 genes from *C. pneumoniae.*
Figs. 7A and 7B show induction of IFN-γ from a human anti-*chlamydia* T-cell line (TCL-8) capable of cross-reacting to *C. trachomatis* and *C. pneumonia* upon activation by monocyte-derived dendritic cells expressing chlamydial proteins.
Fig. 8 shows the identification of T cell epitopes in Chlamydial ribosomal S13 protein with T-cell line TCL 8 EB/DC.
Fig. 9 illustrates the proliferative response of CP-21 T-cells generated against *C. pnuemoniae-*infected dendritic cells to recombinant *C. pneumonia*-SWIBprotein, but not *C. trachomatis* SWIB protein.
Fig. 10 shows the *C. trachomatis*-specific SWIB proliferative responses of a primary T-cell line (TCT-10 EB) from an asymptomatic donor.
Fig. 11 illustrates the identification of T-cell epitope in *C. trachomatis* SWIB with an antigen specific T-cell line (TCL-10 EB).

### DETAILED DESCRIPTION OF THE INVENTION

As noted above, the present invention is generally directed to pharmaceutical compositions and vaccines methods for the diagnosis and treatment of Chlamydial infection. In one aspect, the compositions of the subject invention include polypeptides that comprise at least one immunogenic portion of a *Chlamydia* antigen, or a variant thereof.

In specific embodiments, the subject invention discloses polypeptides comprising an immunogenic portion of a *Chlamydia* antigen, wherein the *Chlamydia* antigen comprises an amino acid sequence set forth in SEQ ID NO. 5 or a variant thereof having at least 90% identity thereto. In other embodiments the polypeptide comprises an amino acid sequence set forth in any one of SEQ ID NO.5 6, 39, 94, 95, 96 or 98, or a variant thereof having at least 90% identity thereto.

As used herein, the term "polypeptide" encompasses amino acid chains of any length, including full length proteins (*i.e*., antigens), wherein the amino acid residues are linked by covalent peptide bonds. Thus, a polypeptide comprising an immunogenic portion of one of the inventive antigens may consist entirely of the immunogenic portion, or may contain additional sequences. The additional sequences may be derived from the native *Chlamydia* antigen or may be heterologous, and such sequences may (but need not) be immunogenic.

The term "polynucleotide(s)," as used herein, means a single or doublestranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments.

An "immunogenic portion" of an antigen is a portion that is capable of reacting with sera obtained from a *Chlamydia-*infected individual (*i.e*., generates an absorbance reading with sera from infected individuals that is at least three standard deviations above the absorbance obtained with sera from uninfected individuals, in a representative ELISA assay described herein). Such immunogenic portions generally comprise at least about 5 amino acid residues, more preferably at least about 10, and most preferably at least about 20 amino acid residues. Methods for preparing and identifying immunogenic portions of antigens of known sequence are well known in the art and include those summarized in Paul, Fundamental Immunology, 3rd ed., Raven Press, 1993, pp. 243-247 and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well known techniques. An immunogenic portion of a native *Chlamydia* protein is a portion that reacts with such antisera and/or T-cells at a level that is not substantially less than the reactivity of the full length polypeptide (*e.g*., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

Examples of immunogenic portions of antigens disclosed herein include, for example, the T cell stimulating epitopes provided in SEQ ID NO: 9, 10, 18, 19, 31, 39, 93-96, 98, 100-102, 106, 108, 138-140, 158, 167, 168, 246, 247 and 254-256. Polypeptides comprising at least an immunogenic portion of one or more *Chlamydia* antigens as described herein may generally be used, alone or in combination, to detect Chlamydial infection in a patient.

The compositions and vaccines of the present invention also encompass variants of the above polypeptides and polynucleotide molecules. Such variants include, but are not limited to, naturally occurring allelic variants of the inventive sequences. In particular, variants include other *Chlamydiae* serovars, such as serovars D, E and F, as well as the several LGV serovars which share homology to the inventive polypeptide and polynucleotide molecules described herein. Preferably, the serovar homologues show 95-99% homology to the corresponding polypeptide sequence(s) described herein.

A polypeptide "variant," as used herein, is a polypeptide that differs from the recited polypeptide only in conservative substitutions and/or modifications, such that the antigenic properties of the polypeptide are retained. In a preferred embodiment, variant polypeptides differ from an identified sequence by substitution, deletion or addition of five amino acids or fewer. Such variants may generally be identified by modifying one of the above polypeptide sequences, and evaluating the antigenic properties of the modified polypeptide using, for example, the representative procedures described herein. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g*., 1-30 amino acids, preferably 5-15 amino acids) has been removed from the N- and/or C-terminal of the mature protein. Polypeptide variants preferably exhibit at least about 70%, more preferably at least about 90% and most preferably at least about 95% identity (determined as described below) to the identified polypeptides.

As used herein, a "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide. Variants may also, or alternatively, contain other modifications, including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g*., poly-His), or to enhance binding of the polypeptide to a solid support. For example, a polypeptide may be conjugated to an immunoglobulin Fc region.

A polynucleotide "variant" is a sequence that differs from the recited nucleotide sequence in having one or more nucleotide deletions, substitutions or additions such that the immunogenicity of the encoded polypeptide is not diminished, relative to the native protein. The effect on the immunogenicity of the encoded polypeptide may generally be assessed as described herein. Such modifications may be readily introduced using standard mutagenesis techniques, such as oligonucleotide-directed site-specific mutagenesis as taught, for example, by Adelman et al. (DNA, 2:183, 1983). Nucleotide variants may be naturally occurring allelic variants as discussed below, or non-naturally occurring variants. Variant nucleotide sequences preferably exhibit at least about 70%, more preferably at least about 80% and most preferably at least about 90% identity (determined as described below) to the recited sequence.

The polypeptides provided for use in the present invention include variants that are encoded by polynucleotide sequences which are substantially homologous to one or more of the polynucleotide sequences specifically recited herein. "Substantial homology," as used herein, refers to polynucleotide sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1% SDS. Such hybridizing polynucleotide sequences are also within the scope of this of this disclosure, as are nucleotide sequences that, due to code degeneracy, encode a polypeptide that is the same as a polypeptide disclosed herein.

Two nucleotide or polypeptide sequences are said to be "identical" if the sequence of nucleotides or amino acid residues in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Resarch Foundaiton, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) Fast and sensitive multiple sequence alignments on a microcomputer CABIOS 5:151-153; Myers, E.W. and Muller W. (1988) Optimal alignments in linear space CABIOS 4:11-17; Robinson, E.D. (1971) Comb. Theor 11:105; Santou, N. Nes, M. (1987) The neighbor joining method. A new method for reconstructing phylogenetic trees Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Rapid similarity searches of nucleic acid and protein data banks Proc. Natl. Acad, Sci. USA 80:726-730.

Preferably, the "percentage of sequence identity" is determined by comparing two optimally aligned sequences over a window of comparison of at least 20 positions, wherein the portion of the polynucleotide sequence in the comparison window may comprise additions or deletions (i.e, gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Also disclosed herein are alleles of the genes encoding the nucleotide sequences recited in herein. As used herein, an "allele" or "allellic sequence" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone or in combination with the others, one or more times in a given sequence. Disclosed herein are polypeptides comprising at least an immunogenic portion of a *Chlamydia* antigen (or a variant of such an antigen), that comprises one or more of the amino acid sequences encoded by (a) a polynucleotide sequence selected from the group consisting of SEQ ID NO: 1-4, 15 21-25, 44-64, 66-76 and 79-88; (b) the complements of such DNA sequences or (c) DNA sequences substantially homologous to a sequence in (a) or (b). As discussed in the Examples below, several of the *Chlamydia* antigens disclosed herein recognize a T cell line that recognizes both *Chlamydia trachomatis* and *Chlamydia pneumoniae* infected monocyte-derived dendritic cells, indicating that they may represent an immunoreactive epitope shared by *Chlamydia trachomatis* and *Chlamydia pneumoniae.* The antigens may thus be employed in a vaccine for both C. *trachomatis* genital tract infections and for *C. pneumonia* infections. Further characterization of these *Chlamydia* antigens from *Chlamydia trachomatis* and *Chlamydia pneumonia* to determine the extent of cross-reactivity is provided in Example 6. Additionally, Example 4 describes cDNA fragments (SEQ ID NO: 15, 16 and 33) isolated from *C. trachomatis* which encode proteins (SEQ ID NO: 17-19 and 32) capable of stimulating a *Chlamydia-specific* murine CD8+ T cell line.

In general, *Chlamydia* antigens, and polynucleotide sequences encoding such antigens, may be prepared using any of a variety of procedures. For example, polynucleotide molecules encoding *Chlamydia* antigens may be isolated from a *Chlamydia* genomic or cDNA expression library by screening with a *Chlamydia*-specific T cell line as described below, and sequenced using techniques well known to those of skill in the art. Additionally, a polynucleotide may be identified, as described in more detail below, by screening a microarray of cDNAs for *Chlamydia*-associated expression (*i.e*., expression that is at least two fold greater in *Chlamydia*-infected cells than in controls, as determined using a representative assay provided herein). Such screens may be performed using a Synteni microarray (Palo Alto, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polypeptides may be amplified from cDNA prepared from cells expressing the proteins described herein.. Such polynucleotides may be amplified via polymerase chain reaction (PCR). For this approach, sequence-specific primers may be designed based on the sequences provided herein, and may be purchased or synthesized.

Antigens may be produced recombinantly, as described below, by inserting a polynucleotide sequence that encodes the antigen into an expression vector and expressing the antigen in an appropriate host. Antigens may be evaluated for a desired property, such as the ability to react with sera obtained from a *Chlamydia*-infected individual as described herein, and may be sequenced using, for example, traditional Edman chemistry. *See* Edman and Berg, Eur. J. Biochem. 80:116-132, 1967.

Polynucleotide sequences encoding antigens may also be obtained by screening an appropriate *Chlamydia* cDNA or genomic DNA library for polynucleotide sequences that hybridize to degenerate oligonucleotides derived from partial amino acid sequences of isolated antigens. Degenerate oligonucleotide sequences for use in such a screen may be designed and synthesized, and the screen may be performed, as described (for example) in Sambrook et al., *Molecular Cloning: A Laboratory Manual,* Cold Spring Harbor Laboratories, Cold Spring Harbor, NY (and references cited therein). Polymerase chain reaction (PCR) may also be employed, using the above oligonucleotides in methods well known in the art, to isolate a nucleic acid probe from a cDNA or genomic library. The library screen may then be performed using the isolated probe.

An amplified portion may be used to isolate a full length gene from a suitable library (*e.g.,* a *Chlamydia* cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g*., by nick-translation or end-labeling with ³²P) using well known techniques. A bacterial or bacteriophage library is then screened by hybridizing filters containing denatured bacterial colonies (or lawns containing phage plaques) with the labeled probe *(see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using techniques well known in the art (*see,* for example, Mullis et al., Cold Spring Harbor Symp. Quant. Biol. 51:263, 1987; Erlich ed., PCR Technology, Stockton Press, NY, 1989), and software well known in the art may also be employed. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR *(see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., PCR Methods Applic. 1:111-19, 1991) and walking PCR (Parker et al., *Nucl. Acids. Res. 19*:3055-60, 1991). Transcription-Mediated Amplification, or TMA is another method that may be utilized for the amplification of DNA, rRNA, or mRNA, as described in Patent No. PCT/US91/03184. This autocatalytic and isothermic non-PCR based method utilizes two primers and two enzymes: RNA polymerase and reverse transcriptase. One primer contains a promoter sequence for RNA polymerase. In the first amplification, the promoter-primer hybridizes to the target rRNA at a defined site. Reverse transcriptase creates a DNA copy of the target rRNA by extension from the 3'end of the promoter-primer. The RNA in the resulting complex is degraded and a second primer binds to the DNA copy. A new strand of DNA is synthesized from the end of the primer by reverse transcriptase creating double stranded DNA. RNA polymerase recognizes the promoter sequence in the DNA template and initiates transcription. Each of the newly synthesized RNA amplicons re-enters the TMA process and serves as a template for a new round of replication leading to the expotential expansion of the RNA amplicon. Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g*., NCBI BLAST searches), and such ESTs may be used to generate a contiguous full length sequence. Full length cDNA sequences may also be obtained by analysis of genomic fragments.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis *(see* Adelman et al., DNA 2:183, 1983). Alternatively, RNA molecules may be generated by *in vitro* or *in vivo* transcription of DNA sequences encoding a *Chlamydial* protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (*e.g.,* by transfecting antigen-presenting cells, such as dendritic cells, with a cDNA construct encoding a *Chlamydial* polypeptide, and administering the transfected cells to the patient).

A portion of a sequence complementary to a coding sequence (*i.e*., an antisense polynucleotide) may also be used as a probe or to modulate gene expression. cDNA constructs that can be transcribed into antisense RNA may also be introduced into cells of tissues to facilitate the production of antisense RNA. An antisense polynucleotide may be used, as described herein, to inhibit expression of a *Chlamydial* protein. Antisense technology can be used to control gene expression through triple-helix formation, which compromises the ability of the double helix to open sufficiently for the binding of polymerases, transcription factors or regulatory molecules (*see* Gee et al., In Huber and Carr, Molecular and Immunologic Approaches, Futura Publishing Co. (Mt. Kisco, NY; 1994)). Alternatively, an antisense molecule may be designed to hybridize with a control region of a gene (*e.g*., promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Synthetic polypeptides having fewer than about 100 amino acids, and generally fewer than about 50 amino acids, may be generated using techniques well known in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division, Foster City, CA, and may be operated according to the manufacturer's instructions.

As noted above, immunogenic portions of *Chlamydia* antigens may be prepared and identified using well known techniques, such as those summarized in Paul, Fundamental Immunology, 3d ed., Raven Press, 1993, pp. 243-247 and references cited therein. Such techniques include screening polypeptide portions of the native antigen for immunogenic properties. The representative ELISAs described herein may generally be employed in these screens. An immunogenic portion of a polypeptide is a portion that, within such representative assays, generates a signal in such assays that is substantially similar to that generated by the full length antigen. In other words, an immunogenic portion of a *Chlamydia* antigen generates at least about 20%, and preferably about 100%, of the signal induced by the full length antigen in a model ELISA as described herein.

Portions and other variants of *Chlamydia* antigens may be generated by synthetic or recombinant means. Variants of a native antigen may generally be prepared using standard mutagenesis techniques, such as oligonucleotide-directed site-specific mutagenesis. Sections of the polynucleotide sequence may also be removed using standard techniques to permit preparation of truncated polypeptides.

Recombinant polypeptides containing portions and/or variants of a native antigen may be readily prepared from a polynucleotide sequence encoding the polypeptide using a variety of techniques well known to those of ordinary skill in the art. For example, supernatants from suitable host/vector systems which secrete recombinant protein into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant protein.

Any of a variety of expression vectors known to those of ordinary skill in the art may be employed to express recombinant polypeptides as described herein. Expression may be achieved in any appropriate host cell that has been transformed or transfected with an expression vector containing a polynucleotide molecule that encodes a recombinant polypeptide. Suitable host cells include prokaryotes, yeast and higher eukaryotic cells. Preferably, the host cells employed are *E. coli,* yeast or a mammalian cell line, such as COS or CHO. The DNA sequences expressed in this manner may encode naturally occurring antigens, portions of naturally occurring antigens, or other variants thereof.

In general, regardless of the method of preparation, the polypeptides disclosed herein are prepared in an isolated, substantially pure, form. Preferably, the polypeptides are at least about 80% pure, more preferably at least about 90% pure and most preferably at least about 99% pure.

Within certain specific embodiments, a polypeptide may be a fusion protein that comprises multiple polypeptides as described herein, or that comprises at least one polypeptide as described herein and an unrelated sequence, such as a known *Chlamydial* protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein. A DNA sequence encoding a fusion protein of the present invention may be constructed using known recombinant DNA techniques to assemble separate DNA sequences encoding, for example, the first and second polypeptides, into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8562, 1986; U.S. Patent No. 4,935,233 and U.S. Patent No. 4,751,180. The linker sequence may be from 1 to about 50 amino acids in length. As an alternative to the use of a peptide linker sequence (when desired), one can utilize non-essential N-terminal amino acid regions (when present) on the first and second polypeptides to separate the functional domains and prevent steric hindrance.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide disclosed herein together with an unrelated immunogenic protein. Preferably the immunogenic protein is capable of eliciting a recall response. Examples of such proteins include tetanus, tuberculosis and hepatitis proteins (*see*, for example, Stoute et al. New Engl. J. Med., 336:86-91, 1997).

Within preferred embodiments; an immunological fusion partner is derived from protein D, a surface protein of the gram-negative bacterium Haemophilus influenza B (WO 91/18926). Preferably, a protein D derivative comprises approximately the first third of the protein (*e.g*., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E*. *coli* C-LYTA expressing plasmids useful for expression of fusion proteins. Purification of hybrid proteins containing the C-LYTA fragment at the amino terminus has been described *(see* Biotechnology 10:795-798, 1992). Within a preferred embodiment, a repeat portion of LYTA may be incorporated into a fusion protein. A repeat portion is found in the C-terminal region starting at residue 178. A particularly preferred repeat portion incorporates residues 188-305. Additionally, the fusion protein Ra12 may be linked to the polynucleotides disclosed herein to facilitate protein expression.

In another aspect, the present invention provides methods of manufacturing a medicament using one or more of the above polypeptides or fusion proteins (or polynucleotides encoding such polypeptides or fusion proteins) to induce protective immunity against Chlamydial infection in a patient. As used herein, a "patient" refers to any warm-blooded animal, preferably a human. A patient may be afflicted with a disease, or may be free of detectable disease and/or infection. In other words, protective immunity may be induced to prevent or treat Chlamydial infection.

In this aspect, the polypeptide, fusion protein or polynucleotide molecule is present within a pharmaceutical composition or a vaccine. Pharmaceutical compositions may comprise one or more polypeptides, each of which may contain one or more of the above sequences (or variants thereof), and a physiologically acceptable carrier. Vaccines may comprise one or more of the above polypeptides and an immunostimulant, such as an adjuvant or a liposome (into which the polypeptide is incorporated). Such pharmaceutical compositions and vaccines may also contain other *Chlamydia* antigens, either incorporated into a combination polypeptide or present within a separate polypeptide.

Alternatively, a vaccine may contain polynucleotides encoding one or more polypeptides or fusion proteins as described above, such that the polypeptide is generated *in situ.* In such vaccines, the polynucleotides may be present within any of a variety of delivery systems known to those of ordinary skill in the art, including nucleic acid expression systems, bacterial and viral expression systems. Appropriate nucleic acid expression systems contain the necessary polynucleotide sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface. In a preferred embodiment, the polynucleotides may be introduced using a viral expression system (*e.g*., vaccinia or other pox virus, retrovirus, or adenovirus), which may involve the use of a non-pathogenic (defective) virus. Techniques for incorporating polynucleotides into such expression systems are well known to those of ordinary skill in the art. The polynucleotides may also be administered as "naked" plasmid vectors as described, for example, in Ulmer et al., Science 259:1745-1749, 1993 and reviewed by Cohen, Science 259:1691-1692, 1993.Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e*., an artificial membrane vesicle). The uptake of naked polynucleotides may be increased by incorporating the polynucleotides into and/or onto biodegradable beads, which are efficiently transported into the cells. The preparation and use of such systems is well known in the art.

In a related aspect, a polynucleotide vaccine as described above may be administered simultaneously with or sequentially to either a polypeptide disclosed herein or a known *Chlamydia* antigen. For example, administration of polynucleotides encoding a polypeptide disclosed herein, either "naked" or in a delivery system as described above, may be followed by administration of an antigen in order to enhance the protective immune effect of the vaccine.

Polypeptides and polynucleotides disclosed herein may also be employed in adoptive immunotherapy for the treatment of *Chlamydial* infection. Adoptive immunotherapy may be broadly classified into either active or passive immunotherapy. In active immunotherapy, treatment relies on the *in vivo* stimulation of the endogenous host immune system with the administration of immune response-modifying agents (for example, vaccines, bacterial adjuvants, and/or cytokines).

In passive immunotherapy, treatment involves the delivery of biologic reagents with established immune reactivity (such as effector cells or antibodies) that can directly or indirectly mediate anti-*Chlamydia* effects and does not necessarily depend on an intact host immune system. Examples of effector cells include T lymphocytes (for example, CD8+ cytotoxic T-lymphocyte, CD4+ T-helper), killer cells (such as Natural Killer cells, lymphokine-activated killer cells), B cells, or antigen presenting cells, (such as dendritic cells and macrophages) expressing the disclosed antigens. The polypeptides disclosed herein may also be used to generate antibodies or anti-idiotypic antibodies (as in U.S. Patent No. 4,918,164), for passive immunotherapy.

The predominant method of procuring adequate numbers of T-cells for adoptive immunotherapy is to grow immune T-cells *in vitro.* Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage, monocyte, fibroblast, or B-cells, may be pulsed with immunoreactive polypeptides, or polynucleotide sequence(s) may be introduced into antigen presenting cells, using a variety of standard techniques well known in the art. For example, antigen presenting cells may be transfected or transduced with a polynucleotide sequence, wherein said sequence contains a promoter region appropriate for increasing expression, and can be expressed as part of a recombinant virus or other expression system. Several viral vectors may be used to transduce an antigen presenting cell, including pox virus, vaccinia virus, and adenovirus; also, antigen presenting cells may be transfected with polynucleotide sequences disclosed herein by a variety of means, including gene-gun technology, lipid-mediated delivery, electroporation, osmotic shock, and particlate delivery mechanisms, resulting in efficient and acceptable expression levels as determined by one of ordinary skill in the art. For cultured T-cells to be effective in therapy, the cultured T-cells must be able to grow and distribute widely and to survive long term *in vivo.* Studies have demonstrated that cultured T-cells can be induced to grow *in vivo* and to survive long term in substantial numbers by repeated stimulation with antigen supplemented with IL-2 (see, for example, Cheever, M., et al, "Therapy With Cultured T Cells: Principles Revisited, " Immunological Reviews, 157:177, 1997).

The polypeptides disclosed herein may also be employed to generate and/or isolate chlamydial-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8+ or CD4+ T-cell clones may be isolated from the patient, expanded using standard tissue culture techniques, and returned to the patient.

Alternatively, peptides corresponding to immunogenic portions of the polypeptides may be employed to generate *Chlamydia* reactive T cell subsets by selective *in vitro* stimulation and expansion of autologous T cells to provide antigen-specific T cells which may be subsequently transferred to the patient as described, for example, by Chang et al, (Crit. Rev. Oncol. Hematol., 22(3), 213, 1996). Cells of the immune system, such as T cells, may be isolated from the peripheral blood of a patient, using a commercially available cell separation system, such as Isolex™ System, available from Nexell Therapeutics, Inc. Irvine, CA. The separated cells are stimulated with one or more of the immunoreactive polypeptides contained within a delivery vehicle, such as a microsphere, to provide antigen-specific T cells. The population of antigen-specific T cells is then expanded using standard techniques and the cells are administered back to the patient.

T-cell and/or antibody receptors specific for the polypeptides disclosed herein can be cloned, expanded, and transferred into other vectors or effector cells for use in adoptive immunotherapy. In particular, T cells may be transfected with the appropriate genes to express the variable domains from chlamydia specific monoclonal antibodies as the extracellular recognition elements and joined to the T cell receptor signaling chains, resulting in T cell activation, specific lysis, and cytokine release. This enables the T cell to redirect its specificity in an MHC-independent manner. See for example, Eshhar, Z., Cancer Immunol Immunother, 45(3-4):131-6, 1997 and Hwu, P., et al, Cancer Res, 55(15):3369-73, 1995. Another embodiment may include the transfection of chlamydia antigen specific alpha and beta T cell receptor chains into alternate T cells, as in Cole, DJ, et al, Cancer Res, 55(4):748-52, 1995.

Syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate disease in a murine model has been demonstrated by Cheever et al, Immunological Reviews, 157:177, 1997). Additionally, vectors expressing the disclosed polynucleotides may be introduced into stem cells taken from the patient and clonally propagated *in vitro* for autologous transplant back into the same patient.

Polypeptides, polynucleotides, T cells and/or binding agents disclosed herein may be incorporated into pharmaceutical compositions or immunogenic compositions (*i.e*., vaccines). Pharmaceutical compositions comprise one or more such compounds and a physiologically acceptable carrier. Vaccines may comprise one or more such compounds and an immunostimulant. An immunostimulant may be any substance that enhances or potentiates an immune response to an exogenous antigen. Examples of immunostimulants include adjuvants, biodegradable microspheres (*e.g*., polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other *Chlamydial* antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface or secretes such an epitope. In a preferred embodiment, the DNA may be introduced using a viral expression system (*e.g*., vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g*., neutral buffered saline or phosphate buffered saline), carbohydrates (*e.g*., glucose, mannose, sucrose or dextrans), mannitol, proteins, polypeptides or amino acids such as glycine, antioxidants, chelating agents such as EDTA or glutathione, adjuvants (*e.g*., aluminum hydroxide) and/or preservatives. Alternatively, compositions of the present invention may be formulated as a lyophilizate. Compounds may also be encapsulated within liposomes using well known technology.

Any of a variety of immunostimulants may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, under select circumstances, the adjuvant composition may be designed to induce an immune response predominantly of the Th1 type or Th2 type. High levels of Th1-type cytokines (*e.g*., IFN-γ, TNFα, IL-2 and IL-12) tend to favor the induction of cell mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT) *(see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation (*i.e*., a formulation such as a capsule, sponge or gel (composed of polysaccharides, for example) that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets *Chlamydia*-infected cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-*Chlamydia* effects *per se* and/or to be immunologically compatible with the receiver (*i.e*., matched HLA haplotype). APCs may generally be isolated from any of a variety of biological fluids and organs, and may be autologous, allogeneic, syngeneic or xenogeneic cells.

The use dendritic cells or progenitors thereof as antigen-presenting cells is disclosed herein. Dendritic cells are highly potent APCs (Banchereau and Steinman, Nature 392:245-251, 1998) and have been shown to be effective as a physiological adjuvant for eliciting prophylactic or therapeutic immunity *(see* Timmerman and Levy, Ann. Rev. Med. 50:507-529, 1999). In general, dendritic cells may be identified based on their typical shape (stellate *in situ,* with marked cytoplasmic processes (dendrites) visible *in vitro*)*,* their ability to take up, process and present antigens with high efficiency, and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated herein. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFa to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g*., CD40, CD80, CD86 and 4-1BB).

APCs may generally be transfected with a polynucleotide encoding a *Chlamydial* protein (or portion or other variant thereof) such that the *Chlamydial* polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the *Chlamydial* polypeptide, DNA (naked or within a plasmid vector) or RNA; or with antigen-expressing recombinant bacterium or viruses (*e.g*., vaccinia, fowlpox, adenovirus or lentivirus vectors). Prior to loading, the polypeptide may be covalently conjugated to an immunological partner that provides T cell help (*e.g.,* a carrier molecule). Alternatively, a dendritic cell may be pulsed with a nonconjugated immunological partner, separately or in the presence of the polypeptide.

Routes and frequency of administration of pharmaceutical compositions and vaccines, as well as dosage, will vary from individual to individual. In general, the pharmaceutical compositions and vaccines may be administered by injection (*e.g.,* intracutaneous, intramuscular, intravenous or subcutaneous), intranasally (*e.g*., by aspiration) or orally. Between 1 and 3 doses may be administered for a 1-36 week period. Preferably, 3 doses are administered, at intervals of 3-4 months, and booster vaccinations may be given periodically thereafter. Alternate protocols may be appropriate for individual patients. A suitable dose is an amount of polypeptide or DNA that, when administered as described above, is capable of raising an immune response in an immunized patient sufficient to protect the patient from *Chlamydial* infection for at least 1-2 years. In general, the amount of polypeptide present in a dose (or produced *in situ* by the DNA in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.1 mL to about 5 mL.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactic galactide) may also be employed as carriers for the pharmaceutical compositions of this invention. Suitable biodegradable microspheres are disclosed, for example, in U.S. Patent Nos. 4,897,268 and 5,075,109.

In general, an appropriate dosage and treatment regimen provides the active compound(s) in an amount sufficient to provide therapeutic and/or prophylactic benefit. Such a response can be monitored by establishing an improved clinical outcome in treated patients as compared to non-treated patients. Increases in preexisting immune responses to a *Chlamydial* protein generally correlate with an improved clinical outcome. Such immune responses may generally be evaluated using standard proliferation, cytotoxicity or cytokine assays, which may be performed using samples obtained from a patient before and after treatment.

Methods for using the polypeptides described above to diagnose Chlamydial infection are disclosed herein. In this aspect, methods are provided for detecting Chlamydial infection in a biological sample, using one or more of the above polypeptides, either alone or in combination. For clarity, the term "polypeptide" will be used when describing specific embodiments of the diagnostic methods. However, it will be clear to one of skill in the art that the fusion proteins of the present invention may also be employed in such methods.

As used herein, a "biological sample" is any antibody-containing sample obtained from a patient. Preferably, the sample is whole blood, sputum, serum, plasma, saliva, cerebrospinal fluid or urine. More preferably, the sample is a blood, serum or plasma sample obtained from a patient. The polypeptides are used in an assay, as described below, to determine the presence or absence of antibodies to the polypeptide(s) in the sample, relative to a predetermined cut-off value. The presence of such antibodies indicates previous sensitization to *Chlamydia* antigens which may be indicative of *Chlamydia*-infection.

When more than one polypeptide is employed, the polypeptides used are preferably complementary (*i.e*., one component polypeptide will tend to detect infection in samples where the infection would not be detected by another component polypeptide). Complementary polypeptides may generally be identified by using each polypeptide individually to evaluate serum samples obtained from a series of patients known to be infected with *Chlamydia.* After determining which samples test positive (as described below) with each polypeptide, combinations of two or more polypeptides may be formulated that are capable of detecting infection in most, or all, of the samples tested.

A variety of assay formats are known to those of ordinary skill in the art for using one or more polypeptides to detect antibodies in a sample. *See, e.g.,* Harlow and Lane, Antibodies: A Laboratory Manual, Cold Spring Harbor Laboratory, 1988, which is incorporated herein by reference. In a preferred embodiment, the assay involves the use of polypeptide immobilized on a solid support to bind to and remove the antibody from the sample. The bound antibody may then be detected using a detection reagent that contains a reporter group. Suitable detection reagents include antibodies that bind to the antibody/polypeptide complex and free polypeptide labeled with a reporter group (*e.g*., in a semi-competitive assay). Alternatively, a competitive assay may be utilized, in which an antibody that binds to the polypeptide is labeled with a reporter group and allowed to bind to the immobilized antigen after incubation of the antigen with the sample. The extent to which components of the sample inhibit the binding of the labeled antibody to the polypeptide is indicative of the reactivity of the sample with the immobilized polypeptide.

The solid support may be any solid material known to those of ordinary skill in the art to which the antigen may be attached. For example, the solid support may be a test well in a microtiter plate, or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The polypeptides may be bound to the solid support using a variety of techniques known to those of ordinary skill in the art. In the context of the present application, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. In such cases, adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of polypeptide ranging from about 10 ng to about 1 µg, and preferably about 100 ng, is sufficient to bind an adequate amount of antigen.

Covalent attachment of polypeptide to a solid support may generally be achieved by first reacting the support with a bifunctional reagent that will react with both the support and a functional group, such as a hydroxyl or amino group, on the polypeptide. For example, the polypeptide may be bound to supports having an appropriate polymer coating using benzoquinone or by condensation of an aldehyde group on the support with an amine and an active hydrogen on the polypeptide (*see, e.g.,* Pierce Immunotechnology Catalog and Handbook, 1991, at A12-A13).

The assay may be an enzyme linked immunosorbent assay (ELISA). This assay may be performed by first contacting a polypeptide antigen that has been immobilized on a solid support, commonly the well of a microtiter plate, with the sample, such that antibodies to the polypeptide within the sample are allowed to bind to the immobilized polypeptide. Unbound sample is then removed from the immobilized polypeptide and a detection reagent capable of binding to the immobilized antibody-polypeptide complex is added. The amount of detection reagent that remains bound to the solid support is then determined using a method appropriate for the specific detection reagent.

More specifically, once the polypeptide is immobilized on the support as described above, the remaining protein binding sites on the support are typically blocked. Any suitable blocking agent known to those of ordinary skill in the art, such as bovine serum albumin (BSA) or Tween 20™ (Sigma Chemical Co., St. Louis, MO) may be employed. The immobilized polypeptide is then incubated with the sample, and antibody is allowed to bind to the antigen. The sample may be diluted with a suitable dilutent, such as phosphate-buffered saline (PBS) prior to incubation. In general, an appropriate contact time (*i.e*., incubation time) is that period of time that is sufficient to detect the presence of antibody within an HGE-infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (such as, for example, Protein A, Protein G, immunoglobulin, lectin or free antigen) conjugated to a reporter group. Preferred reporter groups include enzymes (such as horseradish peroxidase), substrates, cofactors, inhibitors, dyes, radionuclides, luminescent groups, fluorescent groups and biotin. The conjugation of binding agent to reporter group may be achieved using standard methods known to those of ordinary skill in the art. Common binding agents may also be purchased conjugated to a variety of reporter groups from many commercial sources (*e.g*., Zymed Laboratories, San Francisco, CA, and Pierce, Rockford, IL).

The detection reagent is then incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti-*Chlamydia* antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for *Chlamydia-*infection*.* In an alternate preferred embodiment, the cut-off value is determines using a Receiver Operator Curve, according to the method of Sackett et al., Clinical Epidemiology: A Basic Science for Clinical Medicine, Little Brown and Co., 1985, pp. 106-107. Briefly, in this embodiment, the cut-off value may be determined from a plot of pairs of true positive rates (*i.e*., sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for Chlamydial infection.

The assay may also be performed in a rapid flow-through or strip test format, wherein the antigen is immobilized on a membrane, such as nitrocellulose. In the flow-through test, antibodies within the sample bind to the immobilized polypeptide as the sample passes through the membrane. A detection reagent (*e.g*., protein A-colloidal gold) then binds to the antibody-polypeptide complex as the solution containing the detection reagent flows through the membrane. The detection of bound detection reagent may then be performed as described above. In the strip test format, one end of the membrane to which polypeptide is bound is immersed in a solution containing the sample. The sample migrates along the membrane through a region containing detection reagent and to the area of immobilized polypeptide. Concentration of detection reagent at the polypeptide indicates the presence of anti-*Chlamydia* antibodies in the sample. Typically, the concentration of detection reagent at that site generates a pattern, such as a line, that can be read visually. The absence of such a pattern indicates a negative result. In general, the amount of polypeptide immobilized on the membrane is selected to generate a visually discernible pattern when the biological sample contains a level of antibodies that would be sufficient to generate a positive signal in an ELISA, as discussed above. Preferably, the amount of polypeptide immobilized on the membrane ranges from about 25 ng to about 1 µg, and more preferably from about 50 ng to about 500 ng. Such tests can typically be performed with a very small amount (*e.g*., one drop) of patient serum or blood.

Of course, numerous other assay protocols exist that are suitable for use with the polypeptides disclosed herein. The above descriptions are intended to be exemplary only. One example of an alternative assay protocol which may be usefully employed in such methods is a Western blot, wherein the proteins present in a biological sample are separated on a gel, prior to exposure to a binding agent. Such techniques are well known to those of skill in the art.

Agents, such as antibodies and antigen-binding fragments thereof, that specifically bind to a *Chlamydial* protein are also disclosed herein. As used herein, an antibody, or antigen-binding fragment thereof, is said to "specifically bind" to a *Chlamydial* protein if it reacts at a detectable level (within, for example, an ELISA) with a *Chlamydial* protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present disclosure, when the binding constant for complex formation exceeds about 10³ L/mol. The binding constant may be determined using methods well known in the art.

Binding agents may be further capable of differentiating between patients with and without a *Chlamydial* infection using the representative assays provided herein. In other words, antibodies or other binding agents that bind to a *Chlamydial* protein will generate a signal indicating the presence of a *Chlamydial* infection in at least about 20% of patients with the disease, and will generate a negative signal indicating the absence of the disease in at least about 90% of individuals without infection. To determine whether a binding agent satisfies this requirement, biological samples (*e.g*., blood, sera, sputum urine and/or tissue biopsies) from patients with and without *Chlamydial* infection (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g*.; mice, rats, rabbits, sheep or goats). In this step, the polypeptides disclosed herein may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides disclosed herein may be used in the purification process in, for example, an affinity chromatography step.

The use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I. ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e.g*., covalently bonded) to a suitable monoclonal antibody either directly or indirectly (*e.g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g*., U.S. Patent No. 4,671,958, to Rodwell et al.

Where a therapeutic agent is more potent when free from the antibody portion of the immunoconjugates disclosed herein, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g*., U.S. Patent No. 4,489,710, to Spitler), by irradiation of a photolabile bond (*e.g*., U.S. Patent No. 4,625,014, to Senter et al.), by hydrolysis of derivatized amino acid side chains (*e.g*., U.S. Patent No. 4,638,045, to Kohn et al.), by serum complement-mediated hydrolysis (*e.g*., U.S. Patent No. 4,671,958, to Rodwell et al.), and acid-catalyzed hydrolysis (*e.g*., U.S. Patent No. 4,569,789, to Blattler et al.).

It may be desirable to couple more than one agent to an antibody. Multiple molecules of an agent may be coupled to one antibody molecule. Alternatively more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g*., U.S. Patent No. 4,507,234, to Kato et al.), peptides and polysaccharides such as aminodextran (*e.g*., U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in site-specific regions by appropriate methods. It will be evident that the precise dose of the antibody/immunoconjugate will vary depending upon the antibody used, the antigen density, and the rate of clearance of the antibody.

Antibodies may be used in diagnostic tests to detect the presence of *Chlamydia* antigens using assays similar to those detailed above and other techniques well known to those of skill in the art, thereby providing a method for detecting Chlamydial infection in a patient.

Diagnostic reagents may also comprise DNA sequences encoding one or more of the above polypeptides, or one or more portions thereof. For example, at least two oligonucleotide primers may be employed in a polymerase chain reaction (PCR) based assay to amplify *Chlamydia*-specific cNA derived from a biological sample, wherein at least one of the oligonucleotide primers is specific for a DNA molecule encoding a polypeptide disclosed herein. The presence of the amplified cDNA is then detected using techniques well known in the art, such as gel electrophoresis. Similarly oligonucleotide probes specific for a DNA molecule encoding a polypeptide disclosed herein may be used in a hybridization assay to detect the presence of a polypeptide in a biological sample.

As used herein, the term "oligonucleotide primer/probe specific for a DNA molecule" means an oligonucleotide sequence that has at least about 80%, preferably at least about 90% and more preferably at least about 95%, identity to the DNA molecule in question. Oligonucleotide primers and/or probes which may be usefully employed in the diagnostic methods preferably have at least about 10-40 nucleotides. The oligonucleotide primers may comprise at least about 10 contiguous nucleotides of a DNA molecule encoding one of the polypeptides disclosed herein. Preferably, oligonucleotide probes for use in the diagnostic methods comprise at least about 15 contiguous oligonucleotides of a DNA molecule encoding one of the polypeptides disclosed herein. Techniques for both PCR based assays and hybridization assays are well known in the art (see, for example, Mullis *et al. Ibid;* Ehrlich, *Ibid*). Primers or probes may thus be used to detect *Chlamydia*-specific sequences in biological samples. DNA probes or primers comprising oligonucleotide sequences described above may be used alone or in combination with each other.

The following Examples are offered by way of illustration and not by way of limitation. Any examples that may fall outside of the scope of the claimed invention are provided solely to aid the person skilled in the art in their understanding of the technical field of the invention.

### EXAMPLE 1

### ISOLATION OF DNA SEQUENCES ENCODING CHLAMYDIA ANTIGENS

*Chlamydia* antigens of the present invention were isolated by expression cloning of a genomic DNA library of *Chlamydia trachomatis* LGV II essentially as described by Sanderson et al. (J. Exp. Med., 1995, 182:1751-1757) and were shown to induce PBMC proliferation and IFN-γ in an immunoreactive T cell line.

*A Chlamydia*-specific T cell line was generated by stimulating PBMCs from a normal donor with no history of chlamydial genital tract infection with elementary bodies of *Chlamydia trachomatis* LGV II. This T cell line, referred to as TCL-8, was found to recognize both *Chlamydia trachomatis* and *Chlamydia pneumonia* infected monocyte-derived dendritic cells.

A randomly sheared genomic library of *Chlamydia trachomatis* LGV II was constructed in Lambda ZAP (Stratagene, La Jolla, CA) and the amplified library plated out in 96 well microtiter plates at a density of 30 clones/well. Bacteria were induced to express recombinant protein in the presence of 2 mM IPTG for 3 h, then pelleted and resuspended in 200 µl of RPMI 10% FBS. 10 µl of the induced bacterial suspension was transferred to 96 well plates containing autologous monocyte-derived dendritic cells. After a 2 h incubation, dendritic cells were washed to remove free *E. coli,* and *Chlamydia*-specific T cells were added. Positive *E. coli* pools were identified by determining IFN-γ production and proliferation of the T cells in response to the pools.

Four positive pools were identified, which were broken down to yield four pure clones (referred to as 1-B1-66, 4-D7-28, 3-G3-10 and 10-C10-31), with insert sizes of 481 bp, 183 bp, 110 bp and 1400 bp, respectively. The determined DNA sequences for 1-B1-66, 4-D7-28, 3-G3-10 and 10-C10-31 are provided in SEQ ID NO: 1-4, respectively. Clone 1-B1-66 is approximately in region 536690 of the *C. trachomatis* genome (NCBI *C. trachomatis* database). Within clone 1-B1-66, an open reading frame (ORF) has been identified (nucleotides 115 - 375) that encodes a previously identified 9 kDa protein (Stephens, et al. Genbank Accession No. AE001320), the sequence of which is provided in SEQ ID NO: 5). Clone 4-D7-28 is a smaller region of the same ORF (amino acids 22-82 of 1-B1-66). Clone 3-G3-10 is approximately in region 74559 of the *C. trachomatis* genome. The insert is cloned in the antisense orientation with respect to its orientation in the genome. The clone 10-C10-31 contains an open reading frame that corresponds to a previously published sequence for S 13 ribosomal protein from *Chlamydia trachomatis* (Gu, L. et al. J. Bacteriology, 177:2594-2601, 1995). The predicted protein sequences for 4-D7-28 and 10-C 10-31 are provided in SEQ ID NO: 6 and 12, respectively. Predicted protein sequences for 3-G3-10 are provided in SEQ ID NO: 7-11.

In a related series of screening studies, an additional T cell line was used to screen the genomic DNA library of *Chlamydia trachomatis* LGV II described above. A *Chlamydia*-specific T cell line (TCT-1) was derived from a patient with a chlamydial genital tract infection by stimulating patient PBMC with autologous monocyte-derived dendritic cells infected with elementary bodies of *Chlamydia trachomatis* LGV II. One clone, 4C9-18 (SEQ ID NO: 21), containing a 1256 bp insert, elicited a specific immune response, as measured by standard proliferation assays, from the *Chlamydia*-specific T cell line TCT-1. Subsequent analysis revealed this clone to contain three known sequences: lipoamide dehydrogenase (Genbank Accession No. AE001326), disclosed in SEQ ID NO: 22; a hypothetical protein CT429 (Genbank Accession No. AE001316), disclosed in SEQ ID NO: 23; and part of an open reading frame of ubiquinone methyltransferase CT428 (Genbank Accession No. AE001316), disclosed in SEQ ID NO: 24.

In further studies involving clone 4C9-18 (SEQ ID NO: 21), the full-length amino acid sequence for lipoamide dehydrognase (SEQ ID NO: 22) from *C. trachomatis* (LGV II) was expressed in clone CtL2-LPDA-FL, as disclosed in SEQ ID NO: 90.

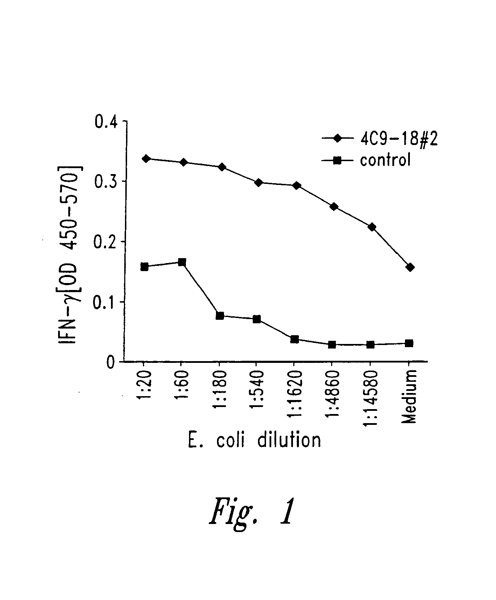

To further characterize the open reading frame containing the T cell stimulating epitope(s), a cDNA fragment containing nucleotides 1-695 of clone 4C9-18 with a cDNA sequence encoding a 6X-Histidine tag on the amino terminus was subcloned into the NdeI/EcoRI site of the pET17b vector (Novagen, Madison, WI), referred to as clone 4C9-18#2 BL21 pLysS (SEQ ID NO: 25, with the corresponding amino acid sequence provided in SEQ ID NO: 26) and transformed into *E*. *coli.* Selective induction of the transformed *E*. *coli* with 2 mM IPTG for three hours resulted in the expression of a 26 kDa protein from clone 4C9-18#2 BL21 pLysS, as evidenced by standard Coomassie-stained SDS-PAGE. To determine the immunogenicity of the protein encoded by clone 4C9-18#2 BL21 pLysS, *E. coli* expressing the 26 kDa protein were titered onto 1 x 10⁴ monocyte-derived dendritic cells and incubated for two hours. The dendritic cell cultures were washed and 2.5 x 10⁴ T cells (TCT-1) added and allowed to incubate for an additional 72 hours, at which time the level of IFN-γ in the culture supernatant was determined by ELISA. As shown in Fig. 1, the T-cell line TCT-1 was found to respond to induced cultures as measured by IFN-g, indicating a *Chlamydia*-specific T-cell response against the lipoamide dehydrogenase sequence. Similarly, the protein encoded by clone 4C9-18#2 BL21 pLysS was shown to stimulate the TCT-1 T-cell line by standard proliferation assays.

Subsequent studies to identify additional *Chlamydia trachomatis* antigens using the above-described CD4+ T-cell expression cloning technique yielded additional clones. The TCT-1 and TCL-8 *Chlamydia*-specific T-cell lines, as well as the TCP-21 T-cell line were utilized to screen the *Chlamydia trachomatis* LGVII genomic library. The TCP-21 T-cell line was derived from a patient having a humoral immune response to *Chlamydia pnuemoniae.* The TCT-1 cell line identified 37 positive pools, the TCT-3 cell line identified 41 positive pools and the TCP-21 cell line identified 2 positive pools. The following clones were derived from 10 of these positive pools. Clone 11-A3-93 (SEQ ID NO: 64), identified by the TCP-21 cell line, is a 1339 bp genomic fragment sharing homology to the HAD superfamily (CT103). The second insert in the same clone shares homology with the fab I gene (CT104) present on the complementary strand. Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of *C*. *pnuemoniae.*

Clone 11-G10-46, (SEQ ID NO: 62), identified using the TCT-3 cell line, contains a 688 bp insert that shares homology to the hypothetical protein CT610. Clone 11-G1-34, (SEQ ID NO: 61), identified using the TCT-3 cell line, has two partial open reading frames (ORF) with an insert size of 1215 bp. One ORF shares homology to the malate dehydrogenase gene (CT376), and the other ORF shares homology to the glycogen hydrolase gene (CT042). Clone 11-H3-68, (SEQ ID NO: 60), identified using the TCT-3 cell line, has two ORFs with a total insert size of 1180 bp. One partial ORF encodes the plasmid-encoded PGP6-D virulence protein while the second ORF is a complete ORF for the L1 ribosomal gene (CT318). Clone 11-H4-28, (SEQ ID NO: 59), identified using the TCT-3 cell line, has an insert size of 552 bp and is part of the ORF for the dnaK gene (CT396). Clone 12-B3-95, (SEQ ID NO: 58), identified using the TCT-1 cell line, has an insert size of 463 bp and is a part of the ORF for for the lipoamide dehydrogenase gene (CT557). Clones 15-G1-89 and 12-B3-95 are identical, (SEQ ID NO: 55 and 58, respectively), identified using the TCT-1 cell line, has an insert size of 463 bp and is part of the ORF for the lipoamide dehydrogenase gene (CT557). Clone 12-G3-83, (SEQ ID NO: 57), identified using the TCT-1 cell line, has an insert size of 1537 bp and has part of the ORF for the hypothetical protein CT622.

Clone 23-G7-68, (SEQ ID NO: 79), identified using the TCT-3 cell line, contains a 950 bp insert and contains a small part of the L11 ribosomal ORF, the entire ORF for L1 ribosomal protein and a part of the ORF for L10 ribosomal protein. Clone 22-F8-91, (SEQ ID NO: 80), identified using the TCT-1 cell line, contains a 395 bp insert that contains a part of the pmpC ORF on the complementary strand of the clone. Clone 21-E8-95, (SEQ ID NO: 81), identified using the TCT-3 cell line, contains a 2,085 bp insert which contains part of CT613 ORF, the complete ORF for CT612, the complete ORF for CT611 and part of the ORF for CT610. Clone 19-F12-57, (SEQ ID NO: 82), identified using the TCT-3 cell line, contains a 405 bp insert which contains part of the CT 858 ORF and a small part of the recA ORF. Clone 19-F12-53, (SEQ ID NO: 83), identified using the TCT-3 cell line, contains a 379 bp insert that is part of the ORF for CT455 encoding glutamyl tRNA synthetase. Clone 19-A5-54, (SEQ ID NO: 84), identified using the TCT-3 cell line, contains a 715 bp insert that is part of the ORF3 (complementary strand of the clone) of the cryptic plasmid. Clone 17-E11-72, (SEQ ID NO: 85), identified using the TCT-1 cell line, contains a 476 bp insert that is part of the ORF for Opp_2 and pmpD. The pmpD region of this clone is covered by the pmpD region of clone 15-H2-76. Clone 17-C1-77, (SEQ ID NO: 86), identified using the TCT-3 cell line, contains a 1551 bp insert that is part of the CT857 ORF, as well as part of the CT858 ORF. Clone 15-H2-76, (SEQ ID NO: 87), identified using the TCT-1 cell line, contains a 3,031 bp insert that contains a large part of the pmpD ORF, part of the CT089 ORF, as well as part of the ORF for SycE. Clone 15-A3-26, (SEQ ID NO: 88), contains a 976 bp insert that contains part of the ORF for CT858. Clone 17-G4-36, (SEQ ID NO: 267), identified using the TCT-10 cell line, contains a 680 bp insert that is in frame with beta-gal in the plasmid and shares homology to part of the ORF for DNA-directed RNA polymerase beta subunit (CT315 in SerD).

Several of the clones described above share homology to various polymorphic membrane proteins. The genomic sequence of *Chlamydia trachomatis* contains a family of nine polymorphic membrane protein genes, referred to as pmp. These genes are designated pmpA, pmpB, pmpC, pmpD, pmpE, pmpF, pmpG, pmpH and pmpI. Proteins expressed from these genes are believed to be of biological relevance in generating a protective immune response to a *Chlamydial* infection. In particular, pmpC, pmpD, pmpE and pmpI contain predictable signal peptides, suggesting they are outer membrane proteins, and therefore, potential immunological targets.

Based on the *Chlamydia trachomatis* LGVII serovar sequence, primer pairs were designed to PCR amplify the full-length fragments of pmpC, pmpD, pmpE, pmpG, pmpH and pmpI. The resulting fragments were subcloned into the DNA vaccine vector JA4304 or JAL, which is JA4304 with a modified linker (SmithKline Beecham, London, England). Specifically, PmpC was subcloned into the JAL vector using the 5' oligo GAT AGG CGC GCC GCA ATC ATG AAA TTT ATG TCA GCT ACT GCT G and the 3' oligo CAG AAC GCG TTT AGA ATG TCA TAC GAG CAC CGC A, as provided in SEQ ID NO: 197 and 198, respectively. PCR amplification of the gene under conditions well known in the art and ligation into the 5' ASCI/3' MluI sites of the JAL vector was completed after inserting the short nucleotide sequence GCAATC (SEQ ID NO: 199) upstream of the ATG to create a Kozak-like sequence. The resulting expression vector contained the full-length pmpC gene comprising 5325 nucleotides (SEQ ID NO: 173) containing the hypothetical signal sequence, which encodes a 187 kD protein (SEQ ID NO: 179). The pmpD gene was subcloned into the JA4304 vaccine vector following PCR amplification of the gene using the following oligos: 5' oligo- TGC AAT CAT GAG TTC GCA GAA AGA TAT AAA AAG C (SEQ ID NO: 200) and 3' oligo- CAG AGC TAG CTT AAA AGA TCA ATC GCA ATC CAG TAT TC (SEQ ID NO: 201). The gene was ligated into the a 5' blunted HIII/3' MluI site of the JA4304 vaccine vector using standard techniques well known in the art. The CAATC (SEQ ID NO: 202) was inserted upstream of the ATG to create a Kozak-like sequence. This clone is unique in that the last threonine of the HindIII site is missing due to the blunting procedure, as is the last glycine of the Kozak-like sequence. The insert, a 4593 nucleotide fragment (SEQ ID NO: 172) is the full-length gene for pmpD containing the hypothetical signal sequence, which encodes a 161 kD protein (SEQ ID NO: 178). PmpE was subcloned into the JA4304 vector using the 5' oligo- TGC AAT CAT GAA AAA AGC GTT TTT CTT TTT C (SEQ ID NO: 203), and the 3' oligo- CAG AAC GCG TCT AGA ATC GCA GAG CAA TTT C (SEQ ID NO: 204). Following PCR amplification, the gene was ligated into the 5' blunted HIII/3' MluI site of JA4304. To facilitate this, a short nucleotide sequence, TGCAATC (SEQ ID NO: 293), was added upstream of the initiation codon for creating a Kozak-like sequence and reconstituting the HindIII site. The insert is the full-length pmpE gene (SEQ ID NO: 171) containing the hypothetical signal sequence. The pmpE gene encodes a 105 kD protein (SEQ ID NO: 177). The pmpG gene was PCR amplified using the 5' oligo- GTG CAA TCA TGA TTC CTC AAG GAA TTT ACG (SEQ ID NO: 205), and the 3' oligo- CAG AAC GCG TTT AGA ACC GGA CTT TAC TTC C (SEQ ID NO: 206) and subcloned into the JA4304 vector. Similar cloning strategies were followed for the pmpI and pmpK genes. In addition, primer pairs were designed to PCR amplify the full-length or overlapping fragments of the pmp genes, which were then subcloned for protein expression in the pET17b vector (Novagen, Madison, WI) and transfected into E. coli BL21 pLysS for expression and subsequent purification utilizing the histidine-nickel chromatographic methodology provided by Novagen. Several of the genes encoding the recombinant proteins, as described below, lack the native signal sequence to facilitate expression of the protein. Full-length protein expression of pmpC was accomplished through expression of two overlapping fragments, representing the amino and carboxy termini. Subcloning of the pmpC-amino terminal portion, which lacks the signal sequence, (SEQ ID NO: 187, with the corresponding amino acid sequence provided in SEQ ID NO: 195) used the 5' oligo- CAG ACA TAT GCA TCA CCA TCA CCA TCA CGA GGC GAG CTC GAT CCA AGA TC (SEQ ID NO: 207), and the 3' oligo- CAG AGG TAC CTC AGA TAG CAC TCT CTC CTA TTA AAG TAG G (SEQ ID NO: 208) into the 5' NdeI/3' KPN cloning site of the vector. The carboxy terminus portion of the gene, pmpC-carboxy terminal fragment (SEQ ID NO: 186, with the corresponding amino acid sequence provided in SEQ ID NO: 194), was subcloned into the 5' NheI/3' KPN cloning site of the expression vector using the following primers: 5' oligo- CAG AGC TAG CAT GCA TCA CCA TCA CCA TCA CGT TAA GAT TGA GAA CTT CTC TGG C (SEQ ID NO: 209), and 3' oligo- CAG AGG TAC CTT AGA ATG TCA TAC GAG CAC CGC AG (SEQ ID NO: 210). PmpD was also expressed as two overlapping proteins. The pmpD-amino terminal portion, which lacks the signal sequence, (SEQ ID NO: 185, with the corresponding amino acid sequence provided in SEQ ID NO: 193) contains the initiating codon of the pET17b and is expressed as a 80 kD protein. For protein expression and purification purposes, a six-histidine tag follows the initiation codon and is fused at the 28^{th} amino acid (nucleotide 84) of the gene. The following primers were used, 5' oligo, CAG ACA TAT GCA TCA CCA TCA CCA TCA CGG GTT AGC (SEQ ID NO: 211), and the 3' oligo-CAG AGG TAC CTC AGC TCC TCC AGC ACA CTC TCT TC (SEQ ID NO: 212), to splice into the 5' NdeI/3' KPN cloning site of the vector. The pmpD-carboxy terminus portion (SEQ ID NO: 184) was expressed as a 92 kD protein (SEQ ID NO: 192). For expression and subsequent purification, an additional methionine, alanine and serine was included, which represent the initiation codon and the first two amino acids from the pET17b vector. A six-histidine tag downstream of the methionine, alanine and serine is fused at the 69151 amino acid (nucleotide 2073) of the gene. The 5' oligo- CAG AGC TAG CCA TCA CCA TCA CCA TCA CGG TGC TAT TTC TTG CTT ACG TGG (SEQ ID NO: 213) and the 3' oligo- CAG AGG TAC TTn AAA AGA TCA ATC GCA ATC CAG TAT TCG (SEQ ID NO: 214) were used to subclone the insert into the 5' NheI/3' KPN cloning site of the expression vector. PmpE was expressed as a 106kD protein (SEQ ID NO: 183 with the corresponding amino acid sequence provided in SEQ ID NO: 191). The pmpE insert also lacks the native signal sequence. PCR amplification of the gene under conditions well known in the art was performed using the following oligo primers: 5' oligo- CAG AGG ATC CAC ATC ACC ATC ACC ATC ACG GAC TAG CTA GAG AGG TTC (SEQ ID NO: 215), and the 3' oligo- CAG AGA ATT CCT AGA ATC GCA GAG CAA TTT C (SEQ ID NO: 216), and the amplified insert was ligated into a 5' BamHI/3' EcoRI site of JA4304. The short nucleotide sequence, as provided in SEQ ID NO: 217, was inserted upstream of the initiation codon for creating the Kozak-like sequence and reconstituting the HindIII site. The expressed protein contains the initiation codon and the downstream 21 amino acids from the pET17b expression vector, i*.*e., MASMTGGQQMGRDSSLVPSSDP (SEQ ID NO: 218). In addition, a six-histidine tag is included upstream of the sequence described above and is fused at the 28^{th} amino acid (nucleotide 84) of the gene, which eliminates the hypothetical signal peptide. The sequences provided in SEQ ID NO: 183 with the corresponding amino acid sequence provided in SEQ ID NO: 191 do not include these additional sequences. The pmpG gene (SEQ ID NO: 182, with the corresponding amino acid sequence provided in SEQ ID No; 190) was PCR amplified under conditions well known in the art using the following oligo primers: 5' oligo- CAG AGG TAC CGC ATC ACC ATC ACC ATC ACA TGA TTC CTC AAG GAA TTT ACG (SEQ ID NO: 219), and the 3' oligo- CAG AGC GGC CGC TTA GAA CCG GAC TTT ACT TCC (SEQ ID NO: 220), and ligated into the 5' KPN/3' NotI cloning site of the expression vector. The expressed protein contains an additional amino acid sequence at the amino end, namely, MASMTGGQQNGRDSSLVPHHHHHH (SEQ ID NO: 221), which comprises the initiation codon and additional sequence from the pET17b expression vector. The pmpI gene (SEQ ID NO: 181, with the corresponding amino acid sequence provided in SEQ ID No; 189) was PCR amplified under conditions well known in the art using the following oligo primers: 5' oligo- CAG AGC TAG CCA TCA CCA TCA CCA TCA CCT CTT TGG CCA GGA TCC C (SEQ ID NO: 222), and the 3' oligo- CAG AAC TAG TCT AGA ACC TGT AAG TGG TCC (SEQ ID NO: 223), and ligted into the expression vector at the 5' NheI/3' SpeI cloning site. The 95 kD expressed protein contains the initiation codon plus an additional alanine and serine from the pET17b vector at the amino end of the protein. In addition, a six-histidine tag is fused at the 21^{st} amino acid of the gene, which eliminates the hypothetical signal peptide.

Clone 14H1-4, (SEQ ID NO: 56), identified using the TCT-3 cell line, contains a complete ORF for the TSA gene, thiol specific antioxidant - CT603 (the CT603 ORF is a homolog of CPn0778 from *C*. *pnuemoniae*)*.* The TSA open reading frame in clone 14-H1-4 was amplified such that the expressed protein possess an additional methionine and a 6x histidine tag (amino terminal end). This amplified insert was sub-cloned into the Nde/EcoRI sites of the pET17b vector. Upon induction of this clone with IPTG, a 22.6 kDa protein was purified by Ni-NTA agarose affinity chromatography. The determined amino acid sequence for the 195 amino acid ORF of clone 14-H1-4 encoding the TSA gene is provided in SEQ ID NO: 65. Further analysis yielded a full-length clone for the TSA gene, referred to as CTL2-TSA-FL, with the full-length amino acid sequence provided in SEQ ID NO: 92.

Further studies yielded 10 additional clones identified by the TCT-1 and TCT-3 T-cell lines, as described above. The clones identified by the TCT-1 line are: 16-D4-22, 17-C5-19, 18-C5-2, 20-G3-45 and 21-C7-66; clones identified by the TCT-3 cell line are: 17-C10-31, 17-E2-9, 22-A1-49 and 22-B3-53. Clone 21-G12-60 was recognized by both the TCT-1 and TCT-3 T cell lines. Clone 16-D4-22 (SEQ ID NO: 119), identified using the TCT-1 cell line contains a 953 bp insert that contains two genes, parts of open reading frame 3 (ORF3) and ORF4 of the *C. trachomatis* plasmid for growth within mammalian cells. Clone 17-C5-19 (SEQ ID NO: 118), contains a 951 bp insert that contains part of the ORF for DT431, encoding for clpP_1 protease and part of the ORF for CT430 (diaminopimelate epimerase). Clone 18-C5-2 (SEQ ID NO: 117) is part of the ORF for S1 ribosomal protein with a 446 bp insert that was identified using the TCT-1 cell line. Clone 20-G3-45 (SEQ ID NO: 116), identified by the TCT-1 cell line, contains a 437 bp insert that is part of the pmpB gene (CT413). Clone 21-C7-66 (SEQ ID NO: 115), identified by the TCT-1 line, contains a 995bp insert that encodes part of the dnaK like protein. The insert of this clone does not overlap with the insert of the TCT-3 clone 11-H4-28 (SEQ ID NO: 59), which was shown to be part of the dnaK gene CT396 Clone 17-C10-31 (SEQ ID NO: 114), identified by the TCT-3 cell line, contains a 976 bp insert. This clone contains part of the ORF for CT858, a protease containing IRBP and DHR domains. Clone 17-E2-9 (SEQ ID NO: 113) contains part of ORFs for two genes, CT611 and CT610, that span a 1142 bp insert. Clone 22-A1-49 (SEQ ID NO: 112), identified using the TCT-3 line, also contains two genes in a 698 bp insert. Part of the ORF for CT660 (DNA gyrase{gyrA_2}) is present on the top strand where as the complete ORF for a hypothetical protein CT659 is present on the complementary strand. Clone 22-B3-53 (SEQ ID NO: 111), identified by the TCT-1 line, has a 267 bp insert that encodes part of the ORF for GroEL (CT110). Clone 21-012-60 (SEQ ID NO: 110), identified by both the TCT-1 and TCT-3 cell lines contains a 1461 bp insert that contains partial ORFs for hypothetical proteins CT875, CT229 and CT228.

Additional *Chlamydia* antigens were obtained by screening a genomic expression library of *Chlamydia trachomatis* (LGV II serovar) in Lambda Screen-1 vector (Novagen, Madison, WI) with sera pooled from several *Chlamydia*-infected individuals using techniques well known in the art. The following immuno-reactive clones were identified and the inserts containing *Chlamydia* genes sequenced: CTL2#1 (SEQ ID NO: 71); CTL2#2 (SEQ ID NO: 70); CTL2#3-5' (SEQ ID NO: 72, a first determined genomic sequence representing the 5' end); CTL2#3-3' (SEQ ID NO: 73, a second determined genomic sequence representing the 3' end); CTL2#4 (SEQ ID NO: 53); CTL2#5 (SEQ ID NO: 69); CTL2#6 (SEQ ID NO: 68); CTL2#7 (SEQ ID NO: 67); CTL2#8b (SEQ ID NO: 54); CTL2#9 (SEQ ID NO: 66); CTL2#10-5' (SEQ ID NO: 74, a first determined genomic sequence representing the 5' end); CTL2#10-3' (SEQ ID NO: 75, a second determined genomic sequence representing the 3' end); CT2#11-5' (SEQ ID NO: 45, a first determined genomic sequence representing the 5' end); CTL2#11-3' (SEQ ID NO: 44, a second determined genomic sequence representing the 3' end); CTL2#12 (SEQ ID NO: 46); CTL2#16-5' (SEQ ID NO: 47); CTL2#18-5' (SEQ ID NO: 49, a first determined genomic sequence representing the 5' end); CTL2#18-3' (SEQ ID NO: 48, a second determined genomic sequence representing the 3' end); CTL2#19-5' (SEQ ID NO: 76, the determined genomic sequence representing the 5' end); CTL2#21 (SEQ ID NO: 50); CTL2#23 (SEQ ID NO: 51; and CTL2#24 (SEQ ID NO: 52).

Additional *Chlamydia trachomatis* antigens were identified by serological expression cloning. These studies used sera pooled from several *Chlamydia*-infected individuals, as described above, but, IgA,and IgM antibodies were used in addition to IgG as a secondary antibody. Clones screened by this method enhance detection of antigens recognized by an early immune response to a *Chlamydial* infection, that is a mucosal humoral immune response. The following immunoreactive clones were characterized and the inserts containing *Chlamydia* genes sequenced: CTL2gam-1 (SEQ ID NO: 290), CTL2gam-2 (SEQ ID NO: 289), CTL2gam-5 (SEQ ID NO: 288), CTL2gam-6-3' (SEQ ID NO: 287, a second determined genomic sequence representing the 3' end), CTL2gam-6-5' (SEQ ID NO: 286, a first determined genomic sequence representing the 5' end), CTL2gam-8 (SEQ ID NO: 285), CTL2gam-10 (SEQ ID NO: 284), CTL2gam-13 (SEQ ID NO: 283), CTL2gam-15-3' (SEQ ID NO: 282, a second determined genomic sequence representing the 3' end), CTL2gam-15-5' (SEQ ID NO: 281, a first determined genomic sequence representing the 5' end), CTL2gam-17 (SEQ ID NO: 280), CTL2gam-18 (SEQ ID NO: 279), CTL2gam-21 (SEQ ID NO: 278), CTL2gam-23 (SEQ ID NO: 277), CTL2gam-24 (SEQ ID NO: 276), CTL2gam-26 (SEQ ID NO: 275), CTL2gam-27 (SEQ ID NO: 274), CTL2gam-28 (SEQ ID NO: 273), CTL2gam-30-3' (SEQ ID NO: 272, a second determined genomic sequence representing the 3' end) and CTL2gam-30-5' (SEQ ID NO: 271, a first determined genomic sequence representing the 5' end).

### EXAMPLE 2

### INDUCTION OF T CELL PROLIFERATION AND INTERFERON-γ PRODUCTION BY CHLAMYDIA TRACHOMATIS ANTIGENS

The ability of recombinant *Chlamydia trachomatis* antigens to induce T cell proliferation and interferon-γ production is determined as follows.

Proteins are induced by IPTG and purified by Ni-NTA agarose affinity chromatograph (Webb et al., J. Immunology 157:5034-5041, 1996). The purified polypeptides are then screened for the ability to induce T-cell proliferation in PBMC preparations. PBMCs from *C. trachomatis* patients as well as from normal donors whose T-cells are known to proliferate in response to *Chlamydia* antigens, are cultured in medium comprising RPMI 1640 supplemented with 10% pooled human serum and 50 µg/ml gentamicin. Purified polypeptides are added in duplicate at concentrations of 0.5 to 10 µ g/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

IFN-γ is measured using an enzyme-linked immunosorbent assay (ELISA). ELISA plates are coated with a mouse monoclonal antibody directed to human IFN-γ (PharMingen, San Diego, CA) in PBS for four hours at room temperature. Wells are then blocked with PBS containing 5% (W/V) non-fat dried milk for 1 hour at room temperature. The plates are washed six times in PBS/0.2% TWEEN-20 and samples diluted 1:2 in culture medium in the ELISA plates are incubated overnight at room temperature. The plates are again washed and a polyclonal rabbit anti-human IFN-γ serum diluted 1:3000 in PBS/10% normal goat serum is added to each well. The plates are then incubated for two hours at room temperature, washed and horseradish peroxidase-coupled anti-rabbit IgG (Sigma Chemical So., St. Louis, MO) is added at a 1:2000 dilution in PBS/5% non-fat dried milk. After a further two hour incubation at room temperature, the plates are washed and TMB substrate added. The reaction is stopped after 20 min with 1 N sulfuric acid. Optical density is determined at 450 nm using 570 nm as a reference wavelength. Fractions that result in both replicates giving an OD two fold greater than the mean OD from cells cultured in medium alone, plus 3 standard deviations, are considered positive.

Using the above methodology, recombinant 1B1-66 protein (SEQ ID NO: 5) as well as two synthetic peptides corresponding to amino acid residues 48-67 (SEQ ID NO: 13; referred to as 1-B1-66/48-67) and 58-77 (SEQ ID NO: 14, referred to as 1B1-66/58-77), respectively, of SEQ ID NO: 5, were found to induce a proliferative response and IFN-γ production in a Chlamydia-specific T cell line used to screen a genomic library of *C. trachomatis* LGV II.

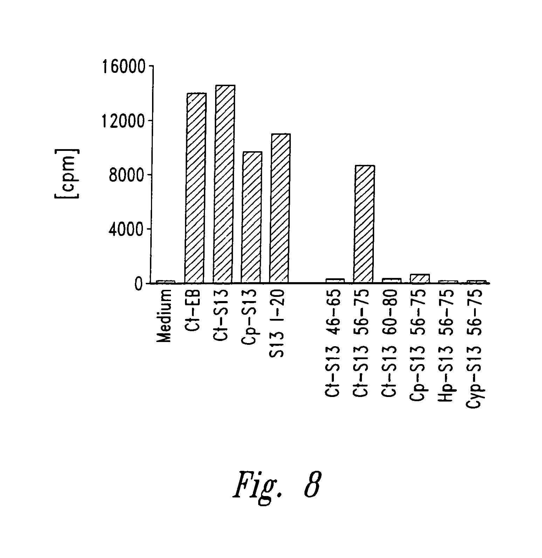

Further studies have identified a *C. trachomatis*-specific T-cell epitope in the ribosomal S 13 protein. Employing standard epitope mapping techniques well known in the art, two T-cell epitopes in the ribosomal S13 protein (rS13) were identified with a *Chlamydia-specific* T-cell line from donor CL-8 (T-cell line TCL-8 EB/DC). Fig. 8 illustrates that the first peptide, rS13 1-20 (SEQ ID NO: 106), is 100% identical with the corresponding *C. pneumoniae* sequence, explaining the cross-reactivity of the T-cell line to recombinant *C. trachomatis-* and *C. pneumoniae-*rS13. The response to the second peptide rS13 56-75 (SEQ ID NO: 108) is *C. trachomatis-specific,* indicating that the rS13 response in this healthy asymptomatic donor was elicited by exposure to *C. trachomatis* and not to *C. pneumoniae,* or any other microbial infection.

As described in Example 1, Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of *C. pneumoniae,* referred to as OMCB. To further define the reactive epitope(s), epitope mapping was performed using a series of overlapping peptides and the immunoassay previously described. Briefly, proliferative responses were determined by stimulating 2.5 x 10⁴ TCP-21 T-cells in the presence of 1 x 10⁴ monocyte-derived dendritic cells with either non-infectious elementary bodies derived from *C. trachomatis* and *C. pneumoniae,* or peptides derived from the protein sequence of *C. trachomatis* or *C. pneumoniae* OMCB protein (0.1 µg/ml). The TCP-21 T-cells responded to epitopes CT-OMCB #167-186, CT-OMCB #171-190, CT-OMCB #171-186, and to a lesser extent, CT-OMCB #175-186 (SEQ ID NO: 249-252, respectively). Notably, the TCP-21 T-cell line also gave a proliferative response to the homologous *C. pneumoniae* peptide CP-OMCB #171-186 (SEQ ID NO: 253), which was equal to or greater than the response to the *C. trachomatis* peptides. The amino acid substitutions in position two (i.e., Asp for Glu) and position four (i.e., Cys for Ser) did not alter the proliferative response of the T-cells and therefore demonstrating this epitope to be a cross-reactive epitope between *C. trachomatis* and *C. pneumoniae.*

To further define the epitope described above, an additional T-cell line, TCT-3, was used in epitope mapping experiments. The immunoassays were performed as described above, except that only peptides from *C. trachomatis* were tested. The T-cells gave a proliferative response to two peptides, CT-OMCB #152-171 and CT-OMCB #157-176 (SEQ ID NO: 246 and 247, respectively), thereby defining an additional immunogenic epitope in the cysteine rich outer membrane protein of *C. trachomatis.*

Clone 14H1-4, (SEQ ID NO: 56, with the corresponding full-length amino acid sequence provided in SEQ ID NO: 92), was identified using the TCT-3 cell line in the CD4 T-cell expression cloning system previously described, and was shown to contain a complete ORF for the, thiol specific antioxidant gene (CT603), referred to as TSA. Epitope mapping immunoassays were performed, as described above, to further define the epitope. The TCT-3 T-cells line exhibited a strong proliferative response to the overlapping peptides CT-TSA #96-115, CT-TSA #101-120 and CT-TSA #106-125 (SEQ ID NO: 254-256, respectively) demonstrating an immunoreactive epitope in the thiol specific antioxidant gene of *C. trachomatis* serovar LGVII.

### EXAMPLE 3

### PREPARATION OF SYNTHETIC POLYPEPTIDES

Polypeptides may be synthesized on a Millipore 9050 peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugating or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1 % trifluoroacetic acid (TFA) and lyophilized prior to purification by C18 reverse phase HPLC. A gradient of 0-60% acetonitrile (containing 0.1% TFA) in water (containing 0.1% TFA) may be used to elute the peptides. Following lyophilization of the pure fractions, the peptides may be characterized using electrospray mass spectrometry and by amino acid analysis.

### EXAMPLE 4

### ISOLATION AND CHARACTERIZATION OF DNA SEQUENCES ENCODING CHLAMYDIA ANTIGENS USING RETROVIRAL EXPRESSION VECTOR SYSTEMS AND SUBSEQUENT IMMUNOLOGICAL ANALYSIS

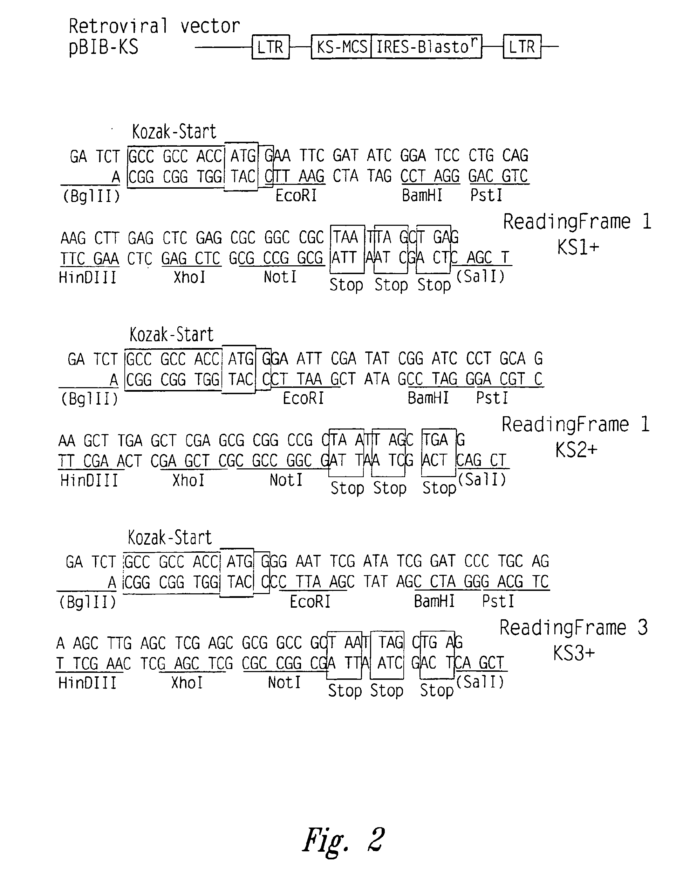

A genomic library of *Chlamydia trachomatis* LGV II was constructed by limited digests using BamHI, BglII, BstYi and MboI restriction enzymes. The restriction digest fragments were subsequently ligated into the BamHI site of the retroviral vectors pBIB-KS1,2,3. This vector set was modified to contain a Kosak translation initiation site and stop codons in order to allow expression of proteins from short DNA genomic fragments, as shown in Fig. 2. DNA pools of 80 clones were prepared and transfected into the retroviral packaging line Phoenix-Ampho, as described in Pear, W.S., Scott, M.L. and Nolan, G.P., Generation of High Titre, Helper-free Retroviruses by Transient Transfection. Methods in Molecular Medicine: Gene Therapy Protocols, Humana Press, Totowa, NJ, pp. 41-57. The *Chlamydia* library in retroviral form was then transduced into H2-Ld expressing P815 cells, which were then used as target cells to stimulate an antigen specific T-cell line.

A *Chlamydia-specific,* murine H2^{d} restricted CD8+ T-cell line was expanded in culture by repeated rounds of stimulation with irradiated *C. trachomatis-infected* J774 cells and irradiated syngeneic spleen cells, as described by Starnbach, M., in J. Immunol., 153:5183, 1994. This *Chlamydia-specific* T-cell line was used to screen the above *Chlamydia* genomic library expressed by the retrovirally-transduced P815 cells. Positive DNA pools were identified by detection of IFN-γ production using Elispot analysis (*SEE* Lalvani et al., J. Experimental Medicine 186:859-865, 1997).

Two positive pools, referred to as 2C7 and 2E10, were identified by IFN-γ Elispot assays. Stable transductants of P815 cells from pool 2C7 were cloned by limiting dilution and individual clones were selected based upon their capacity to elicit IFN-γ production from the *Chlamydia-specific* CTL line. From this screening process, four positive clones were selected, referred to as 2C7-8, 2C7-9, 2C7-19 and 2C7-21. Similarly, the positive pool 2E10 was further screened, resulting in a an additional positive clone, which contains three inserts. The three inserts are fragments of the CT016, tRNA syntase and clpX genes (SEQ ID NO: 268-270, respectively).

Transgenic DNA from these four positive 2C7.8 clones were PCR amplified using pBIB-KS specific primers to selectively amplify the *Chlamydia* DNA insert. Amplified inserts were gel purified and sequenced. One immunoreactive clone, 2C7-8 (SEQ ID NO: 15, with the predicted amino acid sequence provided in SEQ ID NO: 32), is a 160 bp fragment with homology to nucleotides 597304-597145 of *Chlamydia trachomatis,* serovar D (NCBI, BLASTN search; SEQ ID NO: 33, with the predicted amino acid sequence provided in SEQ ID NO: 34). The sequence of clone 2C7-8 maps within two putative open reading frames from the region of high homology described immediately above, and in particular, one of these putative open reading frames, consisting of a 298 amino acid fragment (SEQ ID NO: 16, with the predicted amino acid sequence provided in SEQ ID NO: 17), was demonstrated to exhibit immunological activity.

Full-length cloning of the 298 amino acid fragment (referred to as CT529 and/or the Cap1 gene) from serovar L2 was obtained by PCR amplification using 5'-ttttgaagcaggtaggtgaatatg (forward) (SEQ ID NO: 159) and 5'-ttaagaaatttaaaaaatccctta (reverse) (SEQ ID NO: 160) primers, using purified *C. trachomatis* L2 genomic DNA as template. This PCR product was gel-purified, cloned into pCRBlunt (Invitrogen, Carlsbad, CA) for sequencing, and then subcloned into the *Eco*RI site of pBIB-KMS, a derivative of pBIB-KS for expression. The *Chlamydia pnuemoniae* homlogue of CT529 is provided in SEQ ID NO: 291, with the corresponding amino acid sequence provided in SEQ ID NO: 292.

Full-length DNA encoding various CT529 serovars were amplified by PCR from bacterial lysates containing 10⁵ IFU, essentially as described (Denamur, E., *C.* Sayada, A. Souriau, J. Orfila, A. Rodolakis and J. Elion. 1991. J. Gen. Microbiol. 137: 2525). The following serovars were amplified as described: Ba (SEQ ID NO: 134, with the corresponding predicted amino acid sequence provided in SEQ ID NO: 135); E (BOUR) and E (MTW447) (SEQ ID NO: 122, with the corresponding predicted amino acid sequence provided in SEQ ID NO: 123); F (NI1) (SEQ ID NO: 128, with the corresponding predicted amino acid sequence provided in SEQ ID NO: 129); G; (SEQ ID NO: 126, with the corresponding predicted amino acid sequence provided in SEQ ID NO: 127); Ia (SEQ ID NO: 124, with the corresponding predicted amino acid sequence provided in SEQ ID NO: 125); L1 (SEQ ID NO: 130, with the corresponding predicted amino acid sequence provided in SEQ ID NO: 131); L3 (SEQ ID NO: 132, with the corresponding predicted amino acid sequence provided in SEQ ID NO: 133); I (SEQ ID NO: 263, with the corresponding predicted amino acid sequence provided in SEQ ID NO: 264); K (SEQ ID NO: 265, with the corresponding predicted amino acid sequence provided in SEQ ID NO: 266); and MoPn (SEQ ID NO: 136, with the corresponding predicted amino acid sequence provided in SEQ ID NO: 137). PCR reactions were performed with Advantage Genomic PCR Kit (Clontech, Palo Alto, CA) using primers specific for serovar L2 DNA (external to the ORF). Primers sequences were 5'-ggtataatatctctctaaattttg (forward-SEQ ID NO: 161) and 5'-agataaaaaaggctgtttc' (reverse-SEQ ID NO: 162) except for MoPn which required 5'-ttttgaagcaggtaggtgaatatg (forward-SEQ ID NO: 163) and 5'-tttacaataagaaaagctaagcactttgt (reverse-SEQ ID NO: 164). PCR amplified DNA was purified with QIAquick PCR purification kit (Qiagen, Valencia, CA) and cloned in pCR2.1 (Invitrogen, Carlsbad, CA) for sequencing.

Sequencing of DNA derived from PCR amplified inserts of immunoreactive clones was done on an automated sequencer (ABI 377) using both a pBIB-KS specific forward primer 5'-ccttacacagtcctgctgac (SEQ ID NO: 165) and a reverse primer 3'-gtttccgggccctcacattg (SEQ ID NO: 166). PCRBlunt cloned DNA coding for CT529 serovar L2 and pCR2.1 cloned DNA coding for CT529 serovar Ba, E (BOUR), E (MTW447), F (NI1), G, Ia, K, L1, L3 and MoPn were sequenced using T7 promoter primer and universal M 13 forward and M 13 reverse primers.

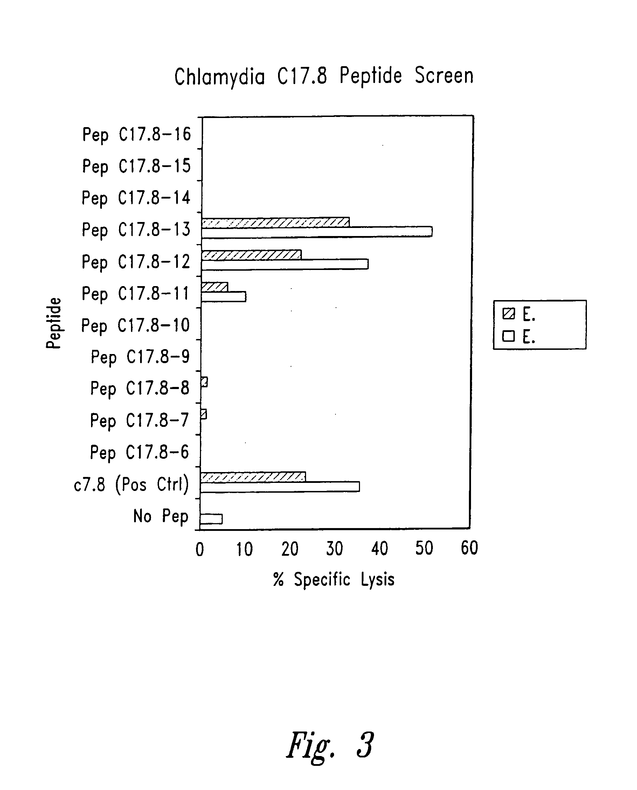

To determine if these two putative open reading frames (SEQ ID NO: 16 and 20) encoded a protein with an associated immunological function, overlapping peptides (17-20 amino acid lengths) spanning the lengths of the two open reading frames were synthesized, as described in Example 3. A standard chromium release assay was utilized to determine the per cent specific lysis of peptide-pulsed H2^{d} restricted target cells. In this assay, aliquots of P815 cells (H2^{d}) were labeled at 37° C for one hour with 100 µCi of ⁵¹Cr in the presence or absence of 1 µg/ml of the indicated peptides. Following this incubation, labeled P815 cells were washed to remove excess ⁵¹Cr and peptide, and subsequently plated in duplicate in microculture plates at a concentration of 1,000 cells/well. Effector CTL (*Chlamydia-specific* CD8 T cells) were added at the indicated effector:target ratios. Following a 4 hour incubation, supernatants were harvested and measured by gamma-counter for release of ⁵¹Cr into the supernatant. Two overlapping peptides from the 298 amino acid open reading frame did specifically stimulate the CTL line. The peptides represented in SEQ ID NO: 138-156 were synthsized, representing the translation of the L2 homologue of the serovar D open reading frame for CT529 (Cap1 gene) and 216 amino acid open reading frame.As shown in Fig. 3, peptides CtC7.8-12 (SEQ ID NO: 18, also referred to as Cap1#132-147, SEQ ID NO: 139) and CtC7.8-13 (SEQ ID NO: 19, also referred to as Cap1#138-155, SEQ ID NO: 140) were able to elicit 38 to 52% specific lysis, respectively, at an effector to target ratio of 10:1. Notably, the overlap between these two peptides contained a predicted H2^{d} (K^{d} and L^{d}) binding peptide. A 10 amino acid peptide was synthesized to correspond to this overlapping sequence (SEQ ID NO: 31) and was found to generate a strong immune response from the anti*-Chlamydia* CTL line by elispot assay. Significantly, a search of the most recent Genbank database revealed no proteins have previously been described for this gene. Therefore, the putative open reading frame encoding clone 2C7-8 (SEQ ID NO: 15) defines a gene which encompasses an antigen from *Chlamydia* capable of stimulating antigen-specific CD8+ T-cells in a MHC-I restricted manner, demonstrating this antigen could be used to develop a vaccine against *Chlamydia.*

To confirm these results and to further map the epitope, truncated peptides (SEQ ID NO: 138-156) were made and tested for recognition by the T-cells in an IFN-g ELISPOT assay. Truncations of either Ser139 (Cap1#140-147, SEQ ID NO: 146) or Leu147 (Cap1#138-146, SEQ ID NO: 147) abrogate T-cell recognition. These results indicate that the 9-mer peptide Cap1#139-147 (SFIGGITYL, SEQ ID NO: 145) is the minimal epitope recognized by the *Chlamydia*-specific T-cells.

Sequence alignments of Cap1 (CT529) from selected serovars of *C. trachomatis* (SEQ ID NO: 121, 123, 125, 127, 129, 131, 133, 135, 137 and 139) shows one of the amino acid differences is found in position 2 of the proposed epitope. The homologous serovar D peptide is SIIGGITYL (SEQ ID NO: 168). The ability of SFIGGITYL and SIIGGITYL to target cells for recognition by the *Chlamydia* specific T-cells was compared. Serial dilutions of each peptide were incubated with P815 cells and tested for recognition by the T-cells in a ⁵¹Cr release assay, as described above. The *Chlamydia-specific* T-cells recognize the serovar L2 peptide at a minimum concentration of 1 nM and the serovar D peptide at a minimum concentration of 10 nM.

Further studies have shown that a Cap1#139-147-specific T-cell clone recognizes *C. trachomatis* infected cells. To confirm that Cap1₁₃₉₋₁₄₇ is presented on the surface of *Chlamydia* infected cells, Balb-3T3 (H-2^{d}) cells were infected with *C. trachomatis* serovar L2 and tested to determine whether these cells are recognized by a CD8+ T-cell clone specific for Cap1#139-147 epitope (SEQ ID NO: 145). The T-cell clone specific for Cap1#139-147 epitope was obtained by limiting dilution of the line 69 T-cells. The T-cell clone specifically recognized the *Chlamydia* infected cells. In these experiments, target cells were *C. trachomatis* infected (positive control) or uninfected Balb/3T3 cells, showing 45%, 36% and 30% specific lysis at 30:1, 10:1 and 3:1 effector to target ratios, respectively; or Cap1#139-147 epitope (SEQ ID NO: 145) coated, or untreated P815 cells, showing 83%, 75% and 58% specific lysis at 30:1, 10:1 and 3:1 effector to target ratios, respectively (negative controls having less than 5% lysis in all cases). This data suggests that the epitope is presented during infection.

In vivo studies show Cap1#139-147 epitope-specific T-cells are primed during murine infection with *C. trachomatis.* To determine if infection with *C. trachomatis* primes a Cap1#139-147 epitope-specific T-cell response, mice were infected i.p. with 10⁸ IFU of *C. trachomatis* serovar L2. Two weeks after infection, the mice were sacrificed and spleen cells were stimulated on irradiated syngeneic spleen cells pulsed with Cap1#139-147 epitope peptide. After 5 days of stimulation, the cultures were used in a standard ⁵¹Cr release assay to determine if there were Cap1#139-147 epitope-specific T-cells present in the culture. Specifically, spleen cells from a *C. trachomatis* serovar L2 immunized mouse or a control mouse injected with PBS after a 5 days culture with Cap1#139-147 peptide-coated syngeneic spleen cells and CD8+ T-cells able to specifically recognize Cap1#139-147 epitope gave 73%, 60% and 32% specific lysis at a30:1, 10:1 and 3:1 effector to target ratios, respectively. The control mice had a percent lysis of approximately 10% at a 30:1 effector to target ratio, and steadily declining with lowering E:T ratios. Target cells were Cap1#139-147 peptide-coated, or untreated P815 cells. These data suggest that Cap1#139-147 peptide-specific T-cells are primed during murine infection with *C. trachomatis.*

### EXAMPLE 5

### GENERATION OF ANTIBODY AND T-CELL RESPONSES IN MICE IMMUNIZED WITH CHLAMYDIA ANTIGENS

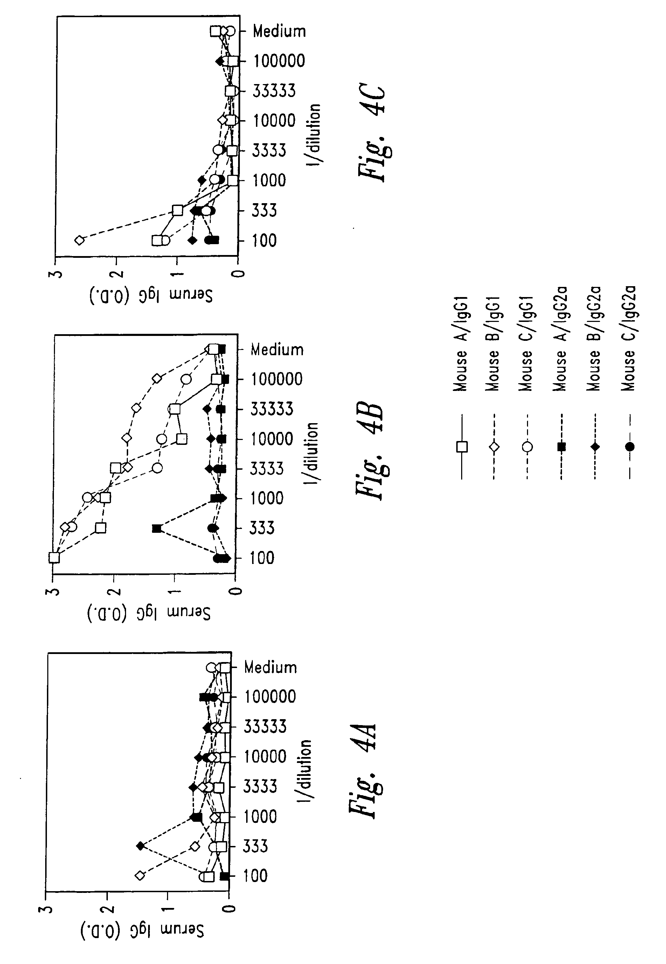

Immunogenicity studies were conducted to determine the antibody and CD4+ T cell responses in mice immunized with either purified SWIB or S13 proteins formulated with Montanide adjuvant, or DNA-based immunizations with pcDNA-3 expression vectors containing the DNA sequences for SWIB or S13. SWIB is also referred to as clone 1-B1-66 (SEQ ID NO: 1, with the corresponding amino acid sequence provided in SEQ ID NO: 5), and S 13 ribosomal protein is also referred to as clone 10-C10-31 (SEQ ID NO: 4, with the corresponding amino acid sequence provided in SEQ ID NO: 12). In the first experiment, groups of three C57BL/6 mice were immunized twice and monitored for antibody and CD4+ T-cell responses. DNA immunizations were intradermal at the base of the tail and polypeptide immunizations were administered by subcutaneous route. Results from standard ³H-incorporation assays of spleen cells from immunized mice shows a strong proliferative response from the group immunized with purified recombinant SWIB polypeptide (SEQ ID NO: 5). Further analysis by cytokine induction assays, as previously described, demonstrated that the group immunized with SWIB polypeptide produced a measurable IFN-γ and IL-4 response. Subsequent ELISA-based assays to determine the predominant antibody isotype response in the experimental group immunized with the SWIB polypeptide were performed. Fig. 4 illustrates the SWIB-immunized group gave a humoral response that was predominantly IgG1.

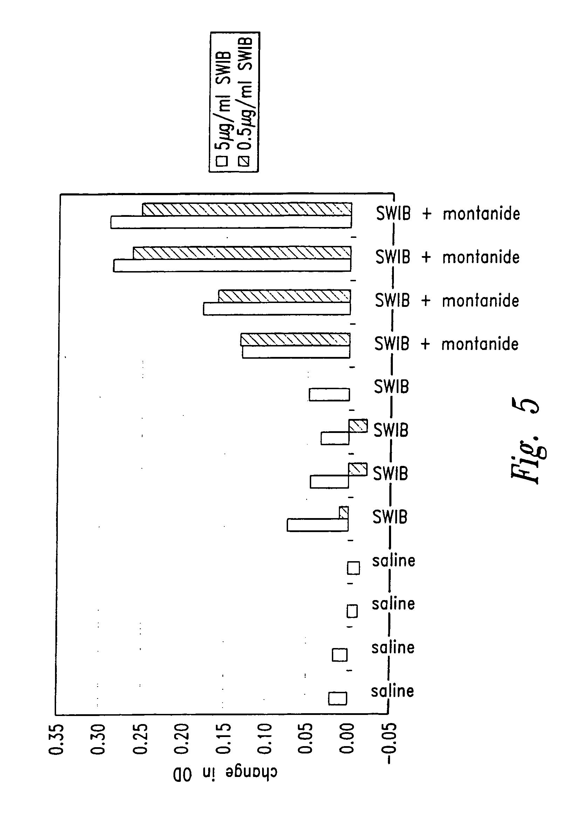

In a second experiment, C3H mice were immunized three times with 10 µg purified SWIB protein (also referred to as clone 1-B1-66, SEQ ID NO: 5) formulated in either PBS or Montanide at three week intervals and harvested two weeks after the third immunization. Antibody titers directed against the SWIB protein were determined by standard ELISA-based techniques well known in the art, demonstrating the SWIB protein formulated with Montanide adjuvant induced a strong humoral immune response. T-cell proliferative responses were determined by a XTT-based assay (Scudiero, et al, Cancer Research, 1988, 48:4827). As shown in Fig. 5, splenocytes from mice immunized with the SWIB polypeptide plus Montanide elicited an antigen specific proliferative response. In addition, the capacity of splenocytes from immunized animals to secrete IFN-γ in response to soluble recombinant SWIB polypeptide was determined using the cytokine induction assay previously described. The splenocytes from all animals in the group immunized with SWIB polypeptide formulated with montanide adjuvant secreted IFN-γ in response to exposure to the SWIB Chlamydia antigen, demonstrating an *Chlamydia*-specific immune response.

In a further experiment, C3H mice were immunized at three separate time points at the base of the tail with 10 µg of purified S WIB or S 13 protein (*C. trachomatis,* SWIB protein, clone 1-B1-66, SEQ ID NO: 5, and S13 protein, clone 10-C10-31, SEQ ID NO: 4) formulated with the SBAS2 adjuvant (SmithKline Beecham, London, England). Antigen-specific antibody titers were measured by ELISA, showing both polypeptides induced a strong IgG response, ranging in titers from 1 x10⁻⁴ to 1 x10⁻⁵. The IgG1 and IgG2a components of this response were present in fairly equal amounts. Antigen-specific T-cell proliferative responses, determined by standard ³H-incorporation assays on spleen cells isolated from immunized mice, were quite strong for SWIB (50,000 cpm above the negative control) and even stronger for s13 (100,000 cpm above the negative control). The IFNγ production was assayed by standard ELISA techniques from supernatant from the proliferating culture. *In vitro* restimulation of the culture with S13 protein induced high levels of IFNγ production, approximately 25 ng/ml versus 2 ng/ml for the negative control. Restimulation with the SWIB protein also induced IFNγ, although to a lesser extent.

In a related experiment, C3H mice were immunized at three separate time points with 10 µg of purified SWIB or S13 protein (*C. trachomatis,* SWIB protein, clone 1-B1-66, SEQ ID NO: 5, and S 13 protein, clone 10-C10-31, SEQ ID NO: 4) mixed with 10 µg of Cholera Toxin. Mucosal immunization was through intranasal inoculation. Antigen-specific antibody responses were determined by standard ELISA techniques. Antigen-specific IgG antibodies were present in the blood of SWIB-immunized mice, with titers ranging from 1 x10⁻³ to 1 x10⁻⁴, but non-detectable in the S13-immunized animals. Antigen-specific T-cell responses from isolated splenocytes, as measured by IFNγ production, gave similar results to those described immediately above for systemic immunization.

An animal study was conducted to determine the immunogenicity of the CT529 serovar LGVII CTL epitope, defined by the CT529 10mer consensus peptide (CSFIGGITYL - SEQ ID NO: 31), which was identified as an H2-Kd restricted CTL epitope. BALB/c mice (3 mice per group) were immunized three times with 25 µg of peptide combined with various adjuvants. The peptide was administered systemically at the base of the tail in either SKB Adjuvant System SBAS-2", SBAS-7 (SmithKline Beecham, London, England) or Montanide. The peptide was also administered intranasally mixed with 10ug of Cholera Toxin (CT). Naive mice were used as a control. Four weeks after the 3rd immunization, spleen cells were restimulated with LPS-blasts pulsed with 10ug/ml CT529 10mer consensus peptide at three different effector to LPS-blasts ratios : 6, 1.5 and 0.4 at 1x10⁶ cell/ml. After 2 restimulations, effector cells were tested for their ability to lyse peptide pulsed P815 cells using a standard chromium release assay. A non-relevant peptide from chicken egg ovalbumin was used as a negative control. The results demonstrate that a significant immune response was elicited towards the CT529 10mer consensus peptide and that antigen-specific T-cells capable of lysing peptide-pulsed targets were elicited in response to immunization with the peptide. Specifically, antigen-specific lytic activities were found in the SBAS-7 and CT adjuvanted group while Montanide and SBAS-2" failed to adjuvant the CTL epitope immunization.

### EXAMPLE 6

### EXPRESSION AND CHARACTERIZATION OF CHLAMYDIA PNEUMONIAE GENES

The human T-cell line, TCL-8, described in Example 1, recognizes *Chlamydia trachomatis* as well as *Chlamydia pneumonia* infected monocyte-derived dendritic cells, suggesting *Chlamydia trachomatis* and *pneumonia* may encode cross-reactive T-cell epitopes. To isolate the *Chlamydia pneumonia* genes homologous to *Chlamydia trachomatis* LGV II clones 1B1-66, also referred to as SWIB (SEQ ID NO: 1) and clone 10C10-31, also referred to as S13 ribosomal protein (SEQ ID NO: 4), HeLa 229 cells were infected with *C. pneumonia* strain TWAR (CDC/CWL-029). After three days incubation, the *C. pneumonia-*infected HeLa cells were harvested, washed and resuspended in 200 µl water and heated in a boiling water bath for 20 minutes. Ten microliters of the disrupted cell suspension was used as the PCR template.

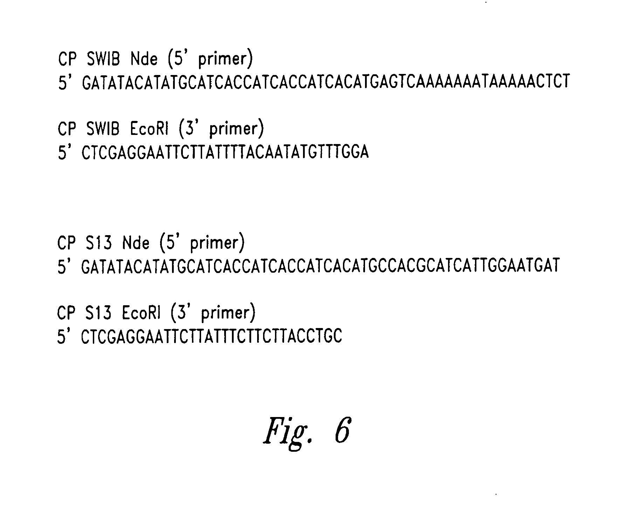

*C. pneumonia* specific primers were designed for clones 1B1-66 and 10C10-31 such that the 5' end had a 6X-Histidine tag and a Nde I site inserted, and the 3' end had a stop codon and a BamHI site included (Fig. 6). The PCR products were amplified and sequenced by standard techniques well known in the art. The *C. pneumonia-specific* PCR products were cloned into expression vector pET17B (Novagen, Madison, WI) and transfected into E. coli BL21 pLysS for expression and subsequent purification utilizing the histidine-nickel chromatographic methodology provided by Novagen. Two proteins from *C. pneumonia* were thus generated, a 10-11 kDa protein referred to as CpSWIB (SEQ ID NO: 27, and SEQ ID NO: 78 having a 6X His tag, with the corresponding amino acid sequence provided in SEQ ID NO: 28, respectively), a 15 kDa protein referred to as CpS 13 (SEQ ID NO: 29, and SEQ ID NO: 77, having a 6X His tag, with the corresponding amino acid sequence provided in SEQ ID NO: 30 and 91, respectively).

### EXAMPLE 7

### INDUCTION OF T CELL PROLIFERATION AND INTERFERON-γ PRODUCTION BY CHLAMYDIA PNEUMONIAE ANTIGENS

The ability of recombinant *Chlamydia pneumoniae* antigens to induce T cell proliferation and interferon-γ production is determined as follows.

Proteins are induced by IPTG and purified by Ni-NTA agarose affinity chromatography (Webb et al., J. Immunology 157:5034-5041, 1996). The purified polypeptides are then screened for the ability to induce T-cell proliferation in PBMC preparations. PBMCs from *C. pneumoniae* patients as well as from normal donors whose T-cells are known to proliferate in response to *Chlamydia* antigens, are cultured in medium comprising RPMI 1640 supplemented with 10% pooled human serum and 50 µg/ml gentamicin. Purified polypeptides are added in duplicate at concentrations of 0.5 to 10µ g/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

IFN-γ was measured using an enzyme-linked immunosorbent assay (ELISA). ELISA plates are coated with a mouse monoclonal antibody directed to human IFN-γ (PharMingen, San Diego, CA) in PBS for four hours at room temperature. Wells are then blocked with PBS containing 5% (W/V) non-fat dried milk for 1 hour at room temperature. The plates are washed six times in PBS/0.2% TWEEN-20 and samples diluted 1:2 in culture medium in the ELISA plates are incubated overnight at room temperature. The plates are again washed and a polyclonal rabbit anti-human IFN-γ serum diluted 1:3000 in PBS/10% normal goat serum is added to each well. The plates are then incubated for two hours at room temperature, washed and horseradish peroxidase-coupled anti-rabbit IgG (Sigma Chemical So., St. Louis, MO) is added at a 1:2000 dilution in PBS/5% non-fat dried milk. After a further two hour incubation at room temperature, the plates are washed and TMB substrate added. The reaction is stopped after 20 min with 1 N sulfuric acid. Optical density is determined at 450 nm using 570 nm as a reference wavelength. Fractions that result in both replicates giving an OD two fold greater than the mean OD from cells cultured in medium alone, plus 3 standard deviations, are considered positive.

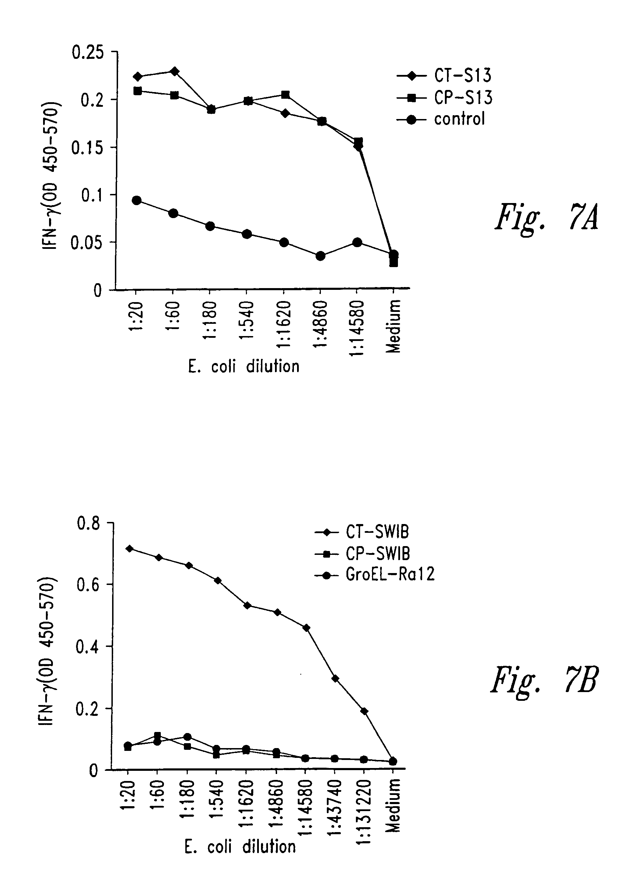

A human anti*-Chlamydia* T-cell line (TCL-8) capable of cross-reacting to *C. trachomatis* and *C. pneumonia* was used to determine whether the expressed proteins described in the example above, (i.e., CpSWIB, SEQ ID NO: 27, and SEQ ID NO: 78 having a 6X His tag, with the corresponding amino acid sequence provided in SEQ ID NO: 28, respectively, and the 15 kDa protein referred to as CpS 13 SEQ ID NO: 29, and SEQ ID NO: 77, having a 6X His tag, with the corresponding amino acid sequence provided in SEQ ID NO: 30 and 91, respectively), possessed T-cell epitopes common to both *C. trachomatis* and *C. pneumonia.* Briefly, *E. coli* expressing *Chlamydial* proteins were titered on 1 x 10⁴ monocyte-derived dendritic cells. After two hours, the dendritic_cells cultures were washed and 2.5 x 10⁴ T cells (TCL-8) added and allowed to incubate for an additional 72 hours. The amount of INF-γ in the culture supernatant was then determined by ELISA. As shown in Figs. 7A and 7B, the TCL-8 T-cell line specifically recognized the S 13 ribosomal protein from both *C. trachomatis* and *C. pneumonia* as demonstrated by the antigen-specific induction of IFN-γ, whereas only the SWIB protein from *C. trachomatis* was recognized by the T-cell line. To validate these results, the T cell epitope of *C. trachomatis* SWIB was identified by epitope mapping using target cells pulsed with a series of overlapping peptides and the T-cell line TCL-8. 3H-thymidine incorporation assays demonstrated that the peptide, referred to as C.t.SWIB 52-67, of SEQ ID NO: 39 gave the strongest proliferation of the TCL-8 line. The homologous peptides corresponding to the SWIB of *C. pneumoniae* sequence (SEQ ID NO: 40), the topoisomerase-SWIB fusion of *C. pneumoniae* (SEQ ID NO: 43) and *C. trachomatis* (SEQ ID NO: 42) as well as the human SWI domain (SEQ ID NO: 41) were synthesized and tested in the above assay. The T-cell line TCL-8 only recognized the *C. trachomatis* peptide of SEQ ID NO: 39 and not the corresponding *C. pneumoniae* peptide (SEQ ID NO: 40), or the other corresponding peptides described above (SEQ ID NO; 41-43).

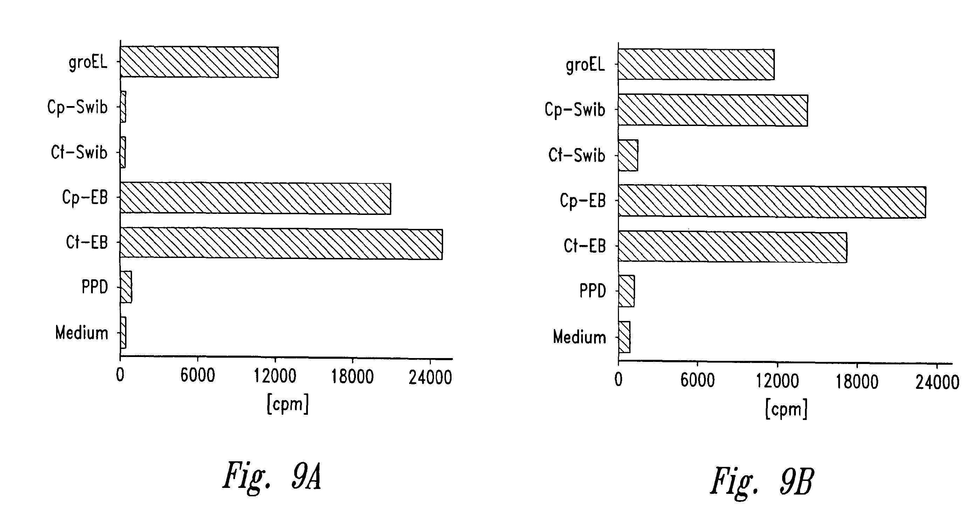

Chlamydia-specific T cell lines were generated from donor CP-21 with a positive serum titer against *C. pneumoniae* by stimulating donor PBMC with either *C. trachomatis* or *C. pneumoniae-infected* monocyte-derived dendritic cells, respectively. T-cells generated against *C. pneumoniae* responded to recombinant *C. pneumoniae-*SWIB but not *C. trachomatis-*SWIB, whereas the T-cell line generated against *C. trachomatis* did not respond to either *C. trachomatis-* or *C. pneumoniae-*SWIB (see Fig. 9). The *C. pneumoniae-*SWIB specific immune response of donor CP-21 confirms the *C. pneumoniae* infection and indicates the elicitation of *C. pneumoniae-*SWIB specific T-cells during *in vivo C. pneumoniae* infection.

Epitope mapping of the T-cell response to *C. pneumoniae-*SWIB has shown that Cp-SWIB-specific T-cells responded to the overlapping peptides Cp-SWIB 32-51 (SEQ ID NO: 101) and Cp-SWIB 37-56 (SEQ ID NO: 102), indicating a C. *pneumoniae-*SWIB-specific T-cell epitope Cp-SWIB 37-51 (SEQ ID NO: 100).

In additional experiments, T-cell lines were generated from donor CP1, also a *C. pneumoniae* seropositive donor, by stimulating PBMC with non-infectious elementary bodies from *C. trachomatis* and *C. pneumoniae,* respectively. In particular, proliferative responses were determined by stimulating 2.5 x 10⁴ T-cells in the presence of 1 x 10⁴ monocyte-derived dendritic cells and non-infectious elementary bodies derived from *C. trachomatis* and *C. pneumoniae,* or either recombinant *C. trachomatis* or *C. pneumoniae* SWIB protein. The T-cell response against SWIB resembled the data obtained with T-cell lines from CP-21 in that *C. pneumoniae-*SWIB, but not *C. trachomatis-*SWIB elicited a response by the *C. pneumoniae* T-cell line. In addition, the *C. trachomatis* T-cell line did not proliferate in response to either *C. trachomatis* or *C. pneumoniae* SWIB, though it did proliferate in response to both CT and CP elementary bodies.As described in Example 1, Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of *C. pneumoniae,* referred to as OMCB. To further define the reactive epitope(s), epitope mapping was performed using a series of overlapping peptides and the immunoassay previously described. Briefly, proliferative responses were determined by stimulating 2.5 x 10⁴ TCP-21 T-cells in the presence of 1 x 10⁴ monocyte-derived dendritic cells with either non-infectious elementary bodies derived from *C. trachomatis* and *C. pneumoniae,* or peptides derived from the protein sequence of *C. trachomatis* or *C. pneumoniae* OMCB protein (0.1 µg/ml). The TCP-21 T-cells responded to epitopes CT-OMCB #167-186, CT-OMCB #171-190, CT-OMCB #171-186, and to a lesser extent, CT-OMCB #175-186 (SEQ ID NO: 249-252, respectively). Notably, the TCP-21 T-cell line also gave a proliferative response to the homologous *C. pneumoniae* peptide CP-OMCB #171-186 (SEQ ID NO: 253), which was equal to or greater than the response to the to the *C. trachomatis* peptides. The amino acid substitutions in position two (i.e., Asp for Glu) and position four (i.e., Cys for Ser) did not alter the proliferative response of the T-cells and therefore demonstrating this epitope to be a cross-reactive epitope between *C. trachomatis* and *C. pneumoniae.*

### EXAMPLE 8

### IMMUNE RESPONSES OF HUMAN PBMC AND T-CELL LINES AGAINST CHLAMYDIA ANTIGENS

The examples provided herein suggest that there is a population of healthy donors among the general population that have been infected with *C. trachomatis* and generated a protective immune response controlling the *C. trachomatis* infection. These donors remained clinically asymptomatic and seronegative for *C. trachomatis.* To characterize the immune responses of normal donors against *chlamydial* antigens which had been identified by CD4 expression cloning, PBMC obtained from 12 healthy donors were tested against a panel of recombinant *chlamydial* antigens including *C. trachomatis-, C. pneumoniae-*SWIB and *C. trachomatis-, C. pneumoniae-*S13. The data are summarized in Table I below. All donors were seronegative for *C. trachomatis,* whereas 6/12 had a positive *C. pneumoniae* titer. Using a stimulation index of >4 as a positive response, 11/12 of the subjects responded to *C. trachomatis* elementary bodies and 12/12 responded to *C. pneumoniae* elementary bodies. One donor, AD104, responded to recombinant *C. pneumoniae-S 13* protein, but not to recombinant *C. trachomatis*-S13 protein, indicating a *C. pneumoniae-specific* response. Three out of 12 donors had a *C. trachomatis*-SWIB, but not a *C. pneumoniae-*SWIB specific response, confirming a *C. trachomatis* infection. *C. trachomatis* and *C. pneumoniae-* S13 elicited a response in 8/12 donors suggesting a chlamydial infection. These data demonstrate the ability of SWIB and S13 to elicit a T-cell response in PBMC of normal study subjects.

**Table I.**

| Immune response of normal study subjects against *Chlamydia* | | | | | | | | | | |
|---|---|---|---|---|---|---|---|---|---|---|
| | | *Chlamydia* | CT | CP | CT | CP | CT | CP | CT | CT |
| onor | Sex | IgGtiter | EB | EB | Swib | Swib | S13 | S13 | lpdA | TSA |
| D100 | male | negative | ++ | +++ | + | - | ++ | ++ | - | nt |
| D104 | female | negative | +++ | ++ | - | - | - | ++ | - | nt |
| D108 | male | CP1:256 | ++ | ++ | + | +/- | + | + | + | nt |
| D112 | female | negative | ++ | ++ | + | - | + | - | +/- | nt |
| D120 | male | negative | - | + | - | - | - | - | - | nt |
| D124 | female | CP1:128 | ++ | ++ | - | - | - | - | - | nt |
| D128 | male | CP 1:512 | + | ++ | - | - | ++ | + | ++ | - |
| D132 | female | negative | ++ | ++ | - | - | + | + | - | - |
| D136 | female | CP 1:128 | + | ++ | - | - | +/- | - | - | - |
| D140 | male | CP 1:256 | ++ | ++ | - | - | + | + | - | - |
| D142 | female | CP1:512 | ++ | ++ | - | - | + | + | + | - |
| D146 | female | negative | ++ | ++ | - | - | ++ | + | + | - |

| | | | | | | | | | | |
|---|---|---|---|---|---|---|---|---|---|---|
| CT= *Chlamydia trachomatis;* CP= *Chlamydia pneumoniae;* EB= *Chlamydia* elementary bodies; Swib= recombinant *Chlamydia* Swib protein; S 13= recombinant *Chlamydia* S 13 protein; lpdA= recombinant *Chlamydia* lpdA protein; TSA= recombinant *Chlamydia* TSA protein.Values represent results from standard proliferation assays. Proliferative responses were determined by stimulating 3 x 10⁵ PBMC with 1 x 10⁴ monocyte-derived dendritic cells pre-incubated with the respective recombinant antigens or elementary bodies (EB). Assays were harvested after 6 days with a ³H-thymidine pulse for the last 18h. SI: Stimulation index +/-: SI ~ 4 +: SI > 4 ++: SI 10-30 +++: SI > 30 | | | | | | | | | | |

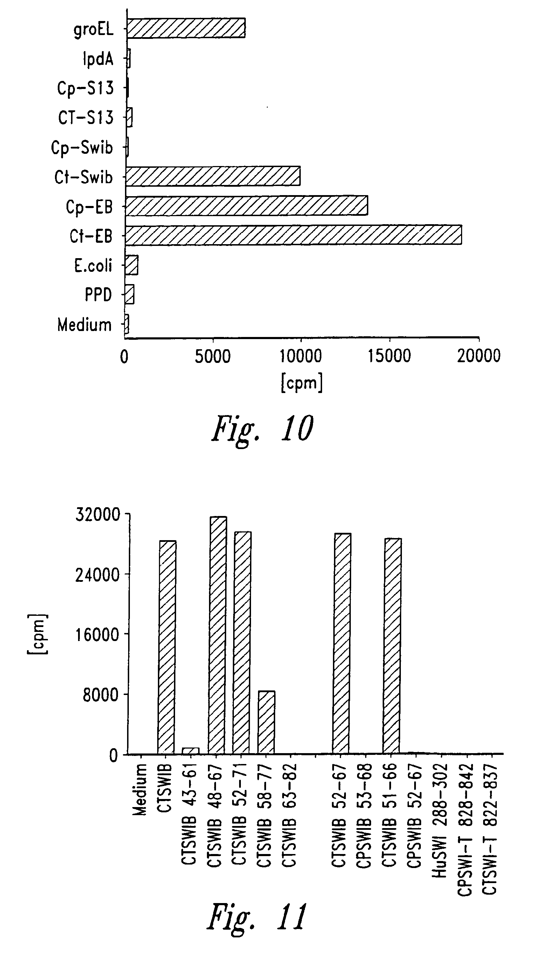

In a first series of experiments, T-cell lines were generated from a healthy female individual (CT-10) with a history of genital exposure to *C. trachomatis* by stimulating T-cells with *C. trachomatis* LGV II elementary bodies as previously described. Although the study subject was exposed to *C. trachomatis,* she did not seroconvert and did not develop clinical symptoms, suggesting donor CT-10 may have developed a protective immune response against *C. trachomatis.* As shown in Fig. 10, a primary *Chlamydia-specific* T-cell line derived from donor CT-10 responded to *C. trachomatis*-SWIB, but not *C. pneumoniae-*SWIB recombinant proteins, confirming the exposure of CT-10 to *C. trachomatis.* Epitope mapping of the T-cell response to *C. trachomatis*-SWIB showed that this donor responded to the same epitope Ct-SWIB 52-67 (SEQ ID NO: 39) as T-cell line TCL-8, as shown in Fig. 11.

Additional T-cell lines were generated as described above for various *C. trachomatis* patients. A summary of the patients' clinical profile and proliferative responses to various *C. trachomatis* and *C. pneumoniae* elementary bodies and recombinant proteins are summarized in Table II.

**Table II**

| **Proliferative response of *C. trachomatis* patients** | | | | | | | | | | |
|---|---|---|---|---|---|---|---|---|---|---|
| | | | **CT** | **CP** | **CT** | **CP** | **CT** | **CP** | **CT** | **CT** |
| **atients** | **Clinical manifestation** | **IgG titer** | **EB** | **EB** | **Swib** | **Swib** | **S13** | **S13** | **IpdA** | **TSA** |
| **CT-1** | **NGU** | **negative** | + | + | - | - | ++ | ++ | ++ | + |
| **CT-2** | **NGU** | **negative** | ++ | ++ | - | - | + | +/- | - | - |
| **CT-3** | **asymptomatic** | **Ct 1:512** | + | + | - | - | + | - | + | - |
| | **shed Eb Dx was HPV** | **Cp 1:1024** | | | | | | | | |
| | | **Cps 1:256** | | | | | | | | |
| **CT-4** | **asymptomatic shed Eb** | **Ct 1:1024** | + | + | - | - | - | - | - | - |
| **CT-5** | **BV** | **Ct 1:256** | ++ | ++ | - | - | + | - | - | - |
| | | **Cp 1:256** | | | | | | | | |
| **CT-6** | **perinial rash discharge** | **Cp 1:1024** | + | + | - | - | - | - | - | - |
| **CT-7** | **BV** | **Ct 1:512** | + | + | - | - | + | + | + | - |
| | **genital ulcer** | **Cp 1:1024** | | | | | | | | |
| **CT-8** | **Not known** | **Nottested** | ++ | ++ | - | - | - | - | - | - |
| **CT-9** | **asymptomatic** | **Ct 1:128** | +++ | ++ | - | - | ++ | + | + | - |
| | | **Cp 1:128** | | | | | | | | |
| **CT-10** | **Itch mild vulvar** | **negative** | ++ | ++ | - | - | - | - | - | - |
| **CT-11** | **BV, abnormal pap** | **Ct 1: 512** | +++ | +++ | - | - | +++ | +/- | ++ | + |
| **CT-12** | **asymptomatic** | **Cp 1: 512** | ++ | ++ | - | - | ++ | + | + | - |

| | | | | | | | | | | |
|---|---|---|---|---|---|---|---|---|---|---|
| NGU= Non-Gonococcal Urethritis; BV= Bacterial Vaginosis; CT= *Chlamydia trachomatis;* CP= *Chlamydia pneumoniae;* EB= *Chlamydia* elementary bodies; Swib= recombinant *Chlamydia* Swib protein; S 13= recombinant *Chlamydia* S 13 protein; lpdA= recombinant *Chlamydia* lpdA protein; TSA= recombinant *Chlamydia* TSA protein Values represent results from standard proliferation assays. Proliferative responses were determined by stimulating 3 x 10⁵ PBMC with 1 x 10⁴ monocyte-derived dendritic cells pre-incubated with the respective recombinant antigens or elementary bodies (EB). Assays were harvested after 6 days with a ³H-thymidine pulse for the last 18 hours. SI: Stimulation index +/-: SI ~ 4 +: SI > 4 ++: SI 10-30 +++: SI > 30 | | | | | | | | | | |

Using the panel of asymptomatic (as defined above) study subjects and *C. trachomatis* patients, as summarized in Tables I and II, a comprehensive study of the immune responses of PBMC derived from the two groups was conducted. Briefly, PBMCs from *C. pneumoniae* patients as well as from normal donors are cultured in medium comprising RPMI 1640 supplemented with 10% pooled human serum and 50 µg/ml gentamicin. Purified polypeptides, a panel of recombinant *chlamydial* antigens including *C. trachomatis-, C. pneumoniae-*SWIB and S 13, as well as. *C. trachomatis* lpdA and TSA are added in duplicate at concentrations of 0.5 to 10 µg/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

Proliferative responses to the recombinant *Chlamydiae* antigens demonstrated that the majority of asymptomatic donors and *C. trachomatis* patients recognized the *C. trachomatis* S 13 antigen (8/12) and a majority of the *C. trachomatis* patients recognized the *C. pneumonia* S13 antigen (8/12), with 4/12 asymptomatic donors also recognizing the *C. pneumonia* S13 antigen. Also, six out of twelve of the *C. trachomatis* patients and four out of twelve of the asymptomatic donors gave a proliferative response to the lpdA antigen of *C. trachomatis.* These results demonstrate that the *C. trachomatis* and *C. pneumonia* S13 antigen, *C. trachomatis* Swib antigen and the *C. trachomatis* lpdA antigen are recognized by the asymptomatic donors, indicating these antigens were recognized during exposure to *Chlamydia* and an immune response elicited against them. This implies these antigens may play a role in conferring protective immunity in a human host. In addition, the *C. trachomatis* and *C. pneumonia* S 13 antigen is recognized equally well among the *C. trachomatis* patients, therefore indicating there may be epitopes shared between *C. trachomatis* and *C. pneumonia* in the S 13 protein. Table III summarizes the results of these studies.

**Table III.**

| **Antigen** | **Normal Donors** | **C.t. Patients** |
|---|---|---|
| C.t.-Swib | 3/12 | 0/12 |
| C.p.-Swib | 0/12 | 0/12 |
| C.t.-S 13 | 8/12 | 8/12 |
| C.p.-S 13 | 4/12 | 8/12 |
| lpdA | 4/12 | 6/12 |
| TSA | 0/12 | 2/12 |

A series of studies were initiated to determine the cellular immune response to short-term T-cell lines generated from asymptomatic donors and *C. trachomatis* patients. Cellular immune responses were measured by standard proliferation assays and IFN-γ, as described in Example 7. Specifically, the majority of the antigens were in the form of single *E. coli* clones expressing Chlamydial antigens, although some recombinant proteins were also used in the assays. The single *E. coli* clones were titered on 1 x 10⁴ monocyte-derived dendritic cells and after two hours, the culture was washed and 2.5 x 10⁴ T-cells were added. The assay using the recombinant proteins were performed as previously described. Proliferation was determined after four days with a standard ³H-thymidine pulse for the last 18 hours. Induction of IFN-γ was determined from culture supernatants harvested after four days using standard ELISA assays, as described above. The results show that all the *C. trachomatis* antigens tested, except for C.T. Swib, elicited a proliferative response from one or more different T-cell lines derived form *C. trachomatis* patients. In addition, proliferative responses were elicited from both the *C. trachomatis* patients and asymptomatic donors for the following *Chlamydia* genes, CT622, groEL, pmpD, CT610 and rS13.

The 12G3-83 clone also contains sequences to CT734 and CT764 in addition to CT622, and therefore these gene sequence may also have immunoreactive epitopes. Similarly, clone 21G12-60 contains sequences to the hypothetical protein genes CT229 and CT228 in addition to CT875; and 15H2-76 also contains sequences from CT812 and CT088, as well as sharing homology to the sycE gene. Clone 11H3-61 also contains sequences sharing homology to the PGP6-D virulence protein.

**Table IV.**

| **Clone** | **C. t. Antigen (putative*)** | **TCL from Asymp. Donors** | **TCL from C. t. Patients** | **SEQ ID NO::** |
|---|---|---|---|---|
| 1B 1-66 (E. coli) | Swib | 2/2 | 0/4 | 5 |
| 1B1-66 (protein) | Swib | 2/2 | 0/4 | 5 |
| 12G3-83 (E. coli) | CT622* | 2/2 | 4/4 | 57 |
| 22B3-53 (E. coli) | groEL | 1/2 | 4/4 | 111 |
| 22B3-53 (protein) | groEL | 1/2 | 4/4 | 111 |
| 15H2-76 (E. coli) | PmpD* | 1/2 | 3/4 | 87 |
| 11H3-61 (E. coli) | rL1* | 0/2 | 3/4 | 60 |
| 14H1-4 (E. coli) | TSA | 0/2 | 3/4 | 56 |
| 14H1-4 (protein) | TSA | 0/2 | 3/4 | 56 |
| 11 G 10-46 (E. coli) | CT610 | 1/2 | 1/4 | 62 |
| 10C10-17 (E. coli) | rS13 | 1/2 | 1/4 | 62 |
| 10C10-17 (protein) | rS13 | 1/2 | 1/4 | 62 |
| 21G12-60 (E. coli) | CT875* | 0/2 | 2/4 | 110 |
| 11H4-32 (E. coli) | dnaK | 0/2 | 2/4 | 59 |
| 21C7-8 (E. coli) | dnaK | 0/2 | 2/4 | 115 |
| 17C10-31 (E. coli) | CT858 | 0/2 | 2/4 | 114 |

### EXAMPLE 9

### PROTECTION STUDIES USING CHLAMYDIA ANTIGENS

Protection studies were conducted in mice to determine whether immunization with chlamydial antigens can impact on the genital tract disease resulting from chlamydial inoculation. Two models were utilized; a model of intravaginal inoculation that uses a human isolate containing a strain of *Chlamydia psittaci* (MTW447), and a model of intrauterine inoculation that involves a human isolate identified as *Chlamydia trachomatis,* serovar F (strain NI1). Both strains induce inflammation in the upper genital tract, which resemble endometritis and salpingitis caused by *Chlamydia trachomatis* in women. In the first experiment, C3H mice (4 mice per group) were immunized three times with 100 µg of pcDNA-3 expression vector containing *C. trachomatis* SWIB DNA (SEQ ID NO: 1, with the corresponding amino acid sequence provided in SEQ ID NO: 5). Inoculations were at the base of the tail for systemic immunization. Two weeks after the last immunization, animals were progesterone treated and infected, either thru the vagina or by injection of the inoculum in the uterus. Two weeks after infection, the mice were sacrificed and genital tracts sectioned, stained and examined for histopathology. Inflammation level was scored (from + for very mild, to +++++ for very severe). Scores attributed to each single oviduct /ovary were summed and divided by the number of organs examined to get a mean score of inflammation for the group. In the model of uterine inoculation, negative control-immunized animals receiving empty vector showed consistent inflammation with an ovary /oviduct mean inflammation score of 6.12, in contrast to 2.62 for the DNA-immunized group. In the model of vaginal inoculation and ascending infection, negative control-immunized mice had an ovary /oviduct mean inflammation score of 8.37, versus 5.00 for the DNA-immunized group. Also, in the later model, vaccinated mice showed no signs of tubal occlusion while negative control vaccinated groups had inflammatory cells in the lumen of the oviduct

In a second experiment, C3H mice (4 mice per group) were immunized three times with 50 µg of pcDNA-3 expression vector containing *C. trachomatis* SWIB DNA (SEQ ID NO: 1, with the corresponding amino acid sequence provided in SEQ ID NO: 5) encapsulated in Poly Lactide co-Glycolide microspheres (PLG); immunizations were made intra-peritoneally. Two weeks after the last immunization, animal were progesterone treated and infected by inoculation of *C. psittaci* in the vagina. Two weeks after infection, mice were sacrificed and genital tracts sectioned, stained and examined for histopathology. Inflammation level was scored as previously described. Scores attributed to each single oviduct /ovary were summed and divided by the number of examined organs to get a mean of inflammation for the group. Negative control-immunized animals receiving PLG-encapsulated empty vector showed consistent infammation with an ovary /oviduct mean inflammation score of 7.28, versus 5.71 for the PLG-encapsulated DNA immunized group. Inflammation in the peritoneum was 1.75 for the vaccinated group versus 3. 75 for the control.

In a third experiment, C3H mice (4 per group) were immunized three times with 10 µg of purified recombinant protein, either SWIB (SEQ ID NO: 1, with the corresponding amino acid sequence provided in SEQ ID NO: 5, or S 13 (SEQ ID NO: 4, with the corresponding amino acid sequence provided in SEQ ID NO: 12) mixed with Cholera Toxin (CT); the preparation was administred intranasally upon anaesthesia in a 20 uL volume. Two weeks after the last immunization, animal were progesterone treated and infected, either by vaginal inoculation of *C. psittaci* or by injection of *C. trachomatis* serovar F in the uterus. Two weeks after infection, the mice were sacrificed and genital tracts sectioned, stained and examined for histopathology. The degree of inflammation was scored as described above. Scores attributed to each single oviduct /ovary were summed and divided by the number of examined organs to get a mean score of inflammation for the group. In the model of uterine inoculation, negative control- immunized animals receiving cholera toxin alone showed an ovary /oviduct mean inflammation score of 4.25 (only 2 mice analyzed ; 2 other died) versus 5.00 for the s13 plus cholera toxin-immunized group, and 1.00 for the SWIB plus cholera toxin. Untreated infected animals had an ovary /oviduct mean inflammation score of 7. In the model of vaginal inoculation and ascending infection, negative control-immunized mice had an ovary /oviduct mean inflammation score of 7.37 versus 6.75 for the s13 plus cholera toxin-immunized group and 5.37 for the SWIB plus cholera toxin-immunized group. Untreated infected animals had an ovary /oviduct mean inflammation score of 8.

The three experiments described above suggest that SWIB-specific protection is obtainable. This protective effect is more marked in the model of homologous infection but is still present when in a heterologous challenge infection with *C. psittaci.*

Although the present invention has been described in some detail by way of illustration and example for purposes of clarity of understanding, changes and modifications can be carried out without departing from the scope of the invention which is intended to be limited only by the scope of the appended claims.

## Claims

1. A pharmaceutical composition comprising a physiologically acceptable carrier and
(a) an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto; or
(b) an isolated polynucleotide encoding an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto; or
(c) a fusion protein comprising an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

2. A pharmaceutical composition according to claim 1, comprising a physiologically acceptable carrier and an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

3. A pharmaceutical composition according to claim 2, comprising a physiologically acceptable carrier and an isolated polypeptide comprising the sequence set forth in SEQ ID NO: 5 or a variant thereof having at least 90% identity thereto.

4. A pharmaceutical composition according to claim 3, comprising a physiologically acceptable carrier and an isolated polypeptide comprising sequence set forth in SEQ ID NO: 5.

5. A pharmaceutical composition according to claim 4, comprising a physiologically acceptable carrier and an isolated polypeptide consisting of the sequence set forth in SEQ ID NO: 5.

6. A pharmaceutical composition according to claim 2, comprising a physiologically acceptable carrier and an isolated polypeptide comprising a sequence set forth in any one of SEQ ID NOS: 6, 39, 94, 95, 96 or 98, or a variant thereof having at least 90% identity thereto.

7. A pharmaceutical composition according to claim 6, comprising a physiologically acceptable carrier and an isolated polypeptide comprising a sequence set forth in any one of SEQ ID NOS: 6, 39, 94, 95, 96 or 98.

8. A pharmaceutical composition according to claim 1, comprising a physiologically acceptable carrier and an isolated polynucleotide encoding a polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

9. A pharmaceutical composition according to claim 8, comprising a physiologically acceptable carrier and an isolated polynucleotide comprising the sequence set forth by nucleotides 115 to 375 of SEQ ID NO: 1.

10. A vaccine comprising an immunostimulant, and
(a) an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto; or
(b) an isolated polynucleotide encoding an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

11. A vaccine according to claim 10, comprising an immunostimulant and an isolated polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

12. A vaccine according to claim 13, wherein the isolated polypeptide comprises the amino acid sequence set forth in SEQ ID NO: 5.

13. A vaccine according to claim 13, wherein the isolated polypeptide comprises the amino acid sequence set forth in any one of SEQ ID NOS: 6, 39, 94, 95, 96 or 98.

14. A vaccine according to claim 10, comprising an immunostimulant and an isolated polynucleotide encoding a polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto.

15. A vaccine according to any one of claims 10 to 15, wherein the immunostimulant is an adjuvant.

16. A pharmaceutical composition according to any one of claims 1 to 9 for use in inducing protective immunity in a patient.

17. A vaccine according to of any one of claims 10 to15 for use in inducing protective immunity in a patient.

18. Use of a polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto;
in the manufacture of a medicament for the treatment or prevention of *Chlamydia trachomatis* infection.

19. Use of a polynucleotide encoding a polypeptide comprising:
(i) the polypeptide sequence set forth in SEQ ID NO: 5 or a variant thereof having at least 90% identity thereto; or
(ii) a polypeptide sequence set forth in any one of SEQ ID NOS: 6, 39, 94, 95, 96 or 98, or a variant thereof having at least 90% identity thereto;
in the manufacture of a medicament for the treatment or prevention of *Chlamydia trachomatis* infection.

## Patentansprüche

1. Pharmazeutische Zusammensetzung, umfassend einen physiologisch unbedenklichen Trägerstoff und
(a) ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit;
oder
(b) ein isoliertes Polynukleotid, codierend für ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit;
oder
(c) ein Fusionsprotein, umfassend ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

2. Pharmazeutische Zusammensetzung nach Anspruch 1, umfassend einen physiologisch unbedenklichen Trägerstoff und ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

3. Pharmazeutische Zusammensetzung nach Anspruch 2, umfassend einen physiologisch unbedenklichen Trägerstoff und ein isoliertes Polypeptid, umfassend die in SEQ ID NO: 5 angegebene Sequenz oder eine Variante davon mit wenigstens 90% Identität damit.

4. Pharmazeutische Zusammensetzung nach Anspruch 3, umfassend einen physiologisch unbedenklichen Trägerstoff und ein isoliertes Polypeptid, umfassend die in SEQ ID NO: 5 angegebene Sequenz.

5. Pharmazeutische Zusammensetzung nach Anspruch 4, umfassend einen physiologisch unbedenklichen Trägerstoff und ein isoliertes Polypeptid, bestehend aus der in SEQ ID NO: 5 angegebenen Sequenz.

6. Pharmazeutische Zusammensetzung nach Anspruch 2, umfassend einen physiologisch unbedenklichen Trägerstoff und ein isoliertes Polypeptid, umfassend eine in einer der SEQ ID NOS: 6, 39, 94, 95, 96 oder 98 angegebene Sequenz oder eine Variante davon mit wenigstens 90% Identität damit.

7. Pharmazeutische Zusammensetzung nach Anspruch 6, umfassend einen physiologisch unbedenklichen Trägerstoff und ein isoliertes Polypeptid, umfassend eine in einer der SEQ ID NOS: 6, 39, 94, 95, 96 oder 98 angegebene Sequenz.

8. Pharmazeutische Zusammensetzung nach Anspruch 1, umfassend einen physiologisch unbedenklichen Trägerstoff und ein isoliertes Polynukleotid, codierend für ein Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

9. Pharmazeutische Zusammensetzung nach Anspruch 8, umfassend einen physiologisch unbedenklichen Trägerstoff und ein isoliertes Polynukleotid, umfassend die durch Nukleotide 115 bis 375 der SEQ ID NO: 1 angegebene Sequenz.

10. Impfstoff, umfassend ein immunstimulierendes Mittel und
(a) ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit;
oder
(b) ein isoliertes Polynukleotid, codierend für ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

11. Impfstoff nach Anspruch 10, umfassend ein immunstimulierendes Mittel und ein isoliertes Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

12. Impfstoff nach Anspruch 13, wobei das isolierte Polypeptid die in SEQ ID NO: 5 angegebene Aminosäuresequenz umfasst.

13. Impfstoff nach Anspruch 13, wobei das isolierte Polypeptid die in einer der SEQ ID NOS: 6, 39, 94, 95, 96 oder 98 angegebene Aminosäuresequenz umfasst.

14. Impfstoff nach Anspruch 10, umfassend ein immunstimulierendes Mittel und ein isoliertes Polynukleotid, codierend für ein Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit.

15. Impfstoff nach einem der Ansprüche 10 bis 15, wobei es sich bei dem immunstimulierenden Mittel um ein Adjuvans handelt.

16. Pharmazeutische Zusammensetzung nach einem der Ansprüche 1 bis 9 zur Verwendung bei der Induktion einer Schutzimmunität bei einem Patienten.

17. Impfstoff nach einem der Ansprüche 10 bis 15 zur Verwendung bei der Induktion einer Schutzimmunität bei einem Patienten.

18. Verwendung eines Polypeptids, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit,
bei der Herstellung eines Arzneimittels zur Behandlung oder Vorbeugung einer Infektion mit *Chlamydia trachomatis.*

19. Verwendung eines Polynukleotids, codierend für ein Polypeptid, umfassend:
(i) die in SEQ ID NO: 5 angegebene Polypeptidsequenz oder eine Variante davon mit wenigstens 90% Identität damit; oder
(ii) eine in einer der SEQ ID NOS: 6, 39, 94, 95, 96 oder 98 angegebene Polypeptidsequenz oder eine Variante davon mit wenigstens 90% Identität damit,
bei der Herstellung eines Arzneimittels zur Behandlung oder Vorbeugung einer Infektion mit *Chlamydia trachomatis.*

## Revendications

1. Composition pharmaceutique comprenant un support physiologiquement acceptable et
(a) un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci ; ou
(b) un polynucléotide isolé codant pour un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci ; ou
(c) une protéine de fusion comprenant un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

2. Composition pharmaceutique selon la revendication 1, comprenant un support physiologiquement acceptable et un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

3. Composition pharmaceutique selon la revendication 2, comprenant un support physiologiquement acceptable et un polypeptide isolé comprenant la séquence présentée dans SEQ ID NO : 5 ou une variante de celle-ci ayant au moins 90 % d'identité avec celle-ci.

4. Composition pharmaceutique selon la revendication 3, comprenant un support physiologiquement acceptable et un polypeptide isolé comprenant la séquence présentée dans SEQ ID NO : 5.

5. Composition pharmaceutique selon la revendication 4, comprenant un support physiologiquement acceptable et un polypeptide isolé constitué de la séquence présentée dans SEQ ID NO : 5.

6. Composition pharmaceutique selon la revendication 2, comprenant un support physiologiquement acceptable et un polypeptide isolé comprenant une séquence présentée dans l'une quelconque de SEQ ID NO : 6, 39, 94, 95, 96 ou 98, ou une variante de celle-ci ayant au moins 90 % d'identité avec celle-ci.

7. Composition pharmaceutique selon la revendication 6, comprenant un support physiologiquement acceptable et un polypeptide isolé comprenant une séquence présentée dans l'une quelconque de SEQ ID NO : 6, 39, 94, 95, 96 ou 98.

8. Composition pharmaceutique selon la revendication 1, comprenant un support physiologiquement acceptable et un polynucléotide isolé codant pour un polypeptide comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

9. Composition pharmaceutique selon la revendication 8, comprenant un support physiologiquement acceptable et un polynucléotide isolé comprenant la séquence présentée par les nucléotides 115 à 375 de SEQ ID NO : 1.

10. Vaccin comprenant un immunostimulant, et
(a) un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci ; ou
(b) un polynucléotide isolé codant pour un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

11. Vaccin selon la revendication 10, comprenant un immunostimulant et un polypeptide isolé comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

12. Vaccin selon la revendication 13, **caractérisé en ce que** le polypeptide isolé comprend la séquence d'acides aminés présentée dans SEQ ID NO : 5.

13. Vaccin selon la revendication 13, **caractérisé en ce que** le polypeptide isolé comprend la séquence d'acides aminés présentée dans l'une quelconque de SEQ ID NO : 6, 39, 94, 95, 96 ou 98.

14. Vaccin selon la revendication 10, comprenant un immunostimulant et un polynucléotide isolé codant pour un polypeptide comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci.

15. Vaccin selon l'une quelconque des revendications 10 à 15, **caractérisé en ce que** l'immunostimulant est un adjuvant.

16. Composition pharmaceutique selon l'une quelconque des revendications 1 à 9, destinée à être utilisée pour induire l'immunité protectrice chez un patient.

17. Vaccin selon l'une quelconque des revendications 10 à 15, destiné à être utilisé pour induire l'immunité protectrice chez un patient.

18. Utilisation d'un polypeptide comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci ;
dans la fabrication d'un médicament destiné au traitement ou à la prévention d'une infection à *Chlamydia trachomatis.*

19. Utilisation d'un polynucléotide codant pour un polypeptide comprenant :
(i) la séquence polypeptidique présentée dans SEQ ID NO : 5 ou une variante de celle-ci ayant au moins 90 % d'identité avec celle-ci ; ou
(ii) une séquence polypeptidique présentée dans l'une quelconque de SEQ ID NO : 6, 39, 94, 95, 96 ou 98, ou une variante de celle-ci ayant au moins 90 % d'identité avec celle-ci ;
dans la fabrication d'un médicament destiné au traitement ou à la prévention d'une infection à *Chlamydia trachomatis.*
